(19) Europäisches Patentamt
European Patent Office
Office européen des brevets

(11) **EP 4 516 897 A1**

(12) **EUROPEAN PATENT APPLICATION**
published in accordance with Art. 153(4) EPC

(43) Date of publication:
**05.03.2025 Bulletin 2025/10**

(21) Application number: **23795550.5**

(22) Date of filing: **27.04.2023**

(51) International Patent Classification (IPC):
*C12N 5/10* $^{(2006.01)}$    *C12N 15/40* $^{(2006.01)}$
*C12N 15/85* $^{(2006.01)}$    *A61K 39/215* $^{(2006.01)}$
*A61P 31/14* $^{(2006.01)}$    *A61P 11/00* $^{(2006.01)}$

(52) Cooperative Patent Classification (CPC):
**A61K 39/215; A61P 11/00; A61P 31/14;
C07K 14/08; C12N 5/10; C12N 15/85**

(86) International application number:
**PCT/CN2023/091194**

(87) International publication number:
**WO 2023/208118 (02.11.2023 Gazette 2023/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.04.2022 CN 202210456261**

(71) Applicant: **Shanghai Regenelead Therapies Co.,
Ltd.
Shanghai 201206 (CN)**

(72) Inventors:
• **HU, Zhanying
Shanghai 200131 (CN)**
• **MING, Xin
Shanghai 200131 (CN)**
• **GAO, Lu
Shanghai 200131 (CN)**

• **WANG, Li
Shanghai 200131 (CN)**
• **ZHU, Qingyuan
Shanghai 200131 (CN)**
• **ZHU, Qihui
Shanghai 200131 (CN)**
• **WAN, Haitao
Shanghai 200131 (CN)**
• **CHEN, Qingxin
Shanghai 200131 (CN)**
• **YANG, Yujie
Shanghai 200131 (CN)**

(74) Representative: **Dragotti & Associati S.R.L.
Via Nino Bixio, 7
20129 Milano (IT)**

Remarks:
The complete document including Reference
Table(s) and the Sequence Listing(s) can be
downloaded from the EPO website

(54) **NUCLEIC ACID CONSTRUCT AND USE THEREOF**

(57) The present disclosure relates to a nucleic acid construct and use thereof. Specifically, the present disclosure relates to a nucleic acid construct comprising an engineered UTR, and use thereof in the prevention and treatment of a disease (for example, prevention of virus infection). The UTR can significantly improve the expression efficiency of a target gene in the nucleic acid construct.

EP 4 516 897 A1

**Description**

The present disclosure claims priority to Chinese Patent Application No. CN202210456261.9, filed on April 27, 2022 and entitled "NUCLEIC ACID CONSTRUCT AND USE THEREOF", which is incorporated herein by reference in its entirety.

**TECHNICAL FIELD**

The present disclosure belongs to the field of nucleic acid drugs and particularly relates to a nucleic acid construct comprising engineered UTRs and use thereof for preventing or treating a disease (e.g., viral infection).

**BACKGROUND**

Vaccines are an important area of medical research. Existing vaccines include inactivated vaccines, adenoviral vector vaccines, attenuated influenza virus vector vaccines, recombinant protein vaccines, and nucleic acid vaccines (including RNA vaccines and DNA vaccines). In RNA vaccines, mRNA is the active ingredient, and it mainly contains a cap structure (Cap), a 5' untranslated region (UTR), an open reading frame (ORF) that encodes an antigen protein, a 3'UTR, and a poly(A) tail structure. The 5'UTR is essential for recruiting ribosomes to the mRNA and initiating codon selection, and it plays an important role in regulating translation efficiency and shaping the cellular proteome (Ivanov, et al. Science. 2016, 352(6292): 1413-1416). Eukaryotic 3'UTRs have various regulatory motifs that can be recognized by microRNAs (miRNAs) and rbp to control mRNA stability, localization, and translation (Mazumder et al., 2003; Mayr, 2017). The polyA tail (located at the 3' end of the mRNA) is another element that determines mRNA stability and protein levels. Optional 3'UTRs not only affect mRNA stability and translation but also control mRNA localization (Tushev et al., 2018). The design and optimization of mRNA, particularly the selection and use of its UTRs, are critical steps in the entire preparation process of mRNA vaccine products. Therefore, developing efficient UTRs remains an urgent need in the field of mRNA drugs.

The present disclosure provides mRNA vaccines with new UTR structures that offer the advantage of enabling stable and efficient expression of the target gene. The vaccines can be generally applied to mRNA vaccines (e.g., vaccines for influenza or COVID-19) to regulate the expression of the target gene, showcasing excellent prospects for clinical pharmaceutical applications.

**SUMMARY**

The present disclosure provides a nucleic acid element capable of regulating the expression of a target gene, and a nucleic acid construct. The nucleotide construct comprises at least one nucleic acid element capable of regulating the expression of a target gene.

Nucleic Acid Element

In some embodiments, the nucleic acid element is a 5' untranslated region element (5'UTR).

In some embodiments, the 5'UTR is selected from the group consisting of or is a 5'UTR derived from any one of the genes Rho GTPase activating protein (ARHGAP), heat shock 27kDa protein 1 (HSPB1), hemoglobin subunit beta (HBB), C-C motif chemokine ligand 13 (CCL13), and the like, or a derivative sequence thereof.

In some embodiments, the 5'UTR in the nucleic acid construct is derived from or is a 5'UTR sequence of an ARHGAP gene or a derivative sequence thereof. In some embodiments, the ARHGAP includes ARHGAP1, ARHGAP2, ARHGAP3, ARHGAP4, ARHGAP5, ARHGAP6, ARHGAP7 (DLC1), ARHGAP8, ARHGAP9, ARHGAP10, ARHGAP12, ARHGAP13 (SRGAP1), ARHGAP14 (SRGAP2), ARHGAP15, ARHGAP17 (RICH1), ARHGAP18, ARHGAP19, ARHGAP20, ARHGAP21, ARHGAP22, ARHGAP23, ARHGAP24, ARHGAP25, and ARHGAP26.

In some embodiments, the 5'UTR is derived from or is a 5'UTR sequence of an ARHGAP15 gene or a derivative sequence thereof. In some embodiments, the ARHGAP15 gene is derived from any species, such as human ARHGAP15, baboon ARHGAP15, monkey ARHGAP15, or mouse ARHGAP15.

In some embodiments, the 5'UTR is derived from or is a 5'UTR sequence of the human ARHGAP15 gene or a derivative sequence thereof. In some embodiments, the 5'UTR of the ARHGAP15 gene comprises or is the nucleotide sequence set forth in SEQ ID NO: 1 or a nucleotide sequence having at least 80%, 85%, 90%, 95%, 96%, 97%, 98%, 99%, or 100% identity thereto.

In some embodiments, the 5'UTR comprises at least one point mutation capable of being used to inhibit an ATG within the UTR from initiating translation. In some embodiments, the point mutation is selected from the group consisting of a mutation at any one or more sites of A, T, or G of the ATG sequence in the 5'UTR. In some embodiments, the point mutation is a mutation at site A of the ATG sequence in the 5'UTR. In some embodiments, the mutation is to mutate A into G, C, or T. In some embodiments, the mutation is to mutate the ATG into a GTG, CTG, or TTG. Further, the mutation is capable of being used to inhibit the ATG within the UTR from initiating translation.

In some embodiments, the aforementioned 5'UTR sequence derived from the ARHGAP15 gene comprises at least one point mutation capable of being used to inhibit an ATG within the UTR from initiating translation. In some embodiments, the point mutation is selected from the group consisting of a mutation at any one or more sites of A, T, or G of the ATG sequence in the 5'UTR. In some embodiments, the point mutation is a mutation at site A of the ATG sequence in the 5'UTR. In some embodiments, the mutation is to mutate A into G, C, or T.

In some embodiments, the aforementioned 5'UTR sequence derived from the ARHGAP15 gene comprises the nucleotide sequence set forth in SEQ ID NO: 44, or, the 5'UTR comprises a nucleotide sequence having at least 80%, 85%, 90%, 95%, 96%, 97%, 98%, or 99% identity to SEQ ID NO: 44, wherein $N_1$ is selected from the group consisting of A, G, C, and T.

In some embodiments, the aforementioned 5'UTR sequence derived from the ARHGAP15 gene comprises a nucleotide sequence in which an ATG is mutated into a GTG. In some embodiments, the 5'UTR of the ARHGAP15 gene comprises or is the nucleotide sequence set forth in SEQ ID NO: 2 or a nucleotide sequence having at least 80%, 85%, 90%, 95%, 96%, 97%, 98%, 99%, or 100% identity thereto.

In some embodiments, the aforementioned 5'UTR sequence derived from the ARHGAP15 gene comprises a nucleotide sequence in which an ATG is mutated into a CTG. In some embodiments, the 5'UTR of the ARHGAP15 gene comprises or is the nucleotide sequence set forth in SEQ ID NO: 42 or a nucleotide sequence having at least 80%, 85%, 90%, 95%, 96%, 97%, 98%, 99%, or 100% identity thereto.

In some embodiments, the aforementioned 5'UTR sequence derived from the ARHGAP15 gene comprises a nucleotide sequence in which an ATG is mutated into a TTG. In some embodiments, the 5'UTR of the ARHGAP15 gene comprises or is the nucleotide sequence set forth in SEQ ID NO: 43 or a nucleotide sequence having at least 80%, 85%, 90%, 95%, 96%, 97%, 98%, 99%, or 100% identity thereto.

In some embodiments, the 5'UTR is a 5'UTR truncation. In some embodiments, the 5'UTR truncation still maintains active functions similar to those of a natural 5'UTR, e.g., still maintains a function of regulating expression of a protein from an encoding target gene of the ORF. In some embodiments, the 5'UTR truncation has enhanced active functions, e.g., an enhanced function of regulating expression of a protein from an encoding target gene of the ORF, as compared to a natural 5'UTR.

In some embodiments, a truncation mode for the 5'UTR truncation comprises: in the sequence direction from the 5' end to the 3' end, deleting a sequence of contiguous nucleotides from the 5' end; and/or, in the sequence direction from the 3' end to the 5' end, deleting a sequence of contiguous nucleotides from the 3' end. In some embodiments, a truncation mode for the 5'UTR truncation comprises, in the sequence direction from the 5' end to the 3' end, deleting a sequence of contiguous nucleotides from the 5' end, and keeping a nucleotide sequence of the 3' end. In some embodiments, a truncation mode for the 5'UTR truncation comprises, in the sequence direction from the 3' end to the 5' end, deleting a sequence of contiguous nucleotides from the 3' end, and keeping a nucleotide sequence of the 5' end. In some embodiments, a truncation mode for the 5'UTR truncation comprises, in the sequence direction from the 5' end to the 3' end, deleting a sequence of contiguous nucleotides from the 5' end; and the truncation mode for the 5'UTR truncation comprises, in the sequence direction from the 3' end to the 5' end, deleting a sequence of contiguous nucleotides from the 3' end. In some embodiments, the 5'UTR truncation also comprises the point mutation according to any of the preceding embodiments. In some embodiments, the 5'UTR truncation does not comprise the point mutation according to any of the preceding embodiments. In some embodiments, the 5'UTR truncation retains a sequence the length of which is at least 90%, 80%, 70%, 60%, 50%, 40%, 30%, 20%, 15%, 10%, 5%, or 1% of the length of a natural 5'UTR sequence.

In some embodiments, the aforementioned 5'UTR sequence derived from the ARHGAP15 gene comprises or is an ARHGAP15 5'UTR truncation. In some embodiments, a truncation mode for the truncation comprises, in the direction from the 5' end to the 3' end of the ARHGAP15 5'UTR, deleting a sequence of contiguous nucleotides from the 5' end; and/or, in the sequence direction from the 3' end to the 5' end, deleting a sequence of contiguous nucleotides from the 3' end.

In some embodiments, a truncation mode for the truncation comprises, in the direction from the 5' end to the 3' end of the ARHGAP15 5'UTR, deleting a sequence of contiguous nucleotides from the 5' end, and keeping a nucleotide sequence of the 3' end. In some embodiments, a truncation mode for the 5'UTR truncation comprises, in the direction from the 3' end to the 5' end of the ARHGAP15 5'UTR, deleting a sequence of contiguous nucleotides from the 3' end, and keeping a nucleotide sequence of the 5' end. In some embodiments, a truncation mode for the 5'UTR truncation is, in the direction from the 5' end to the 3' end of the ARHGAP15 5'UTR, to delete a sequence of contiguous nucleotides from the 5' end; and, in the sequence direction from the 3' end to the 5' end, to delete a sequence of contiguous nucleotides from the 3' end.

In some embodiments, the truncation retains a sequence the length of which is at least 90%, 80%, 70%, 60%, 50%, 40%, 30%, 20%, 15%, 10%, 5%, or 1% of the length of the natural ARHGAP15 5'UTR (SEQ ID NO: 1).

In some embodiments, the 5'UTR truncation comprises a sequence of at least 5 contiguous nucleotides from the sequence set forth in SEQ ID NO: 2 or 44; in some embodiments, the 5'UTR truncation comprises a sequence of 5-62 contiguous nucleotides from the sequence set forth in SEQ ID NO: 2 or 44; in some embodiments, the 5'UTR truncation comprises a sequence of 7-62 contiguous nucleotides from the sequence set forth in SEQ ID NO: 2 or 44; in some embodiments, the 5'UTR truncation comprises a sequence of 7-59 contiguous nucleotides from the sequence set forth in SEQ ID NO: 2; illustratively, the 5'UTR truncation comprises a sequence of 5, 7, 8, 9, 10, 11, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 contiguous nucleotides from the sequence set forth in SEQ ID NO: 2 or 44.

In some embodiments, the nucleotide at the 5' end of the 5'UTR truncation (i.e., the starting nucleotide of the 5'UTR truncation) is the nucleotide at any one of positions 1-57, according to natural counting, of the sequence set forth in SEQ ID NO: 2 or 44. In some embodiments, the nucleotide at the 5' end of the 5'UTR truncation is the nucleotide at any one of positions 1-13 or 17-29, according to natural counting, of the sequence set forth in SEQ ID NO: 2 or 44, for example, the nucleotide at any one of positions 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, and 57.

Illustratively, the nucleotide at the 5' end of the 5'UTR truncation, and the length of the sequence of contiguous nucleotides from the sequence set forth in SEQ ID NO: 2 that the 5'UTR truncation comprises are shown in the table below:

| Starting nucleotide | The length of the sequence of contiguous nucleotides from the sequence set forth in SEQ ID NO: 2 |
|---|---|
| Position 1, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 |
| Position 2, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 |
| Position 3, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, or 61 |
| Position 4, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 |
| Position 5, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 |
| Position 9, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 |
| Position 13, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51 |
| Position 17, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 |
| Position 21, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 |
| Position 23, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41 |
| Position 25, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 |
| Position 29, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 |
| Position 33, G | 5, 6, 7, 8, 9, 10, 11, 12, 13, 14, 15, 16, 17, 18, 19, 20, 21, 22, 23, 24, 25, 26, 27, 28, 29, 30, 31 |
| Position 37, T | 5, 6, 7, 8, 9, 10, 11, 12, 13, 14, 15, 16, 17, 18, 19, 20, 21, 22, 23, 24, 25, 26, 27 |

(continued)

| Starting nucleotide | The length of the sequence of contiguous nucleotides from the sequence set forth in SEQ ID NO: 2 |
|---|---|
| Position 41, C | 5, 6, 7, 8, 9, 10, 11, 12, 13, 14, 15, 16, 17, 18, 19, 20, 21, 22, 23 |
| Position 45, A | 5, 6, 7, 8, 9, 10, 11, 12, 13, 14, 15, 16, 17, 18, 19 |
| Position 49, G | 5, 6, 7, 8, 9, 10, 11, 12, 13, 14, 15 |
| Position 53, A | 5, 6, 7, 8, 9, 10, 11 |
| Position 57, C | 5, 6, 7 |

In some embodiments, the 5'UTR truncation is 59 bp, 55 bp, 51 bp, 47 bp, 43 bp, 39 bp, 35 bp, 31 bp, 27 bp, 23 bp, 19 bp, 15 bp, 11 bp, or 7 bp in length.

In some embodiments, the ARHGAP15 5'UTR truncation also comprises the point mutation according to any of the preceding embodiments. In some embodiments, the ARHGAP15 5'UTR truncation also comprises a nucleotide sequence in which an ATG is mutated into a GTG. In some embodiments, the aforementioned 5'UTR derived from the ARHGAP15 gene comprises or is the nucleotide sequence set forth in any one of SEQ ID NOs: 28-30 or a nucleotide sequence having at least 80%, 85%, 90%, 95%, 96%, 97%, 98%, 99%, or 100% identity thereto.

In some embodiments, the ARHGAP15 5'UTR truncation does not comprise the point mutation according to any of the preceding embodiments. In some embodiments, the aforementioned 5'UTR of the ARHGAP15 gene comprises or is the nucleotide sequences set forth in SEQ ID NOs: 31-40 and comprises CTATAAT; or a nucleotide sequence having at least 80%, 85%, 90%, 95%, 96%, 97%, 98%, 99%, or 100% identity to any one of the aforementioned sequences.

In some embodiments, the 5'UTR truncation comprises at least CTATAAT or the nucleotide sequence set forth in SEQ ID NO: 193.

In some embodiments, the ARHGAP15 5'UTR truncation comprises or is the nucleotide sequence set forth in any one of SEQ ID NOs: 41, 76, and 137-196 or a nucleotide sequence having at least 80%, 85%, 90%, 95%, 96%, 97%, 98%, 99%, or 100% identity thereto.

In some embodiments, the aforementioned 5'UTR derived from the HSPB1 gene comprises or is the nucleotide sequence set forth in any one of SEQ ID NOs: 3-5 or a nucleotide sequence having at least 80%, 85%, 90%, 95%, 96%, 97%, 98%, 99%, or 100% identity to any one of SEQ ID NOs: 3-5.

In some embodiments, the 5'UTR comprises or is the nucleotide sequence set forth in any one of SEQ ID NOs: 1-2, 3-5, 28-43, 76, and 137-196, or the following sequence: CTATAAT, or a nucleotide sequence having at least 80%, 85%, 90%, 95%, 96%, 97%, 98%, 99%, or 100% identity to any one of the aforementioned sequences.

Nucleic Acid Construct

The present disclosure provides a nucleic acid construct comprising:

(a) an open reading frame (ORF), and
(b) a 5' untranslated region element (5'UTR).

In some embodiments, the ORF is a polynucleotide sequence encoding a target gene protein.

In some embodiments, the target gene is heterologous. In some other embodiments, the target gene is endogenous.

In some embodiments, the 5'UTR in the nucleic acid construct is located upstream of the open reading frame. In some embodiments, the 5'UTR in the nucleic acid construct is located at the 5' end of the open reading frame.

In some embodiments, the 5'UTR in the nucleic acid construct is selected from the group consisting of or is a 5'UTR derived from any one of the genes Rho GTPase activating protein (ARHGAP), heat shock 27kDa protein 1 (HSPB1), hemoglobin subunit beta (HBB), C-C motif chemokine ligand 13 (CCL13), and the like, or a derivative sequence thereof.

In some embodiments, the 5'UTR in the nucleic acid construct is derived from or is a 5'UTR sequence of an ARHGAP gene or a derivative sequence thereof. In some embodiments, the ARHGAP includes ARHGAP1, ARHGAP2, ARHGAP3, ARHGAP4, ARHGAP5, ARHGAP6, ARHGAP7 (DLC1), ARHGAP8, ARHGAP9, ARHGAP10, ARHGAP12, ARHGAP13 (SRGAP1), ARHGAP14 (SRGAP2), ARHGAP15, ARHGAP17 (RICH1), ARHGAP18, ARHGAP19, ARHGAP20, ARHGAP21, ARHGAP22, ARHGAP23, ARHGAP24, ARHGAP25, and ARHGAP26.

In some embodiments, the 5'UTR in the nucleic acid construct is derived from or is a 5'UTR sequence of an ARHGAP15 gene or a derivative sequence thereof. In some embodiments, the ARHGAP15 gene is derived from any species, such as human ARHGAP15, baboon ARHGAP15, monkey ARHGAP15, or mouse ARHGAP15.

In some embodiments, the 5'UTR in the nucleic acid construct is derived from or is a 5'UTR sequence of the human ARHGAP15 gene or a derivative sequence thereof. In some embodiments, the 5'UTR of the ARHGAP15 gene comprises or is the nucleotide sequence set forth in SEQ ID NO: 1 or a nucleotide sequence having at least 80%, 85%, 90%, 95%, 96%, 97%, 98%, 99%, or 100% identity thereto.

In some embodiments, the 5'UTR in the nucleic acid construct comprises at least one point mutation capable of being used to inhibit an ATG within the UTR from initiating translation. In some embodiments, the point mutation is selected from the group consisting of a mutation at any one or more sites of A, T, or G of the ATG sequence in the 5'UTR. In some embodiments, the point mutation is a mutation at site A of the ATG sequence in the 5'UTR. In some embodiments, the mutation is to mutate A into G, C, or T. In some embodiments, the mutation is to mutate the ATG into a GTG, CTG, or TTG. Further, the mutation is capable of being used to inhibit the ATG within the UTR from initiating translation.

In some embodiments, the aforementioned 5'UTR sequence derived from the ARHGAP15 gene comprises at least one point mutation capable of being used to inhibit an ATG within the UTR from initiating translation. In some embodiments, the point mutation is selected from the group consisting of a mutation at any one or more sites of A, T, or G of the ATG sequence in the 5'UTR. In some embodiments, the point mutation is a mutation at site A of the ATG sequence in the 5'UTR. In some embodiments, the mutation is to mutate A into G, C, or T.

In some embodiments, the aforementioned 5'UTR sequence derived from the ARHGAP15 gene comprises the nucleotide sequence set forth in SEQ ID NO: 44, or, the 5'UTR comprises a nucleotide sequence having at least 80%, 85%, 90%, 95%, 96%, 97%, 98%, or 99% identity to SEQ ID NO: 44, wherein $N_1$ is selected from the group consisting of A, G, C, and T.

In some embodiments, the aforementioned 5'UTR sequence derived from the ARHGAP15 gene comprises a nucleotide sequence in which an ATG is mutated into a GTG. In some embodiments, the 5'UTR of the ARHGAP15 gene comprises or is the nucleotide sequence set forth in SEQ ID NO: 2 or a nucleotide sequence having at least 80%, 85%, 90%, 95%, 96%, 97%, 98%, 99%, or 100% identity thereto.

In some embodiments, the aforementioned 5'UTR sequence derived from the ARHGAP15 gene comprises a nucleotide sequence in which an ATG is mutated into a CTG. In some embodiments, the 5'UTR of the ARHGAP15 gene comprises or is the nucleotide sequence set forth in SEQ ID NO: 42 or a nucleotide sequence having at least 80%, 85%, 90%, 95%, 96%, 97%, 98%, 99%, or 100% identity thereto.

In some embodiments, the aforementioned 5'UTR sequence derived from the ARHGAP15 gene comprises a nucleotide sequence in which an ATG is mutated into a TTG. In some embodiments, the 5'UTR of the ARHGAP15 gene comprises or is the nucleotide sequence set forth in SEQ ID NO: 43 or a nucleotide sequence having at least 80%, 85%, 90%, 95%, 96%, 97%, 98%, 99%, or 100% identity thereto.

In some embodiments, the 5'UTR in the nucleic acid construct of the present disclosure is a 5'UTR truncation. In some embodiments, the 5'UTR truncation still maintains active functions similar to those of a natural 5'UTR, e.g., still maintains a function of regulating expression of a protein from an encoding target gene of the ORF. In some embodiments, the 5'UTR truncation has enhanced active functions, e.g., an enhanced function of regulating expression of a protein from an encoding target gene of the ORF, as compared to a natural 5'UTR.

In some embodiments, a truncation mode for the 5'UTR truncation comprises: in the sequence direction from the 5' end to the 3' end, deleting a sequence of contiguous nucleotides from the 5' end; and/or, in the sequence direction from the 3' end to the 5' end, deleting a sequence of contiguous nucleotides from the 3' end. In some embodiments, a truncation mode for the 5'UTR truncation comprises, in the sequence direction from the 5' end to the 3' end, deleting a sequence of contiguous nucleotides from the 5' end, and keeping a nucleotide sequence of the 3' end. In some embodiments, a truncation mode for the 5'UTR truncation comprises, in the sequence direction from the 3' end to the 5' end, deleting a sequence of contiguous nucleotides from the 3' end, and keeping a nucleotide sequence of the 5' end. In some embodiments, a truncation mode for the 5'UTR truncation comprises, in the sequence direction from the 5' end to the 3' end, deleting a sequence of contiguous nucleotides from the 5' end; and the truncation mode for the 5'UTR truncation comprises, in the sequence direction from the 3' end to the 5' end, deleting a sequence of contiguous nucleotides from the 3' end.

In some embodiments, the 5'UTR truncation also comprises the point mutation according to any of the preceding embodiments. In some embodiments, the 5'UTR truncation does not comprise the point mutation according to any of the preceding embodiments. In some embodiments, the 5'UTR truncation retains a sequence the length of which is at least 90%, 80%, 70%, 60%, 50%, 40%, 30%, 20%, 15%, 10%, 5%, or 1% of the length of a natural 5'UTR sequence.

In some embodiments, the aforementioned 5'UTR sequence derived from the ARHGAP15 gene comprises or is an ARHGAP15 5'UTR truncation. In some embodiments, a truncation mode for the truncation comprises, in the direction from the 5' end to the 3' end of the ARHGAP15 5'UTR, deleting a sequence of contiguous nucleotides from the 5' end; and/or, in the sequence direction from the 3' end to the 5' end, deleting a sequence of contiguous nucleotides from the 3' end.

In some embodiments, a truncation mode for the truncation comprises, in the direction from the 5' end to the 3' end of the ARHGAP15 5'UTR, deleting a sequence of contiguous nucleotides from the 5' end, and keeping a nucleotide sequence of the 3' end. In some embodiments, a truncation mode for the 5'UTR truncation comprises, in the direction from the 3' end to the 5' end of the ARHGAP15 5'UTR, deleting a sequence of contiguous nucleotides from the 3' end, and keeping a nucleotide sequence of the 5' end. In some embodiments, a truncation mode for the 5'UTR truncation is, in the direction from the 5' end to the 3' end of the ARHGAP15 5'UTR, to delete a sequence of contiguous nucleotides from the 5' end; and, in the sequence direction from the 3' end to the 5' end, to delete a sequence of contiguous nucleotides from the 3' end.

In some embodiments, the truncation retains a sequence the length of which is at least 90%, 80%, 70%, 60%, 50%, 40%, 30%, 20%, 15%, 10%, 5%, or 1% of the length of the natural ARHGAP15 5'UTR (SEQ ID NO: 1).

In some embodiments, the 5'UTR truncation comprises a sequence of at least 5 contiguous nucleotides from the nucleotide sequence set forth in SEQ ID NO: 2 or 44; in some embodiments, the 5'UTR truncation comprises a sequence of 5-62 contiguous nucleotides from the sequence set forth in SEQ ID NO: 2 or 44; in some embodiments, the 5'UTR truncation comprises a sequence of 7-62 contiguous nucleotides from the sequence set forth in SEQ ID NO: 2 or 44; in some embodiments, the 5'UTR truncation comprises a sequence of 7-59 contiguous nucleotides from the sequence set forth in SEQ ID NO: 2; illustratively, the 5'UTR truncation comprises a sequence of 5, 7, 8, 9, 10, 11, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 contiguous nucleotides from the sequence set forth in SEQ ID NO: 2. In some embodiments, the nucleotide at the 5' end of the 5'UTR truncation (i.e., the starting nucleotide of the 5'UTR truncation) is the nucleotide at any one of positions 1-57, according to natural counting, of the sequence set forth in SEQ ID NO: 2 or 44. In some embodiments, the nucleotide at the 5' end of the 5'UTR truncation is the nucleotide at any one of positions 1-13 or 17-29, according to natural counting, of the sequence set forth in SEQ ID NO: 2 or 44, for example, the nucleotide at any one of positions 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, and 57.

Illustratively, the nucleotide at the 5' end of the 5'UTR truncation, and the length of the sequence of contiguous nucleotides from the sequence set forth in SEQ ID NO: 2 that the 5'UTR truncation comprises are shown in the table below:

| Starting nucleotide | The length of the sequence of contiguous nucleotides from the sequence set forth in SEQ ID NO: 2 |
|---|---|
| Position 1, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 |
| Position 2, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 |
| Position 3, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, or 61 |
| Position 4, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 |
| Position 5, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 |
| Position 9, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 |
| Position 13, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51 |
| Position 17, G | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 |
| Position 21, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43 |
| Position 23, T | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41 |

(continued)

| Starting nucleotide | The length of the sequence of contiguous nucleotides from the sequence set forth in SEQ ID NO: 2 |
|---|---|
| Position 25, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 |
| Position 29, A | 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 |
| Position 33, G | 5, 6, 7, 8, 9, 10, 11, 12, 13, 14, 15, 16, 17, 18, 19, 20, 21, 22, 23, 24, 25, 26, 27, 28, 29, 30, 31 |
| Position 37, T | 5, 6, 7, 8, 9, 10, 11, 12, 13, 14, 15, 16, 17, 18, 19, 20, 21, 22, 23, 24, 25, 26, 27 |
| Position 41, C | 5, 6, 7, 8, 9, 10, 11, 12, 13, 14, 15, 16, 17, 18, 19, 20, 21, 22, 23 |
| Position 45, A | 5, 6, 7, 8, 9, 10, 11, 12, 13, 14, 15, 16, 17, 18, 19 |
| Position 49, G | 5, 6, 7, 8, 9, 10, 11, 12, 13, 14, 15 |
| Position 53, A | 5, 6, 7, 8, 9, 10, 11 |
| Position 57, C | 5, 6, 7 |

In some embodiments, the 5'UTR truncation is 59 bp, 55 bp, 51 bp, 47 bp, 43 bp, 39 bp, 35 bp, 31 bp, 27 bp, 23 bp, 19 bp, 15 bp, 11 bp, or 7 bp in length.

In some embodiments, the ARHGAP15 5'UTR truncation also comprises the point mutation according to any of the preceding embodiments. In some embodiments, the ARHGAP15 5'UTR truncation also comprises a nucleotide sequence in which an ATG is mutated into a GTG. In some embodiments, the aforementioned 5'UTR derived from the ARHGAP15 gene comprises or is the nucleotide sequence set forth in any one of SEQ ID NOs: 28-30 or a nucleotide sequence having at least 80%, 85%, 90%, 95%, 96%, 97%, 98%, 99%, or 100% identity thereto.

In some embodiments, the ARHGAP15 5'UTR truncation does not comprise the point mutation according to any of the preceding embodiments. In some embodiments, the aforementioned 5'UTR of the ARHGAP15 gene comprises or is the nucleotide sequences set forth in SEQ ID NOs: 31-40, CTATAAT, or a nucleotide sequence having at least 80%, 85%, 90%, 95%, 96%, 97%, 98%, 99%, or 100% identity thereto.

In some embodiments, the 5'UTR truncation comprises at least CTATAAT or the nucleotide sequence set forth in SEQ ID NO: 193.

In some embodiments, the ARHGAP15 5'UTR truncation comprises or is the nucleotide sequence set forth in any one of SEQ ID NOs: 41, 76, and 137-196 or a nucleotide sequence having at least 80%, 85%, 90%, 95%, 96%, 97%, 98%, 99%, or 100% identity thereto.

In some embodiments, the aforementioned 5'UTR derived from the HSPB1 gene comprises or is the nucleotide sequence set forth in any one of SEQ ID NOs: 3-5 or a nucleotide sequence having at least 80%, 85%, 90%, 95%, 96%, 97%, 98%, 99%, or 100% identity to any one of SEQ ID NOs: 3-5.

In some embodiments, the 5'UTR in the nucleic acid constructs of the present disclosure comprises or is the nucleotide sequence set forth in any one of SEQ ID NOs: 1-2, 3-5, 28-43, 76, and 137-196, or the following sequence: CTATAAT; or a nucleotide sequence having at least 80%, 85%, 90%, 95%, 96%, 97%, 98%, 99%, or 100% identity thereto. In some specific embodiments, the 5'UTR in the nucleic acid construct according to any one of the above comprises or is the nucleotide sequence set forth in any one of SEQ ID NOs: 1-5, 28-43, 76, and 137-196, or the following sequence: CTATAAT.

In some embodiments, the nucleic acid construct of the present disclosure also further comprises: (c) a 3' untranslated region element (3'UTR).

In some embodiments, the open reading frame of the present disclosure is derived from a different gene than the 5'UTR and/or the 3'UTR. In some embodiments, the nucleic acid construct of the present disclosure comprises at least one open reading frame, at least one 5'UTR, or at least one 3'UTR. In some embodiments, the 5'UTR and 3'UTR in the nucleic acid construct of the present disclosure are from the same source or different sources, e.g., derived from the same gene or different genes. In some embodiments, the 5'UTR and 3'UTR in the nucleic acid construct of the present disclosure are derived from the same species or different species.

In some embodiments, the 3'UTR in the nucleic acid construct of the present disclosure is located downstream of the open reading frame. In some embodiments, the 3'UTR in the nucleic acid construct is located at the 3' end of the open reading frame.

In some embodiments, the 3'UTR in the nucleic acid construct of the present disclosure is selected from the group consisting of or is a 3'UTR derived from any one of the genes hemoglobin subunit beta (HBB), ARHGAP15, coronin 1A (CORO1A), hemopexin (HPX), and the like, or a derivative sequence thereof.

In some embodiments, the 3'UTR is derived from or is a 3'UTR sequence of the HBB or ARHGAP15 gene or a derivative sequence thereof.

In some embodiments, the aforementioned 3'UTR derived from the HBB gene comprises or is the nucleotide sequence set forth in SEQ ID NO: 7 or a nucleotide sequence having at least 80%, 85%, 90%, 95%, 96%, 97%, 98%, 99%, or 100% identity thereto.

In some embodiments, the aforementioned 3'UTR derived from the ARHGAP15 gene comprises or is the nucleotide sequence set forth in SEQ ID NO: 8 or a nucleotide sequence having at least 80%, 85%, 90%, 95%, 96%, 97%, 98%, 99%, or 100% identity thereto.

In some embodiments, the aforementioned 3'UTR derived from the CORO1A gene comprises or is the nucleotide sequence set forth in SEQ ID NO: 9 or a nucleotide sequence having at least 80%, 85%, 90%, 95%, 96%, 97%, 98%, 99%, or 100% identity thereto.

In some embodiments, the aforementioned 3'UTR derived from the HPX gene comprises or is the nucleotide sequence set forth in SEQ ID NO: 10 or a nucleotide sequence having at least 80%, 85%, 90%, 95%, 96%, 97%, 98%, 99%, or 100% identity thereto.

In some embodiments, the 3'UTR in the nucleic acid construct of the present disclosure comprises or is the nucleotide sequence set forth in any one of SEQ ID NOs: 7-10 or a nucleotide sequence having at least 80%, 85%, 90%, 95%, 96%, 97%, 98%, 99%, or 100% identity thereto. In some specific embodiments, the 3'UTR comprises or is the nucleotide sequence set forth in SEQ ID NO: 7 or 8.

In some embodiments, the nucleic acid construct comprises a 5'UTR and a 3'UTR, wherein:

the 5'UTR is selected from the group consisting of or is a 5'UTR derived from any one of the genes ARHGAP (e.g., ARHGAP15), HSPB1, HBB, CCL13, and the like, or a derivative sequence thereof, and the 3'UTR is selected from the group consisting of or is a 3'UTR derived from any one of the genes HBB, ARHGAP15, CORO1A, HPX, and the like, or a derivative sequence thereof.

In some embodiments, the nucleic acid construct comprises a 5'UTR and a 3'UTR, the 5'UTR and the 3'UTR being selected from the group consisting of any one of the following:

the 5'UTR is derived from or is a 5'UTR of the ARHGAP15 gene or a derivative sequence thereof, and the 3'UTR is derived from or is a 3'UTR of the HBB gene or a derivative sequence thereof;

the 5'UTR is derived from or is a 5'UTR of the ARHGAP15 gene or a derivative sequence thereof, and the 3'UTR is derived from or is a 3'UTR of the ARHGAP15 gene or a derivative sequence thereof;

the 5'UTR is derived from or is a 5'UTR of the ARHGAP15 gene or a derivative sequence thereof, and the 3'UTR is derived from or is a 3'UTR of the CORO1A gene or a derivative sequence thereof;

the 5'UTR is derived from or is a 5'UTR of the ARHGAP15 gene or a derivative sequence thereof, and the 3'UTR is derived from or is a 3'UTR of the HPX gene or a derivative sequence thereof;

the 5'UTR is derived from or is a 5'UTR of the HBB gene or a derivative sequence thereof, and the 3'UTR is derived from or is a 3'UTR of the HBB gene or a derivative sequence thereof;

the 5'UTR is derived from or is a 5'UTR of the HBB gene or a derivative sequence thereof, and the 3'UTR is derived from or is a 3'UTR of the ARHGAP15 gene or a derivative sequence thereof;

the 5'UTR is derived from or is a 5'UTR of the HBB gene or a derivative sequence thereof, and the 3'UTR is derived from or is a 3'UTR of the CORO1A gene or a derivative sequence thereof;

the 5'UTR is derived from or is a 5'UTR of the HBB gene or a derivative sequence thereof, and the 3'UTR is derived from or is a 3'UTR of the HPX gene or a derivative sequence thereof;

the 5'UTR is derived from or is a 5'UTR of the HSPB1 gene or a derivative sequence thereof, and the 3'UTR is derived from or is a 3'UTR of the HBB gene or a derivative sequence thereof;

the 5'UTR is derived from or is a 5'UTR of the HSPB1 gene or a derivative sequence thereof, and the 3'UTR is derived from or is a 3'UTR of the ARHGAP15 gene or a derivative sequence thereof;

the 5'UTR is derived from or is a 5'UTR of the HSPB1 gene or a derivative sequence thereof, and the 3'UTR is derived from or is a 3'UTR of the CORO1A gene or a derivative sequence thereof;

the 5'UTR is derived from or is a 5'UTR of the HSPB1 gene or a derivative sequence thereof, and the 3'UTR is derived from or is a 3'UTR of the HPX gene or a derivative sequence thereof; or

the 5'UTR is derived from or is a 5'UTR of the CCL13 gene or a derivative sequence thereof, and the 3'UTR is derived from or is a 3'UTR of the HBB gene or a derivative sequence thereof;

the 5'UTR is derived from or is a 5'UTR of the CCL13 gene or a derivative sequence thereof, and the 3'UTR is derived from or is a 3'UTR of the ARHGAP15 gene or a derivative sequence thereof;

the 5'UTR is derived from or is a 5'UTR of the CCL13 gene or a derivative sequence thereof, and the 3'UTR is derived from or is a 3'UTR of the CORO1A gene or a derivative sequence thereof; or

the 5'UTR is derived from or is a 5'UTR of the CCL13 gene or a derivative sequence thereof, and the 3'UTR is derived from or is a 3'UTR of the HPX gene or a derivative sequence thereof.

In some embodiments, the nucleic acid construct of the present disclosure comprises a 5'UTR and a 3'UTR, the 5'UTR and the 3'UTR being selected from the group consisting of any one of the following:

the 5'UTR comprises or is the nucleotide sequence set forth in any one of SEQ ID NOs: 1-2, 28-43, 76, and 137-196 and CTATAAT or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 7 or a nucleotide sequence having at least 80%, 85%, 90%, 95%, 96%, 97%, 98%, 99%, or 100% identity thereto;

the 5'UTR comprises or is the nucleotide sequence set forth in any one of SEQ ID NOs: 1-2, 28-43, 76, and 137-196 and CTATAAT or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 8 or a nucleotide sequence having at least 80%, 85%, 90%, 95%, 96%, 97%, 98%, 99%, or 100% identity thereto;

the 5'UTR comprises or is the nucleotide sequence set forth in any one of SEQ ID NOs: 1-2, 28-43, 76, and 137-196 and CTATAAT or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 9 or a nucleotide sequence having at least 80%, 85%, 90%, 95%, 96%, 97%, 98%, 99%, or 100% identity thereto;

the 5'UTR comprises or is the nucleotide sequence set forth in any one of SEQ ID NOs: 1-2, 28-43, 76, and 137-196 and CTATAAT or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 10 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 3 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 7 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 3 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 8 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 3 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 9 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 3 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 10 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 4 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 7 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 4 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 8 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 4 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 9 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 4 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 10 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 5 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 7 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 5 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 8 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 5 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 9 or a nucleotide sequence having at least 80%, 85%, 90%, 95%, 96%, 97%, 98%, 99%, or 100% identity thereto; and

the 5'UTR comprises or is the nucleotide sequence set forth in SEQ ID NO: 5 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 10 or a nucleotide sequence having at least 80%, 85%, 90%, 95%, 96%, 97%, 98%, 99%, or 100% identity thereto.

In some embodiments, the nucleic acid construct comprises a 5'UTR and a 3'UTR, wherein: the 5'UTR comprises or is the nucleotide sequence set forth in any one of SEQ ID NOs: 1-2, 28-43, 76, and 137-196 and CTATAAT, and the 3'UTR is derived from or is a 3'UTR derived from the HBB, ARHGAP15, CORO1A, or HPX gene or a derivative sequence thereof.

In some specific embodiments, the nucleic acid construct comprises a 5'UTR and a 3'UTR, wherein: the 5'UTR comprises or is the nucleotide sequence set forth in any one of SEQ ID NOs: 1-2, 28-43, 76, and 137-196 and CTATAAT, and the 3'UTR comprises or is the nucleotide sequence set forth in any one of SEQ ID NOs: 7-10.

In some embodiments, the nucleic acid construct of the present disclosure also further comprises: (d) a polyadenylic acid (poly-A) tail.

In some embodiments, the poly-A tail in the nucleic acid construct is located downstream of the 3'UTR. In some embodiments, the poly-A tail in the nucleic acid construct is located at the 3' end of the 3'UTR. In some embodiments, the poly-A tail is at the 3' end of the nucleic acid construct. In some embodiments, the poly-A tail is at least about 50, 100, 150, 200, 300, 400, or 500 nucleotides in length.

In some embodiments, the poly-A tail includes, but is not limited to, HGH polyA, SV40polyA, BGH polyA, rbGlob polyA, or SV40late polyA.

In some specific embodiments, the poly-A tail comprises or is the nucleotide sequence set forth in SEQ ID NO: 16 or 135.

In some embodiments, in the 5'-to-3' direction, the nucleic acid construct according to any one of the above comprises any one of i)-iv):

    i) a 5'UTR, and an open reading frame (ORF);
    ii) an open reading frame (ORF), and a 3'UTR;
    iii) a 5'UTR, an open reading frame (ORF), and a 3'UTR; and
    iv) a 5'UTR, an open reading frame (ORF), a 3'UTR, and a poly-A tail;

wherein the ORF is derived from a different gene than the 5'UTR and/or the 3'UTR.

In some embodiments, the 5'UTR in i) and iii) to iv) is selected from the group consisting of a 5'UTR derived from any one of the genes ARHGAP, HSPB1, HBB, CCL13, and the like, or a derivative sequence thereof (e.g., a 5'UTR of ARHGAP15 or a derivative sequence thereof).

In some embodiments, the 3'UTR in ii) to iv) is selected from the group consisting of a 3'UTR derived from any one of the genes HBB, ARHGAP15, CORO1A, HPX, and the like, or a derivative sequence thereof (e.g., a 3'UTR of HBB or ARHGAP15 or a derivative sequence thereof).

In some embodiments, the poly-A tail in iv) to iv) comprises, for example, the nucleotide sequence set forth in SEQ ID NO: 16 or 135.

In some embodiments, the 5'UTR in i) comprises or is the nucleotide sequence set forth in any one of SEQ ID NOs: 1-5, 28-43, 76, and 137-196 and CTATAAT.

In some embodiments, the 3'UTR in ii) comprises or is the nucleotide sequence set forth in any one of SEQ ID NOs: 7-10.

In some embodiments, the 5'UTR in iii) comprises or is the nucleotide sequence set forth in any one of SEQ ID NOs: 1-5, 28-43, 76, and 137-196 and CTATAAT, and the 3'UTR comprises or is the nucleotide sequence set forth in any one of SEQ ID NOs: 7-10.

In some embodiments, the 5'UTR in iv) comprises or is the nucleotide sequence set forth in any one of SEQ ID NOs: 1-5, 28-43, 76, and 137-196 and CTATAAT, the 3'UTR comprises or is the nucleotide sequence set forth in any one of SEQ ID NOs: 7-10, and the poly-A tail comprises or is the nucleotide sequence set forth in SEQ ID NO: 16 or 135.

In the present disclosure, in the nucleic acid construct according to any one of the above, the ORF comprises a nucleotide sequence encoding at least one polypeptide or protein. In some embodiments, the nucleotide sequence may be a codon-optimized nucleotide sequence.

In some embodiments, the polypeptide or protein encoded by the ORF is a fluorescent protein or a luciferase.

In some embodiments, the polypeptide or protein encoded by the ORF is a viral antigen. Illustratively, the viral antigen includes, but is not limited to, antigens of influenza viruses, coronaviruses, respiratory syncytial virus, human immunodeficiency viruses, herpes simplex virus, rabies virus, or EB virus, among others.

In some embodiments, the viral antigen is a coronavirus antigen. In some embodiments, the coronavirus is a human-infecting coronavirus, such as SARS-CoV-2 (COVID-19), SARS-CoV, HCoV-229E, HCoV-OC43, HCoV-NL63, HCoV-HKU1, or MERS-CoV. In some embodiments, the coronavirus is SARS-COV-2. In some embodiments, the coronavirus antigen is a structural protein. In some embodiments, the structural protein is selected from the group consisting of a spike protein (S protein), an envelope protein (E protein), a membrane protein (M protein), and a nucleocapsid protein (N protein). In some embodiments, the structural protein is a spike protein. In some embodiments, the spike protein is a SARS-COV-2 spike protein. In some embodiments, the SARS-COV-2 spike protein is selected from the group consisting of a spike protein of any one of the viral strains SARS-COV-2 (e.g., wild-type SARS-COV-2), SARS-COV-2 Alpha(B.1.1.7), SARS-COV-2 Beta(B.1.351), SARS-COV-2 Gamma(P.1), SARS-COV-2 Kappa(B.1.617.1), SARS-COV-2 Delta(B.1.617.2), SARS-COV-2 Omicron(B.1.1.529), SARS-COV-2 Omicron(B.A.4), and the like.

In some embodiments, the SARS-COV-2 spike protein comprises or is the amino acid sequence set forth in any one of SEQ ID NOs: 12, 14, 21, 23, 25, 80, and 136 or a sequence having at least 80%, 85%, 90%, 95%, 96%, 97%, 98%, 99%, or 100% amino acid identity thereto.

In some embodiments, the ORF comprises a codon-optimized nucleotide sequence comprising a sequence having at least 80%, 85%, 90%, 95%, 96%, 97%, 98%, 99%, or 100% identity to a wild-type nucleotide sequence encoding a SARS-COV-2 antigen (e.g., wild-type SARS-COV-2, SEQ ID NO: 81).

In some embodiments, the ORF encoding the polypeptide or protein comprises or is the nucleotide sequence set forth in any one of SEQ ID NOs: 13, 15, 20, 22, 24, 81, and 97 or a nucleotide sequence having at least 80%, 85%, 90%, 95%, 96%, 97%, 98%, 99%, or 100% identity thereto.

In some embodiments, the ORF encodes an influenza virus antigen. In some embodiments, the influenza virus is selected from the group consisting of influenza A virus and influenza B virus; illustratively, the influenza virus is influenza A virus H1N1, influenza A virus H3N2, influenza A virus H3N8, influenza A virus H2N2, influenza A virus H5N1, influenza A virus H9N2, influenza A virus H7N7, influenza B virus/Victoria (e.g., influenza B virus/Washington/02/2019), influenza B virus/Yamagata (e.g., influenza B/Phuket/3073/2013), or the like. In some embodiments, the influenza virus antigen is a structural protein of an influenza virus, e.g., hemagglutinin (HA), neuraminidase (NA), M2 ion channel, matrix protein M1, or nucleoprotein (NP). In some specific embodiments, the influenza virus antigen is an HA protein of an influenza virus, e.g., an HA protein of influenza A virus H1N1, influenza A virus H3N2, influenza B virus Victoria (e.g., influenza B/Washington/02/2019), or influenza B virus Yamagata (e.g., influenza B/Phuket/3073/2013). In some specific embodiments, the influenza virus antigen is an NA protein of an influenza virus, e.g., an NA protein of influenza A virus H1N1, influenza A virus H3N2, influenza B virus Victoria (e.g., influenza B/Washington/02/2019), or influenza B virus Yamagata (e.g., influenza B/Phuket/3073/2013).

In some embodiments, the HA protein comprises or is the amino acid sequence set forth in any one of SEQ ID NOs: 83-86 and 98-101 or a sequence having at least 80%, 85%, 90%, 95%, 96%, 97%, 98%, 99%, or 100% amino acid identity thereto.

In some embodiments, the ORF comprises a codon-optimized nucleotide sequence comprising a sequence having at least 80%, 85%, 90%, 95%, 96%, 97%, 98%, 99%, or 100% identity to a wild-type nucleotide sequence encoding an HA antigen.

In some embodiments, the ORF comprises or is the nucleotide sequence set forth in any one of SEQ ID NOs: 87-90 and 102-105 or a sequence having at least 80%, 85%, 90%, 95%, 96%, 97%, 98%, 99%, or 100% identity thereto.

In some embodiments, the ORF comprises or is the nucleotide sequence set forth in any one of SEQ ID NOs: 93-96 or a sequence having at least 80%, 85%, 90%, 95%, 96%, 97%, 98%, 99%, or 100% identity thereto.

In some embodiments, the present disclosure provides a nucleic acid construct comprising, in the 5'-to-3' direction, a 5'UTR, an ORF, a 3'UTR, and a poly-A tail in sequence. In some embodiments, the 5'UTR comprises or is the nucleotide sequence set forth in any one of SEQ ID NOs: 1-5, 28-43, 76, and 137-196 and CTATAAT; the ORF comprises or is the nucleotide sequence set forth in any one of SEQ ID NOs: 13, 15, 20, 22, 24, 81, 97, 87-90, 93-96, and 102-105, the 3'UTR comprises or is the nucleotide sequence set forth in any one of SEQ ID NOs: 7-10, and the poly-A tail comprises or is the nucleotide sequence set forth in SEQ ID NO: 16 or 135.

Illustratively, the nucleic acid construct comprises the nucleotide sequences set forth in SEQ ID NOs: 18-19.

In the present disclosure, including any embodiment thereof, the nucleic acid construct is a nucleic acid molecule, e.g., a cDNA.

RNA Molecule

The present disclosure provides an RNA molecule comprising:

(a) an open reading frame (ORF), and
(b) a 5' untranslated region element (5'UTR).

In some embodiments, the ORF is a polynucleotide sequence encoding a target gene protein.

In some embodiments, the target gene is heterologous. In some other embodiments, the target gene is endogenous.

In some embodiments, the 5'UTR in the RNA molecule is located upstream of the open reading frame. In some embodiments, the 5'UTR in the RNA molecule is located at the 5' end of the open reading frame.

In some embodiments, the 5'UTR in the RNA molecule is selected from the group consisting of or is a 5'UTR derived from any one of the genes Rho GTPase activating protein (ARHGAP), heat shock 27kDa protein 1 (HSPB1), hemoglobin subunit beta (HBB), C-C motif chemokine ligand 13 (CCL13), and the like, or a derivative sequence thereof.

In some embodiments, the 5'UTR in the RNA molecule is derived from or is a 5'UTR sequence of an ARHGAP gene or a derivative sequence thereof. In some embodiments, the ARHGAP includes ARHGAP1, ARHGAP2, ARHGAP3, ARHGAP4, ARHGAP5, ARHGAP6, ARHGAP7 (DLC1), ARHGAP8, ARHGAP9, ARHGAP10, ARHGAP12, ARHGAP13 (SRGAP1), ARHGAP14 (SRGAP2), ARHGAP15, ARHGAP17 (RICH1), ARHGAP18, ARHGAP19, ARHGAP20, ARHGAP21, ARHGAP22, ARHGAP23, ARHGAP24, ARHGAP25, and ARHGAP26.

In some embodiments, the 5'UTR is derived from or is a 5'UTR sequence of an ARHGAP15 gene or a derivative sequence thereof. In some embodiments, the ARHGAP15 gene is derived from any species, such as human ARHGAP15, baboon ARHGAP15, or mouse ARHGAP15.

In some embodiments, the 5'UTR is derived from or is a 5'UTR sequence of the human ARHGAP15 gene or a derivative sequence thereof. In some embodiments, the 5'UTR of the ARHGAP15 gene comprises or is the nucleotide sequence set forth in SEQ ID NO: 45 or a nucleotide sequence having at least 80%, 85%, 90%, 95%, 96%, 97%, 98%, 99%, or 100% identity thereto.

In some embodiments, the 5'UTR in the RNA molecule of the present disclosure comprises at least one point mutation, the point mutation including the point mutation in any embodiment of the aforementioned nucleic acid construct. In some embodiments, the mutation is to mutate A into G, C, or U.

In some embodiments, the aforementioned 5'UTR sequence derived from the ARHGAP15 gene comprises the nucleotide sequence set forth in SEQ ID NO: 79, or, the 5'UTR comprises a nucleotide sequence having at least 80%, 85%, 90%, 95%, 96%, 97%, 98%, or 99% identity to SEQ ID NO: 79, wherein $N_2$ is selected from the group consisting of A, G, C, and U.

In some embodiments, the aforementioned 5'UTR sequence derived from the ARHGAP15 gene comprises a nucleotide sequence in which an AUG is mutated into a GUG. In some embodiments, the 5'UTR derived from the ARHGAP15 gene comprises or is the nucleotide sequence set forth in SEQ ID NO: 46 or a nucleotide sequence having at least 80%, 85%, 90%, 95%, 96%, 97%, 98%, 99%, or 100% identity thereto. In some embodiments, the aforementioned 5'UTR sequence derived from the ARHGAP15 gene comprises a nucleotide sequence in which an AUG is mutated into a CUG. In some embodiments, the 5'UTR derived from the ARHGAP15 gene comprises or is the nucleotide sequence set forth in SEQ ID NO: 77 or a nucleotide sequence having at least 80%, 85%, 90%, 95%, 96%, 97%, 98%, 99%, or 100% identity thereto. In some embodiments, the aforementioned 5'UTR sequence derived from the ARHGAP15 gene comprises a nucleotide sequence in which an AUG is mutated into a UUG. In some embodiments, the 5'UTR derived from the ARHGAP15 gene comprises or is the nucleotide sequence set forth in SEQ ID NO: 78 or a nucleotide sequence having at least 80%, 85%, 90%, 95%, 96%, 97%, 98%, 99%, or 100% identity thereto. In some embodiments, the 5'UTR in the RNA molecule of the present disclosure is a 5'UTR truncation. In some embodiments, the 5'UTR truncation still maintains active functions similar to those of a natural 5'UTR, e.g., still maintains a function of regulating expression of a protein from an encoding target gene of the ORF.

In some embodiments, a truncation mode for the 5'UTR truncation comprises: in the sequence direction from the 5' end to the 3' end, deleting a sequence of contiguous nucleotides from the 5' end; and/or, in the sequence direction from the 3' end to the 5' end, deleting a sequence of contiguous nucleotides from the 3' end. In some embodiments, a truncation mode for the 5'UTR truncation comprises, in the sequence direction from the 5' end to the 3' end, deleting a sequence of contiguous nucleotides from the 5' end, and keeping a nucleotide sequence of the 3' end. In some embodiments, a truncation mode for the 5'UTR truncation comprises, in the sequence direction from the 3' end to the 5' end, deleting a sequence of contiguous nucleotides from the 3' end, and keeping a nucleotide sequence of the 5' end. In some embodiments, a truncation mode for the 5'UTR truncation comprises, in the sequence direction from the 5' end to the 3' end, deleting a sequence of contiguous nucleotides from the 5' end; and the truncation mode for the 5'UTR truncation comprises, in the sequence direction from the 3' end to the 5' end, deleting a sequence of contiguous nucleotides from the 3' end. In some embodiments, the 5'UTR truncation also comprises the point mutation according to any of the preceding embodiments. In some embodiments, the 5'UTR truncation does not comprise the point mutation according to any of the preceding embodiments. In some embodiments, the 5'UTR truncation retains a sequence the length of which is at least 90%, 80%, 70%, 60%, 50%, 40%, 30%, 20%, 15%, 10%, 5%, or 1% of the length of a natural 5'UTR sequence.

In some embodiments, the aforementioned 5'UTR sequence derived from the ARHGAP15 gene comprises or is an ARHGAP15 5'UTR truncation. In some embodiments, a truncation mode for the truncation comprises, in the direction from the 5' end to the 3' end of the ARHGAP15 5'UTR, deleting a sequence of contiguous nucleotides from the 5' end; and/or, in the sequence direction from the 3' end to the 5' end, deleting a sequence of contiguous nucleotides from the 3' end.

In some embodiments, a truncation mode for the truncation comprises, in the direction from the 5' end to the 3' end of the ARHGAP15 5'UTR, deleting a sequence of contiguous nucleotides from the 5' end, and keeping a nucleotide sequence of the 3' end. In some embodiments, a truncation mode for the 5'UTR truncation comprises, in the direction from the 3' end to the 5' end of the ARHGAP15 5'UTR, deleting a sequence of contiguous nucleotides from the 3' end, and keeping a nucleotide sequence of the 5' end. In some embodiments, a truncation mode for the 5'UTR truncation is, in the direction from the 5' end to the 3' end of the ARHGAP15 5'UTR, to delete a sequence of contiguous nucleotides from the 5' end; and, in the sequence direction from the 3' end to the 5' end, to delete a sequence of contiguous nucleotides from the 3' end.

In some embodiments, the truncation retains a sequence the length of which is at least 90%, 80%, 70%, 60%, 50%, 40%, 30%, 20%, 15%, 10%, 5%, or 1% of the length of the natural ARHGAP15 5'UTR (SEQ ID NO: 45).

In some embodiments, the 5'UTR truncation comprises a sequence of at least 5 contiguous nucleotides from the nucleotide sequence set forth in SEQ ID NO: 46 or 79; in some embodiments, the 5'UTR truncation comprises a sequence of 5-62 contiguous nucleotides from the sequence set forth in SEQ ID NO: 46 or 79; in some embodiments, the 5'UTR truncation comprises a sequence of 7-62 contiguous nucleotides from the sequence set forth in SEQ ID NO: 46 or 79; in some embodiments, the 5'UTR truncation comprises a sequence of 7-59 contiguous nucleotides from the sequence set forth in SEQ ID NO: 46 or 79; illustratively, the 5'UTR truncation comprises a sequence of 5, 7, 8, 9, 10, 11, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 contiguous nucleotides from the sequence set forth in SEQ ID NO: 2.

In some embodiments, the nucleotide at the 5' end of the 5'UTR truncation (i.e., the starting nucleotide of the 5'UTR truncation) is the nucleotide at any one of positions 1-57, according to natural counting, of the sequence set forth in SEQ ID NO: 46 or 79.

In some embodiments, the nucleotide at the 5' end of the 5'UTR truncation is the nucleotide at any one of positions 1-13 or 17-29, according to natural counting, of the sequence set forth in SEQ ID NO: 46 or 79, for example, the nucleotide at any one of positions 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, and 57.

In some embodiments, the 5'UTR truncation is 59 bp, 55 bp, 51 bp, 47 bp, 43 bp, 39 bp, 35 bp, 31 bp, 27 bp, 23 bp, 19 bp, 15 bp, 11 bp, or 7 bp in length.

In some embodiments, the ARHGAP15 5'UTR truncation also comprises the point mutation according to any of the preceding embodiments. In some embodiments, the ARHGAP15 5'UTR truncation also comprises a nucleotide sequence in which an AUG is mutated into a GUG. In some embodiments, the aforementioned 5'UTR derived from the ARHGAP15 gene comprises or is the nucleotide sequences set forth in SEQ ID NOs: 63-65 or nucleotide sequences having at least 80%, 85%, 90%, 95%, 96%, 97%, 98%, 99%, or 100% identity thereto.

In some embodiments, the ARHGAP15 5'UTR truncation does not comprise the point mutation according to any of the preceding embodiments. In some embodiments, the aforementioned 5'UTR derived from the ARHGAP15 gene comprises or is the nucleotide sequences set forth in SEQ ID NOs: 66-75, CUAUAAU, or nucleotide sequences having at least 80%, 85%, 90%, 95%, 96%, 97%, 98%, 99%, or 100% identity thereto.

In some embodiments, the aforementioned 5'UTR derived from the ARHGAP15 gene comprises or is sequences corresponding to the sequences set forth in SEQ ID NOs: 41, 76, and 137-196 (replacing T in the sequences with U) or nucleotide sequences having at least 80%, 85%, 90%, 95%, 96%, 97%, 98%, 99%, or 100% identity thereto.

In some embodiments, the aforementioned 5'UTR derived from the HSPB1 gene comprises or is the nucleotide sequence set forth in SEQ ID NO: 47 or a nucleotide sequence having at least 80%, 85%, 90%, 95%, 96%, 97%, 98%, 99%, or 100% identity thereto.

In some embodiments, the aforementioned 5'UTR derived from the HBB gene comprises or is the nucleotide sequence set forth in SEQ ID NO: 48 or a nucleotide sequence having at least 80%, 85%, 90%, 95%, 96%, 97%, 98%, 99%, or 100% identity thereto.

In some embodiments, the aforementioned 5'UTR derived from the CCL13 gene comprises or is the nucleotide sequence set forth in SEQ ID NO: 49 or a nucleotide sequence having at least 80%, 85%, 90%, 95%, 96%, 97%, 98%, 99%, or 100% identity thereto.

In some embodiments, the 5'UTR in the RNA molecule described in the present disclosure comprises or is the nucleotide sequence set forth in any one of SEQ ID NOs: 45-49, 63-75, and 77-78 and CUAUAAU or a nucleotide sequence having at least 80%, 85%, 90%, 95%, 96%, 97%, 98%, 99%, or 100% identity thereto. Alternatively, the 5'UTR in the RNA molecule comprises sequences corresponding to the sequences set forth in SEQ ID NOs: 41, 76, and 137-196 (replacing T in the sequences with U) or nucleotide sequences having at least 80%, 85%, 90%, 95%, 96%, 97%, 98%, 99%, or 100% identity thereto.

In some specific embodiments, the 5'UTR described in the RNA molecule according to any one of the above comprises or is the nucleotide sequence set forth in any one of SEQ ID NOs: 45-49, 63-75, and 77-78 and CUAUAAU. Alternatively, the 5'UTR in the RNA molecule comprises sequences corresponding to the sequences set forth in SEQ ID NOs: 41, 76, and 137-196 (replacing T in the sequences with U).

In some embodiments, the RNA molecule of the present disclosure also further comprises: (c) a 3' untranslated region element (3'UTR).

In some embodiments, the open reading frame of the present disclosure is derived from a different gene than the 5'UTR and/or the 3'UTR. In some embodiments, the RNA molecule of the present disclosure comprises at least one open reading frame, at least one 5'UTR, or at least one 3'UTR. In some embodiments, the 5'UTR and 3'UTR in the RNA molecule of the present disclosure are from the same source or different sources, e.g., derived from the same gene or different genes. In some embodiments, the 5'UTR and 3'UTR in the RNA molecule of the present disclosure are derived from the same species or different species.

In some embodiments, the 3'UTR in the RNA molecule of the present disclosure is located downstream of the open reading frame. In some embodiments, the 3'UTR in the RNA molecule is located at the 3' end of the open reading frame. In some embodiments, the 3'UTR in the RNA molecule of the present disclosure is selected from the group consisting of or is a 3'UTR derived from any one of the genes hemoglobin subunit beta (HBB), ARHGAP15, coronin 1A (CORO1A), hemopexin (HPX), and the like, or a derivative sequence thereof.

In some embodiments, the 3'UTR is derived from or is a 3'UTR sequence of the HBB or ARHGAP15 gene or a derivative sequence thereof.

In some embodiments, the aforementioned 3'UTR derived from the HBB gene comprises or is the nucleotide sequence set forth in SEQ ID NO: 50 or a nucleotide sequence having at least 80%, 85%, 90%, 95%, 96%, 97%, 98%, 99%, or 100% identity thereto.

In some embodiments, the aforementioned 3'UTR derived from the ARHGAP15 gene comprises or is the nucleotide sequence set forth in SEQ ID NO: 51 or a nucleotide sequence having at least 80%, 85%, 90%, 95%, 96%, 97%, 98%, 99%, or 100% identity thereto.

In some embodiments, the aforementioned 3'UTR derived from the CORO1A gene comprises or is the nucleotide sequence set forth in SEQ ID NO: 52 or a nucleotide sequence having at least 80%, 85%, 90%, 95%, 96%, 97%, 98%, 99%, or 100% identity thereto.

In some embodiments, the aforementioned 3'UTR derived from the HPX gene comprises or is the nucleotide sequence set forth in SEQ ID NO: 53 or a nucleotide sequence having at least 80%, 85%, 90%, 95%, 96%, 97%, 98%, 99%, or 100% identity thereto.

In some embodiments, the 3'UTR in the RNA molecule of the present disclosure comprises or is the nucleotide sequence set forth in any one of SEQ ID NOs: 50-53 or a nucleotide sequence having at least 80%, 85%, 90%, 95%, 96%, 97%, 98%, 99%, or 100% identity thereto.

In some specific embodiments, the 3'UTR comprises or is the nucleotide sequence set forth in SEQ ID NO: 50 or 51.

In some embodiments, the RNA molecule comprises a 5'UTR and a 3'UTR, wherein:
the 5'UTR is selected from the group consisting of or is a 5'UTR derived from any one of the genes ARHGAP (e.g., ARHGAP15), HSPB1, HBB, CCL13, and the like, or a derivative sequence thereof, and the 3'UTR is selected from the group consisting of or is a 3'UTR derived from any one of the genes HBB, ARHGAP15, CORO1A, HPX, and the like, or a derivative sequence thereof.

In some embodiments, the RNA molecule comprises a 5'UTR and a 3'UTR, the 5'UTR and the 3'UTR being selected from the group consisting of any one of the following: the 5'UTR is derived from or is a 5'UTR of the ARHGAP15 gene or a derivative sequence thereof, and the 3'UTR is derived from or is a 3'UTR of the HBB gene or a derivative sequence thereof;

the 5'UTR is derived from or is a 5'UTR of the ARHGAP15 gene or a derivative sequence thereof, and the 3'UTR is derived from or is a 3'UTR of the ARHGAP15 gene or a derivative sequence thereof;
the 5'UTR is derived from or is a 5'UTR of the ARHGAP15 gene or a derivative sequence thereof, and the 3'UTR is derived from or is a 3'UTR of the CORO1A gene or a derivative sequence thereof;
the 5'UTR is derived from or is a 5'UTR of the ARHGAP15 gene or a derivative sequence thereof, and the 3'UTR is derived from or is a 3'UTR of the HPX gene or a derivative sequence thereof;
the 5'UTR is derived from or is a 5'UTR of the HBB gene or a derivative sequence thereof, and the 3'UTR is derived from or is a 3'UTR of the HBB gene or a derivative sequence thereof;

the 5'UTR is derived from or is a 5'UTR of the HBB gene or a derivative sequence thereof, and the 3'UTR is derived from or is a 3'UTR of the ARHGAP15 gene or a derivative sequence thereof;

the 5'UTR is derived from or is a 5'UTR of the HBB gene or a derivative sequence thereof, and the 3'UTR is derived from or is a 3'UTR of the CORO1A gene or a derivative sequence thereof;

the 5'UTR is derived from or is a 5'UTR of the HBB gene or a derivative sequence thereof, and the 3'UTR is derived from or is a 3'UTR of the HPX gene or a derivative sequence thereof;

the 5'UTR is derived from or is a 5'UTR of the HSPB1 gene or a derivative sequence thereof, and the 3'UTR is derived from or is a 3'UTR of the HBB gene or a derivative sequence thereof;

the 5'UTR is derived from or is a 5'UTR of the HSPB1 gene or a derivative sequence thereof, and the 3'UTR is derived from or is a 3'UTR of the ARHGAP15 gene or a derivative sequence thereof;

the 5'UTR is derived from or is a 5'UTR of the HSPB1 gene or a derivative sequence thereof, and the 3'UTR is derived from or is a 3'UTR of the CORO1A gene or a derivative sequence thereof;

the 5'UTR is derived from or is a 5'UTR of the HSPB1 gene or a derivative sequence thereof, and the 3'UTR is derived from or is a 3'UTR of the HPX gene or a derivative sequence thereof; or

the 5'UTR is derived from or is a 5'UTR of the CCL13 gene or a derivative sequence thereof, and the 3'UTR is derived from or is a 3'UTR of the HBB gene or a derivative sequence thereof;

the 5'UTR is derived from or is a 5'UTR of the CCL13 gene or a derivative sequence thereof, and the 3'UTR is derived from or is a 3'UTR of the ARHGAP15 gene or a derivative sequence thereof;

the 5'UTR is derived from or is a 5'UTR of the CCL13 gene or a derivative sequence thereof, and the 3'UTR is derived from or is a 3'UTR of the CORO1A gene or a derivative sequence thereof; or

the 5'UTR is derived from or is a 5'UTR of the CCL13 gene or a derivative sequence thereof, and the 3'UTR is derived from or is a 3'UTR of the HPX gene or a derivative sequence thereof.

In some embodiments, the RNA molecule of the present disclosure comprises a 5'UTR and a 3'UTR, the 5'UTR and the 3'UTR being selected from the group consisting of any one of the following:

the 5'UTR comprises or is the nucleotide sequence set forth in any one of SEQ ID NOs: 45-46, 63-75, and 77-78 and CUAUAAU or a nucleotide sequence having at least 80%, 85%, 90%, 95%, 96%, 97%, 98%, 99%, or 100% identity thereto, or the 5'UTR comprises sequences corresponding to the sequences set forth in SEQ ID NOs: 41, 76, and 137-196 (replacing T in the sequences with U) or nucleotide sequences having at least 80%, 85%, 90%, 95%, 96%, 97%, 98%, 99%, or 100% identity thereto; and the 3'UTR comprises or is the nucleotide sequence set forth in SEQ ID NO: 50 or a nucleotide sequence having at least 80%, 85%, 90%, 95%, 96%, 97%, 98%, 99%, or 100% identity thereto;

the 5'UTR comprises or is the nucleotide sequence set forth in any one of SEQ ID NOs: 45-46, 63-75, and 77-78 and CUAUAAU or a nucleotide sequence having at least 80%, 85%, 90%, 95%, 96%, 97%, 98%, 99%, or 100% identity thereto, or the 5'UTR comprises sequences corresponding to the sequences set forth in SEQ ID NOs: 41, 76, and 137-196 (replacing T in the sequences with U) or nucleotide sequences having at least 80%, 85%, 90%, 95%, 96%, 97%, 98%, 99%, or 100% identity thereto; and the 3'UTR comprises or is the nucleotide sequence set forth in SEQ ID NO: 51 or a nucleotide sequence having at least 80%, 85%, 90%, 95%, 96%, 97%, 98%, 99%, or 100% identity thereto;

the 5'UTR comprises or is the nucleotide sequence set forth in any one of SEQ ID NOs: 45-46, 63-75, and 77-78 and CUAUAAU or a nucleotide sequence having at least 80%, 85%, 90%, 95%, 96%, 97%, 98%, 99%, or 100% identity thereto, or the 5'UTR comprises sequences corresponding to the sequences set forth in SEQ ID NOs: 41, 76, and 137-196 (replacing T in the sequences with U) or nucleotide sequences having at least 80%, 85%, 90%, 95%, 96%, 97%, 98%, 99%, or 100% identity thereto; and the 3'UTR comprises or is the nucleotide sequence set forth in SEQ ID NO: 52 or a nucleotide sequence having at least 80%, 85%, 90%, 95%, 96%, 97%, 98%, 99%, or 100% identity thereto;

the 5'UTR comprises or is the nucleotide sequence set forth in any one of SEQ ID NOs: 45-46, 63-75, and 77-78 and CUAUAAU or a nucleotide sequence having at least 80%, 85%, 90%, 95%, 96%, 97%, 98%, 99%, or 100% identity thereto, or the 5'UTR comprises sequences corresponding to the sequences set forth in SEQ ID NOs: 41, 76, and 137-196 (replacing T in the sequences with U) or nucleotide sequences having at least 80%, 85%, 90%, 95%, 96%, 97%, 98%, 99%, or 100% identity thereto; and the 3'UTR comprises or is the nucleotide sequence set forth in SEQ ID NO: 53 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 47 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 50 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 47 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 51 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 47 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 52 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 47 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 53 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 48 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 50 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 48 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 51 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 48 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 52 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 48 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 53 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 49 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 50 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 49 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 51 or a nucleotide sequence having at least 80%, 85%, 90%, 95%, 96%, 97%, 98%, 99%, or 100% identity thereto;

the 5'UTR comprises or is the nucleotide sequence set forth in SEQ ID NO: 49 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 52 or a nucleotide sequence having at least 80%, 85%, 90%, 95%, 96%, 97%, 98%, 99%, or 100% identity thereto; and

the 5'UTR comprises or is the nucleotide sequence set forth in SEQ ID NO: 49 or a nucleotide sequence having at least 80%, 85%, 90%, 95%, 96%, 97%, 98%, 99%, or 100% identity thereto, and the 3'UTR comprises or is the nucleotide sequence set forth in SEQ ID NO: 53 or a nucleotide sequence having at least 80%, 85%, 90%, 95%, 96%, 97%, 98%, 99%, or 100% identity thereto.

In some embodiments, the RNA molecule comprises a 5'UTR and a 3'UTR, wherein: the 5'UTR comprises or is the nucleotide sequence set forth in any one of SEQ ID NOs: 45-46, 63-75, and 77-78 and CUAUAAU, or the 5'UTR comprises sequences corresponding to the sequences set forth in SEQ ID NOs: 41, 76, and 137-196 (replacing T in the sequences with U); and the 3'UTR is derived from or is a 3'UTR of any one of the genes HBB, ARHGAP15, CORO1A, and HPX gene or a derivative sequence thereof.

In some specific embodiments, the RNA molecule comprises a 5'UTR and a 3'UTR, wherein: the 5'UTR comprises or is the nucleotide sequence set forth in any one of SEQ ID NOs: 45-46, 63-75, and 77-78 and CUAUAAU, or the 5'UTR comprises sequences corresponding to the sequences set forth in SEQ ID NOs: 41, 76, and 137-196 (replacing T in the sequences with U); and the 3'UTR comprises or is the nucleotide sequence set forth in any one of SEQ ID NOs: 50-53.

In some embodiments, the RNA molecule of the present disclosure also further comprises: (d) a polyadenylic acid (poly-A) tail.

In some embodiments, the poly-A tail in the RNA molecule is located downstream of the 3'UTR. In some embodiments, the poly-A tail in the RNA molecule is located at the 3' end of the 3'UTR. In some embodiments, the poly-A tail is at the 3' end of the RNA molecule. In some embodiments, the poly-A tail is at least about 50, 100, 150, 200, 300, 400, or 500 nucleotides in length.

In some embodiments, the poly-A tail includes, but is not limited to, HGH polyA, SV40polyA, BGH polyA, rbGlob polyA, or SV40late polyA.

In some specific embodiments, the poly-A tail comprises or is the nucleotide sequence set forth in SEQ ID NO: 56 or 135.

In some embodiments, the RNA molecule of the present disclosure also further comprises: (e) a 5' cap structure (5'Cap).

In some embodiments, the 5' cap structure in the RNA molecule is located upstream of the 5'UTR. In some embodiments, the 5' cap structure in the RNA molecule is located at the 5' end of the 5'UTR. In some embodiments, the 5' cap structure is a cap structure known to those skilled in the art, such as CapO (methylation of the first nucleobase, e.g., m7GpppN), Cap1 (additional methylation of the ribose of a nucleotide adjacent to m7GpppN, e.g., m7G(5')ppp(5') (2'OMeA)pG), Cap2 (additional methylation of the ribose of the 3rd nucleotide downstream of m7GpppN), Cap3 (additional methylation of the ribose of the 3rd nucleotide downstream of m7GpppN), Cap4 (additional methylation of the ribose of the 4th nucleotide downstream of m7GpppN), ARCA (anti-reverse cap analog), modified ARCA (e.g., phosphorothioate-modified ARCA), inosine, N1-methyl-guanosine, 2'-fluoro-guanosine, 7-deaza-guanosine, 8-oxo-guanosine, 2-amino-guanosine, LNA-guanosine, and 2-azido-guanosine.

In some embodiments, the 5'-cap structure (e.g., CapO or Cap1) is formed using chemical RNA synthesis or RNA *in vitro* transcription (co-transcriptional capping).

In some embodiments, the 5'-cap structure (e.g., Cap0 or Cap1) is formed via enzymatic capping using a capping enzyme (e.g., vaccinia virus capping enzyme and/or cap-dependent 2'-O methyltransferase). In some embodiments, the 5' cap structure (Cap0 or Cap1) is added using an immobilized capping enzyme. The capping method and means in WO2016/193226 are incorporated herein by reference in their entirety.

In some embodiments, the 5' cap structure includes, but is not limited to, ARCA, 3'OMe-m7G(5')ppp(5')G, m7G(5')ppp(5')(2'OMeA)pU, m7Gppp(A2'O-MOE)pG, m7G(5')ppp(5')(2'OMeA)pG, m7G(5')ppp(5')(2'OMeG)pG, m7(3'OMeG)(5')ppp(5') (2'OMeG)pG, or m7(3'OMeG)(5')ppp(5')(2'OMeA)pG.

In some specific embodiments, the 5' cap structure is m7G(5')ppp(5')(2'OMeA)pG. Other 5' cap structures or cap structure analogs may also be used.

In some embodiments, in the 5'-to-3' direction, the RNA molecule according to any one of the above comprises any one of i)-v):

    i) a 5'UTR, and an open reading frame (ORF);
    ii) an open reading frame (ORF), and a 3'UTR;
    iii) a 5'UTR, an open reading frame (ORF), and a 3'UTR; and
    iv) a 5'UTR, an open reading frame (ORF), a 3'UTR, and a poly-A tail;
    v) a 5' cap structure (5'Cap), a 5'UTR, an open reading frame (ORF), a 3'UTR, and a poly-A tail;

wherein the ORF is derived from a different gene than the 5'UTR and/or the 3'UTR.

In some embodiments, the 5'UTR in i) and iii) to v) is selected from the group consisting of a 5'UTR derived from any one of the genes ARHGAP, HSPB1, HBB, CCL13, and the like, or a derivative sequence thereof (e.g., a 5'UTR of ARHGAP15 or a derivative sequence thereof).

In some embodiments, the 3'UTR in ii) to v) is selected from the group consisting of a 3'UTR derived from any one of the genes HBB, ARHGAP15, CORO1A, HPX, and the like, or a derivative sequence thereof (e.g., a 3'UTR of HBB or ARHGAP15 or a derivative sequence thereof).

In some embodiments, the poly-A tail in iv) to v) comprises, for example, the nucleotide sequence set forth in SEQ ID NO: 56.

In some embodiments, the 5' cap structure in v) includes, but is not limited to, Cap0, Cap1 (e.g., m7G(5')ppp(5') (2'OMeA)pG), Cap2, Cap3, Cap4, and ARCA.

In some embodiments, the 5'UTR in i) comprises or is the nucleotide sequence set forth in any one of SEQ ID NOs: 45-49, 63-75, and 77-78 and CUAUAAU; or the 5'UTR comprises sequences corresponding to the sequences set forth in SEQ ID NOs: 41, 76, and 137-196 (replacing T in the sequences with U).

In some embodiments, the 3'UTR in ii) comprises or is the nucleotide sequence set forth in any one of SEQ ID NOs: 50-53.

In some embodiments, the 5'UTR in iii) comprises or is the nucleotide sequence set forth in any one of SEQ ID NOs: 45-49, 63-75, and 77-78 and CUAUAAU, or the 5'UTR comprises sequences corresponding to the sequences set forth in SEQ ID NOs: 41, 76, and 137-196 (replacing T in the sequences with U); and the 3'UTR comprises or is the nucleotide sequence set forth in any one of SEQ ID NOs: 50-53.

In some embodiments, the 5'UTR in iv) comprises or is the nucleotide sequence set forth in any one of SEQ ID NOs: 45-49, 63-75, and 77-78 and CUAUAAU, or the 5'UTR comprises sequences corresponding to the sequences set forth in SEQ ID NOs: 41, 76, and 137-196 (replacing T in the sequences with U); the 3'UTR comprises or is the nucleotide sequence set forth in any one of SEQ ID NOs: 50-53, and the poly-A tail comprises or is the nucleotide sequence set forth in SEQ ID NO: 56.

In some embodiments, the 5'UTR in v) comprises or is the nucleotide sequence set forth in any one of SEQ ID NOs: 45-49, 63-75, and 77-78 and CUAUAAU, or the 5'UTR comprises sequences corresponding to the sequences set forth in SEQ ID NOs: 41, 76, and 137-196 (replacing T in the sequences with U); the 3'UTR comprises or is the nucleotide sequence set forth in any one of SEQ ID NOs: 50-53, the poly-A tail comprises or is the nucleotide sequence set forth in SEQ ID NO: 56, and the 5' cap structure is m7G(5')ppp(5')(2'OMeA)pG.

In the present disclosure, in the RNA molecule according to any one of the above, the ORF comprises a nucleotide sequence encoding at least one polypeptide or protein. In some embodiments, the nucleotide sequence may be a codon-optimized nucleotide sequence.

In some embodiments, the polypeptide or protein encoded by the ORF is a fluorescent protein or a luciferase.

In some embodiments, the polypeptide or protein encoded by the ORF is a viral antigen. Illustratively, the viral antigen includes, but is not limited to, antigens of influenza viruses, coronaviruses, respiratory syncytial virus, human immunodeficiency viruses, herpes simplex virus, rabies virus, or EB virus, among others.

In some embodiments, the viral antigen is a coronavirus antigen. In some embodiments, the coronavirus is a human-infecting coronavirus, such as SARS-CoV-2 (COVID-19), SARS-CoV, HCoV-229E, HCoV-OC43, HCoV-NL63, HCoV-HKU1, or MERS-CoV. In some embodiments, the coronavirus is SARS-COV-2. In some embodiments, the coronavirus antigen is a structural protein. In some embodiments, the structural protein is selected from the group consisting of a spike protein (S protein), an envelope protein (E protein), a membrane protein (M protein), and a nucleocapsid protein (N protein). In some embodiments, the structural protein is a spike protein. In some embodiments, the spike protein is a SARS-COV-2 spike protein. In some embodiments, the SARS-COV-2 spike protein is selected from the group consisting of a spike protein of any one of the viral strains SARS-COV-2 (e.g., wild-type SARS-COV-2), SARS-COV-2 Alpha(B.1.1.7), SARS-COV-2 Beta(B.1.351), SARS-COV-2 Gamma(P.1), SARS-COV-2 Kappa(B.1.617.1), SARS-COV-2 Delta(B.1.617.2), SARS-COV-2 Omicron(B.1.1.529), SARS-COV-2 Omicron(BA.4), and the like. In some embodiments, the SARS-COV-2 spike protein comprises or is the amino acid sequence set forth in any one of SEQ ID NOs: 12, 14, 21, 23, 25, 80, and 136 or a sequence having at least 80%, 85%, 90%, 95%, 96%, 97%, 98%, 99%, or 100% amino acid identity thereto.

In some embodiments, the ORF comprises a codon-optimized nucleotide sequence comprising a sequence having at least 80%, 85%, 90%, 95%, 96%, 97%, 98%, 99%, or 100% identity to a wild-type nucleotide sequence encoding a SARS-COV-2 antigen (e.g., wild-type SARS-COV-2, SEQ ID NO: 82).

In some embodiments, the ORF encoding the polypeptide or protein comprises or is the nucleotide sequence set forth in any one of SEQ ID NOs: 54-55, 59-61, 82, and 130 or a nucleotide sequence having at least 80%, 85%, 90%, 95%, 96%, 97%, 98%, 99%, or 100% identity thereto.

In some embodiments, the ORF encodes an influenza virus antigen. In some embodiments, the influenza virus is selected from the group consisting of influenza A virus and influenza B virus; illustratively, the influenza virus is influenza A virus H1N1, influenza A virus H3N2, influenza A virus H3N8, influenza A virus H2N2, influenza A virus H5N1, influenza A virus H9N2, influenza A virus H7N7, influenza B virus/Victoria (e.g., influenza B/Washington/02/2019), influenza B virus/Yamagata (e.g., influenza B/Phuket/3073/2013), or the like. In some embodiments, the influenza virus antigen is a structural protein of an influenza virus, e.g., hemagglutinin (HA), neuraminidase (NA), M2 ion channel, matrix protein M1, or nucleoprotein (NP). In some specific embodiments, the influenza virus antigen is an HA protein of an influenza virus, e.g., an HA protein of influenza A virus H1N1, influenza A virus H3N2, influenza B virus/Victoria (e.g., influenza B/Washington/02/2019), or influenza B virus/Yamagata (e.g., influenza B/Phuket/3073/2013). In some specific embodiments, the influenza virus antigen is an NA protein of an influenza virus, e.g., an NA protein of influenza A virus H1N1, influenza A virus H3N2, influenza B virus Victoria (e.g., influenza B/Washington/02/2019), or influenza B virus Yamagata (e.g., influenza B/Phuket/3073/2013). In some embodiments, the HA protein comprises or is the amino acid sequence set forth in any one of SEQ ID NOs: 83-86 and 98-101 or a sequence having at least 80%, 85%, 90%, 95%, 96%, 97%, 98%, 99%, or 100% amino acid identity thereto.

In some embodiments, the ORF comprises a codon-optimized nucleotide sequence comprising a sequence having at least 80%, 85%, 90%, 95%, 96%, 97%, 98%, 99%, or 100% identity to a wild-type nucleotide sequence encoding an HA antigen.

In some embodiments, the ORF encoding the polypeptide or protein comprises or is the nucleotide sequence set forth in any one of SEQ ID NOs: 126-129 and 131-134 or a sequence having at least 80%, 85%, 90%, 95%, 96%, 97%, 98%, 99%, or 100% identity thereto.

In some embodiments, the present disclosure provides an RNA molecule comprising, in the 5'-to-3' direction, a 5'UTR, an ORF, a 3'UTR, and a poly-A tail in sequence. In some embodiments, the 5'UTR comprises or is the nucleotide sequence set forth in any one of SEQ ID NOs: 45-49, 45-49, 63-75, and 77-78 and CUAUAAU, or the 5'UTR comprises sequences corresponding to the sequences set forth in SEQ ID NOs: 41, 76, and 137-196 (replacing T in the sequences with U); the ORF comprises or is the nucleotide sequences set forth in SEQ ID NOs: 54-55, 59-61, 82, and 126-134, the 3'UTR comprises or is the nucleotide sequence set forth in any one of SEQ ID NOs: 50-53, and the poly-A tail comprises or is the nucleotide sequence set forth in SEQ ID NO: 56 or 135. In some specific embodiments, the RNA molecule comprises the nucleotide sequences set forth in SEQ ID NOs: 57-58 and 106-125.

In the present disclosure, including any embodiment thereof, the RNA molecule may be an mRNA.

The present disclosure uses a 5'UTR sequence derived from human Rho GTPase activating protein (ARHGAP) for recombinant expression of a target gene (e.g., SARS-CoV-2 viral protein) for the first time, and screens, designs, and successfully verifies that a 5'UTR derived from ARHGAP15 and an engineered and modified 5'UTR thereof can initiate efficient expression of different target genes. Rho GTPase activating protein 15 (ARHGAP15), a Rac1-specific GTPase activating protein (GAP), belongs to the ARHGAP family, and it is a major negative regulator of Rho family GTPase activity. The present disclosure provides more 5'UTR and 3'UTR options and combinations with excellent expression efficiency. Additionally, The present disclosure provides mRNA vaccines with the potential to efficiently prevent infection with SARS-CoV-2 and influenza viruses, particularly against various variants thereof.

Polynucleotide

The present disclosure also provides an isolated polynucleotide encoding one or more polypeptides or proteins, wherein the polypeptides or proteins are coronavirus antigens. In some embodiments, the coronavirus is SARS-COV-2. In some embodiments, the coronavirus antigen is a structural protein. In some embodiments, the structural protein is selected from the group consisting of a spike protein (S protein), an envelope protein (E protein), a membrane protein (M protein), and a nucleocapsid protein (N protein). In some embodiments, the structural protein is a spike protein. In some embodiments, the spike protein is a SARS-COV-2 spike protein. In some embodiments, the SARS-COV-2 spike protein is selected from the group consisting of a spike protein of any one of the viral strains SARS-COV-2 (e.g., wild-type SARS-COV-2), SARS-COV-2 Alpha(B.1.1.7), SARS-COV-2 Beta(B.1.351), SARS-COV-2 Gamma(P.1), SARS-COV-2 Kappa(B.1.617.1), SARS-COV-2 Delta(B.1.617.2), SARS-COV-2 Omicron(B.1.1.529), SARS-COV-2 Omicron(BA.4), and the like. In some embodiments, the SARS-COV-2 spike protein comprises or is the amino acid sequence set forth in any one of SEQ ID NOs: 12, 14, 21, 23, 25, 80, and 136 or a sequence having at least 80%, 85%, 90%, 95%, 96%, 97%, 98%, 99%, or 100% amino acid identity thereto.

In some embodiments, the ORF encodes an influenza virus antigen. In some embodiments, the influenza virus is selected from the group consisting of influenza A virus and influenza B virus; illustratively, the influenza virus is influenza A virus H1N1, influenza A virus H3N2, influenza A virus H3N8, influenza A virus H2N2, influenza A virus H5N1, influenza A virus H9N2, influenza A virus H7N7, influenza B virus/Victoria (e.g., influenza B/Washington/02/2019), influenza B virus/Yamagata (e.g., influenza B/Phuket/3073/2013), or the like. In some embodiments, the influenza virus antigen is a structural protein of an influenza virus, e.g., hemagglutinin (HA), neuraminidase (NA), M2 ion channel, matrix protein M1, or nucleoprotein (NP). In some specific embodiments, the influenza virus antigen is an HA protein of an influenza virus, e.g., an HA protein of influenza A virus H1N1, influenza A virus H3N2, influenza B virus/Victoria (e.g., influenza B/Washington/02/2019), or influenza B virus/Yamagata (e.g., influenza B/Phuket/3073/2013). In some specific embodiments, the influenza virus antigen is an NA protein of an influenza virus, e.g., an NA protein of influenza A virus H1N1, influenza A virus H3N2, influenza B virus Victoria (e.g., influenza B/Washington/02/2019), or influenza B virus Yamagata (e.g., influenza B/Phuket/3073/2013). In some embodiments, the influenza virus HA protein comprises or is the amino acid sequence set forth in any one of SEQ ID NOs: 83-86 and 98-101 or a sequence having at least 80%, 85%, 90%, 95%, 96%, 97%, 98%, 99%, or 100% amino acid identity thereto.

In some embodiments, the ORF comprises a codon-optimized nucleotide sequence comprising a sequence having at least 80%, 85%, 90%, 95%, 96%, 97%, 98%, 99%, or 100% identity to a wild-type nucleotide sequence encoding a SARS-COV-2 antigen (e.g., wild-type SARS-COV-2 mRNA, SEQ ID NO: 82).

In some embodiments, the isolated polynucleotide comprises or is the nucleotide sequences set forth in SEQ ID NOs: 54-55, 59-61, 82, and 126-134 or nucleotide sequences having at least 80%, 85%, 90%, 95%, 96%, 97%, 98%, 99%, or 100% identity thereto.

In some embodiments, the polynucleotide is an RNA, e.g., an mRNA. In some embodiments, the polynucleotide is an ORF region of an mRNA.

In some embodiments, the present disclosure provides use of any one of the aforementioned nucleic acid constructs or RNA molecules in any one of the following: (1) preparing a vaccine; (2) encoding a viral antigen in a subject or in vitro; (3) preparing a medicament encoding a viral antigen in a subject or in vitro; and (4) preparing a medicament.

In some embodiments, the nucleic acid construct or RNA molecule in the present disclosure encodes a target protein, wherein the open reading frame (ORF) has enhanced expression of the target protein.

Modification

To further improve the stability of protein expression of the RNA or polynucleotide molecule of the present disclosure, the RNA or polynucleotide molecule may further comprise one or more modifications (including chemical modifications), such as backbone modifications, sugar modifications, base modifications, and/or lipid modifications. In some embodiments, the RNA or polynucleotide molecule is uniformly modified to a particular modification (e.g., complete modification throughout the sequence). For example, the RNA may be uniformly modified with pseudouridines such that each U in the sequence is a pseudouridine.

Backbone modifications in connection with the present disclosure refer to chemical modifications of the phosphate of the backbone of the nucleotides that the RNA or polynucleotide molecule of the present disclosure comprises. In some embodiments, the backbone modifications include, but are not limited to, complete substitution of unmodified phosphate moieties in the backbone with modified phosphates; for example, backbone phosphate groups may be modified by substituting one or more oxygen atoms with a different substituent. In some embodiments, the modified phosphates include, but are not limited to, phosphorothioates, phosphoroselenates, boranophosphates, boranophosphates, hydrogen phosphonates, phosphoramidates, alkyl or aryl phosphonates, and phosphotriesters.

Sugar modifications in connection with the present disclosure refer to chemical modifications of the sugar of the nucleotides that the RNA or polynucleotide molecule of the present disclosure comprises. In some embodiments, the sugar modifications include, but are not limited to, modifications or replacements of the 2' hydroxy (OH) of the RNA molecule with many different "oxy" or "deoxy" substituents. In some embodiments, the "oxy" modifications include, but are not limited to, substitution modifications with alkoxy, aryloxy, polyethylene glycol (PEG), and the like. In some embodiments, "deoxy" modifications include, but are not limited to, hydrogen and amino (e.g., NH2, alkylamino, dialkylamino, heterocyclyl, arylamino, diarylamino, heteroarylamino, diheteroarylamino, or amino acid) modifications.

Base modifications in connection with the present disclosure refer to chemical modifications of the base moiety of the nucleotides that the RNA or polynucleotide molecule of the present disclosure comprises. In some embodiments, the base modifications include modifications to adenine, guanine, cytosine, and uracil in nucleotides. For example, the nucleosides and nucleotides described herein may be chemically modified on the surface of the major groove. In some embodiments, chemical modifications to the major groove may include amino, thiol, alkyl, or halogen groups. In some embodiments, the base modifications include, but are not limited to, modifications with pseudouridine, 1-methyl-pseudouridine, 5-azacytidine, 5-methylcytosine-5'-triphosphate, or 2-methoxyadenine. In some embodiments, the base modifications are pseudouridine modifications. In some specific embodiments, the RNA may be uniformly modified with pseudouridines such that each U in the sequence is a pseudouridine.

Lipid modifications in connection with the present disclosure mean that the RNA or polynucleotide molecule of the present disclosure comprises lipid modifications. In some embodiments, the lipid modifications include, but are not limited to, covalent attachment of the RNA or polynucleotide molecule of the present disclosure to at least one adapter, and covalent attachment of the corresponding adapter to at least one lipid. In some embodiments, the lipid modifications include, but are not limited to, covalent attachment of the RNA or polynucleotide molecule of the present disclosure to at least one lipid (no adapter).

UTRs

UTRs (5'UTR and/or 3'UTR) may be provided as a flanking region to the nucleic acid construct, RNA, or polynucleotide molecule of the present disclosure. UTRs may be homologous or heterologous with coding regions in the nucleic acid construct, RNA, or polynucleotide molecule of the present disclosure. The flanking region may comprise one or more 5'UTRs and/or 3'UTRs, and the UTRs may be identical or different sequences. Any portion of the flanking region may be codon-optimized. Before and/or after codon optimization, any portion of the flanking region may independently comprise one or more different structural or chemical modifications.

To alter one or more properties of the nucleic acid construct, RNA, or polynucleotide of the present disclosure, UTRs heterologous with the ORF of the present disclosure are introduced or engineering-synthesized into the nucleic acid construct, RNA, or polynucleotide of the present disclosure. The recombinant nucleic acid construct, RNA, or polynucleotide is then administered to a cell, tissue, or organism, and the results, such as protein levels, localization, and/or half-life, are measured to assess the beneficial effects of the heterologous UTRs on the RNA or polynucleotide of the present disclosure. In some embodiments, the UTRs include a wild UTR or a variant thereof, the UTR variant comprising the addition or removal of one or more nucleotides, including A, T, C, or G, to or from an end. In some embodiments, the UTR variant also comprises codon optimization or modification performed in any way. In some embodiments, the UTR variant also comprises the derivative sequence according to any embodiment of the present disclosure; for example, on the basis of a natural UTR sequence, an ATG is point-mutated into a GTG in order to inhibit the ATG within the UTR from initiating translation.

Vector

The present disclosure also provides a vector comprising the nucleic acid construct, RNA, or polynucleotide according to any one of the foregoing. The nucleic acid construct, RNA, or polynucleotide may be present in the vector and/or may be part of the vector, the vector being, for example, a plasmid, cosmid, YAC, or viral vector. The vector may be an expression vector, i.e., a vector capable of providing for the expression of a polypeptide encoded by the nucleic acid construct, RNA, or polynucleotide. The expression vector generally comprises at least one of the nucleic acids of the present disclosure, which is operatively linked to one or more suitable expression regulatory elements (e.g., promoters and terminators). The selection of the elements and their sequences for expression in a particular host is within the knowledge of those skilled in the art. Regulatory elements and other elements useful or necessary for expression of the encoded polypeptide of the present disclosure include, for example, promoters, terminators, selection labels, leader sequences, and reporter genes.

The nucleic acid construct of the present disclosure may be prepared or obtained by known means (e.g., by automatic DNA synthesis and/or recombinant DNA technology) based on nucleotide sequence information of the present disclosure, and/or may be isolated from a suitable natural source.

In some embodiments, the vector of the present disclosure also comprises a promoter; for example, the promoter is at the 5' end of the 5'UTR of the nucleic acid construct, and for example, the promoter is a T7 promoter, a T7 lac promoter, a Tac promoter, a Lac promoter, or a Trp promoter.

In some specific embodiments, the T7 promoter comprises or is the nucleotide sequence set forth in SEQ ID NO: 17.

Host Cell

The present disclosure also provides a host cell comprising the nucleic acid construct, RNA, or polynucleotide according to any one of the foregoing. In some embodiments, the cell is capable of expressing one or more polypeptides encoded by the nucleic acid construct, RNA, or polynucleotide of the present disclosure. In some embodiments, the host cell is a bacterial cell, a fungal cell, or a mammalian cell.

Bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, e.g., cells of the species *Trichoderma, Neurospora,* and *Aspergillus*; or cells of the species *Saccharomyces* (e.g., *Saccharomyces cerevisiae), Schizosaccharomyces* (e.g., *Schizosaccharomyces pombe), Pichia* (*Pichia pastoris* and *Pichia methanolica*), and *HansenuLa.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

Production or Preparation Method

The present disclosure provides a method for preparing the nucleic acid construct, RNA, or polynucleotide of the present disclosure, and a method for preparing the encoded polypeptide thereof.

Methods and reagents for preparation and production of the nucleic acid construct, RNA, or polynucleotide and the encoded polypeptide thereof, e.g., specific suitable vectors, transformation or transfection methods, selection labels, methods for inducing protein expression, and culture conditions, are known in the art. Similarly, protein isolation and purification techniques suitable for use in the method for manufacturing the encoded polypeptide of the present disclosure are well known to those skilled in the art.

In some embodiments, the method for preparing the nucleic acid construct, RNA, or polynucleotide comprises culturing the aforementioned host cell and collecting the produced nucleic acid construct, RNA, or polynucleotide from the culture. The nucleic acid construct, RNA, or polynucleotide of the present disclosure and the encoded polypeptide thereof may also be obtained by other production methods known in the art, such as chemical synthesis, including solid-phase, or liquid-phase synthesis.

In some embodiments, a method for preparing an RNA molecule comprises: preparing a nucleic acid construct or a vector, and then using the nucleic acid construct or vector to perform reverse transcription to obtain the RNA molecule. In some specific embodiments, the method also comprises adding a 5'Cap to the 5' end of the RNA molecule.

Vaccine

The present disclosure also provides a vaccine comprising the nucleic acid construct according to any one of the foregoing, the RNA according to any one of the foregoing, and/or the polynucleotide according to any one of the foregoing. In some embodiments, the nucleic acid construct, RNA, or polynucleotide encodes one or more antigens of one or more viral strains. The vaccine provided by the present disclosure may be a monovalent vaccine, a multivalent vaccine, or a combination vaccine.

Monovalent Vaccine

The present disclosure provides a monovalent vaccine comprising encoding an antigen of an organism. In some embodiments, the monovalent vaccine comprises encoding one antigen of one viral strain.

Multivalent/Combination Vaccine

In some embodiments, the vaccine may comprise a nucleic acid construct, RNA, or polynucleotide molecule or multiple nucleic acid constructs, RNAs, or polynucleotide molecules encoding two or more antigens of the same species or different species. In some embodiments, the vaccine comprises an RNA or multiple RNAs encoding two or more antigens of the same viral strain or different viral strains. In some embodiments, the RNA may encode 1, 2, 3, 4, 5, 6, 7, 8, 9, 10, 11, 12, or more viral antigens.

In some embodiments, in the monovalent vaccine and the multivalent/combination vaccine described above, the antigen is a coronavirus antigen, the coronavirus being, for example, SARS-CoV-2 (COVID-19), SARS-CoV, HCoV-229E, HCoV-OC43, HCoV-NL63, HCoV-HKU1, or MERS-CoV. In some embodiments, the coronavirus antigen is a structural protein, e.g., selected from the group consisting of a spike protein (S protein), an envelope protein (E protein), a membrane protein (M protein), and a nucleocapsid protein (N protein). In some embodiments, the structural protein is a spike protein, e.g., a SARS-COV-2 spike protein. In some embodiments, the SARS-COV-2 spike protein is selected from the group consisting of a spike protein of any one of the viral strains SARS-COV-2 (e.g., wild-type SARS-COV-2 mRNA), SARS-COV-2 Alpha(B.1.1.7), SARS-COV-2 Beta(B.1.351), SARS-COV-2 Gamma(P.1), SARS-COV-2 Kappa(B.1.617.1), SARS-COV-2 Delta(B.1.617.2), SARS-COV-2 Omicron(B.1.1.529), SARS-COV-2 Omicron(BA.4), and the like. In some embodiments, the SARS-COV-2 spike protein comprises or is the amino acid sequence set forth in any one of SEQ ID NOs: 12, 14, 21, 23, 25, 80, and 136 or a sequence having at least 80%, 85%, 90%, 95%, 96%, 97%, 98%, 99%, or 100% amino acid identity thereto. In some embodiments, a DNA sequence encoding the SARS-COV-2 spike protein comprises or is the nucleotide sequence set forth in any one of SEQ ID NOs: 13, 15, 20, 22, 24, 81, and 97 or a nucleotide sequence having at least 80%, 85%, 90%, 95%, 96%, 97%, 98%, 99%, or 100% identity thereto. In some embodiments, an RNA sequence encoding the SARS-COV-2 spike protein comprises or is the nucleotide sequences set forth in SEQ ID NOs: 54-55, 59-61, 82, and 130 or nucleotide sequences having at least 80%, 85%, 90%, 95%, 96%, 97%, 98%, 99%, or 100% identity thereto. In some embodiments, the ORF encodes an influenza virus antigen. In some embodiments, the influenza virus is selected from the group consisting of influenza A virus and influenza B virus; illustratively, the influenza virus is influenza A virus H1N1, influenza A virus H3N2, influenza A virus H3N8, influenza A virus H2N2, influenza A virus H5N1, influenza A virus H9N2, influenza A virus H7N7, influenza B virus/Victoria (e.g., influenza B/Washington/02/2019), influenza B virus/Yamagata (e.g., influenza B/Phuket/3073/2013), or the like. In some embodiments, the influenza virus antigen is a structural protein of an influenza virus, e.g., hemagglutinin (HA), neuraminidase (NA), M2 ion channel, matrix protein M1, or nucleoprotein (NP). In some specific embodiments, the influenza virus antigen is an HA protein of an influenza virus, e.g., an HA protein of influenza A virus H1N1, influenza A virus H3N2, influenza B virus/Victoria (e.g., influenza B/Washington/02/2019), or influenza B virus/Yamagata (e.g., influenza B/Phuket/3073/2013). In some embodiments, the HA protein comprises or is the amino acid sequence set forth in any one of SEQ ID NOs: 83-86 and 98-101 or a sequence having at least 80%, 85%, 90%, 95%, 96%, 97%, 98%, 99%, or 100% amino acid identity thereto. In some embodiments, a DNA sequence encoding the HA protein comprises or is the nucleotide sequence set forth in any one of SEQ ID NOs: 87-90, 93-97, and 102-105 or a nucleotide sequence having at least 80%, 85%, 90%, 95%, 96%, 97%, 98%, 99%, or 100% identity thereto. In some embodiments, an RNA sequence encoding the HA protein comprises or is the nucleotide sequence set forth in any one of SEQ ID NOs: 126-129 and 131-134 or a nucleotide sequence having at least 80%, 85%, 90%, 95%, 96%, 97%, 98%, 99%, or 100% identity thereto.

In some embodiments, two or more different RNAs (e.g., mRNAs) may be formulated in the same lipid nanoparticle. In other embodiments, two or more different RNAs may be formulated separately in separate lipid nanoparticles, and then the lipid nanoparticles may be combined and administered as a single vaccine composition (e.g., comprising multiple RNAs encoding multiple antigens), or may be administered separately.

The present disclosure also provides a multivalent/combination vaccine comprising an RNA encoding one or more coronaviruses or antigens of one or more different organisms. That is, the vaccine of the present disclosure may be a multivalent/combination vaccine that targets one or more antigens of the same viral strain/species, or one or more antigens of different viral strains/species.

Delivery System

The present disclosure also provides a delivery system comprising the nucleic acid construct according to any one of the foregoing, or the RNA molecule according to any one of the foregoing, wherein the delivery vehicle is a cationic lipid delivery particle. In some embodiments, the particle is a nanoparticle. In some embodiments, the delivery vehicle is a lipid nanoparticle. The RNA molecule in the present disclosure may be delivered into cells and/or *in vivo* using any type of lipid nanoparticle in the art; illustratively, lipid nanoparticles include, but are not limited to, lipid particles disclosed in WO2017075531, WO2018081480A1, WO2017049245A2, WO2017099823A1, WO2022245888AI, WO2022150717A1, CN101291653A, CN102119217A, WO2011000107A1, and CN107028886A, which are incorporated herein by reference in their entirety.

Pharmaceutical Composition

The present disclosure also provides a pharmaceutical composition comprising the nucleic acid construct according to any one of the foregoing, the polynucleotide according to any one of the foregoing, the vaccine according to any one of the foregoing, the vector according to any one of the foregoing, and/or the delivery vehicle according to any one of the foregoing, and a pharmaceutically acceptable carrier, diluent, or excipient; specifically, the pharmaceutical composition is a solid formulation, an injection, a topical formulation, a spray, a liquid formulation, or a composite formulation.

Product or Kit

The present disclosure provides a product or kit comprising the nucleic acid construct according to any one of the foregoing, the polynucleotide according to any one of the foregoing, the vaccine according to any one of the foregoing, the vector according to any one of the foregoing, the delivery vehicle according to any one of the foregoing, and/or the pharmaceutical composition according to any one of the foregoing. The kit may be used to provide relevant detection or diagnostic use.

Method for Treating and/or Preventing Disease and Pharmaceutical Use

The present disclosure also provides use of administering to a subject in need thereof a therapeutically and/or prophylactically effective amount of the nucleic acid construct according to any one of the foregoing, the polynucleotide according to any one of the foregoing, the vaccine according to any one of the foregoing, the vector according to any one of the foregoing, the delivery vehicle according to any one of the foregoing, the pharmaceutical composition according to any one of the foregoing, and/or the product or kit according to any one of the foregoing in the preparation of a medicament for treating and/or preventing a disease. In some embodiments, the disease comprises a viral infectious disease or a respiratory disease associated with viral infection. In some embodiments, the virus is a coronavirus. In some embodiments, the coronavirus is a human-infecting coronavirus, such as SARS-CoV-2 (COVID-19), SARS-CoV, HCoV-229E, HCoV-OC43, HCoV-NL63, HCoV-HKU1, or MERS-CoV. In some embodiments, the coronavirus is SARS-CoV-2. In some embodiments, the virus is an influenza virus. In some embodiments, the coronavirus is an influenza virus that infects humans, such as influenza A virus or influenza B virus. Illustratively, the influenza virus is influenza A virus H1N1, influenza A virus H3N2, influenza B virus/Victoria (e.g., influenza B/Washington/02/2019), influenza B virus/Yamagata (e.g., influenza B/Phuket/3073/2013), or the like. In some embodiments, the respiratory disease associated with viral infection (e.g., respiratory disease associated with SARS-CoV-2 infection) includes simple infection, such as fever, cough, and sore throat, headache, rhinitis, and the like, pneumonia, acute respiratory infection, severe acute respiratory infection (SARI), hypoxemic respiratory failure and acute respiratory distress syndrome, sepsis and septic shock, severe acute respiratory syndrome (SARS), and the like.

The present disclosure also provides a method for treating and/or preventing a disease, the method comprising administering to a subject in need thereof a therapeutically and/or prophylactically effective amount of the nucleic acid construct according to any one of the foregoing, the polynucleotide according to any one of the foregoing, the vaccine according to any one of the foregoing, the vector according to any one of the foregoing, the delivery vehicle according to any one of the foregoing, the pharmaceutical composition according to any one of the foregoing, and/or the product or kit according to any one of the foregoing. In some embodiments, the disease comprises a viral infectious disease or a respiratory disease associated with viral infection. In some embodiments, the virus is a coronavirus. In some embodiments, the coronavirus is a human-infecting coronavirus, such as SARS-CoV-2 (COVID-19), SARS-CoV, HCoV-229E, HCoV-OC43, HCoV-NL63, HCoV-HKU1, or MERS-CoV. In some embodiments, the coronavirus is SARS-CoV-2. In some embodiments, the virus is an influenza virus. In some embodiments, the coronavirus is an influenza virus that infects humans, such as influenza A virus or influenza B virus. Illustratively, the influenza virus is influenza A virus H1N1, influenza A virus H3N2, influenza B virus/Victoria (e.g., influenza B/Washington/02/2019), influenza B virus/Yamagata (e.g., influenza B/Phuket/3073/2013), or the like. In some embodiments, the respiratory disease associated with viral infection (e.g., respiratory disease associated with SARS-CoV-2 infection) includes simple infection, such as fever, cough, and sore throat, headache, rhinitis, and the like, pneumonia, acute respiratory infection, severe acute respiratory infection (SARI), hypoxemic respiratory failure and acute respiratory distress syndrome, sepsis and septic shock, severe acute respiratory syndrome (SARS), and the like.

The present disclosure also provides a method comprising administering to a subject in need thereof an effective amount of the nucleic acid construct according to any one of the foregoing, the polynucleotide according to any one of the foregoing, the vaccine according to any one of the foregoing, the vector according to any one of the foregoing, the delivery vehicle according to any one of the foregoing, the pharmaceutical composition according to any one of the foregoing, and/or the product or kit according to any one of the foregoing, which is capable of inducing in the subject a neutralizing antibody reaction and/or a T cell immune response, e.g., a neutralizing antibody reaction against a viral antigen, e.g., a CD4+ and/or CD8+ T cell immune response against a viral antigen. The antigen-antibody titer of the subject increases after vaccination relative to the antigen-antibody titer of a subject vaccinated with a prophylactically effective dose of a conventional vaccine against the antigen. In some embodiments, the virus is a coronavirus. In some embodiments, the coronavirus is a human-infecting coronavirus, such as SARS-CoV-2 (COVID-19), SARS-CoV, HCoV-229E, HCoV-OC43, HCoV-NL63, HCoV-HKU1, or MERS-CoV. In some embodiments, the coronavirus is SARS-CoV-2. In some embodiments, the virus is an influenza virus. In some embodiments, the coronavirus is an influenza virus that infects humans, such as influenza A virus or influenza B virus. Illustratively, the influenza virus is influenza A virus H1N1, influenza A virus H3N2, influenza B virus/Victoria (e.g., influenza B/Washington/02/2019), influenza B virus/Yamagata (e.g., influenza B/Phuket/3073/2013), or the like.

In some embodiments, the subject is immunized. In some embodiments, the subject has a lung disease. In some embodiments, the subject is 5 years old or younger, or 65 years old or older.

In some embodiments, the method comprises administering to the subject at least one, two, three, four, and more doses of the nucleic acid construct according to any one of the foregoing, the polynucleotide according to any one of the foregoing, the vaccine according to any one of the foregoing, the vector according to any one of the foregoing, the delivery vehicle according to any one of the foregoing, the pharmaceutical composition according to any one of the foregoing, or the product or kit according to any one of the foregoing.

In some embodiments, a detectable level of a viral antigen (such as a coronavirus antigen) is produced in the serum of the subject 1-72 hours after administration of the nucleic acid construct according to any one of the foregoing, the polynucleotide according to any one of the foregoing, the vaccine according to any one of the foregoing, the vector according to any one of the foregoing, the delivery vehicle according to any one of the foregoing, the pharmaceutical composition according to any one of the foregoing, or the product or kit according to any one of the foregoing. In some embodiments, a neutralizing antibody titer of at least 100 NU/mL, 200 NU/mL, 300 NU/mL, 400 NU/mL, 500 NU/mL, 600 NU/mL, 700 NU/mL, 800 NU/mL, 900 NU/mL, or 1000 NU/mL is produced in the serum of the subject 1-72 hours after administration.

In some embodiments, the antibody titer produced in the subject is increased by at least 1 log relative to a control. For example, the antibody titer produced in the subject may be increased by at least 2, 3, 4, 5, 6, 7, 8, 9, or 10 log relative to the control.

In some embodiments, the antibody titer produced in the subject is increased at least 2-fold relative to the control. For example, the antibody titer produced in the subject is increased at least 3, 4, 5, 6, 7, 8, 9, or 10-fold relative to the control. In some examples, a geometric mean is the nth power of the product of n numbers and is generally used to describe proportional growth. In some embodiments, the geometric mean is used to characterize the antibody titer produced in the subject. In some embodiments, the control may be a subject who has not been vaccinated, or a subject who has been administered a live attenuated virus vaccine, an inactivated virus vaccine, or a protein subunit vaccine.

Vaccine Efficacy

In some embodiments, an antigen-specific immune response is characterized by measuring an anti-(corona) virus antigen antibody titer produced in a subject who has undergone the administration of the preceding embodiments. An antibody titer is a measurement of the amount of antibodies within a subject, e.g., antibodies specific to a particular antigen or epitope of an antigen. Antibody titers are typically expressed as the reciprocal of the maximum dilution that provides a positive result. Enzyme-linked immunosorbent assay (ELISA) is a common assay for determining antibody titers.

In some embodiments, the antibody titer is used to assess whether a subject has been infected or to determine whether the immunization is required. In some embodiments, the antibody titer is used to determine the intensity of the autoimmune response, to determine whether the boost immunization is required, to determine whether a previous vaccine dose is effective, and to identify any recent or previous infections. According to the present disclosure, the antibody titer can be used to determine the intensity of an immune response in a subject induced by an immune composition (e.g., an RNA vaccine).

In some embodiments, the antibody titer against a virus (coronavirus) antigen produced in the subject is increased by at least 1 log relative to the control. For example, the antibody titer produced in the subject may be increased by at least 2, 3, 4, 5, 6, 7, 8, 9, or 10 log relative to the control.

In some embodiments, the antibody titer produced in the subject is increased at least 2-fold relative to the control. For example, the antibody titer produced in the subject is increased at least 3, 4, 5, 6, 7, 8, 9, or 10-fold relative to the control.

In some embodiments, the antigen-specific immune response is measured by the ratio of the neutralizing antibody titer in serum to the geometric mean titer (GMT) of the coronavirus, referred to as the geometric mean ratio (GMR). The geometric mean titer (GMT) is the mean antibody titer in a group of subjects calculated by multiplying all values and taking the n-th root, where n is the number of subjects with available data.

In some embodiments, the control may be a subject who has not been vaccinated, or a subject who has been administered a live attenuated virus vaccine, an inactivated virus vaccine, or a protein subunit vaccine.

In some embodiments, the vaccine efficacy can be assessed using standard analytical methods (see, e.g., Weinberg et al., J. Infect. Dis., Jun 1, 2010; 201 (11):1607-10). For example, the vaccine efficacy can be measured in a double-blind, randomized, controlled clinical trial. The vaccine efficacy may be expressed as a proportional reduction in the attack rates (ARs) of unvaccinated study cohort (ARU) and vaccinated study cohort (ARV) and can be calculated from the relative risk (RR) of a disease in the vaccinated group using the following equation.

$$\text{Efficacy} = (\text{ARU} - \text{ARV})/\text{ARU} \times 100;$$

and

$$\text{Efficacy} = (1 - \text{RR}) \times 100$$

In some other embodiments, the vaccine effectiveness can be assessed using standard analytical methods (see, e.g., Weinberg et al., J. Infect. Dis., Jun 1, 2010; 201 (11):1607-10). The vaccine effectiveness is an assessment of how a vaccine (which may have been demonstrated to have high vaccine efficacy) reduces a disease in a population. This measure allows to assess the net balance between advantageous and disadvantageous effects of vaccination programs but not the vaccine in a natural condition rather than in a controlled clinical trial. The vaccine effectiveness is proportional to the vaccine efficacy, but is also affected by the degree of immunity of the target population, as well as other non-vaccine related factors such as hospitalization, outpatient visits, or costs. For example, a retrospective case-control analysis can be conducted, i.e., comparing the vaccination rates in a group of infected subjects with an appropriate control group. The vaccine effectiveness can be expressed as a ratio difference, i.e., the probability of infection after vaccination (odds ratio, OR).

$$\text{Effectiveness} = (1 - \text{OR}) \times 100.$$

**BRIEF DESCRIPTION OF THE DRAWINGS**

FIG. 1 illustrates a schematic representation of a plasmid template containing a T7 promoter, a 5'UTR, a 3'UTR, a polyA tail, and an exogenous gene CDS sequence.

FIG. 2 illustrates the chip electrophoresis photo of the mRNA synthesized by *in vitro* transcription using a linearized plasmid as the template. L denotes the RNA ladder, and 1 denotes the purified mRNA sample.

FIG. 3 illustrates the difference in expression level of SAS-Cov-2 B.1.351 spike protein caused by different combinations of 5'UTRs and 3'UTRs as measured by ELISA. The 5'UTRs and 3'UTRs are abbreviated as shown in Table 3.

FIG. 4 illustrates the difference in expression level of SAS-Cov-2 B.1.1.7 spike protein caused by different combinations of 5'UTRs and 3'UTRs as measured by ELISA. The 5'UTRs and 3'UTRs are abbreviated as shown in Table 3.

FIG. 5 illustrates a schematic representation of the point mutation from 5'UTR I to 5'UTR I'.

FIG. 6 illustrates the difference in expression level of SAS-Cov-2 B.1.617.2 spike protein caused by the 5'UTR I point mutation as measured by Western Blot. 5'UTR I was used for samples 1 and 3, and 5'UTR I' was used for samples 2 and 4. The mRNA synthesis templates for samples 1 and 2 were from plasmid minipreparation, and the mRNA synthesis templates for samples 3 and 4 were from plasmid maxipreparation;

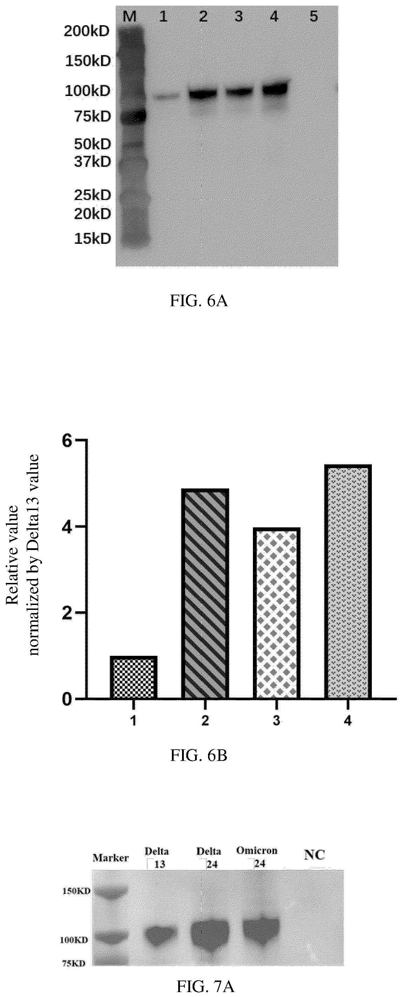

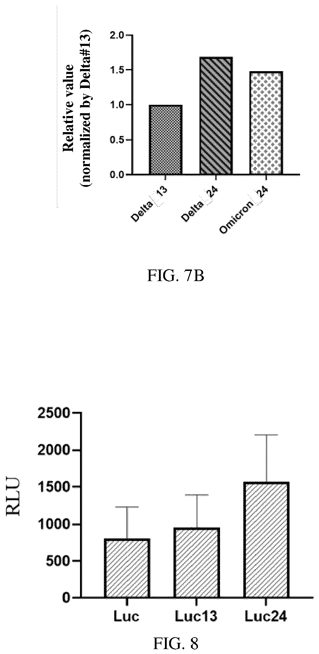

sample 5 was an untransfected cell supernatant control; m denotes the molecular weight markers. FIG. 6A illustrates a Western Blot assay, and FIG. 6B illustrates a sample ratio graph after quantification of the Western Blot assay results. FIG. 7 illustrates the effects of 5'UTR I' on expression levels of different heterologous genes as measured by Western Blot. FIG. 7A illustrates a Western Blot assay, and FIG. 7B illustrates a sample relative value graph after quantification of the Western Blot assay results.

FIG. 8 illustrates the luciferase expression mediated by 5'UTR I'-3'UTR B and 5'UTR I-3'UTR B compared to the BNT162b2 UTR combination.

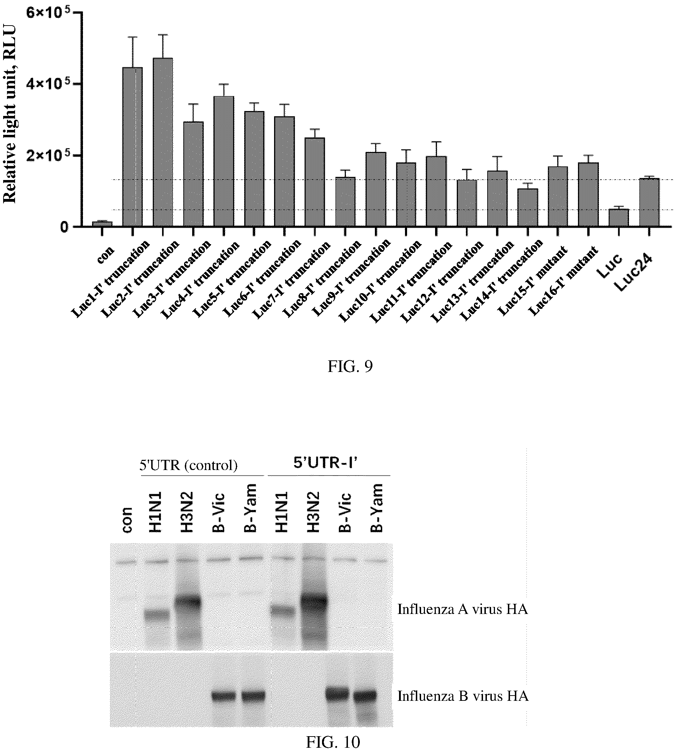

FIG. 9 illustrates the luciferase expression regulated by 5'UTR I' truncations and 5'UTR I' mutants.

FIG. 10 illustrates the difference in HA mRNA expression level regulated by 5'UTR I' versus a control 5'UTR.

## DETAILED DESCRIPTION

### Terminology

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. The "2019-nCoV" is known as severe acute respiratory syndrome coronavirus 2 (SARS-CoV-2).

The "disease caused by 2019-nCoV" is known as COVID-19.

The "nucleic acid" or "nucleotide" includes RNA, DNA, and cDNA molecules. It will be appreciated that due to the degeneracy of the genetic codes, a large number of nucleotide sequences encoding a given protein may be generated. The term nucleic acid can be used interchangeably with the term "polynucleotide". The "oligonucleotide" refers to a short nucleic acid molecule. The "primer" is an oligonucleotide, whether naturally occurring in a purified restriction enzymatic digest or produced synthetically, that is capable of acting as a start point of synthesis when placed in a condition inducing the synthesis of a primer extension product complementary to a nucleic acid strand (i.e., in the presence of nucleotides and an inducing agent such as DNA polymerase, and at a suitable temperature and pH). To maximize the amplification efficiency, the primer is preferably single-stranded, but may optionally be double-stranded. If double-stranded, the primer is first treated to separate the strands before use in preparing an extension product. Preferably, the primer is a deoxyribonucleotide. The primer must be long enough to initiate the synthesis of the extension product in the presence of an inducer. The exact length of the primer will depend on many factors, including temperature, source of the primer, and the method used.

The "vector" or "expression vector" is meant a replicon, such as a plasmid, bacmid, phage, virus, virion, or cosmid, to which another DNA segment, i.e., an "insert", may be attached, so as to achieve the replication of the attached segment in a cell. The vector may be a nucleic acid construct designed for the delivery to a host cell or for the transfer among different host cells. As used herein, the vector may be viral or non-viral in its original and/or final form, such as the PUC57 DNA vector used herein. The term "vector" encompasses any genetic element capable of replicating when combined with appropriate control elements and capable of transferring a gene sequence to a cell. In some embodiments, the vector may be an expression vector or a recombinant vector.

The "promoter" refers to any nucleic acid sequence that regulates the expression of another nucleic acid sequence by driving the transcription of the nucleic acid sequence, which may be a heterologous target gene encoding a protein or RNA. The promoter may be constitutive, inducible, repressible, or tissue-specific, or any combination thereof. The promoter is a control region of a nucleic acid sequence where the initiation and rate of the transcription of the remainder of the nucleic acid sequence is controlled.

The "gene" refers to a segment of DNA involved in the production of a polypeptide chain, which may or may not include preceding and succeeding coding regions, e.g., 5' untranslated region (5'UTR) or "leader" sequence and 3'UTR or "non-transcribed tail" sequence, as well as intervening sequences (introns) between the coding segments (exons).

The "recombinant" refers to that a polynucleotide is a product of various combinations of cloning, restriction, or ligation procedures, as well as other procedures resulting in constructs different and/or distinct from naturally occurring polynucleotides.

The "introduction", in the context of inserting a nucleic acid sequence into a cell, refers to "transfection", "transformation", or "transduction" and includes reference to the incorporation of a nucleic acid sequence into a eukaryotic or prokaryotic cell, where the nucleic acid sequence may be incorporated into the genome of the cell (e.g., chromosome, plasmid, plastidial, or mitochondrial DNA), transformed to autonomous replication, or transiently expressed (e.g., transfected mRNA).

The "nucleic acid construct" refers to a nucleic acid molecule, e.g., a DNA fragment, either single- or double-stranded, that is modified or synthesized to comprise a nucleic acid segment in a manner that does not naturally occur; the nucleic acid molecule comprises one or more control sequences or regulatory elements. In the context of the present disclosure, the nucleic acid construct contains a recombinant nucleotide sequence consisting essentially of optionally one, two, three, or more isolated nucleotide sequences including 5'UTR, open reading frame (ORF), and 3'UTR. In embodiments involving constructs comprising two or more sequences, the sequences are operably linked to each other in the construct.

The "derivative sequence" refers to a nucleotide sequence that is highly homologous (e.g., having at least 80%, 85%, 88%, 90%, 93%, 95%, 96%, 97%, 98%, 99%, or 100% identity) to the UTR sequence of the present disclosure and still retains the same or similar functional activity as the UTR sequence of the present disclosure. In some embodiments, the derivative sequence is a nucleotide sequence having one or more substituted, deleted, or added nucleotides based on a natural UTR sequence. In some embodiments, the derivative sequence is a nucleotide sequence that is truncated on the basis of the natural UTR sequence. In some embodiments, the derivative sequence has a point mutation of an ATG into a GTG, CTG, or TTG on the basis of a natural UTR sequence, for inhibiting the ATG within the UTR from initiating translation.

The "operably linked" is defined herein as a structure where a control sequence, i.e., a promoter sequence and/or a 5'UTR sequence, are suitably positioned relative to the coding DNA sequence such that the control sequence directs the transcription of the coding sequence and the translation of the mRNA into a polypeptide sequence encoded by the coding DNA.

The "open reading frame", abbreviated as "ORF", refers to a segment or region encoding an mRNA molecule of a polypeptide. The ORF comprises contiguous, non-overlapping, in-frame codons, starting with a start codon and ending with a stop codon, which are translated by the ribosome.

The "endogenous" refers to any substance derived from or produced in an organism, cell, tissue, or system.

The "exogenous" refers to any substance introduced or produced from outside an organism, cell, tissue, or system.

The "sequence identity" refers to sequence identity between genes or proteins at the nucleotide or amino acid level, respectively. The "sequence identity" is a measure of identity between proteins at the amino acid level and between nucleic acids at the nucleotide level. The protein sequence identity can be determined by comparing the amino acid sequences at given positions in the aligned sequences. Similarly, the nucleic acid sequence identity can be determined by comparing the nucleotide sequences at given positions in the aligned sequences. Methods for aligning sequences for comparison are well known in the art, including GAP, BESTFIT, BLAST, FASTA, and TFASTA. The BLAST algorithm calculates the percent sequence identity and performs a statistical analysis of the similarity between two sequences. Software for performing BLAST analysis is publicly available through the National Center for Biotechnology Information (NCBI) website.

The "homologous" or "homology" is defined as the percentage of nucleotide residues in a target chromosome identical to those in a corresponding sequence after aligning the sequences and introducing gaps, if necessary, to achieve the maximum percentage sequence identity. Alignment to determine percentage nucleotide sequence homology can be accomplished in a variety of ways within the skill in the art, for example, using publicly available computer software such as BLAST, BLAST-2, ALIGN, ClustalW2, or Megalign (DNASTAR). In some specific embodiments, the present disclosure calculates percentage sequence homology on the basis of BLAST. Those skilled in the art can determine suitable parameters for aligning the sequences, including any algorithms necessary to achieve the maximum alignment over the full length of the sequences being compared. In some embodiments, the sequences are considered "homologous" when, for example, a nucleic acid sequence (e.g., a DNA sequence) of a homology arm has at least 70%, at least 75%, at least 80%, at least 85%, at least 90%, at least 91%, at least 92%, at least 93%, at least 94%, at least 95%, at least 96%, at least 97%, at least 98%, at least 99%, or higher identity to a corresponding natural or unedited nucleic acid sequence (e.g., a genomic sequence) of a host cell.

The "substitution" is defined as a change in amino acid or nucleotide sequence as follows: one or more amino acids or nucleotides are substituted with a different amino acid or nucleotide, as compared to the amino acid sequence or nucleotide sequence of a reference polypeptide. If the substitution is conservative, the substituent amino acid has similar structural or chemical properties (e.g., charge, polarity, hydrophobicity, etc.) as the substituted amino acid. In some embodiments, a polypeptide variant may have a "non-conservative" change, where the amino acids before and after the substitution differ in structural and/or chemical properties.

The "deletion" is defined as a change in amino acid or nucleotide sequence as follows: one or more amino acid or nucleotide residues are deleted, as compared to the amino acid sequence or nucleotide sequence of a reference polypeptide. In the case of a polypeptide or polynucleotide sequence, considering the length of the polypeptide or polynucleotide sequence being modified, the deletion may involve deletions of 2, 5, 10, up to 20, up to 30, or up to 50 or more amino acid or nucleotide residues.

The "insertion" or "addition" refers to a change in amino acid or nucleotide sequence as follows: one or more amino acids or nucleotides are added, as compared to the amino acid sequence or nucleotide sequence of a reference polypeptide. The "insertion" generally refers to the addition of one or more amino acid residues within the amino acid sequence of a polypeptide (or nucleotide residues within a polynucleotide), while the "addition" may be an insertion or the addition of amino acid residues at the N- or C-terminus of a polypeptide (or the addition of nucleotide residues at the 5' or 3' terminus of a polynucleotide). In the case of a polypeptide or polynucleotide sequence, the addition or insertion may involve up to 10, up to 20, up to 30, or up to 50 or more amino acids (or nucleotide residues).

The "codon optimization" refers to the replacement of a codon in the target sequence that is rarely seen in highly expressed genes of a given species with a codon commonly seen in highly expressed genes of the species, with the codons before and after the replacement encoding the same amino acid. Various species may exhibit specific usage biases for certain codons for a particular amino acid. The codon usage bias (the difference in codon usage between organisms) is often correlated with the translation efficiency of messenger RNA (mRNA), which in turn is believed to particularly depend on the characteristics of the codons translated and the availability of specific transfer RNA (tRNA) molecules. The predominance of a selected tRNA in a cell is typically a reflection of the codons most frequently used in peptide synthesis. Thus, on the basis of codon optimization, genes can be modified for the optimal gene expression in a given organism. Thus, the option of optimal codons depends on the codon usage bias of the host genome.

The "antigen antibody" refers to a serum antibody that specifically binds to an antigen. The "cell" or "host cell" includes any cell type susceptible to transformation, transfection, transduction, and the like with the nucleic acid construct or vector of the present disclosure. As a non-limiting example, the host cell may be an isolated primary cell, a pluripotent stem cell, a CD34+ cell, an induced pluripotent stem cell, or any one of a number of immortalized cell lines (e.g., a HepG2 cell). Alternatively, the host cell may be an *in situ* or *in vivo* cell in a tissue, organ, or organism.

The "treatment" refers to administering a therapeutic agent, such as a composition comprising any one of the nucleic acid constructs of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate any particular symptom of the disease (also known as a "therapeutically effective amount") may vary depending on a variety of factors, such as the disease state, age, and weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom.

The "effective amount" or "pharmaceutically effective amount" comprises an amount sufficient to ameliorate or prevent a symptom or condition of a medical disorder. The effective amount also refers to an amount sufficient to allow or facilitate diagnosis. The effective amount for a particular patient or veterinary subject may vary depending on the factors such as the disorder to be treated, the general health of the patient, the method and route and dose of administration, and the severity of adverse effects. The effective amount may be the maximum dose or administration regimen to avoid significant adverse effects or toxic effects. In some embodiments, the "effective amount" is an effective amount of RNA that can produce an antigen-specific immune response.

The "prophylactically effective dose" refers to an effective dose to prevent viral infection at a clinically acceptable level. In some embodiments, the effective dose is a dose listed in the vaccine package insert. The conventional vaccine, as used herein, refers to a vaccine other than the mRNA vaccine of the present disclosure. For example, the conventional vaccine includes, but is not limited to, a live microbial vaccine, an inactivated microbial vaccine, a subunit vaccine, a protein antigen vaccine, a DNA vaccine, a virus-like particle (VLP) vaccine, and the like. In exemplary embodiments, the conventional vaccine is one that has been approved by regulatory authorities and/or listed by a national drug administration, such as the Chinese National Medical Products Administration.

The term "pharmaceutically acceptable" refers to those therapeutic agents, materials, compositions, and/or dosage forms that are, within the scope of sound medical judgment, suitable for use in contact with the tissues of patients without excessive toxicity, irritation, allergic reaction, or other problems or complications, and are commensurate with a proper benefit/risk ratio and effective for the intended use.

The "monovalent vaccine" refers to a vaccine preparation for immunization made from an antigen of one serotype from a pathogenic organism. For example, a vaccine containing only one antigen of one SARS-CoV-2 strain of the present disclosure is a monovalent vaccine.

The "multivalent vaccine" refers to a vaccine preparation for immunization made from antigens of more than one serotype from the same pathogenic organism. For example, a vaccine containing an antigen from one SARS-CoV-2 variant mixed with one or more antigens from other SARS-CoV-2 variants of different subtypes is a multivalent vaccine.

The "combined vaccine" refers to a vaccine preparation for immunization made from antigens of more than one serotype from different pathogenic organisms. For example, a vaccine containing an antigen of the SARS-CoV-2 strain of the present disclosure mixed with several pathogenic microorganisms that can prevent and/or treat other viral infections is a combined vaccine. The combined vaccine is generally prepared from monovalent vaccines prepared by the same preparation method, and then is mixed in the same injection for immunization; alternatively, antigens of several pathogenic microorganisms are mixed and then prepared into a vaccine product. In some specific embodiments, antigens of pathogenic microorganisms are first mixed and then prepared into a vaccine preparation. Combined and multivalent vaccines are the future development trends due to their advantages of: (1) cost-efficiency in vaccination; (2) cost-efficiency in packaging in doses, transportation, and assembly; (3) reduced damage to vaccinees, particularly infants; (4) increased vaccination coverage; (5) efficiency in vaccination; (6) compactness for vaccine storage, etc. The combined or multivalent vaccine can save direct and indirect costs including multiple visits, missed vaccination doses, and the like. For example, combined, multivalent vaccines commonly used at present include vaccines against *Streptococcus pneumoniae, Neisseria meningitidis,* poliovirus, rotavirus, influenza, HPV, and the like.

The "immune response" of the vaccine of the present disclosure refers to the development of a humoral and/or cellular immune response in a subject to protein(s) of a virus (e.g., coronavirus) present in the vaccine. For the purposes of the present disclosure, the "humoral" immune response refers to an immune response mediated by antibody molecules, including, for example, secretory IgA or IgG molecules, while the "cellular" immune response is an immune response mediated by T lymphocytes (e.g., CD4+ helper and/or CD8+ T cells (e.g., CTLs) and/or other leukocytes). An important aspect of the cellular immunity involves the antigen-specific reactions of cytolytic T lymphocytes (CTLs). The CTLs are specific for peptide antigens that are presented in combination with proteins encoded by the major histocompatibility complex (MHC) and expressed on the cell surface, and help induce and promote the destruction of intracellular microorganisms or lyse cells infected with these microorganisms. Another aspect of the cellular immunity involves antigen-specific responses by helper T cells, which help stimulate function and concentrate the activities of non-specific effector cells on cells with peptidic antigens associated with MHC molecules. The cellular immune responses also result in cytokines, chemokines, and other like molecules produced by activated T cells and/or other leukocytes.

## Examples

The present disclosure is further described below with reference to examples; however, these examples are not intended to limit the scope of the present disclosure. Experimental procedures without conditions specified in the examples of the present disclosure were generally conducted according to conventional conditions such as cell culture manuals or molecular cloning manuals, or according to conditions recommended by the manufacturers of the raw material or the goods. Reagents without specific origins indicated are commercially available conventional reagents.

The following general experimental procedures 1) to 4) are given by means of examples:

1) mRNA production

To generate *in vitro* transcribed mRNA, the plasmid was linearized downstream of the polyA tail using BspQ 1 (Vazyme, DD4302-PC, China) and purified by a PCR purification kit (QIAGEN, 28106, Germany). *In vitro* transcription was performed using the T7 *in vitro* transcription kit (Invitrogen, AM1333, USA) with the purified linearized plasmid as template. The mRNA synthesized by *in vitro* transcription was purified by MEGAclear™ Kit (Invitrogen, AM1908, USA) to give an mRNA with a relatively higher purity for subsequent *in vitro* cell transfection and other experiments.

2) Chip electrophoresis

To examine the integrity and purity of the mRNA obtained by *in vitro* transcription, the mRNA was analyzed using an on-chip electrophoresis system (Agilent 2100, USA) and the RNA 6000 Nano kit (Agilent, 5067-1511, USA). The procedures are as follows: the mRNA was denatured (incubated for 2 min at 70 °C and quickly transferred on ice), and the treated samples were sequentially transferred on corresponding wells of the chip, loaded for analysis until the analysis results were generated.

3) Cell culture

Materials: FBS (Gibco), DMEM medium (Gibco), Opti-MEM (Gibco), PBS (Gibco), trypsin-EDTA (Gibco), antibiotics (pen/strep, Gibco), and Lipofectamine™ 2000 (Invitrogen, 52887).
Human embryonic kidney cells (293T, ATCC) were cultured in DMEM medium supplemented with 10% of FBS and 1% of the antibiotics in a humidified environment at 5% $CO_2$.

4) *In vitro* transfection

Cells were seeded in 12-well cell culture plates 8 h before the transfection at $8 \times 10^5$ cells/well. The cells were transfected using a commercially available transfection reagent Lipofectamine™ 2000 in a ratio of 1 μg of mRNA to 2.5 μL of Lipofectamine™ 2000, at 2 μg of mRNA/well. The procedures are as follows: Lipofectamine™ 2000 and mRNA were separately diluted in Opti-MEM medium in a final volume of 100 μL, and the Lipofectamine™ 2000 solution and the mRNA solution were mixed and incubated at room temperature for 10-15 min to give a liposome complex containing mRNA. After the DMEM medium in the cell culture plates was discarded, the liposome complexes containing different mRNAs were added to the corresponding plates. Opti-MEM medium containing 10% of FBS was then added at 400 μL/well, and the cells were cultured at 37 °C (5% $CO_2$) for 4-6 h. The transfection medium was then discarded. DMEM medium containing 10% of FBS and 1% of antibiotics was added at 1 mL/well, and the cells were cultured at 37 °C (5% $CO_2$) for 48 h. Meanwhile, part of the cells were transfected with Lipofectamine™ 2000 solution with no mRNA as a negative control in the above manner.

The sequences of the target genes used in the present disclosure are summarized in Table 1.

Table 1. Sequence information of target genes of the present disclosure

| Protein encoded by target gene | Polypeptide sequence | DNA sequence | mRNA sequence | Optimized DNA sequence | Optimized mRNA sequence | Mutation position compared to wildtype SARS-CoV-2 (KV-PP) |
|---|---|---|---|---|---|---|
| B.1.1.7 (Alpha) spike protein | SEQ ID NO:23 | / | / | SEQ ID NO:22 | SEQ ID NO:60 | 983-984 |
| B.1.351 (Beta) spike protein | SEQ ID NO:21 | / | / | SEQ ID NO:20 | SEQ ID NO:59 | 983-984 |
| B.1.617.2 (Delta) spike protein | SEQ ID NO:12 | / | / | SEQ ID NO: 13 | SEQ ID NO:54 | 984-985 |
| B.1.1.529 (Omicron) spike protein | SEQ ID NO:14 | / | / | SEQ ID NO:15 | SEQ ID NO:55 | 983-985 |
| SARS-CoV-2 spike protein | SEQ ID NO:25 | / | / | SEQ ID NO:24 | SEQ ID NO:61 | 986-987 |
| Wildtype SARS-CoV-2 spike protein | SEQ ID NO:80 | SEQ ID NO:81 | SEQ ID NO:82 | / | / | / |

The mutation from amino acids KV to PP in SARS-CoV-2 spike protein can change the spike protein from an unstable prefusion conformation to a stable postfusion conformation (Structure-based design of prefusion-stabilized SARS-CoV-2 spikes, Science, VOL. 369, NO. 6510), such that the spike protein is in a stable state in conformation, which is favorable for vaccine design and production by taking the spike protein as immunogen.

**Example 1. Screening and preparation of 5'UTR sequences**

1. Screening of UTRs

In order to give a regulatory element capable of highly expressing a target protein in dendritic cells (DCs), this example was conducted with four-stage screening to give a new UTR.

The procedures are as follows: I, a high-protein-expression gene library was established, including genes with a high protein expression in monocytes, genes with a higher protein expression in DCs than in monocytes, genes with a higher protein level, and genes of a UTR with confirmed capability of increasing the protein expression, thus giving 2140 genes. II, a UTR library was established by downloading all sequences in the high-protein-expression gene library from Genbank database (https://www.ncbi.nlm.nih.gov/genbank/), analyzed for the integrity, and then analyzed by methods such as repeat combination, CDS (coding sequence) interception and the like to give UTR data, thus giving 940 UTRs. III, a UTR selection library was established by analyzing the UTR library, screening sequences with a proper sequence length (40 to 70 for 5'UTR sequences and 100 to 150 or 300 to 400 for 3'UTR sequences) and free energy, predicting the free energy of the sequences one by one, selecting sequences with a high 5'UTR free energy, and selecting sequences with a low 3'UTR free energy, thus acquiring 64 UTRs from the selection library. IV, final candidate sequences were determined by a screening strategy with a 5'UTR free energy of -10 or higher and a low 3'UTR free energy, finally giving 20 5'UTRs and 3'UTRs for screening and intracellular verification.

On this basis, we believe that the 5'UTR sequence (designated as 5'UTR I, with the DNA sequence set forth in SEQ ID NO: 1 and the RNA sequence set forth in SEQ ID NO: 45) derived from human Rho GTPase activating protein 15 (abbreviated as ARHGAP15) has the potential of efficiently expressing target proteins in human cells (particularly DCs).

2. Preparation of UTR

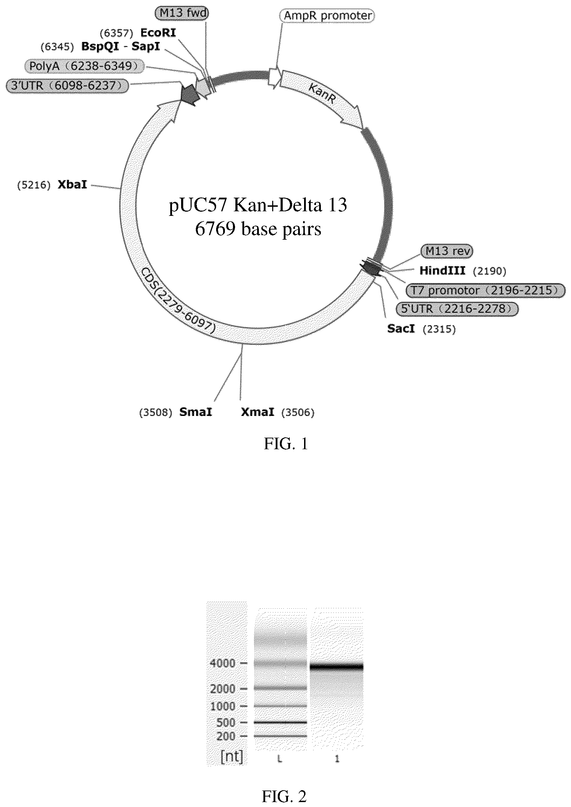

This example illustrates the preparation of 5'UTR I-containing mRNA. First, a sequence containing 5'UTR, 3'UTR, polyA tail, and the CDS of a heterologous target gene was artificially synthesized and inserted into pUC57 vector to give a template vector, as shown in FIG. 1. The sequence of the target gene inserted in the vector was confirmed by sequencing.

As shown in the pUC57 Delta13 vector in FIG. 1, the 5'UTR is set forth in SEQ ID NO: 1; the 3'UTR was a human hemoglobin beta subunit (HBB) 3'UTR sequence (SEQ ID NO: 7, designated as 3'UTR B); the CDS sequence was a base sequence (SEQ ID NO: 13) with optimized codons encoding the B.1.617.2 (Delta) spike protein; the polyA tail was a polyadenylic acid sequence (with the DNA sequence set forth in SEQ ID NO: 16 and the RNA sequence set forth in SEQ ID NO: 56); the T7 promoter is set forth in SEQ ID NO: 17.

To generate *in vitro* transcribed mRNA, the plasmid was linearized downstream of the poly A tail using BspQ I (Vazyme, DD4302-PC, China) and purified by a PCR purification kit (QIAGEN, 28106, Germany). *In vitro* transcription was performed to synthesize the mRNA with the purified linearized plasmid as template according to the following procedures. A 100 μL mRNA reaction system, as shown in Table 2, was synthesized. In the reaction system, 2 μg of linearized template was diluted to 100 μL with the non-nucleoside enzyme water. The reaction system was incubated for 4 h at 37 °C and digested for 30 min by DNase I. Detections showed a small amount of byproduct dsRNA in the synthesized mRNA and thus a good yield. The capping process was achieved by chemical capping in the *in vitro* transcription synthesis, with the cap structure being m7G(5')ppp(5')(2'OMeA)pG·NH$_4$ (Hongene, ON-134). The synthesized mRNA was purified by MEGAclear™ Kit (Invitrogen, AM1908, USA) to give an mRNA with a relatively higher purity for subsequent *in vitro* cell transfection and other experiments.

Table 2. mRNA reaction system

| Component | Cat. No. | Manufacturer | System (μL) |
|---|---|---|---|
| ATP solution (100 mM) | R1331 | Hongene | 4.41 |
| CTP solution (100 mM) | R3331 | Hongene | 5 |
| GTP solution (100 mM) | R2331 | Hongene | 2.79 |
| 10× transcription buffer | DD4101R-02 | Vazyme | 10 |
| T7 RNA polymerase | DD4101-PC-02 | Vazyme | 6.6 |
| Mouse RNase inhibitor | DD4102-PA | Vazyme | 2.75 |
| Pyrophosphatase | DD4103-PC-01 | Vazyme | 0.66 |
| Pseudo-UTP | RS-022 | Hongene | 2.79 |
| Cap-GAG Trimer | m7G(5')ppp(5')(2'OMeA)pG | Hongene | 1 |

Through screenings and functional verification, the 5'UTR I with the potential of efficiently expressing the target protein was acquired, with a sequence set forth SEQ ID NO: 1; and the construct containing the 5'UTR I can efficiently transcribe the corresponding mRNA, with a size consistent with the theoretical value as determined by chip electrophoresis (see FIG. 2).

**Example 2. Screening of combinations of different 5'UTRs and 3'UTRs for efficiently expressing heterologous proteins**

In this example, different combinations of 5 'UTRs and different combinations of 3'UTRs were screened to give UTR combinations capable of efficiently expressing a target gene.

The procedures are as follows: Constructs of plasmids containing the CDS of the same target gene with different UTR combinations were artificially synthesized. The basic plasmid was constructed according to the procedures in Example 1. The target gene CDS sequence was introduced by recombinant construction via artificial synthesis and HindIII/XhoI digestion. The reconstructed plasmid was linearized and purified mRNA was synthesized according to the procedures in Example 1. mRNA was transfected into 293T cells by Lipofectamine™ 2000. 293T cell culture supernatant was collected after 48 h and detected by Western Blot and ELISA to determine the level of secreted spike proteins in the cell supernatant.

The target genes used were: the gene encoding the SARS-CoV-2 B.1.351 spike protein (with a CDS set forth in SEQ ID NO: 20), or the gene encoding the SARS-CoV-2 B.1.1.7 spike protein (with a CDS set forth in SEQ ID NO: 22). The information of different 5'UTRs and 3'UTRs and their combinations are shown in Tables 3 and 4.

Western Blot procedures: To detect the *in vitro* expression level of the constructed mRNAs, the cell supernatant in the plate was collected after the transfection was completed, and the *in vitro* expression level of the constructed mRNAs was finally obtained by polyacrylamide gel electrophoresis, membrane transfer, co-incubation with the primary antibody and secondary antibody, and color development. The primary antibody was a SARS-CoV-2 (2019-nCoV) spike RBD antibody (Sino Biology, 40592-T62), and the secondary antibody was an HRP-linked anti-rabbit IgG (Transgene, HS101).

ELISA procedures: after the transfection was completed, the cell supernatant was collected, diluted in a ratio of 1:50 to 1:100, and detected for the expression level of the constructed mRNAs *in vitro* using an ELISA kit (SARS-CoV-2 (2019-nCoV) spike detection ELISA KIT, KIT40591, Sino Biology). The absorbance at 450 nm was read and the concentration of the relevant antigen was calculated.

Table 3. Sources and sequences of 5'UTR and 3'UTR

| UTR | Abbreviation | Source | GenBank ID | DNA sequence (SEQ ID NO:) | RNA sequence (SEQ ID NO:) |
|---|---|---|---|---|---|
| 5'UTR | I | Human ARHGAP15 | XM_003822828.3 | 1 | 45 |
| | G | Human heat shock 27kDa protein 1 (HSPB1) | BC012292.1 | 3 | 47 |
| | A | Human DNA, containing HBB gene | LC507571.1 | 4 | 48 |
| | J | Human C-C motif chemokine ligand 13 (CCL13) | NM_005408.3 | 5 | 49 |
| 3'UTR | B | Human HBB | MK476504.1 | 7 | *50* |
| | F | Human ARHGAP15 | NM_018460.4 | 8 | 51 |
| | E | Coronin 1A (CORO1A) | NM_007074.4 | 9 | 52 |
| | D | Hemopexin (HPX) | NM_000613.3 | 10 | 53 |

Table 4. Different combinations of 5'UTR and 3'UTR

| Name | 5'UTR | 3'UTR |
|---|---|---|
| A-B | A | B |
| A-E | A | E |
| A-D | A | D |
| A-F | A | F |
| G-E | G | E |
| G-B | G | B |
| G-D | G | D |
| I-E | I | E |
| I-B | I | B |
| I-F | I | F |
| I-D | I | D |
| J-E | J | E |

In Table 4, 5'UTRs A, G, I, and J are set forth in SEQ ID NOs: 4. 3, 1, and 5, respectively; 3'UTRs B, E, D, and F are set forth in SEQ ID NO: 7. 9, 10, and 8, respectively.

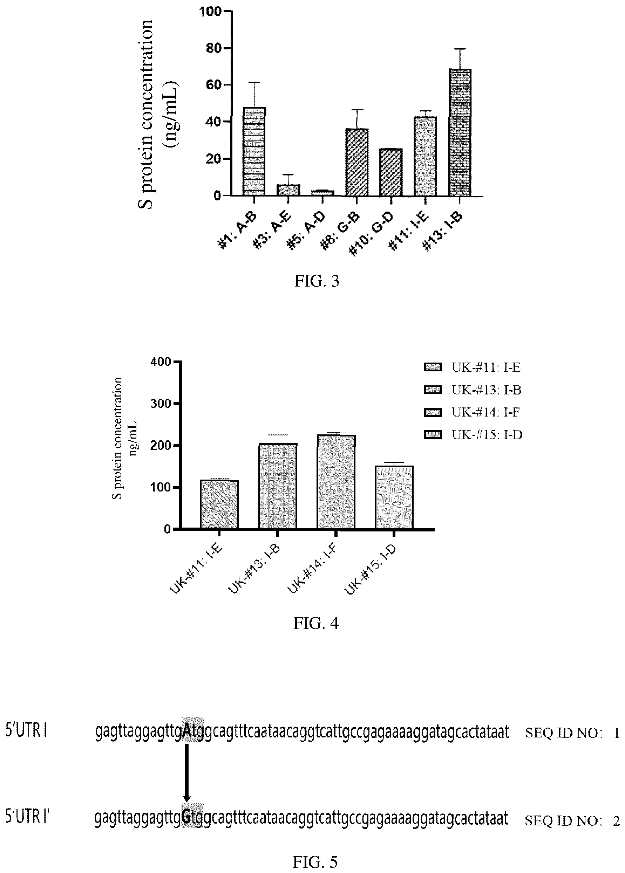

The effects of different combinations of 5'UTR with 3'UTR on the expression of SARS-CoV-2 B.1.351 spike protein and SARS-CoV-2 B.1.1.7 spike protein are shown in FIG. 3 and Table 5 and FIG. 4 and Table 6, respectively.

Table 5. Regulatory result of different UTR combinations on B.1.351 spike protein expression level (ELISA)

| Sample No. | #1: A-B | #3: A-E | #5: A-D | #8: G-B | #10: G-D | #11: I-E | #13: I-B |
|---|---|---|---|---|---|---|---|
| Spike protein concentration (mean±SD, ng/mL) | 48.2 ±13.4 | 6.3 ±5.4 | 2.9 ±0.28 | 36.7 ±10.5 | 26±0.14 | 43.4 ±2.97 | 69.4±10.88 |
| (sample numbering rule is that, for example, #1: A-B denotes sample #1 and a UTR combination of A-B) | | | | | | | |

Table 6. Regulatory result of different UTR combinations on B.1.1.7 spike protein expression level (ELISA)

| Sample No. | UK-#11: I-E | UK-#13: I-B | UK-#14: I-F | UK-#15: I-D | NC (Lipo control) |
|---|---|---|---|---|---|
| Spike protein concentration (mean±SD, ng/mL) | 118.35±3.61 | 206.1 ±19.66 | 227.6 ±4.38 | 153.55 ±7.99 | -9.65±0.64 |
| (sample numbering rule is that, for example, UK-#11: I-E denotes sample UK-#11 and a UTR combination of I-E) | | | | | |

As can be seen from FIG. 3 and Table 5, the comparison of B.1.351 spike protein expression level mediated by different UTR combinations is as follows: I-B > A-B > G-B, and I-B > I-E. For the 5'UTR, I is superior to A and G; for relevant combinations of I, I-B is superior to I-E. As can be seen from FIG. 4 and Table 6, the comparison of B.1.1.7 spike protein expression level mediated by different UTR combinations is as follows: I-F > I-B > I-D > I-E. By analyzing the results of B.1.351 and B.1.1.7 spike protein expression levels, the I-B UTR combination exhibited more significant advantages in mediating the efficient expression of the target gene, while the I-F combination also exhibited advantages in high-level expression of the target gene. Furthermore, the mRNAs containing I-B or I-F regulated B.1.617.2 spike protein were prepared, transfected into cells, and tested by ELISA. The spike protein expression level results are shown in Table 7.

Table 7. I-B and I-F UTR regulated B.1.617.2 spike protein expression level (ELISA)

| Sample | I-B | I-F |
|---|---|---|
| Spike protein concentration (ng/mL) | 57.1 | 58.8 |

Unlike the 5'UTR and 3'UTR in the I-B combination that are from different genes, the 5'UTR and 3'UTR in the I-F combination come from the same gene ARHGAP15. The results in Table 7 show that the I-B (SEQ ID NO: 1 and SEQ ID NO: 7) and I-F (SEQ ID NO: 1 and SEQ ID NO: 8) combinations regulate the expression of the target gene at comparable levels, further illustrating the universality of 5'UTR I for combination with different 3'UTRs to mediate efficient expression of the target gene (e.g., the gene encoding the spike protein).

**Example 3. Sequence engineering and preliminary functional validation of 5'UTR I**

1) Bioinformatic analysis and mutation design

5'UTR I (SEQ ID NO: 1) is a naturally occurring promoter of the human Rho GTPase activating protein family. The data of Examples 1-2 support that 5'UTR I assists in efficiently expressing the SARS-CoV-2 spike protein. However, 5'UTR I contains an ATG and may express a 27aa short peptide, which may affect normal protein expression of the target gene. In order to avoid the phenomenon that ribosome initiates the translation from the ATG inside the UTR, increase the probability of translation from the ATG in the CDS region, and increase the expression, a point mutation (A→G) was introduced to give a mutant 5'UTR I' (with a DNA sequence set forth in SEQ ID NO: 2 and an RNA sequence set forth in SEQ ID NO: 46). The mutation positions are shown in FIG. 5.

2) Point mutation from 5'UTR I to 5'UTR I' effectively increasing the expression of target genes

Plasmids containing 5'UTR I-B and 5'UTR I'-B to regulate the target gene expression were prepared according to the method in Example 1. The target gene was the gene encoding B.1.617.2 spike protein (SEQ ID NO: 13). The plasmids were designated as Delta 13 and Delta 24, respectively, and the mRNAs were synthesized by PCR and transcription and purified (the cap-free I'-Delta24-B mRNA sequence is set forth in SEQ ID NO: 57). The prepared mRNA was transfected into 293T cells, and the B.1.617.2 spike protein expression was detected using the Western Blot method in Example 2. By gray scanning using Image software, the grayscale value was calculated and normalized by the expression level of sample 1 (Delta 13) to give the ratio in Table 8.

The results are shown in FIGs. 6A and 6B and Table 8. The results show that for mRNAs prepared using either the minipreparation plasmid (with no endotoxin removal) or the maxipreparation plasmid (with endotoxins removed) as the template, the expression level of the spike protein mediated by the construct of 5'UTR I' after point mutation was greater than that mediated by the construct containing 5'UTR I. Thus, the 5'UTR I modified by the point mutation in Example 3 effectively improves the expression of heterologous proteins.

Table 8. Target gene expression levels regulated by I-B and I'-B UTRs (Western Blot assay)

| Sample | Plasmid mini-pre | | Plasmid maxi-pre | |
|---|---|---|---|---|
| | Sample 1 (Delta 13) | Sample 2 (Delta 24) | Sample 3 (Delta 13) | Sample 4 (Delta 24) |
| Ratio to expression of Sample 1 | 1.00 | 4.88 | 3.98 | 5.44 |

## Example 4. Assay of mRNA expression of different target genes regulated by 5'UTR I

Plasmids containing the gene encoding B.1.617.2 (Delta) spike protein (SEQ ID NO: 13) and the gene encoding B.1.1.529 (Omicron) spike protein (SEQ ID NO: 15) were constructed by replacing the target gene via digestion, with the 5'UTR I' and the 3'UTR B retained. The target gene sequences contained in the plasmids, as set forth in SEQ ID NO: 13 and SEQ ID NO: 15, were codon-optimized sequences encoding target protein sequences set forth in SEQ ID NO: 12 and SEQ ID NO: 14. mRNAs were prepared and purified as in Example 1. 2 $\mu$g of mRNA was transfected to 293T cells by Lipofectamine™ 2000, and the cell supernatant was harvested after 2 days. As per the Western Blot method in Example 2, the expression of I-B regulated B.1.617.2 spike protein mRNA (designated as Delta 13), I'-B regulated B.1.617.2 spike protein mRNA (designated as Delta 24), and I'-B regulated B.1.1.529 spike protein mRNA (designated as Omicron 24) were determined. The sequences of cap-free Delta 24 mRNA and Omicron 24 mRNA are set forth in SEQ ID NO: 57 and SEQ ID NO: 58. By gray scanning using Image software, the grayscale value was calculated and normalized by the expression level of Delta 13 to give the ratio in Table 9.

FIGs. 7A and 7B show that under the regulation of I'-B UTR combination, the SARS-CoV-2 B.1.617.2 and B.1.1.529 genes were expressed efficiently. The results suggest that 5'UTR I' has universality and can initiate the efficient expression of different target genes. Comparing 5'UTRs I and I', the Delta 24 spike protein exhibited an expression level significantly higher than that of the Delta 13 spike protein, indicating that the point mutation modification of 5'UTR I' effectively improves the protein expression efficiency, and the result further confirms the conclusion of Example 3.

Table 9. Target gene expression levels regulated by I-B and I'-B UTR combinations (Western Blot assay)

| Sample | Delta 13 | Delta 24 | Omicron 24 |
|---|---|---|---|
| Ratio to expression of sample Delta 13 | 1.00 | 1.69 | 1.48 |

## Example 5. Assay of luciferase expression regulated by I'-B UTR combination

A vector containing the luciferase CDS sequence (SEQ ID NO: 26) and the same T7 promoter (SEQ ID NO: 17) and polyA tail as in the previous vector containing the SARS-CoV-2 gene was constructed. Three vectors Luc, Luc13, and Luc24 with different UTRs were separately constructed, and the UTRs contained in the vectors are shown in Table 10. Among these, the 5'UTR and 3'UTR combination of BNT162b2 in Luc was used as the control, which is from CN113521269A. Capped mRNA was synthesized by *in vitro* transcription as in Example 1 and transfected *in vitro.* The supernatant was collected and luciferase substrate was added for detection. The plate was read, and the results are shown in Table 11 and FIG. 8.

Table 10. Vector structure

| Sample No. / Component | Luc | Luc13 | Luc24 |
|---|---|---|---|
| 5'UTR | BNT162b2(SEQ ID NO: 6) | I | I' |
| 3'UTR | BNT162b2(SEQ ID NO: 11) | B | B |

Table 11. Luciferase expression levels regulated by I'-B and I-B

| Sample | Luc | Luc13 | Luc24 |
|---|---|---|---|
| Run I | 774.81 | 992.36 | 1619.76 |
| Run II | 841.14 | 923.83 | 1519.50 |
| Note: The parameters are expressed in relative light unit (RLU). | | | |

The results show that the luciferase protein expression regulated by 5'UTR I' after point mutation was higher than that by 5'UTR I. Compared to the 5'UTR and 3'UTR combination of BNT162b2 in Luc control, the I-B combination of the present disclosure results in a higher expression level of the target gene, while the I'-B combination results in a significant increase in the expression level of the target gene. The assay results using luciferase as the target gene also suggest that the 5'UTR I or 5'UTR I' can regulate the efficient expression of any target protein with universality.

**Example 6. Assay of luciferase expression regulated by 5'UTR I' truncations and mutants**

6.1 The 5' end of the 5'UTR I' was successively truncated, and the efficiency of the 5'UTR I' truncations of different lengths in regulating protein expression was analyzed by bioinformatic methods (Nat Biotechnol. Jul., 2019; 37(7):803-809). The relative MRL values of the 5'UTR I' truncations to the 5'UTR I' were calculated, and the results are shown in Table 12 below. The results in Table 12 show that successive truncations of the nucleotide sequence at the 5' end of the 5'UTR I' did not cause significant reduction in the MRL value, indicating that the 5'UTR I' truncations can retain the functional activity of regulating the expression of the target protein. The 3' end of the 5'UTR I' truncation set forth in SEQ ID NO: 153 was successively truncated, and the relative MRL values of the 3' end truncations to the nucleotide sequence set forth in SEQ ID NO: 153 were calculated, as shown in Table 13. As can be seen from Table 13, the 3' end truncations did not exhibit a significant effect on the regulatory efficiency of the 5'UTR I' truncations.

Table 12. Relative MRL values of 5'UTR I' truncations and 5'UTR I'

| 5'UTR I' truncation | Relative MRL value | SEQ ID NO: |
|---|---|---|
| AGTTAGGAGTTGGTGGCAGTTTCAATAACAGGTCATTGCCGAGAAAAGGATAGCACTATAAT | 1 | SEQ ID NO: 137 |
| GTTAGGAGTTGGTGGCAGTTTCAATAACAGGTCATTGCCGAGAAAAGGATAGCACTATAAT | 1 | SEQ ID NO: 138 |
| TTAGGAGTTGGTGGCAGTTTCAATAACAGGTCATTGCCGAGAAAAGGATAGCACTATAAT | 1 | SEQ ID NO: 139 |
| TAGGAGTTGGTGGCAGTTTCAATAACAGGTCATTGCCGAGAAAAGGATAGCACTATAAT | 1 | SEQ ID NO: 28 |

(continued)

| 5'UTR I' truncation | Relative MRL value | SEQ ID NO: |
|---|---|---|
| AGGAGTTGGTGGCAGTTTCAATAACAGGTCATTGCCGAGAAAAGGATAGCACTATAAT | 1 | SEQ ID NO: 140 |
| GGAGTTGGTGGCAGTTTCAATAACAGGTCATTGCCGAGAAAAGGATAGCACTATAAT | 1 | SEQ ID NO: 141 |
| GAGTTGGTGGCAGTTTCAATAACAGGTCATTGCCGAGAAAAGGATAGCACTATAAT | 1 | SEQ ID NO: 142 |
| AGTTGGTGGCAGTTTCAATAACAGGTCATTGCCGAGAAAAGGATAGCACTATAAT | 1 | SEQ ID NO: 29 |
| GTTGGTGGCAGTTTCAATAACAGGTCATTGCCGAGAAAAGGATAGCACTATAAT | 1 | SEQ ID NO: 143 |
| TTGGTGGCAGTTTCAATAACAGGTCATTGCCGAGAAAAGGATAGCACTATAAT | 1 | SEQ ID NO: 144 |
| TGGTGGCAGTTTCAATAACAGGTCATTGCCGAGAAAAGGATAGCACTATAAT | 1 | SEQ ID NO: 145 |
| GGTGGCAGTTTCAATAACAGGTCATTGCCGAGAAAAGGATAGCACTATAAT | 1 | SEQ ID NO: 30 |
| GTGGCAGTTTCAATAACAGGTCATTGCCGAGAAAAGGATAGCACTATAAT | 1 | SEQ ID NO: 146 |
| TGGCAGTTTCAATAACAGGTCATTGCCGAGAAAAGGATAGCACTATAAT | 0.8 | SEQ ID NO: 147 |
| GGCAGTTTCAATAACAGGTCATTGCCGAGAAAAGGATAGCACTATAAT | 1 | SEQ ID NO: 148 |
| GCAGTTTCAATAACAGGTCATTGCCGAGAAAAGGATAGCACTATAAT | 1.02 | SEQ ID NO: 31 |
| CAGTTTCAATAACAGGTCATTGCCGAGAAAAGGATAGCACTATAAT | 1.02 | SEQ ID NO: 149 |
| AGTTTCAATAACAGGTCATTGCCGAGAAAAGGATAGCACTATAAT | 1.02 | SEQ ID NO: 150 |
| GTTTCAATAACAGGTCATTGCCGAGAAAAGGATAGCACTATAAT | 1 | SEQ ID NO: 151 |

(continued)

| 5'UTR I' truncation | Relative MRL value | SEQ ID NO: |
|---|---|---|
| TTTCAATAACAGGTCATTGCCGAGAAAAGGATAGCACTATAAT | 1.01 | SEQ ID NO: 32 |
| TTCAATAACAGGTCATTGCCGAGAAAAGGATAGCACTATAAT | 1.01 | SEQ ID NO: 152 |
| TCAATAACAGGTCATTGCCGAGAAAAGGATAGCACTATAAT | 1.01 | SEQ ID NO: 153 |
| CAATAACAGGTCATTGCCGAGAAAAGGATAGCACTATAAT | 1.02 | SEQ ID NO: 154 |
| AATAACAGGTCATTGCCGAGAAAAGGATAGCACTATAAT | 1.02 | SEQ ID NO: 33 |
| ATAACAGGTCATTGCCGAGAAAAGGATAGCACTATAAT | 1.02 | SEQ ID NO: 155 |
| TAACAGGTCATTGCCGAGAAAAGGATAGCACTATAAT | 1.01 | SEQ ID NO: 156 |
| AACAGGTCATTGCCGAGAAAAGGATAGCACTATAAT | 1.01 | SEQ ID NO: 157 |
| ACAGGTCATTGCCGAGAAAAGGATAGCACTATAAT | 1 | SEQ ID NO: 34 |
| CAGGTCATTGCCGAGAAAAGGATAGCACTATAAT | 1 | SEQ ID NO: 158 |
| AGGTCATTGCCGAGAAAAGGATAGCACTATAAT | 1 | SEQ ID NO: 159 |
| GGTCATTGCCGAGAAAAGGATAGCACTATAAT | 0.97 | SEQ ID NO: 160 |
| GTCATTGCCGAGAAAAGGATAGCACTATAAT | 0.98 | SEQ ID NO: 35 |
| TCATTGCCGAGAAAAGGATAGCACTATAAT | 0.99 | SEQ ID NO: 161 |
| CATTGCCGAGAAAAGGATAGCACTATAAT | 1 | SEQ ID NO: 162 |
| ATTGCCGAGAAAAGGATAGCACTATAAT | 1 | SEQ ID NO: 163 |
| TTGCCGAGAAAAGGATAGCACTATAAT | 0.98 | SEQ ID NO: 36 |
| TGCCGAGAAAAGGATAGCACTATAAT | 0.93 | SEQ ID NO: 164 |
| GCCGAGAAAAGGATAGCACTATAAT | 0.99 | SEQ ID NO: 165 |
| CCGAGAAAAGGATAGCACTATAAT | 1 | SEQ ID NO: 166 |

(continued)

| 5'UTR I' truncation | Relative MRL value | SEQ ID NO: |
|---|---|---|
| CGAGAAAAGGATAGCACTATAAT | 1 | SEQ ID NO: 37 |
| GAGAAAAGGATAGCACTATAAT | 1 | SEQ ID NO: 167 |
| AGAAAAGGATAGCACTATAAT | 1.01 | SEQ ID NO: 168 |
| GAAAAGGATAGCACTATAAT | 1 | SEQ ID NO: 169 |
| AAAAGGATAGCACTATAAT | 1 | SEQ ID NO: 38 |
| AAAGGATAGCACTATAAT | 1 | SEQ ID NO: 170 |
| AAGGATAGCACTATAAT | 0.99 | SEQ ID NO: 171 |
| AGGATAGCACTATAAT | 0.99 | SEQ ID NO: 172 |
| GGATAGCACTATAAT | 0.97 | SEQ ID NO: 39 |

Table 13. Relative MRL values of 3' end-truncated 5'UTR I' truncations to untruncated form

| 5'UTR I' truncation | Relative MRL value | SEQ NO ID: |
|---|---|---|
| TCAATAACAGGTCATTGCCGAGAAAAGGATAGCA | 0.99 | SEQ ID NO: 173 |
| TCAATAACAGGTCATTGCCGAGAAAAGGATAGCACTA | 0.98 | SEQ ID NO: 174 |
| TCAATAACAGGTCATTGCCGAGAAAAGGATAGCACT | 1 | SEQ ID NO: 175 |
| TCAATAACAGGTCATTGCCGAGAAAAGGATAGCACTATA | 0.99 | SEQ ID NO: 176 |
| TCAATAACAGGTCATTGCCGAGAAAAG | 0.97 | SEQ ID NO: 177 |
| TCAATAACAGGTCATTGCCGAGAAAAGGATA | 0.97 | SEQ ID NO: 178 |
| TCAATAACAGGTCATTGCCGAGAAAAGGAT | 0.98 | SEQ ID NO: 179 |
| TCAATAACAGGTCATTGCCGAGAAAAGGATAGC | 0.99 | SEQ ID NO: 180 |
| TCAATAACAGGTCATTGCCGAGAAAA | 0.95 | SEQ ID NO: 181 |
| TCAATAACAGGTCATTGCCGAGAAAAGG | 0.95 | SEQ ID NO: 182 |
| TCAATAACAGGTCATTGCCGAGAAA | 0.96 | SEQ ID NO: 183 |
| TCAATAACAGGTCATT | 0.93 | SEQ ID NO: 184 |
| TCAATAACAGGTCATTGCCGAGAAAAGGATAGCACTATAA | 1.01 | SEQ ID NO: 185 |
| TCAATAACAGGTCATTGCCGAGAAAAGGA | 0.97 | SEQ ID NO: 186 |
| TCAATAACAGGTCATTGCCGAG | 0.94 | SEQ ID NO: 187 |
| TCAATAACAGGTCATTGCCGAGAAAAGGATAGCACTAT | 0.93 | SEQ ID NO: 188 |
| TCAATAACAGGTCATTGCCGAGAA | 0.96 | SEQ ID NO: 189 |
| TCAATAACAGGTCATTG | 0.9 | SEQ ID NO: 190 |
| TCAATAACAGGTCATTGCCGAGAAAAGGATAG | 0.98 | SEQ ID NO: 191 |
| TCAATAACAGGTCATTGCCGAGA | 0.94 | SEQ ID NO: 192 |

(continued)

| 5'UTR I' truncation | Relative MRL value | SEQ NO ID: |
|---|---|---|
| TCAATAACAGGTCAT | 0.91 | SEQ ID NO: 193 |
| TCAATAACAGGTCATTGCC | 0.92 | SEQ ID NO: 194 |
| TCAATAACAGGTCATTGCCGAGAAAAGGATAGCAC | 0.94 | SEQ ID NO: 195 |
| TCAATAACAGGTCATTGCCG | 0.93 | SEQ ID NO: 196 |
| TCAATAACAGGTCATTGCCGA | 0.92 | SEQ ID NO: 41 |
| TCAATAACAGGTCATTGC | 0.85 | SEQ ID NO: 76 |

6.2 16 constructs containing 5'UTR I' truncations with different lengths (SEQ ID NOs: 28-40, CTATAAT) and 2 point mutations A→C and A→T (SEQ ID NOs: 42 and 43) different from the A→G point mutation were synthesized by Genewiz, Suzhou. The 16 synthesized sequences were inserted into Luc 24 in Example 5 by HindIII and ScaI digestion and ligation, thereby given constructs with corresponding luciferase 5'UTR I' truncations and point mutations, designated as LUC1-16, with corresponding sequences of the 5'UTR I' truncations (1-I' to 14-I') and mutants (15-I' and 16-I') set forth in SEQ ID NO: 63-78, respectively. Capped mRNA was synthesized by *in vitro* transcription as in Example 1 and transfected into 293T cells *in vitro* for 24 h. The luciferase substrate was added for detection, and the plate was read. The results are shown in FIG. 9. The luciferase expression levels mediated by Luc24 (I'-B combination) and 5'UTR truncations and mutants thereof were significantly higher than that mediated by Luc (5' and 3'UTRs of BNT162b2). The 5'UTR I' truncations can retain the activity of 5'UTR I' in regulating the expression of the target protein. Part of the 5'UTR I' truncations (1-I', 2-I', 3-I', 4-I', 5-I', 6-I', 7-I', 9-I', 10-I', 11-I', and 13-I') exhibited increased activity in regulating the expression of the target protein. Meanwhile, the mutants 15-I' and 16-I' of the 5'UTR I' mediated higher expression levels of the target gene than that of 5'UTR I', indicating that the functional activity of 5'UTR in regulating the expression of the target gene can be further enhanced by truncating the 5' end of 5'UTR I' or point mutation inside.

**Example 7. Differences in influenza HA mRNA expression levels regulated by control 5'UTR and 5'UTR I'**

Plasmids of the gene encoding influenza A virus H1N1 (A/Wisconsin/588/2019) HA protein (SEQ ID NO: 83), the gene encoding influenza A virus H3N2 (A/Cambodia/e0826360/2020) HA protein (SEQ ID NO: 84), the gene encoding influenza B virus (Washington/02/2019) HA protein (SEQ ID NO: 85), and the gene encoding influenza B virus (Phuket/3073/2013) HA protein (SEQ ID NO: 86) were constructed by replacing the target gene via digestion, with the 5'UTR I' and the 3'UTR B retained. The target gene sequences contained in the plasmids were SEQ ID NOs: 87-90, which were all codon-optimized sequences. Four HA CDS sequences encoding SEQ ID NOs: 83-86 were synthesized with reference to 5' and 3'UTR sequences (SEQ ID NOs: 91 and 92) in Patent Nos. WO2022/245888A1 and WO2022/150717A1, as set forth in SEQ ID NOs: 93-96, respectively. mRNAs were prepared and purified as in Example 1. 1 μg of mRNA was transfected to 293T cells by Lipofectamine™ 2000, and the lysate was harvested after 1 day. The expression of the four influenza HA protein mRNAs regulated by I'-B was detected by the Western Blot method in Example 2. The primary antibody was an influenza A virus hemagglutinin/HA antibody (Sino Biology, 86001-RM01) or an influenza B virus hemagglutinin/HA antibody against (Sino Biology, 11053-R004), and the secondary antibody was an HRP-linked anti-rabbit IgG (Transgene, HS101). The results are shown in FIGs. 10 and 11. The expression levels of the four influenza HA regulated by 5'UTR-I' were significantly higher as compared to the control 5'UTR.

Part of the sequences of the present disclosure are shown as follows:

>5'UTR I (63bp)

GAGTTAGGAGTTGATGGCAGTTTCAATAACAGGTCATTGCCGAGAAAAGGAT
AGCACTATAAT

SEQ ID NO: 1

>5'UTR I' (63bp)

GAGTTAGGAGTTGGTGGCAGTTTCAATAACAGGTCATTGCCGAGAAAAGGAT
AGCACTATAAT

SEQ ID NO: 2

>5'UTR G
GCACTTTTCTGAGCAGACGTCCAGAGCAGAGTCAGCCGCCACC
SEQ ID NO: 3
>5'UTR A ACATTTGCTTCTGACACAACTGTGTTCACTAGCAACCTCAAACGCCGCCACC
SEQ ID NO: 4
>5'UTR J

AGAGAGGCAAAGAAACATTGTGAAATCTCCAACTCTTAACCTTCAAGCCGCC
ACC

SEQ ID NO: 5

>5'UTR BNT162b2 GAATAAACTAGTATTCTTCTGGTCCCCACAGACTCAGAGAGAACCCGCCACC
SEQ ID NO: 6
>3'UTR B

GCTCGCTTTCTTGCTGTCCAATTTCTATTAAAGGTTCCTTTGTTCCCTAAGTCC
AACTACTAAACTGGGGGATATTATGAAGGGCCTTGAGCATCTGGATTCTGCCT
AATAAAAAACATTTATTTTCATTGCAA

SEQ ID NO: 7

>3'UTR F

CAGACAAGACAAGCTACTGAATACGTTCACATCTGTCTTGATGCCTAATATTT
TTACATTTCTGTAAACATATTTCTGAAATATTTTTTGCCTTTCAAGCGACAGAT
GCCTCATTTTGTGAAAACTTAATGATGATTTTGTGTTTAAGTTCCAAACATTTG
AATAAAATAATTGACAATA

SEQ ID NO: 8

>3'UTR E

CCTGAGGCCTCAGAACACAGGTTTCAGATTGATAGGCCTGCAGGTCTCCAGG
CAGCAACCAGCTGAGCGACTAAAGGGCCCAAGGCCAGGGCTCTAGGGATGG
GGCTCAGCAGAGGCTGGGGTAAGGGGAGCCAGGGAGGAGCTGGGCCTAATG
CAGCACCGGGTCCCCAGGATGCCGTGTCTCGGCTGGAGGAGGAGATGCGGA
AGCTCCAGGCCACGGTGCAGGAGCTCCAGAAGCGCTTGGACAGGCTGGAGG
AGACAGTCCAGGCCAAGTAGAGCCCCGCAGGGCCTCCAGCAGGGTCAGCCA
TTCACACCCATCCACTCACCTCCCATTCCCAGCCACATGGCAGAGAAAAAAA
TCATAATAAAATGGCTTTATTTTCTGGTA

SEQ ID NO: 9

>3'UTR D

GGGGCCTTCTGACATGAGTCTGGCCTGGCCCCACCTCCTAGTTCCTCATAATA
AAGACAGATTGCTTCTTCGCTTCTCACTGAGGGGCCTTCTGACATGAGTCTG
GCCTGGCCCCACCTCCCCAGTTCTCATAATAAAGACAGATTGCTTCTTCACT
TGAAGCTCGCTTTCTTGCTGTCCAATTTCTATTAAAGGTTCCTTTGTTCCCTAA
GTCCAACTACTAAACTGGGGGATATTATGAAGGGCCTTGAGCATCTGGATTCT
GCCTAATAAAAACATTTATTTTCATTGCAA

SEQ ID NO: 10

>3'UTR BNT162b2

CTCGAGCTGGTACTGCATGCACGCAATGCTAGCTGCCCCTTTCCCGTCCTGG
GTACCCCGAGTCTCCCCCGACCTCGGGTCCCAGGTATGCTCCCACCTCCACC
TGCCCCACTCACCACCTCTGCTAGTTCCAGACACCTCCCAAGCACGCAGCA
ATGCAGCTCAAAACGCTTAGCCTAGCCACACCCCACGGGAAACAGCAGTG
ATTAACCTTTAGCAATAAACGAAAGTTTAACTAAGCTATACTAACCCCAGGG
TTGGTCAATTTCGTGCCAGCCACACCCTGGA

SEQ
ID NO: 11

>B.1.617.2 (Delta) spike protein amino acid sequence

MFVFLVLLPLVSSQCVNLRTRTQLPPAYTNSFTRGVYYPDKVFRSSVLHSTQDLF
LPFFSNVTWFHAIHVSGTNGTKRFDNPVLPFNDGVYFASTEKSNIIRGWIFGTTL
DSKTQSLLIVNNATNVVIKVCEFQFCNDPFLDVYYHKNNKSWMESGVYSSANN
CTFEYVSQPFLMDLEGKQGNFKNLREFVFKNIDGYFKIYSKHTPINLVRDLPQGF
SALEPLVDLPIGINITRFQTLLALHRSYLTPGDSSSGWTAGAAAYYVGYLQPRTFL
LKYNENGTITDAVDCALDPLSETKCTLKSFTVEKGIYQTSNFRVQPTESIVRFPNI
TNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNL
DSKVGGNYNYRYRLFRKSNLKPFERDISTEIYQAGSKPCNGVEGFNCYFPLQSY
GFQPTNGVGYQPYRVVVLSFELLHAPATVCGPKKSTNLVKNKCVNFNFNGLTG
TGVLTESNKKFLPFQQFGRDIADTTDAVRDPQTLEILDITPCSFGGVSVITPGTNT

SNQVAVLYQGVNCTEVPVAIHADQLTPTWRVYSTGSNVFQTRAGCLIGAEHVN
NSYECDIPIGAGICASYQTQTNSRRRARSVASQSIIAYTMSLGAENSVAYSNNSIAI
PTNFTISVTTEILPVSMTKTSVDCTMYICGDSTECSNLLLQYGSFCTQLNRALTGI
AVEQDKNTQEVFAQVKQIYKTPPIKDFGGFNFSQILPDPSKPSKRSFIEDLLFNKV
TLADAGFIKQYGDCLGDIAARDLICAQKFNGLTVLPPLLTDEMIAQYTSALLAG
TITSGWTFGAGAALQIPFAMQMAYRFNGIGVTQNVLYENQKLIANQFNSAIGKI
QDSLSSTASALGKLQNVVNQNAQALNTLVKQLSSNFGAISSVLNDILSRLDPPEA
EVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFV
SNGTHWFVTQRNFYEPQIITTDNTFVSGNCDVVIGIVNNTVYDPLQPELDSFKEE
LDKYFKNHTSPDVDLGDISGINASVVNIQKEIDRLNEVAKNLNESLIDLQELGKY
EQYIKWPWYIWLGFIAGLIAIVMVTIMLCCMTSCCSCLKGCCSCGSCCKFDEDD
SEPVLKGVKLHYT

SEQ ID NO: 12

>B.1.617.2 (Delta) spike protein CDS

ATGTTCGTGTTCCTGGTGCTGCTGCCCCTGGTGAGCTCTCAGTGCGTGAACC
TGAGGACAAGAACACAGCTGCCCCCCGCCTACACCAACAGCTTCACAAGAG
GCGTGTACTACCCCGACAAGGTGTTCAGAAGCAGCGTGCTGCACTCCACCCA
AGACCTGTTTCTGCCCTTCTTCAGCAACGTGACCTGGTTCCACGCCATCCAC
GTCAGCGGCACCAACGGCACCAAGAGATTCGACAACCCCGTGCTGCCCTTC
AACGACGGCGTGTACTTCGCTAGCACCGAAAAGAGCAACATCATCAGAGGC
TGGATCTTCGGCACCACCCTAGACTCCAAGACACAAAGCCTGCTGATCGTTA
ACAACGCGACCAACGTGGTGATCAAGGTGTGCGAGTTTCAGTTCTGCAACG
ACCCCTTCCTGGACGTGTACTACCACAAGAACAACAAGAGCTGGATGGAGA
GCGGGGTCTACAGCAGCGCCAACAACTGCACCTTCGAGTACGTGTCTCAGCC
CTTCCTGATGGACCTGGAGGGCAAGCAAGGCAATTTCAAGAACCTAAGAGA
GTTCGTGTTCAAGAACATCGACGGCTACTTCAAGATCTACAGCAAGCACACC
CCCATCAACCTGGTGAGAGACCTGCCCCAAGGCTTCAGCGCCCTGGAGCCC
CTGGTGGACCTGCCCATCGGCATCAACATCACAAGATTTCAGACCCTGCTGG
CCCTGCACCGTAGCTACCTAACCCCCGGCGATAGTTCCTCTGGCTGGACGGC
CGGCGCTGCCGCCTACTACGTGGGCTACCTGCAGCCTAGAACCTTCCTGCTG
AAGTACAACGAGAACGGCACTATCACTGATGCCGTGGACTGTGCCCTGGACC
CCCTGAGCGAGACCAAGTGCACCCTCAAGAGCTTCACCGTGGAGAAGGGCA
TCTATCAGACAAGCAACTTCAGAGTGCAGCCCACCGAGAGCATCGTGAGATT
CCCCAACATCACCAACCTGTGCCCCTTCGGCGAGGTGTTCAACGCCACAAGA
TTCGCTAGCGTGTACGCGTGGAACAGAAAGAGAATCAGCAACTGCGTGGCC
GACTACAGCGTGCTGTACAACAGCGCTAGCTTCAGCACCTTCAAGTGCTATG
GCGTCAGCCCCACCAAGCTGAACGACCTGTGCTTCACCAACGTGTACGCCG
ACAGCTTCGTGATCAGAGGCGACGAGGTGAGACAGATTGCCCCGGGCAAA
CCGGCAAGATCGCCGACTACAACTACAAGCTGCCCGACGACTTCACCGGCTG
CGTGATCGCGTGGAACAGCAATAACCTGGATTCCAAGGTGGGCGGCAACTAC

AACTACCGGTACAGACTGTTCAGAAAGAGCAACCTGAAGCCCTTCGAGAGA
GACATCAGCACCGAGATCTACCAAGCCGGCAGCAAGCCCTGCAACGGCGTG
GAGGGCTTCAACTGCTACTTCCCCCTGCAGAGCTACGGCTTTCAGCCCACCA
ACGGCGTGGGCTATCAGCCCTACAGAGTGGTGGTGCTGAGCTTCGAGCTGCT
GCACGCCCCGCAACCGTGTGCGGCCCCAAAAAGAGCACCAACCTGGTGAA
GAACAAGTGCGTGAACTTCAACTTTAATGGCCTTACCGGCACGGGCGTGCTG
ACCGAGAGCAACAAGAAGTTCCTGCCATTTCAGCAATTCGGCAGAGACATC
GCCGACACCACCGACGCGGTCAGAGACCCTCAGACCCTGGAGATCCTGGAC
ATCACCCCCTGTAGCTTCGGCGGCGTGAGCGTGATCACCCCCGGCACTAACA
CAAGCAACCAAGTGGCCGTGCTGTACCAAGGCGTGAACTGCACCGAGGTGC
CCGTGGCCATCCACGCCGATCAGCTGACCCCCACCTGGAGAGTATACAGCAC
CGGAAGCAACGTGTTTCAGACAAGAGCCGGCTGCCTGATCGGCGCCGAGCA
CGTGAACAACAGCTACGAGTGCGACATCCCCATCGGCGCCGGCATCTGCGCT
AGCTATCAGACACAGACCAACAGCCGCCGGAGAGCTAGAAGCGTGGCTTCT
CAGAGCATCATCGCCTACACCATGAGCCTGGGCGCCGAGAACAGCGTGGCCT
ACAGCAACAACAGCATCGCCATCCCCACCAACTTCACCATCAGCGTGACCAC
CGAGATCCTGCCCGTAAGTATGACCAAGACAAGCGTGGACTGCACCATGTAC
ATCTGCGGCGACAGCACCGAGTGCAGCAACCTGCTGCTGCAGTACGGCAGC
TTCTGCACACAGCTGAACAGAGCCCTGACCGGCATCGCCGTGGAGCAAGAC
AAGAACACCCAAGAGGTGTTCGCCCAAGTGAAGCAGATCTACAAGACCCCC
CCCATCAAGGACTTCGGCGGCTTCAACTTCAGCCAAATCCTGCCCGACCCTA
GCAAGCCTAGCAAGCGGAGCTTCATCGAGGACCTGCTGTTCAACAAGGTGA
CCCTGGCCGACGCCGGCTTCATCAAGCAGTACGGCGACTGCCTAGGCGACAT
CGCAGCTAGAGACCTGATCTGCGCTCAGAAGTTCAATGGACTCACCGTGCTC
CCCCCCCTGCTGACCGACGAGATGATCGCTCAGTACACCTCTGCCCTGCTGG
CGGGAACCATAACAAGCGGTTGGACATTTGGCGCCGGGGCCGCACTGCAAA
TCCCCTTCGCCATGCAGATGGCCTACAGATTCAACGGCATCGGCGTGACACA
GAACGTGCTGTACGAGAATCAGAAGCTGATCGCCAATCAGTTCAACAGCGCC
ATCGGCAAGATCCAAGACAGCCTGAGCAGCACCGCTAGCGCCCTGGGCAAG
CTGCAAAACGTGGTGAATCAGAACGCCCAAGCCCTGAACACCCTGGTGAAG
CAGCTGAGCAGCAACTTCGGCGCCATCAGCAGCGTATTGAATGACATCCTGT
CTAGACTGGACCCCCCCGAGGCCGAGGTGCAGATCGACAGACTGATCACCG
GCAGACTGCAGAGCCTGCAGACCTACGTGACACAGCAGCTGATCAGAGCCG
CCGAGATCAGAGCTAGCGCCAACCTGGCCGCCACCAAGATGAGCGAGTGCG
TGCTGGGGCAGAGCAAGAGAGTGGACTTCTGCGGCAAGGGCTACCACCTGA
TGAGCTTCCCTCAGAGCGCCCCCCACGGCGTGGTGTTCCTGCACGTGACCTA
CGTGCCCGCCCAAGAGAAGAACTTCACCACCGCTCCCGCCATCTGCCACGA
CGGCAAGGCCCACTTCCCTAGAGAGGGCGTGTTCGTGAGCAACGGCACCCA
CTGGTTCGTGACACAGAGAAACTTCTACGAGCCTCAGATCATCACCACCGAC
AACACCTTCGTGAGCGGCAACTGCGACGTGGTGATCGGCATAGTGAACAAC
ACCGTATACGACCCCCTGCAGCCCGAGCTGGACAGCTTCAAGGAGGAGCTG

GACAAGTACTTCAAAAACCATACTAGCCCCGACGTGGACCTGGGAGACATCT
CTGGCATCAACGCTAGCGTGGTGAACATTCAGAAGGAGATCGACCGGCTGAA
TGAGGTGGCCAAGAACCTGAACGAGAGCCTGATCGACCTGCAAGAGCTGGG
CAAGTACGAGCAGTACATCAAGTGGCCCTGGTACATCTGGCTGGGCTTCATC
GCCGGCCTGATCGCCATCGTGATGGTGACCATCATGCTGTGCTGCATGACAA
GCTGTTGCAGCTGCCTGAAAGGTTGCTGCAGCTGTGGCTCGTGCTGCAAGTT
CGACGAGGACGACAGCGAGCCCGTGCTGAAGGGCGTGAAGCTGCACTACAC
CTGATGA

SEQ ID NO: 13

>B.1.1.529 (Omicron) spike protein amino acid sequence

MFVFLVLLPLVSSQCVNLTTRTQLPPAYTNSFTRGVYYPDKVFRSSVLHSTQDLF
LPFFSNVTWFHVISGTNGTKRFDNPVLPFNDGVYFASIEKSNIIRGWIFGTTLDSK
TQSLLIVNNATNVVIKVCEFQFCNDPFLDHKNNKSWMESEFRVYSSANNCTFEY
VSQPFLMDLEGKQGNFKNLREFVFKNIDGYFKIYSKHTPIIVREPEDLPQGFSAL
EPLVDLPIGINITRFQTLLALHRSYLTPGDSSSGWTAGAAAYYVGYLQPRTFLLK
YNENGTITDAVDCALDPLSETKCTLKSFTVEKGIYQTSNFRVQPTESIVRFPNITN
LCPFDEVFNATRFASVYAWNRKRISNCVADYSVLYNLAPFFTFKCYGVSPTKLN
DLCFTNVYADSFVIRGDEVRQIAPGQTGNIADYNYKLPDDFTGCVIAWNSNKLD
SKVSGNYNYLYRLFRKSNLKPFERDISTEIYQAGNKPCNGVAGFNCYFPLRSYSF
RPTYGVGHQPYRVVVLSFELLHAPATVCGPKKSTNLVKNKCVNFNFNGLKGTG
VLTESNKKFLPFQQFGRDIADTTDAVRDPQTLEILDITPCSFGGVSVITPGTNTSN
QVAVLYQGVNCTEVPVAIHADQLTPTWRVYSTGSNVFQTRAGCLIGAEYVNNS
YECDIPIGAGICASYQTQTKSHRRARSVASQSIIAYTMSLGAENSVAYSNNSIAIPT
NFTISVTTEILPVSMTKTSVDCTMYICGDSTECSNLLLQYGSFCTQLKRALTGIAV
EQDKNTQEVFAQVKQIYKTPPIKYFGGFNFSQILPDPSKPSKRSFIEDLLFNKVTL
ADAGFIKQYGDCLGDIAARDLICAQKFKGLTVLPPLLTDEMIAQYTSALLAGTIT
SGWTFGAGAALQIPFAMQMAYRFNGIGVTQNVLYENQKLIANQFNSAIGKIQDS
LSSTASALGKLQDVVNHNAQALNTLVKQLSSKFGAISSVLNDIFSRLDPPEAEVQ
IDRLITGRLQSLQTYVTQQLIRAAEIRASANLAATKMSECVLGQSKRVDFCGKG
YHLMSFPQSAPHGVVFLHVTYVPAQEKNFTTAPAICHDGKAHFPREGVFVSNGT
HWFVTQRNFYEPQIITTDNTFVSGNCDVVIGIVNNTVYDPLQPELDSFKEELDK
YFKNHTSPDVDLGDISGINASVVNIQKEIDRLNEVAKNLNESLIDLQELGKYEQY
IKWPWYIWLGFIAGLIAIVMVTIMLCCMTSCCSCLKGCCSCGSCCKFDEDDSEP
VLKGVKLHYT

SEQ ID NO: 14

>B.1.1.529 (Omicron) spike protein CDS

ATGTTCGTGTTCCTGGTGCTGCTGCCCCTGGTGAGCTCTCAGTGCGTGAACC
TGACCACAAGAACACAGCTGCCCCCGCCTACACCAACAGCTTCACAAGAG
GCGTGTACTACCCCGACAAGGTGTTCAGAAGCAGCGTGCTGCACTCCACCCA
AGACCTGTTTCTGCCCTTCTTCAGCAACGTGACCTGGTTCCACGTGATCAGC
GGCACCAACGGCACCAAGAGATTCGACAACCCCGTGCTGCCCTTCAACGAC

GGCGTGTACTTCGCTAGCATCGAAAAGAGCAACATCATCAGAGGCTGGATCT
TCGGCACCACCCTAGACTCCAAGACACAAAGCCTGCTGATCGTTAACAACGC
GACCAACGTGGTGATCAAGGTGTGCGAGTTTCAGTTCTGCAACGACCCCTTC
CTGGACCACAAGAACAACAAGAGCTGGATGGAGAGCGAGTTCAGAGTGTAC
AGCAGCGCCAACAACTGCACCTTCGAGTACGTGTCTCAGCCCTTCCTGATGG
ACCTGGAGGGCAAGCAAGGCAATTTCAAGAACCTAAGAGAGTTCGTGTTCA
AGAACATCGACGGCTACTTCAAGATCTACAGCAAGCACACCCCATCATCGT
GAGAGAGCCCGAGGACCTGCCCCAAGGCTTCAGCGCCCTGGAGCCCCTGGT
GGACCTGCCCATCGGCATCAACATCACAAGATTTCAGACCCTGCTGGCCCTG
CACCGTAGCTACCTAACCCCCGGCGATAGTTCCTCTGGCTGGACGGCCGGCG
CTGCCGCCTACTACGTGGGCTACCTGCAGCCTAGAACCTTCCTGCTGAAGTA
CAACGAGAACGGCACTATCACTGATGCCGTGGACTGTGCCCTGGACCCCCTG
AGCGAGACCAAGTGCACCCTCAAGAGCTTCACCGTGGAGAAGGGCATCTAT
CAGACAAGCAACTTCAGAGTGCAGCCCACCGAGAGCATCGTGAGATTCCCC
AACATCACCAACCTGTGCCCCTTCGACGAGGTGTTCAACGCCACCAGATTTG
CTAGCGTGTACGCCTGGAACCGGAAGAGAATCAGCAACTGCGTGGCCGACT
ACAGCGTGCTGTACAACCTGGCCCCCTTCTTCACCTTCAAGTGCTATGGCGTC
AGCCCCACCAAGCTGAACGACCTGTGCTTCACCAACGTGTACGCCGACAGC
TTCGTGATCAGAGGCGACGAGGTGAGACAGATCGCCCCCGGGCAGACCGGC
AACATCGCCGACTACAACTACAAGCTGCCCGACGACTTCACCGGCTGCGTGA
TCGCCTGGAACAGCAACAAGCTGGATAGCAAGGTGAGCGGCAACTACAATT
ACCTGTACAGACTGTTCAGAAAGAGCAACCTGAAGCCCTTCGAGAGAGACA
TCAGCACCGAGATCTACCAAGCCGGCAACAAGCCCTGCAACGGCGTGGCGG
GCTTCAACTGCTACTTCCCCCTCAGGAGCTACAGCTTCAGACCCACCTACGG
CGTGGGCCATCAGCCCTACAGAGTGGTCGTGCTGAGCTTCGAGCTGCTGCAC
GCCCCTGCCACAGTGTGCGGCCCCAAAAAGAGCACCAACCTGGTGAAGAAC
AAGTGCGTGAACTTCAACTTCAACGGCCTGAAGGGCACCGGCGTGCTGACC
GAGAGCAACAAGAAGTTCCTGCCATTTCAGCAATTCGGCAGAGACATCGCC
GACACCACCGACGCGGTCAGAGACCCTCAGACCCTGGAGATCCTGGACATC
ACCCCCTGTAGCTTCGGCGGCGTGAGCGTGATCACCCCCGGCACTAACACAA
GCAACCAAGTGGCCGTGCTGTACCAAGGCGTGAACTGCACCGAGGTGCCCG
TGGCCATCCACGCCGATCAGCTGACCCCCACCTGGAGAGTGTATAGCACCGG
CAGCAACGTGTTTCAGACAAGAGCCGGCTGCCTGATCGGCGCCGAGTACGT
GAACAACAGCTACGAGTGCGACATCCCCATCGGCGCCGGCATCTGCGCTAGC
TATCAGACACAGACCAAGAGCCACCGGAGAGCTAGAAGCGTGGCTAGCCAA
AGCATCATCGCCTACACCATGAGCCTGGGCGCCGAGAACAGCGTGGCCTACA
GCAACAACAGCATCGCCATCCCCACCAACTTCACCATCAGCGTGACCACCGA
GATCCTGCCCGTAAGTATGACCAAGACAAGCGTGGACTGCACCATGTACATC
TGCGGCGACAGCACCGAGTGCAGCAACCTGCTCCTGCAGTACGGCAGCTTC
TGCACACAGCTGAAGAGAGCCCTGACCGGCATCGCCGTGGAGCAAGACAAG
AACACCCAAGAGGTGTTCGCCCAAGTGAAGCAGATCTACAAGACCCCCCCC

ATCAAGTACTTCGGCGGCTTCAACTTCAGCCAAATCCTGCCCGACCCTAGCA
AGCCTAGCAAGCGGAGCTTCATCGAGGACCTGCTGTTCAACAAGGTGACCCT
GGCCGACGCCGGCTTCATCAAGCAGTACGGCGACTGCCTAGGCGACATCGCA
GCTAGAGACCTGATCTGCGCTCAGAAGTTCAAGGGACTCACCGTGCTCCCCC
CCCTGCTGACCGACGAGATGATCGCTCAGTACACCTCTGCCCTGCTGGCGGG
AACCATAACAAGCGGTTGGACATTTGGCGCCGGGGCCGCACTGCAAATCCCC
TTCGCCATGCAGATGGCCTACAGATTCAACGGCATCGGCGTGACACAGAACG
TGCTGTACGAGAATCAGAAGCTGATCGCCAATCAGTTCAACAGCGCCATCGG
CAAGATCCAAGACAGCCTGAGCAGCACCGCTAGCGCCCTGGGCAAGCTGCA
AGACGTGGTGAACCACAACGCCCAAGCCCTGAACACCCTGGTGAAGCAGCT
GAGCAGCAAGTTCGGCGCCATCAGCAGCGTGCTGAATGACATCTTCTCTAGA
CTGGACCCCCCCGAGGCCGAGGTGCAGATCGACAGACTGATCACCGGCAGA
CTGCAGAGCCTGCAGACCTACGTGACACAGCAGCTGATCAGAGCCGCCGAG
ATCAGAGCTAGCGCCAACCTGGCCGCCACCAAGATGAGCGAGTGCGTGCTG
GGGCAGAGCAAGAGAGTGGACTTCTGCGGCAAGGGCTACCACCTGATGAGC
TTCCCTCAGAGCGCCCCCCACGGCGTGGTGTTCCTGCACGTGACCTACGTGC
CCGCCCAAGAGAAGAACTTCACCACCGCTCCCGCCATCTGCCACGACGGCA
AGGCCCACTTCCCTAGAGAGGGCGTGTTCGTGAGCAACGGCACCCACTGGT
TCGTGACACAGAGAAACTTCTACGAGCCTCAGATCATCACCACCGACAACAC
CTTCGTGAGCGGCAACTGCGACGTGGTGATCGGCATAGTGAACAACACCGTA
TACGACCCCCTGCAGCCCGAGCTGGACAGCTTCAAGGAGGAGCTGGACAAG
TACTTCAAAAACCATACTAGCCCCGACGTGGACCTGGGAGACATCTCTGGCA
TCAACGCTAGCGTGGTGAACATTCAGAAGGAGATCGACCGGCTGAATGAGG
TGGCCAAGAACCTGAACGAGAGCCTGATCGACCTGCAAGAGCTGGGCAAGT
ACGAGCAGTACATCAAGTGGCCCTGGTACATCTGGCTGGGCTTCATCGCCGG
CCTGATCGCCATCGTGATGGTGACCATCATGCTGTGCTGCATGACAAGCTGTT
GCAGCTGCCTGAAAGGTTGCTGCAGCTGTGGCTCGTGCTGCAAGTTCGACG
AGGACGACAGCGAGCCCGTGCTGAAGGGCGTGAAGCTGCACTACCTGAT
GA

SEQ ID NO: 15

>poly A

AAAAAAAAAAAAAAAAAAAAAAAAAAAAAAAAAGCATATGACTAAAAAAAAAA
AAAAAAAAAAAAAAAAAAAAAAAAAAAAAAAAAAAAAAAAAAAAAAAAAAA
AAAAAAAAAAAA

SEQ ID NO: 16

>T7 promoter
TAATACGACTCACTATAAGG
SEQ ID NO: 17
>1'-Delta24-B

GAGTTAGGAGTTGGTGGCAGTTTCAATAACAGGTCATTGCCGAGAAAAGGAT
AGCACTATAATATGTTCGTGTTCCTGGTGCTGCTGCCCCTGGTGAGCTCTCAG

TGCGTGAACCTGAGGACAAGAACACAGCTGCCCCCCGCCTACACCAACAGC
TTCACAAGAGGCGTGTACTACCCCGACAAGGTGTTCAGAAGCAGCGTGCTG
CACTCCACCCAAGACCTGTTTCTGCCCTTCTTCAGCAACGTGACCTGGTTCC
ACGCCATCCACGTCAGCGGCACCAACGGCACCAAGAGATTCGACAACCCCG
TGCTGCCCTTCAACGACGGCGTGTACTTCGCTAGCACCGAAAAGAGCAACAT
CATCAGAGGCTGGATCTTCGGCACCACCCTAGACTCCAAGACACAAAGCCTG
CTGATCGTTAACAACGCGACCAACGTGGTGATCAAGGTGTGCGAGTTTCAGT
TCTGCAACGACCCCTTCCTGGACGTGTACTACCACAAGAACAACAAGAGCT
GGATGGAGAGCGGGGTCTACAGCAGCGCCAACAACTGCACCTTCGAGTACG
TGTCTCAGCCCTTCCTGATGGACCTGGAGGGCAAGCAAGGCAATTTCAAGAA
CCTAAGAGAGTTCGTGTTCAAGAACATCGACGGCTACTTCAAGATCTACAGC
AAGCACACCCCCATCAACCTGGTGAGAGACCTGCCCCAAGGCTTCAGCGCC
CTGGAGCCCCTGGTGGACCTGCCCATCGGCATCAACATCACAAGATTTCAGA
CCCTGCTGGCCCTGCACCGTAGCTACCTAACCCCCGGCGATAGTTCCTCTGGC
TGGACGGCCGGCGCTGCCGCCTACTACGTGGGCTACCTGCAGCCTAGAACCT
TCCTGCTGAAGTACAACGAGAACGGCACTATCACTGATGCCGTGGACTGTGC
CCTGGACCCCCTGAGCGAGACCAAGTGCACCCTCAAGAGCTTCACCGTGGA
GAAGGGCATCTATCAGACAAGCAACTTCAGAGTGCAGCCCACCGAGAGCAT
CGTGAGATTCCCCAACATCACCAACCTGTGCCCCTTCGGCGAGGTGTTCAAC
GCCACAAGATTCGCTAGCGTGTACGCGTGGAACAGAAAGAGAATCAGCAAC
TGCGTGGCCGACTACAGCGTGCTGTACAACAGCGCTAGCTTCAGCACCTTCA
AGTGCTATGGCGTCAGCCCCACCAAGCTGAACGACCTGTGCTTCACCAACGT
GTACGCCGACAGCTTCGTGATCAGAGGCGACGAGGTGAGACAGATTGCCCC
CGGGCAAACCGGCAAGATCGCCGACTACAACTACAAGCTGCCCGACGACTT
CACCGGCTGCGTGATCGCGTGGAACAGCAATAACCTGGATTCCAAGGTGGGC
GGCAACTACAACTACCGGTACAGACTGTTCAGAAAGAGCAACCTGAAGCCC
TTCGAGAGAGACATCAGCACCGAGATCTACCAAGCCGGCAGCAAGCCCTGC
AACGGCGTGGAGGGCTTCAACTGCTACTTCCCCCTGCAGAGCTACGGCTTTC
AGCCCACCAACGGCGTGGGCTATCAGCCCTACAGAGTGGTGGTGCTGAGCTT
CGAGCTGCTGCACGCCCCCGCAACCGTGTGCGGCCCCAAAAAGAGCACCAA
CCTGGTGAAGAACAAGTGCGTGAACTTCAACTTTAATGGCCTTACCGGCACG
GGCGTGCTGACCGAGAGCAACAAGAAGTTCCTGCCATTTCAGCAATTCGGC
AGAGACATCGCCGACACCACCGACGCGGTCAGAGACCCTCAGACCCTGGAG
ATCCTGGACATCACCCCCTGTAGCTTCGGCGGCGTGAGCGTGATCACCCCCG
GCACTAACACAAGCAACCAAGTGGCCGTGCTGTACCAAGGCGTGAACTGCA
CCGAGGTGCCCGTGGCCATCCACGCCGATCAGCTGACCCCCACCTGGAGAGT
ATACAGCACCGGAAGCAACGTGTTTCAGACAAGAGCCGGCTGCCTGATCGG
CGCCGAGCACGTGAACAACAGCTACGAGTGCGACATCCCCATCGGCGCCGG
CATCTGCGCTAGCTATCAGACACAGACCAACAGCCGCCGGAGAGCTAGAAG
CGTGGCTTCTCAGAGCATCATCGCCTACACCATGAGCCTGGGCGCCGAGAAC
AGCGTGGCCTACAGCAACAACAGCATCGCCATCCCCACCAACTTCACCATCA

GCGTGACCACCGAGATCCTGCCCGTAAGTATGACCAAGACAAGCGTGGACT
GCACCATGTACATCTGCGGCGACAGCACCGAGTGCAGCAACCTGCTGCTGCA
GTACGGCAGCTTCTGCACACAGCTGAACAGAGCCCTGACCGGCATCGCCGT
GGAGCAAGACAAGAACACCCAAGAGGTGTTCGCCCAAGTGAAGCAGATCTA
CAAGACCCCCCCCATCAAGGACTTCGGCGGCTTCAACTTCAGCCAAATCCTG
CCCGACCCTAGCAAGCCTAGCAAGCGGAGCTTCATCGAGGACCTGCTGTTCA
ACAAGGTGACCCTGGCCGACGCCGGCTTCATCAAGCAGTACGGCGACTGCCT
AGGCGACATCGCAGCTAGAGACCTGATCTGCGCTCAGAAGTTCAATGGACTC
ACCGTGCTCCCCCCCCTGCTGACCGACGAGATGATCGCTCAGTACACCTCTG
CCCTGCTGGCGGGAACCATAACAAGCGGTTGGACATTTGGCGCCGGGGCCG
CACTGCAAATCCCCTTCGCCATGCAGATGGCCTACAGATTCAACGGCATCGG
CGTGACACAGAACGTGCTGTACGAGAATCAGAAGCTGATCGCCAATCAGTTC
AACAGCGCCATCGGCAAGATCCAAGACAGCCTGAGCAGCACCGCTAGCGCC
CTGGGCAAGCTGCAAAACGTGGTGAATCAGAACGCCCAAGCCCTGAACACC
CTGGTGAAGCAGCTGAGCAGCAACTTCGGCGCCATCAGCAGCGTATTGAATG
ACATCCTGTCTAGACTGGACCCCCCCGAGGCCGAGGTGCAGATCGACAGACT
GATCACCGGCAGACTGCAGAGCCTGCAGACCTACGTGACACAGCAGCTGAT
CAGAGCCGCCGAGATCAGAGCTAGCGCCAACCTGGCCGCCACCAAGATGAG
CGAGTGCGTGCTGGGGCAGAGCAAGAGAGTGGACTTCTGCGGCAAGGGCTA
CCACCTGATGAGCTTCCCTCAGAGCGCCCCCCACGGCGTGGTGTTCCTGCAC
GTGACCTACGTGCCCGCCCAAGAGAAGAACTTCACCACCGCTCCCGCCATCT
GCCACGACGGCAAGGCCCACTTCCCTAGAGAGGGCGTGTTCGTGAGCAACG
GCACCCACTGGTTCGTGACACAGAGAAACTTCTACGAGCCTCAGATCATCAC
CACCGACAACACCTTCGTGAGCGGCAACTGCGACGTGGTGATCGGCATAGTG
AACAACACCGTATACGACCCCCTGCAGCCCGAGCTGGACAGCTTCAAGGAG
GAGCTGGACAAGTACTTCAAAAACCATACTAGCCCCGACGTGGACCTGGGA
GACATCTCTGGCATCAACGCTAGCGTGGTGAACATTCAGAAGGAGATCGACC
GGCTGAATGAGGTGGCCAAGAACCTGAACGAGAGCCTGATCGACCTGCAAG
AGCTGGGCAAGTACGAGCAGTACATCAAGTGGCCCTGGTACATCTGGCTGGG
CTTCATCGCCGGCCTGATCGCCATCGTGATGGTGACCATCATGCTGTGCTGCA
TGACAAGCTGTTGCAGCTGCCTGAAAGGTTGCTGCAGCTGTGGCTCGTGCTG
CAAGTTCGACGAGGACGACAGCGAGCCCGTGCTGAAGGGCGTGAAGCTGC
ACTACACCTGATGACTCGAGGCTCGCTTTCTTGCTGTCCAATTTCTATTAAAG
GTTCCTTTGTTCCCTAAGTCCAACTACTAAACTGGGGGATATTATGAAGGGCC
TTGAGCATCTGGATTCTGCCTAATAAAAACATTTATTTTCATTGCAAAAAAA
AAAAAAAAAAAAAAAAAAAAAAAAAGCATATGACTAAAAAAAAAAAAAAA
AAAAAAAAAAAAAAAAAAAAAAAAAAAAAAAAAAAAAAAAAAAAAAAAAA
AAAAAAA

SEQ ID NO: 18

> I'-OC24-B

GAGTTAGGAGTTGGTGGCAGTTTCAATAACAGGTCATTGCCGAGAAAAGGAT

AGCACTATAATATGTTCGTGTTCCTGGTGCTGCTGCCCCTGGTGAGCTCTCAG
TGCGTGAACCTGACCACAAGAACACAGCTGCCCCCGCCTACACCAACAGC
TTCACAAGAGGCGTGTACTACCCCGACAAGGTGTTCAGAAGCAGCGTGCTG
CACTCCACCCAAGACCTGTTTCTGCCCTTCTTCAGCAACGTGACCTGGTTCC
ACGTGATCAGCGGCACCAACGGCACCAAGAGATTCGACAACCCCGTGCTGC
CCTTCAACGACGGCGTGTACTTCGCTAGCATCGAAAAGAGCAACATCATCAG
AGGCTGGATCTTCGGCACCACCCTAGACTCCAAGACACAAAGCCTGCTGATC
GTTAACAACGCGACCAACGTGGTGATCAAGGTGTGCGAGTTTCAGTTCTGCA
ACGACCCCTTCCTGGACCACAAGAACAACAAGAGCTGGATGGAGAGCGAGT
TCAGAGTGTACAGCAGCGCCAACAACTGCACCTTCGAGTACGTGTCTCAGCC
CTTCCTGATGGACCTGGAGGGCAAGCAAGGCAATTTCAAGAACCTAAGAGA
GTTCGTGTTCAAGAACATCGACGGCTACTTCAAGATCTACAGCAAGCACACC
CCCATCATCGTGAGAGAGCCCGAGGACCTGCCCCAAGGCTTCAGCGCCCTG
GAGCCCCTGGTGGACCTGCCCATCGGCATCAACATCACAAGATTTCAGACCC
TGCTGGCCCTGCACCGTAGCTACCTAACCCCCGGCGATAGTTCCTCTGGCTGG
ACGGCCGGCGCTGCCGCCTACTACGTGGGCTACCTGCAGCCTAGAACCTTCC
TGCTGAAGTACAACGAGAACGGCACTATCACTGATGCCGTGGACTGTGCCCT
GGACCCCCTGAGCGAGACCAAGTGCACCCTCAAGAGCTTCACCGTGGAGAA
GGGCATCTATCAGACAAGCAACTTCAGAGTGCAGCCCACCGAGAGCATCGTG
AGATTCCCCAACATCACCAACCTGTGCCCCTTCGACGAGGTGTTCAACGCCA
CCAGATTTGCTAGCGTGTACGCCTGGAACCGGAAGAGAATCAGCAACTGCGT
GGCCGACTACAGCGTGCTGTACAACCTGGCCCCCTTCTTCACCTTCAAGTGC
TATGGCGTCAGCCCCACCAAGCTGAACGACCTGTGCTTCACCAACGTGTACG
CCGACAGCTTCGTGATCAGAGGCGACGAGGTGAGACAGATCGCCCCCGGGC
AGACCGGCAACATCGCCGACTACAACTACAAGCTGCCCGACGACTTCACCG
GCTGCGTGATCGCCTGGAACAGCAACAAGCTGGATAGCAAGGTGAGCGGCA
ACTACAATTACCTGTACAGACTGTTCAGAAAGAGCAACCTGAAGCCCTTCGA
GAGAGACATCAGCACCGAGATCTACCAAGCCGGCAACAAGCCCTGCAACGG
CGTGGCGGGCTTCAACTGCTACTTCCCCCTCAGGAGCTACAGCTTCAGACCC
ACCTACGGCGTGGGCCATCAGCCCTACAGAGTGGTCGTGCTGAGCTTCGAGC
TGCTGCACGCCCCTGCCACAGTGTGCGGCCCCAAAAAGAGCACCAACCTGG
TGAAGAACAAGTGCGTGAACTTCAACTTCAACGGCCTGAAGGGCACCGGCG
TGCTGACCGAGAGCAACAAGAAGTTCCTGCCATTTCAGCAATTCGGCAGAG
ACATCGCCGACACCACCGACGCGGTCAGAGACCCTCAGACCCTGGAGATCC
TGGACATCACCCCCTGTAGCTTCGGCGGCGTGAGCGTGATCACCCCCGGCAC
TAACACAAGCAACCAAGTGGCCGTGCTGTACCAAGGCGTGAACTGCACCGA
GGTGCCCGTGGCCATCCACGCCGATCAGCTGACCCCCACCTGGAGAGTGTAT
AGCACCGGCAGCAACGTGTTTCAGACAAGAGCCGGCTGCCTGATCGGCGCC
GAGTACGTGAACAACAGCTACGAGTGCGACATCCCCATCGGCGCCGGCATCT
GCGCTAGCTATCAGACACAGACCAAGAGCCACCGGAGAGCTAGAAGCGTGG
CTAGCCAAAGCATCATCGCCTACACCATGAGCCTGGGCGCCGAGAACAGCGT

GGCCTACAGCAACAACAGCATCGCCATCCCCACCAACTTCACCATCAGCGTG
ACCACCGAGATCCTGCCCGTAAGTATGACCAAGACAAGCGTGGACTGCACCA
TGTACATCTGCGGCGACAGCACCGAGTGCAGCAACCTGCTCCTGCAGTACGG
CAGCTTCTGCACACAGCTGAAGAGAGCCCTGACCGGCATCGCCGTGGAGCA
AGACAAGAACACCCAAGAGGTGTTCGCCCAAGTGAAGCAGATCTACAAGAC
CCCCCCCATCAAGTACTTCGGCGGCTTCAACTTCAGCCAAATCCTGCCCGAC
CCTAGCAAGCCTAGCAAGCGGAGCTTCATCGAGGACCTGCTGTTCAACAAG
GTGACCCTGGCCGACGCCGGCTTCATCAAGCAGTACGGCGACTGCCTAGGCG
ACATCGCAGCTAGAGACCTGATCTGCGCTCAGAAGTTCAAGGGACTCACCGT
GCTCCCCCCCTGCTGACCGACGAGATGATCGCTCAGTACACCTCTGCCCTG
CTGGCGGGAACCATAACAAGCGGTTGGACATTTGGCGCCGGGGCCGCACTG
CAAATCCCCTTCGCCATGCAGATGGCCTACAGATTCAACGGCATCGGCGTGA
CACAGAACGTGCTGTACGAGAATCAGAAGCTGATCGCCAATCAGTTCAACAG
CGCCATCGGCAAGATCCAAGACAGCCTGAGCAGCACCGCTAGCGCCCTGGG
CAAGCTGCAAGACGTGGTGAACCACAACGCCCAAGCCCTGAACACCCTGGT
GAAGCAGCTGAGCAGCAAGTTCGGCGCCATCAGCAGCGTGCTGAATGACAT
CTTCTCTAGACTGGACCCCCCCGAGGCCGAGGTGCAGATCGACAGACTGATC
ACCGGCAGACTGCAGAGCCTGCAGACCTACGTGACACAGCAGCTGATCAGA
GCCGCCGAGATCAGAGCTAGCGCCAACCTGGCCGCCACCAAGATGAGCGAG
TGCGTGCTGGGGCAGAGCAAGAGAGTGGACTTCTGCGGCAAGGGCTACCAC
CTGATGAGCTTCCCTCAGAGCGCCCCCCACGGCGTGGTGTTCCTGCACGTGA
CCTACGTGCCCGCCCAAGAGAAGAACTTCACCACCGCTCCCGCCATCTGCCA
CGACGGCAAGGCCCACTTCCCTAGAGAGGGCGTGTTCGTGAGCAACGGCAC
CCACTGGTTCGTGACACAGAGAAACTTCTACGAGCCTCAGATCATCACCACC
GACAACACCTTCGTGAGCGGCAACTGCGACGTGGTGATCGGCATAGTGAAC
AACACCGTATACGACCCCCTGCAGCCCGAGCTGGACAGCTTCAAGGAGGAG
CTGGACAAGTACTTCAAAAACCATACTAGCCCCGACGTGGACCTGGGAGACA
TCTCTGGCATCAACGCTAGCGTGGTGAACATTCAGAAGGAGATCGACCGGCT
GAATGAGGTGGCCAAGAACCTGAACGAGAGCCTGATCGACCTGCAAGAGCT
GGGCAAGTACGAGCAGTACATCAAGTGGCCCTGGTACATCTGGCTGGGCTTC
ATCGCCGGCCTGATCGCCATCGTGATGGTGACCATCATGCTGTGCTGCATGAC
AAGCTGTTGCAGCTGCCTGAAAGGTTGCTGCAGCTGTGGCTCGTGCTGCAA
GTTCGACGAGGACGACAGCGAGCCCGTGCTGAAGGGCGTGAAGCTGCACTA
CACCTGATGACTCGAGGCTCGCTTTCTTGCTGTCCAATTTCTATTAAAGGTTC
CTTTGTTCCCTAAGTCCAACTACTAAACTGGGGGATATTATGAAGGGCCTTGA
GCATCTGGATTCTGCCTAATAAAAACATTTATTTTCATTGCAAAAAAAAAA
AAAAAAAAAAAAAAAAAAAAAGCATATGACTAAAAAAAAAAAAAAAAAAAA
AAAAAAAAAAAAAAAAAAAAAAAAAAAAAAAAAAAAAAAAAAAAAAAAAAA
AAAA

SEQ ID NO: 19

>B.1.351 spike protein CDS

ATGTTCGTGTTCCTGGTGCTGCTGCCCCTGGTGAGCTCTCAGTGCGTGAACTT
CACGACAAGAACACAGCTGCCCCCCGCCTACACCAACAGCTTCACAAGAGG
CGTGTACTACCCCGACAAGGTGTTCAGAAGCAGCGTGCTGCACTCCACCCAA
GACCTGTTTCTGCCCTTCTTCAGCAACGTGACCTGGTTCCACGCCATCCACGT
GAGCGGCACCAACGGCACCAAGAGATTCGCCAACCCCGTGCTGCCCTTCAA
CGACGGCGTGTACTTCGCTAGCACCGAAAAGAGCAACATCATCAGAGGCTG
GATCTTCGGCACCACCCTAGACTCCAAGACACAAAGCCTGCTGATCGTTAAC
AACGCGACCAACGTGGTGATCAAGGTGTGCGAGTTTCAGTTCTGCAACGAC
CCCTTCCTGGGCGTGTACTACCACAAGAACAACAAGAGCTGGATGGAGAGC
GAGTTCCGTGTCTACAGCAGCGCCAACAACTGCACCTTCGAGTACGTGTCTC
AGCCCTTCCTGATGGACCTGGAGGGCAAGCAAGGCAATTTCAAGAACCTAA
GAGAGTTCGTGTTCAAGAACATCGACGGCTACTTCAAGATCTACAGCAAGCA
CACCCCCATCAACCTGGTGAGAGGCCTGCCCCAAGGCTTCAGCGCCCTGGA
GCCCTGGTGGACCTGCCCATCGGCATCAACATCACAAGATTTCAGACCCTG
CACCGTAGCTACCTAACCCCCGGCGATAGTTCCTCTGGCTGGACGGCCGGCG
CTGCCGCCTACTACGTGGGCTACCTGCAGCCTAGAACCTTCCTGCTGAAGTA
CAACGAGAACGGCACTATCACTGATGCCGTGGACTGTGCCCTGGACCCCCTG
AGCGAGACCAAGTGCACCCTCAAGAGCTTCACCGTGGAGAAGGGCATCTAT
CAGACAAGCAACTTCAGAGTGCAGCCCACCGAGAGCATCGTGAGATTCCCC
AACATCACCAACCTGTGCCCCTTCGGCGAGGTGTTCAACGCCACAAGATTCG
CTAGCGTGTACGCGTGGAACAGAAAGAGAATCAGCAACTGCGTGGCCGACT
ACAGCGTGCTGTACAACAGCGCTAGCTTCAGCACCTTCAAGTGCTATGGCGT
CAGCCCCACCAAGCTGAACGACCTGTGCTTCACCAACGTGTACGCCGACAG
CTTCGTGATCAGAGGCGACGAGGTGAGACAGATTGCCCCCGGGCAAACCGG
CAACATCGCCGACTACAACTACAAGCTGCCCGACGACTTCACCGGCTGCGTG
ATCGCGTGGAACAGCAATAACCTGGATTCCAAGGTGGGCGGCAACTACAACT
ACCTGTACAGACTGTTCAGAAAGAGCAACCTGAAGCCCTTCGAGAGAGACA
TCAGCACCGAGATCTACCAAGCCGGCAGCACCCCCTGCAACGGCGTGAAGG
GCTTCAACTGCTACTTCCCCCTGCAGAGCTACGGCTTTCAGCCCACCTACGG
CGTGGGCTATCAGCCCTACAGAGTGGTGGTGCTGAGCTTCGAGCTGCTGCAC
GCCCCCGCAACCGTGTGCGGCCCCAAAAAGAGCACCAACCTGGTGAAGAAC
AAGTGCGTGAACTTCAACTTTAATGGCCTTACCGGCACGGGCGTGCTGACCG
AGAGCAACAAGAAGTTCCTGCCATTTCAGCAATTCGGCAGAGACATCGCCG
ACACCACCGACGCGGTCAGAGACCCTCAGACCCTGGAGATCCTGGACATCA
CCCCCTGTAGCTTCGGCGGCGTGAGCGTGATCACCCCCGGCACTAACACAAG
CAACCAAGTGGCCGTGCTGTACCAAGGCGTGAACTGCACCGAGGTGCCCGT
GGCCATCCACGCCGATCAGCTGACCCCCACCTGGAGAGTATACAGCACCGGA
AGCAACGTGTTTCAGACAAGAGCCGGCTGCCTGATCGGCGCCGAGCACGTG
AACAACAGCTACGAGTGCGACATCCCCATCGGCGCCGGCATCTGCGCTAGCT
ATCAGACACAGACCAACAGCCCCCGGAGAGCTAGAAGCGTGGCTTCTCAGA
GCATCATCGCCTACACCATGAGCCTGGGCGTCGAGAACAGCGTGGCCTACAG

CAACAACAGCATCGCCATCCCCACCAACTTCACCATCAGCGTGACCACCGAG
ATCCTGCCCGTAAGTATGACCAAGACAAGCGTGGACTGCACCATGTACATCT
GCGGCGACAGCACCGAGTGCAGCAACCTGCTGCTGCAGTACGGCAGCTTCT
GCACACAGCTGAACAGAGCCCTGACCGGCATCGCCGTGGAGCAAGACAAGA
ACACCCAAGAGGTGTTCGCCCAAGTGAAGCAGATCTACAAGACCCCCCCCAT
CAAGGACTTCGGCGGCTTCAACTTCAGCCAAATCCTGCCCGACCCTAGCAAG
CCTAGCAAGCGGAGCTTCATCGAGGACCTGCTGTTCAACAAGGTGACCCTGG
CCGACGCCGGCTTCATCAAGCAGTACGGCGACTGCCTAGGCGACATCGCAGC
TAGAGACCTGATCTGCGCTCAGAAGTTCAATGGACTCACCGTGCTCCCCCCC
CTGCTGACCGACGAGATGATCGCTCAGTACACCTCTGCCCTGCTGGCGGGAA
CCATAACAAGCGGTTGGACATTTGGCGCCGGGGCCGCACTGCAAATCCCCTT
CGCCATGCAGATGGCCTACAGATTCAACGGCATCGGCGTGACACAGAACGTG
CTGTACGAGAATCAGAAGCTGATCGCCAATCAGTTCAACAGCGCCATCGGCA
AGATCCAAGACAGCCTGAGCAGCACCGCTAGCGCCCTGGGCAAGCTGCAAG
ACGTGGTGAATCAGAACGCCCAAGCCCTGAACACCCTGGTGAAGCAGCTGA
GCAGCAACTTCGGCGCCATCAGCAGCGTATTGAATGACATCCTGTCTAGACT
GGACCCCCCCGAGGCCGAGGTGCAGATCGACAGACTGATCACCGGCAGACT
GCAGAGCCTGCAGACCTACGTGACACAGCAGCTGATCAGAGCCGCCGAGAT
CAGAGCTAGCGCCAACCTGGCCGCCACCAAGATGAGCGAGTGCGTGCTGGG
GCAGAGCAAGAGAGTGGACTTCTGCGGCAAGGGCTACCACCTGATGAGCTT
CCCTCAGAGCGCCCCCACGGCGTGGTGTTCCTGCACGTGACCTACGTGCCC
GCCCAAGAGAAGAACTTCACCACCGCTCCCGCCATCTGCCACGACGGCAAG
GCCCACTTCCCTAGAGAGGGCGTGTTCGTGAGCAACGGCACCCACTGGTTCG
TGACACAGAGAAACTTCTACGAGCCTCAGATCATCACCACCGACAACACCTT
CGTGAGCGGCAACTGCGACGTGGTGATCGGCATAGTGAACAACACCGTATAC
GACCCCCTGCAGCCCGAGCTGGACAGCTTCAAGGAGGAGCTGGACAAGTAC
TTCAAAAACCATACTAGCCCCGACGTGGACCTGGGAGACATCTCTGGCATCA
ACGCTAGCGTGGTGAACATTCAGAAGGAGATCGACCGGCTGAATGAGGTGG
CCAAGAACCTGAACGAGAGCCTGATCGACCTGCAAGAGCTGGGCAAGTACG
AGCAGTACATCAAGTGGCCCTGGTACATCTGGCTGGGCTTCATCGCCGGCCT
GATCGCCATCGTGATGGTGACCATCATGCTGTGCTGCATGACAAGCTGTTGCA
GCTGCCTGAAAGGTTGCTGCAGCTGTGGCTCGTGCTGCAAGTTCGACGAGG
ACGACAGCGAGCCCGTGCTGAAGGGCGTGAAGCTGCACTACCTGATGA

SEQ ID NO: 20

>B.1.351 spike protein amino acid sequence

MFVFLVLLPLVSSQCVNFTTRTQLPPAYTNSFTRGVYYPDKVFRSSVLHSTQDLF
LPFFSNVTWFHAIHVSGTNGTKRFANPVLPFNDGVYFASTEKSNIIRGWIFGTTL
DSKTQSLLIVNNATNVVIKVCEFQFCNDPFLGVYYHKNNKSWMESEFRVYSSA
NNCTFEYVSQPFLMDLEGKQGNFKNLREFVFKNIDGYFKIYSKHTPINLVRGLP
QGFSALEPLVDLPIGINITRFQTLHRSYLTPGDSSSGWTAGAAAYYVGYLQPRTF
LLKYNENGTITDAVDCALDPLSETKCTLKSFTVEKGIYQTSNFRVQPTESIVRFPN

ITNLCPFGEVFNATRFASVYAWNRKRISNCVADYSVLYNSASFSTFKCYGVSPTK
LNDLCFTNVYADSFVIRGDEVRQIAPGQTGNIADYNYKLPDDFTGCVIAWNSNN
LDSKVGGNYNYLYRLFRKSNLKPFERDISTEIYQAGSTPCNGVKGFNCYFPLQS
YGFQPTYGVGYQPYRVVVLSFELLHAPATVCGPKKSTNLVKNKCVNFNFNGLT
GTGVLTESNKKFLPFQQFGRDIADTTDAVRDPQTLEILDITPCSFGGVSVITPGTN
TSNQVAVLYQGVNCTEVPVAIHADQLTPTWRVYSTGSNVFQTRAGCLIGAEHVN
NSYECDIPIGAGICASYQTQTNSPRRARSVASQSIIAYTMSLGVENSVAYSNNSIAI
PTNFTISVTTEILPVSMTKTSVDCTMYICGDSTECSNLLLQYGSFCTQLNRALTGI
AVEQDKNTQEVFAQVKQIYKTPPIKDFGGFNFSQILPDPSKPSKRSFIEDLLFNKV
TLADAGFIKQYGDCLGDIAARDLICAQKFNGLTVLPPLLTDEMIAQYTSALLAG
TITSGWTFGAGAALQIPFAMQMAYRFNGIGVTQNVLYENQKLIANQFNSAIGKI
QDSLSSTASALGKLQDVVNQNAQALNTLVKQLSSNFGAISSVLNDILSRLDPPEA
EVQIDRLITGRLQSLQTYVTQQLIRAAEIRASANLAATKMSECVLGQSKRVDFC
GKGYHLMSFPQSAPHGVVFLHVTYVPAQEKNFTTAPAICHDGKAHFPREGVFV
SNGTHWFVTQRNFYEPQIITTDNTFVSGNCDVVIGIVNNTVYDPLQPELDSFKEE
LDKYFKNHTSPDVDLGDISGINASVVNIQKEIDRLNEVAKNLNESLIDLQELGKY
EQYIKWPWYIWLGFIAGLIAIVMVTIMLCCMTSCCSCLKGCCSCGSCCKFDEDD
SEPVLKGVKLHYT

SEQ ID NO: 21

>B.1.1.7 spike protein CDS

ATGTTCGTGTTCCTGGTGCTGCTGCCCCTGGTGAGCTCTCAGTGCGTGAACC
TGACCACAAGAACACAGCTGCCCCCCGCCTACACCAACAGCTTCACAAGAG
GCGTGTACTACCCCGACAAGGTGTTCAGAAGCAGCGTGCTGCACTCCACCCA
AGACCTGTTTCTGCCCTTCTTCAGCAACGTGACCTGGTTCCACGCCATCAGC
GGCACCAACGGCACCAAGAGATTCGACAACCCCGTGCTGCCCTTCAACGAC
GGCGTGTACTTCGCTAGCACCGAAAAGAGCAACATCATCAGAGGCTGGATCT
TCGGCACCACCCTAGACTCCAAGACACAAAGCCTGCTGATCGTTAACAACGC
GACCAACGTGGTGATCAAGGTGTGCGAGTTTCAGTTCTGCAACGACCCCTTC
CTGGGCGTGTACCACAAGAACAACAAGAGCTGGATGGAGAGCGAGTTCCGT
GTCTACAGCAGCGCCAACAACTGCACCTTCGAGTACGTGTCTCAGCCCTTCC
TGATGGACCTGGAGGGCAAGCAAGGCAATTTCAAGAACCTAAGAGAGTTCG
TGTTCAAGAACATCGACGGCTACTTCAAGATCTACAGCAAGCACACCCCCAT
CAACCTGGTGAGAGACCTGCCCCAAGGCTTCAGCGCCCTGGAGCCCCTGGT
GGACCTGCCCATCGGCATCAACATCACAAGATTTCAGACCCTGCTGGCCCTG
CACCGTAGCTACCTAACCCCCGGCGATAGTTCCTCTGGCTGGACGGCCGGCG
CTGCCGCCTACTACGTGGGCTACCTGCAGCCTAGAACCTTCCTGCTGAAGTA
CAACGAGAACGGCACTATCACTGATGCCGTGGACTGTGCCCTGGACCCCCTG
AGCGAGACCAAGTGCACCCTCAAGAGCTTCACCGTGGAGAAGGGCATCTAT
CAGACAAGCAACTTCAGAGTGCAGCCCACCGAGAGCATCGTGAGATTCCCC
AACATCACCAACCTGTGCCCCTTCGGCGAGGTGTTCAACGCCACAAGATTCG
CTAGCGTGTACGCGTGGAACAGAAAGAGAATCAGCAACTGCGTGGCCGACT

ACAGCGTGCTGTACAACAGCGCTAGCTTCAGCACCTTCAAGTGCTATGGCGT
CAGCCCCACCAAGCTGAACGACCTGTGCTTCACCAACGTGTACGCCGACAG
CTTCGTGATCAGAGGCGACGAGGTGAGACAGATTGCCCCCGGGCAAACCGG
CAAGATCGCCGACTACAACTACAAGCTGCCCGACGACTTCACCGGCTGCGTG
ATCGCGTGGAACAGCAATAACCTGGATTCCAAGGTGGGCGGCAACTACAACT
ACCTGTACAGACTGTTCAGAAAGAGCAACCTGAAGCCCTTCGAGAGAGACA
TCAGCACCGAGATCTACCAAGCCGGCAGCACCCCCTGCAACGGCGTGGAGG
GCTTCAACTGCTACTTCCCCCTGCAGAGCTACGGCTTTCAGCCCACCTACGG
CGTGGGCTATCAGCCCTACAGAGTGGTGGTGCTGAGCTTCGAGCTGCTGCAC
GCCCCCGCAACCGTGTGCGGCCCCAAAAAGAGCACCAACCTGGTGAAGAAC
AAGTGCGTGAACTTCAACTTTAATGGCCTTACCGGCACGGGCGTGCTGACCG
AGAGCAACAAGAAGTTCCTGCCATTTCAGCAATTCGGCAGAGACATCGACG
ACACCACCGACGCGGTCAGAGACCCTCAGACCCTGGAGATCCTGGACATCA
CCCCCTGTAGCTTCGGCGGCGTGAGCGTGATCACCCCCGGCACTAACACAAG
CAACCAAGTGGCCGTGCTGTACCAAGGCGTGAACTGCACCGAGGTGCCCGT
GGCCATCCACGCCGATCAGCTGACCCCCACCTGGAGAGTATACAGCACCGGA
AGCAACGTGTTTCAGACAAGAGCCGGCTGCCTGATCGGCGCCGAGCACGTG
AACAACAGCTACGAGTGCGACATCCCCATCGGCGCCGGCATCTGCGCTAGCT
ATCAGACACAGACCAACAGCCACCGGAGAGCTAGAAGCGTGGCTTCTCAGA
GCATCATCGCCTACACCATGAGCCTGGGCGCCGAGAACAGCGTGGCCTACAG
CAACAACAGCATCGCCATCCCCATCAACTTCACCATCAGCGTGACCACCGAG
ATCCTGCCCGTAAGTATGACCAAGACAAGCGTGGACTGCACCATGTACATCT
GCGGCGACAGCACCGAGTGCAGCAACCTGCTGCTGCAGTACGGCAGCTTCT
GCACACAGCTGAACAGAGCCCTGACCGGCATCGCCGTGGAGCAAGACAAGA
ACACCCAAGAGGTGTTCGCCCAAGTGAAGCAGATCTACAAGACCCCCCCCAT
CAAGGACTTCGGCGGCTTCAACTTCAGCCAAATCCTGCCCGACCCTAGCAAG
CCTAGCAAGCGGAGCTTCATCGAGGACCTGCTGTTCAACAAGGTGACCCTGG
CCGACGCCGGCTTCATCAAGCAGTACGGCGACTGCCTAGGCGACATCGCAGC
TAGAGACCTGATCTGCGCTCAGAAGTTCAATGGACTCACCGTGCTCCCCCCC
CTGCTGACCGACGAGATGATCGCTCAGTACACCTCTGCCCTGCTGGCGGGAA
CCATAACAAGCGGTTGGACATTTGGCGCCGGGGCCGCACTGCAAATCCCCTT
CGCCATGCAGATGGCCTACAGATTCAACGGCATCGGCGTGACACAGAACGTG
CTGTACGAGAATCAGAAGCTGATCGCCAATCAGTTCAACAGCGCCATCGGCA
AGATCCAAGACAGCCTGAGCAGCACCGCTAGCGCCCTGGGCAAGCTGCAAG
ACGTGGTGAATCAGAACGCCCAAGCCCTGAACACCCTGGTGAAGCAGCTGA
GCAGCAACTTCGGCGCCATCAGCAGCGTATTGAATGACATCCTGGCTAGACT
GGACCCCCCCGAGGCCGAGGTGCAGATCGACAGACTGATCACCGGCAGACT
GCAGAGCCTGCAGACCTACGTGACACAGCAGCTGATCAGAGCCGCCGAGAT
CAGAGCTAGCGCCAACCTGGCCGCCACCAAGATGAGCGAGTGCGTGCTGGG
GCAGAGCAAGAGAGTGGACTTCTGCGGCAAGGGCTACCACCTGATGAGCTT
CCCTCAGAGCGCCCCCCACGGCGTGGTGTTCCTGCACGTGACCTACGTGCCC

GCCCAAGAGAAGAACTTCACCACCGCTCCCGCCATCTGCCACGACGGCAAG
GCCCACTTCCCTAGAGAGGGCGTGTTCGTGAGCAACGGCACCCACTGGTTCG
TGACACAGAGAAACTTCTACGAGCCTCAGATCATCACCACCCACAACACCTT
CGTGAGCGGCAACTGCGACGTGGTGATCGGCATAGTGAACAACACCGTATAC
GACCCCCTGCAGCCCGAGCTGGACAGCTTCAAGGAGGAGCTGGACAAGTAC
TTCAAAAACCATACTAGCCCCGACGTGGACCTGGGAGACATCTCTGGCATCA
ACGCTAGCGTGGTGAACATTCAGAAGGAGATCGACCGGCTGAATGAGGTGG
CCAAGAACCTGAACGAGAGCCTGATCGACCTGCAAGAGCTGGGCAAGTACG
AGCAGTACATCAAGTGGCCCTGGTACATCTGGCTGGGCTTCATCGCCGGCCT
GATCGCCATCGTGATGGTGACCATCATGCTGTGCTGCATGACAAGCTGTTGCA
GCTGCCTGAAAGGTTGCTGCAGCTGTGGCTCGTGCTGCAAGTTCGACGAGG
ACGACAGCGAGCCCGTGCTGAAGGGCGTGAAGCTGCACTACCTGATGA

SEQ ID NO: 22

>B.1.1.7 spike protein amino acid sequence

MFVFLVLLPLVSSQCVNLTTRTQLPPAYTNSFTRGVYYPDKVFRSSVLHSTQDLF
LPFFSNVTWFHAISGTNGTKRFDNPVLPFNDGVYFASTEKSNIIRGWIFGTTLDS
KTQSLLIVNNATNVVIKVCEFQFCNDPFLGVYHKNNKSWMESEFRVYSSANNC
TFEYVSQPFLMDLEGKQGNFKNLREFVFKNIDGYFKIYSKHTPINLVRDLPQGFS
ALEPLVDLPIGINITRFQTLLALHRSYLTPGDSSSGWTAGAAAYYVGYLQPRTFL
LKYNENGTITDAVDCALDPLSETKCTLKSFTVEKGIYQTSNFRVQPTESIVRFPNI
TNLCPFGEVFNATRFASVYAWNRKRISNCVADYSVLYNSASFSTFKCYGVSPTKL
NDLCFTNVYADSFVIRGDEVRQIAPGQTGKIADYNYKLPDDFTGCVIAWNSNNL
DSKVGGNYNYLYRLFRKSNLKPFERDISTEIYQAGSTPCNGVEGFNCYFPLQSY
GFQPTYGVGYQPYRVVVLSFELLHAPATVCGPKKSTNLVKNKCVNFNFNGLTG
TGVLTESNKKFLPFQQFGRDIDDTTDAVRDPQTLEILDITPCSFGGVSVITPGTNT
SNQVAVLYQGVNCTEVPVAIHADQLTPTWRVYSTGSNVFQTRAGCLIGAEHVN
NSYECDIPIGAGICASYQTQTNSHRRARSVASQSIIAYTMSLGAENSVAYSNNSIAI
PINFTISVTTEILPVSMTKTSVDCTMYICGDSTECSNLLLQYGSFCTQLNRALTGI
AVEQDKNTQEVFAQVKQIYKTPPIKDFGGFNFSQILPDPSKPSKRSFIEDLLFNKV
TLADAGFIKQYGDCLGDIAARDLICAQKFNGLTVLPPLLTDEMIAQYTSALLAG
TITSGWTFGAGAALQIPFAMQMAYRFNGIGVTQNVLYENQKLIANQFNSAIGKI
QDSLSSTASALGKLQDVVNQNAQALNTLVKQLSSNFGAISSVLNDILARLDPPE
AEVQIDRLITGRLQSLQTYVTQQLIRAAEIRASANLAATKMSECVLGQSKRVDF
CGKGYHLMSFPQSAPHGVVFLHVTYVPAQEKNFTTAPAICHDGKAHFPREGVF
VSNGTHWFVTQRNFYEPQIITTHNTFVSGNCDVVIGIVNNTVYDPLQPELDSFKE
ELDKYFKNHTSPDVDLGDISGINASVVNIQKEIDRLNEVAKNLNESLIDLQELGK
YEQYIKWPWYIWLGFIAGLIAIVMVTIMLCCMTSCCSCLKGCCSCGSCCKFDED
DSEPVLKGVKLHYT

SEQ ID NO: 23

>SARS-CoV-2 spike protein CDS

ATGTTCGTGTTCCTGGTGCTGCTGCCCCTGGTGAGCTCTCAGTGCGTGAACC

TGACCACAAGAACACAGCTGCCCCCCGCCTACACCAACAGCTTCACAAGAG
GCGTGTACTACCCCGACAAGGTGTTCAGAAGCAGCGTGCTGCACTCCACCCA
AGACCTGTTTCTGCCCTTCTTCAGCAACGTGACCTGGTTCCACGCCATCCAC
GTGAGCGGCACCAACGGCACCAAGAGATTCGACAACCCCGTGCTGCCCTTC
AACGACGGCGTGTACTTCGCTAGCACCGAAAAGAGCAACATCATCAGAGGC
TGGATCTTCGGCACCACCCTAGACTCCAAGACACAAAGCCTGCTGATCGTTA
ACAACGCGACCAACGTGGTGATCAAGGTGTGCGAGTTTCAGTTCTGCAACG
ACCCCTTCCTGGGCGTGTACTACCACAAGAACAACAAGAGCTGGATGGAGA
GCGAGTTCCGTGTCTACAGCAGCGCCAACAACTGCACCTTCGAGTACGTGTC
TCAGCCCTTCCTGATGGACCTGGAGGGCAAGCAAGGCAATTTCAAGAACCTA
AGAGAGTTCGTGTTCAAGAACATCGACGGCTACTTCAAGATCTACAGCAAGC
ACACCCCCATCAACCTGGTGAGAGACCTGCCCCAAGGCTTCAGCGCCCTGG
AGCCCCTGGTGGACCTGCCCATCGGCATCAACATCACAAGATTTCAGACCCT
GCTGGCCCTGCACCGTAGCTACCTAACCCCCGGCGATAGTTCCTCTGGCTGG
ACGGCCGGCGCTGCCGCCTACTACGTGGGCTACCTGCAGCCTAGAACCTTCC
TGCTGAAGTACAACGAGAACGGCACTATCACTGATGCCGTGGACTGTGCCCT
GGACCCCCTGAGCGAGACCAAGTGCACCCTCAAGAGCTTCACCGTGGAGAA
GGGCATCTATCAGACAAGCAACTTCAGAGTGCAGCCCACCGAGAGCATCGTG
AGATTCCCCAACATCACCAACCTGTGCCCCTTCGGCGAGGTGTTCAACGCCA
CAAGATTCGCTAGCGTGTACGCGTGGAACAGAAAGAGAATCAGCAACTGCG
TGGCCGACTACAGCGTGCTGTACAACAGCGCTAGCTTCAGCACCTTCAAGTG
CTATGGCGTCAGCCCCACCAAGCTGAACGACCTGTGCTTCACCAACGTGTAC
GCCGACAGCTTCGTGATCAGAGGCGACGAGGTGAGACAGATTGCCCCCGGG
CAAACCGGCAAGATCGCCGACTACAACTACAAGCTGCCCGACGACTTCACC
GGCTGCGTGATCGCGTGGAACAGCAATAACCTGGATTCCAAGGTGGGCGGC
AACTACAACTACCTGTACAGACTGTTCAGAAAGAGCAACCTGAAGCCCTTCG
AGAGAGACATCAGCACCGAGATCTACCAAGCCGGCAGCACCCCCTGCAACG
GCGTGGAGGGCTTCAACTGCTACTTCCCCCTGCAGAGCTACGGCTTTCAGCC
CACCAATGGCGTGGGCTATCAGCCCTACAGAGTGGTGGTGCTGAGCTTCGAG
CTGCTGCACGCCCCCGCAACCGTGTGCGGCCCCAAAAAGAGCACCAACCTG
GTGAAGAACAAGTGCGTGAACTTCAACTTTAATGGCCTTACCGGCACGGGCG
TGCTGACCGAGAGCAACAAGAAGTTCCTGCCATTTCAGCAATTCGGCAGAG
ACATCGCCGACACCACCGACGCGGTCAGAGACCCTCAGACCCTGGAGATCC
TGGACATCACCCCCTGTAGCTTCGGCGGCGTGAGCGTGATCACCCCCGGCAC
TAACACAAGCAACCAAGTGGCCGTGCTGTACCAAGACGTGAACTGCACCGA
GGTGCCCGTGGCCATCCACGCCGATCAGCTGACCCCCACCTGGAGAGTATAC
AGCACCGGAAGCAACGTGTTTCAGACAAGAGCCGGCTGCCTGATCGGCGCC
GAGCACGTGAACAACAGCTACGAGTGCGACATCCCCATCGGCGCCGGCATCT
GCGCTAGCTATCAGACACAGACCAACAGCCCTCGGAGAGCTAGAAGCGTGG
CTTCTCAGAGCATCATCGCCTACACCATGAGCCTGGGCGCCGAGAACAGCGT
GGCCTACAGCAACAACAGCATCGCCATCCCCACCAACTTCACCATCAGCGTG

ACCACCGAGATCCTGCCCGTAAGTATGACCAAGACAAGCGTGGACTGCACCA
TGTACATCTGCGGCGACAGCACCGAGTGCAGCAACCTGCTGCTGCAGTACGG
CAGCTTCTGCACACAGCTGAACAGAGCCCTGACCGGCATCGCCGTGGAGCA
AGACAAGAACACCCAAGAGGTGTTCGCCCAAGTGAAGCAGATCTACAAGAC
CCCCCCCATCAAGGACTTCGGCGGCTTCAACTTCAGCCAAATCCTGCCCGAC
CCTAGCAAGCCTAGCAAGCGGAGCTTCATCGAGGACCTGCTGTTCAACAAG
GTGACCCTGGCCGACGCCGGCTTCATCAAGCAGTACGGCGACTGCCTAGGCG
ACATCGCAGCTAGAGACCTGATCTGCGCTCAGAAGTTCAATGGACTCACCGT
GCTCCCCCCCCTGCTGACCGACGAGATGATCGCTCAGTACACCTCTGCCCTG
CTGGCGGGAACCATAACAAGCGGTTGGACATTTGGCGCCGGGGCCGCACTG
CAAATCCCCTTCGCCATGCAGATGGCCTACAGATTCAACGGCATCGGCGTGA
CACAGAACGTGCTGTACGAGAATCAGAAGCTGATCGCCAATCAGTTCAACAG
CGCCATCGGCAAGATCCAAGACAGCCTGAGCAGCACCGCTAGCGCCCTGGG
CAAGCTGCAAGACGTGGTGAATCAGAACGCCCAAGCCCTGAACACCCTGGT
GAAGCAGCTGAGCAGCAACTTCGGCGCCATCAGCAGCGTATTGAATGACATC
CTGAGCAGACTGGACCCCCCCGAGGCCGAGGTGCAGATCGACAGACTGATC
ACCGGCAGACTGCAGAGCCTGCAGACCTACGTGACACAGCAGCTGATCAGA
GCCGCCGAGATCAGAGCTAGCGCCAACCTGGCCGCCACCAAGATGAGCGAG
TGCGTGCTGGGGCAGAGCAAGAGAGTGGACTTCTGCGGCAAGGGCTACCAC
CTGATGAGCTTCCCTCAGAGCGCCCCCCACGGCGTGGTGTTCCTGCACGTGA
CCTACGTGCCCGCCCAAGAGAAGAACTTCACCACCGCTCCCGCCATCTGCCA
CGACGGCAAGGCCCACTTCCCTAGAGAGGGCGTGTTCGTGAGCAACGGCAC
CCACTGGTTCGTGACACAGAGAAACTTCTACGAGCCTCAGATCATCACCACC
GACAACACCTTCGTGAGCGGCAACTGCGACGTGGTGATCGGCATAGTGAAC
AACACCGTATACGACCCCCTGCAGCCCGAGCTGGACAGCTTCAAGGAGGAG
CTGGACAAGTACTTCAAAAACCATACTAGCCCCGACGTGGACCTGGGAGACA
TCTCTGGCATCAACGCTAGCGTGGTGAACATTCAGAAGGAGATCGACCGGCT
GAATGAGGTGGCCAAGAACCTGAACGAGAGCCTGATCGACCTGCAAGAGCT
GGGCAAGTACGAGCAGTACATCAAGTGGCCCTGGTACATCTGGCTGGGCTTC
ATCGCCGGCCTGATCGCCATCGTGATGGTGACCATCATGCTGTGCTGCATGAC
AAGCTGTTGCAGCTGCCTGAAAGGTTGCTGCAGCTGTGGCTCGTGCTGCAA
GTTCGACGAGGACGACAGCGAGCCCGTGCTGAAGGGCGTGAAGCTGCACTA
CACCTGATGA

SEQ ID NO: 24

>SARS-CoV-2 spike protein amino acid sequence

MFVFLVLLPLVSSQCVNLTTRTQLPPAYTNSFTRGVYYPDKVFRSSVLHSTQDLF
LPFFSNVTWFHAIHVSGTNGTKRFDNPVLPFNDGVYFASTEKSNIIRGWIFGTTL
DSKTQSLLIVNNATNVVIKVCEFQFCNDPFLGVYYHKNNKSWMESEFRVYSSA
NNCTFEYVSQPFLMDLEGKQGNFKNLREFVFKNIDGYFKIYSKHTPINLVRDLP
QGFSALEPLVDLPIGINITRFQTLLALHRSYLTPGDSSSGWTAGAAAYYVGYLQP
RTFLLKYNENGTITDAVDCALDPLSETKCTLKSFTVEKGIYQTSNFRVQPTESIVR

FPNITNLCPFGEVFNATRFASVYAWNRKRISNCVADYSVLYNSASFSTFKCYGVS
PTKLNDLCFTNVYADSFVIRGDEVRQIAPGQTGKIADYNYKLPDDFTGCVIAWN
SNNLDSKVGGNYNYLYRLFRKSNLKPFERDISTEIYQAGSTPCNGVEGFNCYFP
LQSYGFQPTNGVGYQPYRVVVLSFELLHAPATVCGPKKSTNLVKNKCVNFNFN
GLTGTGVLTESNKKFLPFQQFGRDIADTTDAVRDPQTLEILDITPCSFGGVSVITP
GTNTSNQVAVLYQDVNCTEVPVAIHADQLTPTWRVYSTGSNVFQTRAGCLIGAE
HVNNSYECDIPIGAGICASYQTQTNSPRRARSVASQSIIAYTMSLGAENSVAYSN
NSIAIPTNFTISVTTEILPVSMTKTSVDCTMYICGDSTECSNLLLQYGSFCTQLNR
ALTGIAVEQDKNTQEVFAQVKQIYKTPPIKDFGGFNFSQILPDPSKPSKRSFIEDLL
FNKVTLADAGFIKQYGDCLGDIAARDLICAQKFNGLTVLPPLLTDEMIAQYTSA
LLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLD
PPEAEVQIDRLITGRLQSLQTYVTQQLIRAAEIRASANLAATKMSECVLGQSKRV
DFCGKGYHLMSFPQSAPHGVVFLHVTYVPAQEKNFTTAPAICHDGKAHFPREG
VFVSNGTHWFVTQRNFYEPQIITTDNTFVSGNCDVVIGIVNNTVYDPLQPELDSF
KEELDKYFKNHTSPDVDLGDISGINASVVNIQKEIDRLNEVAKNLNESLIDLQEL
GKYEQYIKWPWYIWLGFIAGLIAIVMVTIMLCCMTSCCSCLKGCCSCGSCCKFD
EDDSEPVLKGVKLHYT

SEQ ID NO: 25

>Luciferase CDS

ATGGAGGACGCCAAGAACATCAAGAAAGGCCCCGCCCCCTTCTACCCCCTG
GAGGACGGCACCGCCGGCGAGCAGCTGCACAAAGCCATGAAAAGATACGC
TCTGGTGCCCGGCACCATCGCCTTCACCGACGCCCACATCGAGGTGAACAT
CACCTACGCCGAGTACTTCGAGATGAGCGTGAGACTGGCCGAGGCTATGAA
GAGATACGGCTTGAACACCAACCACAGAATCGTGGTGTGCAGCGAGAACA
GCCTGCAGTTCTTCATGCCCGTGCTGGGCGCCCTGTTCATCGGCGTGGCCGT
TGCCCCCGCCAACGACATCTACAACGAGAGAGAGCTGCTGAACAGCATGAA
CATCTCTCAGCCCACCGTGGTTTTCGTGTCCAAAAAGGGCCTGCAGAAGAT
CCTGAACGTGCAGAAGAAGCTGCCCATCATTCAGAAGATCATCATCATGGAC
AGCAAGACCGACTACCAAGGCTTTCAGAGCATGTACACCTTCGTGACAAGC
CACCTGCCCCCCGGCTTCAACGAGTACGACTTCGTGCCCGAGAGCTTCGAC
AGAGACAAGACCATCGCCCTGATCATGAACAGCAGCGGCAGCACCGGCCTG
CCCAAGGGCGTGGCCCTGCCCCACAGAACCGCCTGCGTGAGATTCAGCCAC
GCTAGAGACCCCATCTTCGGCAATCAGATCATCCCCGACACCGCCATCCTGA
GCGTAGTGCCTTTCCATCATGGCTTCGGCATGTTCACCACCCTGGGCTACCT
GATCTGCGGCTTCAGAGTGGTGCTGATGTACAGATTCGAGGAGGAGCTGTT
CCTGAGAAGCCTGCAAGACTACAAGATTCAGAGCGCCCTGCTGGTGCCCAC
CCTGTTCAGCTTCTTCGCCAAGAGCACCCTGATCGACAAGTATGACCTGAGC

AACCTGCACGAAATTGCGAGCGGCGGAGCGCCGCTCAGCAAAGAGGTGGG
CGAGGCCGTGGCCAAGAGATTCCACCTGCCCGGCATCAGACAAGGCTACGG
CCTGACCGAGACCACAAGCGCCATCCTGATCACCCCCGAGGGCGACGATAA
GCCCGGCGCCGTGGGCAAGGTGGTACCATTCTTCGAGGCCAAGGTGGTGGA
CCTGGACACCGGCAAGACCCTGGGCGTGAATCAGAGAGGCGAGCTGTGCG
TGAGAGGCCCCATGATCATGAGCGGCTACGTGAACAACCCCGAGGCCACCA
ACGCCCTCATCGATAAGGATGGGTGGCTGCACAGCGGCGACATCGCCTACT
GGGACGAGGACGAGCACTTCTTCATCGTGGACAGACTCAAGAGCCTGATCA
AGTACAAGGGCTACCAAGTGGCCCCCGCCGAGTTGGAGAGCATCCTGCTGC
AGCACCCCAATATCTTCGACGCTGGCGTGGCCGGACTACCCGACGACGACG
CCGGCGAGTTACCCGCCGCCGTGGTGGTGCTGGAGCACGGCAAGACCATGA
CCGAGAAGGAGATCGTGGACTACGTGGCTAGCCAAGTGACCACCGCCAAG
AAGCTGAGAGGCGGCGTGGTGTTCGTTGACGAAGTGCCCAAGGGCCTGAC
CGGCAAGCTGGACGCTAGAAAGATCAGAGAGATCCTGATCAAGGCCAAGA
AGGGCGGCAAGAGCAAGCTGTGA

SEQ ID NO: 26

>Luciferase amino acid sequence

MEDAKNIKKGPAPFYPLEDGTAGEQLHKAMKRYALVPGTIAFTDAHIEVNITYA
EYFEMSVRLAEAMKRYGLNTNHRIVVCSENSLQFFMPVLGALFIGVAVAPANDI
YNERELLNSMNISQPTVVFVSKKGLQKILNVQKKLPIIQKIIIMDSKTDYQGFQS
MYTFVTSHLPPGFNEYDFVPESFDRDKTIALIMNSSGSTGLPKGVALPHRTACVR
FSHARDPIFGNQIIPDTAILSVVPFHHGFGMFTTLGYLICGFRVVLMYRFEEELFL
RSLQDYKIQSALLVPTLFSFFAKSTLIDKYDLSNLHEIASGGAPLSKEVGEAVAKR
FHLPGIRQGYGLTETTSAILITPEGDDKPGAVGKVVPFFEAKVVDLDTGKTLGVN
QRGELCVRGPMIMSGYVNNPEATNALIDKDGWLHSGDIAYWDEDEHFFIVDRL
KSLIKYKGYQVAPAELESILLQHPNIFDAGVAGLPDDDAGELPAAVVVLEHGKT
MTEKEIVDYVASQVTTAKKLRGGVVFVDEVPKGLTGKLDARKIREILIKAKKGG
KSKL

SEQ ID NO: 27

>LUC1- I' truncation

TAGGAGTTGGTGGCAGTTTCAATAACAGGTCATTGCCGAGAAAAGGATAGCA
CTATAAT

SEQ ID NO: 28

>LUC2- I' truncation

AGTTGGTGGCAGTTTCAATAACAGGTCATTGCCGAGAAAAGGATAGCACTAT
AAT

SEQ ID NO: 29

>LUC3- I' truncation
GGTGGCAGTTTCAATAACAGGTCATTGCCGAGAAAAGGATAGCACTATAAT
SEQ ID NO: 30
>LUC4- I' truncation
GCAGTTTCAATAACAGGTCATTGCCGAGAAAAGGATAGCACTATAAT
SEQ ID NO: 31
>LUC5- I' truncation
TTTCAATAACAGGTCATTGCCGAGAAAAGGATAGCACTATAAT
SEQ ID NO: 32
>LUC6- I' truncation
AATAACAGGTCATTGCCGAGAAAAGGATAGCACTATAAT
SEQ ID NO: 33
> LUC7 - I' truncation
ACAGGTCATTGCCGAGAAAAGGATAGCACTATAAT
SEQ ID NO: 34
>LUC8- I' truncation
GTCATTGCCGAGAAAAGGATAGCACTATAAT
SEQ ID NO: 35
>LUC9- I' truncation
TTGCCGAGAAAAGGATAGCACTATAAT
SEQ ID NO: 36
>LUC10- I' truncation
CGAGAAAAGGATAGCACTATAAT

SEQ ID NO: 37
>LUC11- I' truncation
AAAAGGATAGCACTATAAT
SEQ ID NO: 38
>LUC12- I' truncation
GGATAGCACTATAAT
SEQ ID NO: 39
>LUC13- I' truncation
AGCACTATAAT
SEQ ID NO: 40
>LUC14- I' truncation
CTATAAT
> LUC15- I' mutant

GAGTTAGGAGTTG**C**TGGCAGTTTCAATAACAGGTCATTGCCGAGAAAAGGA
TAGCACTATAAT

<div align="right">SEQ ID NO: 42</div>

>LUC16- I' mutant

GAGTTAGGAGTTG**T**TGGCAGTTTCAATAACAGGTCATTGCCGAGAAAAGGA
TAGCACTATAAT

<div align="right">SEQ ID NO: 43</div>

>5'UTR I general formula

GAGTTAGGAGTTGN$_1$TGGCAGTTTCAATAACAGGTCATTGCCGAGAAAAGGA
TAGCACTATAAT

<div align="right">SEQ ID NO: 44</div>

N$_1$ is selected from the group consisting of A, G, C, and T

The following SEQ ID NOs: 45-79 are RNA sequences.

>5'UTR I (63bp)

GAGUUAGGAGUUGAUGGCAGUUUCAAUAACAGGUCAUUGCCGAGAAAAG
GAUAGCACUAUAAU

<div align="right">SEQ ID NO: 45</div>

>5'UTR I' (63bp)

GAGUUAGGAGUUGGUGGCAGUUUCAAUAACAGGUCAUUGCCGAGAAAAG
GAUAGCACUAUAAU

<div align="right">SEQ ID NO: 46</div>

>5'UTR G

GCACUUUCUGAGCAGACGUCCAGAGCAGAGUCAGCCGCCACC

<div align="right">SEQ ID NO: 47</div>

>5'UTR A

ACAUUUGCUUCUGACACAACUGUGUUCACUAGCAACCUCAAACGCCGCCA
CC

SEQ ID NO: 48

>5'UTR J

AGAGAGGCAAAGAAACAUUGUGAAAUCUCCAACUCUUAACCUUCAAGCC
GCCACC

SEQ ID NO: 49

>3'UTR B

GCUCGCUUUCUUGCUGUCCAAUUUCUAUUAAAGGUUCCUUUGUUCCCUAA
GUCCAACUACUAAACUGGGGGAUAUUAUGAAGGGCCUUGAGCAUCUGGA
UUCUGCCUAAUAAAAAACAUUUAUUUUCAUUGCAA

SEQ ID NO: 50

>3'UTR F

CAGACAAGACAAGCUACUGAAUACGUUCACAUCUGUCUUGAUGCCUAAU
AUUUUUACAUUUCUGUAAACAUAUUUCUGAAAUAUUUUUUGCCUUUCAA
GCGACAGAUGCCUCAUUUUGUGAAACUUAAUGAUGAUUUUGUGUUUAA
GUUCCAAACAUUUGAAUAAAAUAAUUGACAAUA

SEQ ID NO: 51

>3'UTR E

CCUGAGGCCUCAGAACACAGGUUUCAGAUUGAUAGGCCUGCAGGUCUCCA
GGCAGCAACCAGCUGAGCGACUAAAGGGCCCAAGGCCAGGGCUCUAGGGA
UGGGGCUCAGCAGAGGCUGGGGUAAGGGGAGCCAGGGAGGAGCUGGGCC
UAAUGCAGCACCGGGUCCCCAGGAUGCCGUGUCUCGGCUGGAGGAGGAGA
UGCGGAAGCUCCAGGCCACGGUGCAGGAGCUCCAGAAGCGCUUGGACAGG
CUGGAGGAGACAGUCCAGGCCAAGUAGAGCCCCGCAGGGCCUCCAGCAGG
GUCAGCCAUUCACACCCAUCCACUCACCUCCCAUUCCCAGCCACAUGGCA
GAGAAAAAAUCAUAAUAAAAUGGCUUUAUUUUCUGGUA

SEQ ID NO: 52

>3'UTR D

GGGGCCUUCUGACAUGAGUCUGGCCUGGCCCCACCUCCUAGUUCCUCAUA
AUAAAGACAGAUUGCUUCUUCGCUUCUCACUGAGGGGCCUUCUGACAUG
AGUCUGGCCUGGCCCCACCUCCCCAGUUUCUCAUAAUAAAGACAGAUUGC
UUCUUCACUUGAAGCUCGCUUUCUUGCUGUCCAAUUUCUAUUAAAGGUU
CCUUUGUUCCCUAAGUCCAACUACUAAACUGGGGGAUAUUAUGAAGGGC
CUUGAGCAUCUGGAUUCUGCCUAAUAAAAACAUUUAUUUUCAUUGCAA

SEQ ID NO: 53

>B.1.617.2 (Delta) spike protein mRNA

AUGUUCGUGUUCCUGGUGCUGCUGCCCCUGGUGAGCUCUCAGUGCGUGAA
CCUGAGGACAAGAACACAGCUGCCCCCGCCUACACCAACAGCUUCACAA
GAGGCGUGUACUACCCCGACAAGGUGUUCAGAAGCAGCGUGCUGCACUCC
ACCCAAGACCUGUUUCUGCCCUUCUUCAGCAACGUGACCUGGUUCCACGC
CAUCCACGUCAGCGGCACCAACGGCACCAAGAGAUUCGACAACCCCGUGC
UGCCCUUCAACGACGGCGUGUACUUCGCUAGCACCGAAAAGAGCAACAUC
AUCAGAGGCUGGAUCUUCGGCACCACCCUAGACUCCAAGACACAAAGCCU
GCUGAUCGUUAACAACGCGACCAACGUGGUGAUCAAGGUGUGCGAGUUU
CAGUUCUGCAACGACCCCUUCCUGGACGUGUACUACCACAAGAACAACAA
GAGCUGGAUGGAGAGCGGGGUCUACAGCAGCGCCAACAACUGCACCUUCG
AGUACGUGUCUCAGCCCUUCCUGAUGGACCUGGAGGGCAAGCAAGGCAAU
UUCAAGAACCUAAGAGAGUUCGUGUUCAAGAACAUCGACGGCUACUUCA
AGAUCUACAGCAAGCACACCCCCAUCAACCUGGUGAGAGACCUGCCCCAA
GGCUUCAGCGCCCUGGAGCCCCUGGUGGACCUGCCCAUCGGCAUCAACAU
CACAAGAUUUCAGACCCUGCUGGCCCUGCACCGUAGCUACCUAACCCCCG
GCGAUAGUUCCUCUGGCUGGACGGCCGGCGCUGCCGCCUACUACGUGGGC
UACCUGCAGCCUAGAACCUUCCUGCUGAAGUACAACGAGAACGGCACUAU
CACUGAUGCCGUGGACUGUGCCCUGGACCCCCUGAGCGAGACCAAGUGCA
CCCUCAAGAGCUUCACCGUGGAGAAGGGCAUCUAUCAGACAAGCAACUUC
AGAGUGCAGCCCACCGAGAGCAUCGUGAGAUUCCCCAACAUCACCAACCU
GUGCCCCUUCGGCGAGGUGUUCAACGCCACAAGAUUCGCUAGCGUGUACG

CGUGGAACAGAAAGAGAAUCAGCAACUGCGUGGCCGACUACAGCGUGCU
GUACAACAGCGCUAGCUUCAGCACCUUCAAGUGCUAUGGCGUCAGCCCCA
CCAAGCUGAACGACCUGUGCUUCACCAACGUGUACGCCGACAGCUUCGUG
AUCAGAGGCGACGAGGUGAGACAGAUUGCCCCCGGGCAAACCGGCAAGAU
CGCCGACUACAACUACAAGCUGCCCGACGACUUCACCGGCUGCGUGAUCG
CGUGGAACAGCAAUAACCUGGAUUCCAAGGUGGGCGGCAACUACAACUAC
CGGUACAGACUGUUCAGAAAGAGCAACCUGAAGCCCUUCGAGAGAGACA
UCAGCACCGAGAUCUACCAAGCCGGCAGCAAGCCCUGCAACGGCGUGGAG
GGCUUCAACUGCUACUUCCCCCUGCAGAGCUACGGCUUUCAGCCCACCAA
CGGCGUGGGCUAUCAGCCCUACAGAGUGGUGGUGCUGAGCUUCGAGCUGC
UGCACGCCCCCGCAACCGUGUGCGGCCCCAAAAAGAGCACCAACCUGGUG
AAGAACAAGUGCGUGAACUUCAACUUUAAUGGCCUUACCGGCACGGGCG
UGCUGACCGAGAGCAACAAGAAGUUCCUGCCAUUUCAGCAAUUCGGCAGA
GACAUCGCCGACACCACCGACGCGGUCAGAGACCCUCAGACCCUGGAGAU
CCUGGACAUCACCCCCUGUAGCUUCGGCGGCGUGAGCGUGAUCACCCCCG
GCACUAACACAAGCAACCAAGUGGCCGUGCUGUACCAAGGCGUGAACUGC
ACCGAGGUGCCCGUGGCCAUCCACGCCGAUCAGCUGACCCCCACCUGGAG
AGUAUACAGCACCGGAAGCAACGUGUUUCAGACAAGAGCCGGCUGCCUGA
UCGGCGCCGAGCACGUGAACAACAGCUACGAGUGCGACAUCCCCAUCGGC
GCCGGCAUCUGCGCUAGCUAUCAGACACAGACCAACAGCCGCCGGAGAGC
UAGAAGCGUGGCUUCUCAGAGCAUCAUCGCCUACACCAUGAGCCUGGGCG
CCGAGAACAGCGUGGCCUACAGCAACAACAGCAUCGCCAUCCCCACCAAC
UUCACCAUCAGCGUGACCACCGAGAUCCUGCCCGUAAGUAUGACCAAGAC
AAGCGUGGACUGCACCAUGUACAUCUGCGGCGACAGCACCGAGUGCAGCA
ACCUGCUGCUGCAGUACGGCAGCUUCUGCACACAGCUGAACAGAGCCCUG
ACCGGCAUCGCCGUGGAGCAAGACAAGAACACCCAAGAGGUGUUCGCCCA
AGUGAAGCAGAUCUACAAGACCCCCCCCAUCAAGGACUUCGGCGGCUUCA
ACUUCAGCCAAAUCCUGCCCGACCCUAGCAAGCCUAGCAAGCGGAGCUUC
AUCGAGGACCUGCUGUUCAACAAGGUGACCCUGGCCGACGCCGGCUUCAU
CAAGCAGUACGGCGACUGCCUAGGCGACAUCGCAGCUAGAGACCUGAUCU
GCGCUCAGAAGUUCAAUGGACUCACCGUGCUCCCCCCCCUGCUGACCGAC
GAGAUGAUCGCUCAGUACACCUCUGCCCUGCUGGCGGGAACCAUAACAAG
CGGUUGGACAUUUGGCGCCGGGGCCGCACUGCAAAUCCCCUUCGCCAUGC
AGAUGGCCUACAGAUUCAACGGCAUCGGCGUGACACAGAACGUGCUGUAC
GAGAAUCAGAAGCUGAUCGCCAAUCAGUUCAACAGCGCCAUCGGCAAGAU
CCAAGACAGCCUGAGCAGCACCGCUAGCGCCCUGGGCAAGCUGCAAAACG
UGGUGAAUCAGAACGCCCAAGCCCUGAACACCCUGGUGAAGCAGCUGAGC
AGCAACUUCGGCGCCAUCAGCAGCGUAUUGAAUGACAUCCUGUCUAGACU
GGACCCCCCCGAGGCCGAGGUGCAGAUCGACAGACUGAUCACCGGCAGAC
UGCAGAGCCUGCAGACCUACGUGACACAGCAGCUGAUCAGAGCCGCCGAG
AUCAGAGCUAGCGCCAACCUGGCCGCCACCAAGAUGAGCGAGUGCGUGCU

GGGGCAGAGCAAGAGAGUGGACUUCUGCGGCAAGGGCUACCACCUGAUG
AGCUUCCCUCAGAGCGCCCCCCACGGCGUGGUGUUCCUGCACGUGACCUA
CGUGCCCGCCCAAGAGAAGAACUUCACCACCGCUCCCGCCAUCUGCCACG
ACGGCAAGGCCCACUUCCCUAGAGAGGGCGUGUUCGUGAGCAACGGCACC
CACUGGUUCGUGACACAGAGAAACUUCUACGAGCCUCAGAUCAUCACCAC
CGACAACACCUUCGUGAGCGGCAACUGCGACGUGGUGAUCGGCAUAGUGA
ACAACACCGUAUACGACCCCCUGCAGCCCGAGCUGGACAGCUUCAAGGAG
GAGCUGGACAAGUACUUCAAAAACCAUACUAGCCCCGACGUGGACCUGGG
AGACAUCUCUGGCAUCAACGCUAGCGUGGUGAACAUUCAGAAGGAGAUC
GACCGGCUGAAUGAGGUGGCCAAGAACCUGAACGAGAGCCUGAUCGACCU
GCAAGAGCUGGGCAAGUACGAGCAGUACAUCAAGUGGCCCUGGUACAUC
UGGCUGGGCUUCAUCGCCGGCCUGAUCGCCAUCGUGAUGGUGACCAUCAU
GCUGUGCUGCAUGACAAGCUGUUGCAGCUGCCUGAAAGGUUGCUGCAGC
UGUGGCUCGUGCUGCAAGUUCGACGAGGACGACAGCGAGCCCGUGCUGAA
GGGCGUGAAGCUGCACUACACCUGAUGA

SEQ ID NO: 54

>B.1.1.529 (Omicron) spike protein mRNA

AUGUUCGUGUUCCUGGUGCUGCUGCCCCUGGUGAGCUCUCAGUGCGUGAA
CCUGACCACAAGAACACAGCUGCCCCCGCCUACACCAACAGCUUCACAA
GAGGCGUGUACUACCCCGACAAGGUGUUCAGAAGCAGCGUGCUGCACUCC
ACCCAAGACCUGUUUCUGCCCUUCUUCAGCAACGUGACCUGGUUCCACGU
GAUCAGCGGCACCAACGGCACCAAGAGAUUCGACAACCCCGUGCUGCCCU
UCAACGACGGCGUGUACUUCGCUAGCAUCGAAAAGAGCAACAUCAUCAGA
GGCUGGAUCUUCGGCACCACCCUAGACUCCAAGACACAAAGCCUGCUGAU
CGUUAACAACGCGACCAACGUGGUGAUCAAGGUGUGCGAGUUUCAGUUC
UGCAACGACCCCUUCCUGGACCACAAGAACAACAAGAGCUGGAUGGAGAG
CGAGUUCAGAGUGUACAGCAGCGCCAACAACUGCACCUUCGAGUACGUGU
CUCAGCCCUUCCUGAUGGACCUGGAGGGCAAGCAAGGCAAUUUCAAGAAC
CUAAGAGAGUUCGUGUUCAAGAACAUCGACGGCUACUUCAAGAUCUACA
GCAAGCACACCCCCAUCAUCGUGAGAGAGCCCGAGGACCUGCCCCAAGGC
UUCAGCGCCCUGGAGCCCCUGGUGGACCUGCCCAUCGGCAUCAACAUCAC
AAGAUUUCAGACCCUGCUGGCCCUGCACCGUAGCUACCUAACCCCCGGCG
AUAGUUCCUCUGGCUGGACGGCCGGCGCUGCCGCCUACUACGUGGGCUAC
CUGCAGCCUAGAACCUUCCUGCUGAAGUACAACGAGAACGGCACUAUCAC
UGAUGCCGUGGACUGUGCCCUGGACCCCCUGAGCGAGACCAAGUGCACCC
UCAAGAGCUUCACCGUGGAGAAGGGCAUCUAUCAGACAAGCAACUUCAG
AGUGCAGCCCACCGAGAGCAUCGUGAGAUUCCCCAACAUCACCAACCUGU
GCCCCUUCGACGAGGUGUUCAACGCCACCAGAUUUGCUAGCGUGUACGCC
UGGAACCGGAAGAGAAUCAGCAACUGCGUGGCCGACUACAGCGUGCUGU
ACAACCUGGCCCCCUUCUUCACCUUCAAGUGCUAUGGCGUCAGCCCCACC
AAGCUGAACGACCUGUGCUUCACCAACGUGUACGCCGACAGCUUCGUGAU

CAGAGGCGACGAGGUGAGACAGAUCGCCCCGGGCAGACCGGCAACAUCG
CCGACUACAACUACAAGCUGCCCGACGACUUCACCGGCUGCGUGAUCGCC
UGGAACAGCAACAAGCUGGAUAGCAAGGUGAGCGGCAACUACAAUUACC
UGUACAGACUGUUCAGAAAGAGCAACCUGAAGCCCUUCGAGAGAGACAU
CAGCACCGAGAUCUACCAAGCCGGCAACAAGCCCUGCAACGGCGUGGCGG
GCUUCAACUGCUACUUCCCCUCAGGAGCUACAGCUUCAGACCCACCUAC
GGCGUGGGCCAUCAGCCCUACAGAGUGGUCGUGCUGAGCUUCGAGCUGCU
GCACGCCCCUGCCACAGUGUGCGGCCCCAAAAAGAGCACCAACCUGGUGA
AGAACAAGUGCGUGAACUUCAACUUCAACGGCCUGAAGGGCACCGGCGUG
CUGACCGAGAGCAACAAGAAGUUCCUGCCAUUUCAGCAAUUCGGCAGAGA
CAUCGCCGACACCACCGACGCGGUCAGAGACCCUCAGACCCUGGAGAUCC
UGGACAUCACCCCCUGUAGCUUCGGCGGCGUGAGCGUGAUCACCCCCGGC
ACUAACACAAGCAACCAAGUGGCCGUGCUGUACCAAGGCGUGAACUGCAC
CGAGGUGCCCGUGGCCAUCCACGCCGAUCAGCUGACCCCCACCUGGAGAG
UGUAUAGCACCGGCAGCAACGUGUUUCAGACAAGAGCCGGCUGCCUGAUC
GGCGCCGAGUACGUGAACAACAGCUACGAGUGCGACAUCCCCAUCGGCGC
CGGCAUCUGCGCUAGCUAUCAGACACAGACCAAGAGCCACCGGAGAGCUA
GAAGCGUGGCUAGCCAAAGCAUCAUCGCCUACACCAUGAGCCUGGGCGCC
GAGAACAGCGUGGCCUACAGCAACAACAGCAUCGCCAUCCCCACCAACUU
CACCAUCAGCGUGACCACCGAGAUCCUGCCCGUAAGUAUGACCAAGACAA
GCGUGGACUGCACCAUGUACAUCUGCGGCGACAGCACCGAGUGCAGCAAC
CUGCUCCUGCAGUACGGCAGCUUCUGCACACAGCUGAAGAGAGCCCUGAC
CGGCAUCGCCGUGGAGCAAGACAAGAACACCCAAGAGGUGUUCGCCCAAG
UGAAGCAGAUCUACAAGACCCCCCCCAUCAAGUACUUCGGCGGCUUCAAC
UUCAGCCAAAUCCUGCCCGACCCUAGCAAGCCUAGCAAGCGGAGCUUCAU
CGAGGACCUGCUGUUCAACAAGGUGACCCUGGCCGACGCCGGCUUCAUCA
AGCAGUACGGCGACUGCCUAGGCGACAUCGCAGCUAGAGACCUGAUCUGC
GCUCAGAAGUUCAAGGGACUCACCGUGCUCCCCCCCCUGCUGACCGACGA
GAUGAUCGCUCAGUACACCUCUGCCCUGCUGGCGGGAACCAUAACAAGCG
GUUGGACAUUUGGCGCCGGGGCCGCACUGCAAAUCCCCUUCGCCAUGCAG
AUGGCCUACAGAUUCAACGGCAUCGGCGUGACACAGAACGUGCUGUACGA
GAAUCAGAAGCUGAUCGCCAAUCAGUUCAACAGCGCCAUCGGCAAGAUCC
AAGACAGCCUGAGCAGCACCGCUAGCGCCCUGGGCAAGCUGCAAGACGUG
GUGAACCACAACGCCCAAGCCCUGAACACCCUGGUGAAGCAGCUGAGCAG
CAAGUUCGGCGCCAUCAGCAGCGUGCUGAAUGACAUCUUCUCUAGACUGG
ACCCCCCCGAGGCCGAGGUGCAGAUCGACAGACUGAUCACCGGCAGACUG
CAGAGCCUGCAGACCUACGUGACACAGCAGCUGAUCAGAGCCGCCGAGAU
CAGAGCUAGCGCCAACCUGGCCGCCACCAAGAUGAGCGAGUGCGUGCUGG
GCAGAGCAAGAGAGUGGACUUCUGCGGCAAGGGCUACCACCUGAUGAG
CUUCCCUCAGAGCGCCCCCCACGGCGUGGUGUUCCUGCACGUGACCUACG
UGCCCGCCCAAGAGAAGAACUUCACCACCGCUCCCGCCAUCUGCCACGAC

GGCAAGGCCCACUUCCCUAGAGAGGGCGUGUUCGUGAGCAACGGCACCCA
CUGGUUCGUGACACAGAGAAACUUCUACGAGCCUCAGAUCAUCACCACCG
ACAACACCUUCGUGAGCGGCAACUGCGACGUGGUGAUCGGCAUAGUGAAC
AACACCGUAUACGACCCCCUGCAGCCCGAGCUGGACAGCUUCAAGGAGGA
GCUGGACAAGUACUUCAAAAACCAUACUAGCCCCGACGUGGACCUGGGAG
ACAUCUCUGGCAUCAACGCUAGCGUGGUGAACAUUCAGAAGGAGAUCGA
CCGGCUGAAUGAGGUGGCCAAGAACCUGAACGAGAGCCUGAUCGACCUGC
AAGAGCUGGGCAAGUACGAGCAGUACAUCAAGUGGCCCUGGUACAUCUG
GCUGGGCUUCAUCGCCGGCCUGAUCGCCAUCGUGAUGGUGACCAUCAUGC
UGUGCUGCAUGACAAGCUGUUGCAGCUGCCUGAAAGGUUGCUGCAGCUG
UGGCUCGUGCUGCAAGUUCGACGAGGACGACAGCGAGCCCGUGCUGAAGG
GCGUGAAGCUGCACUACACCUGAUGA

SEQ ID NO: 55

>poly A

AAAAAAAAAAAAAAAAAAAAAAAAAAAAAAAAGCAUAUGACUAAAAAAAAA
AAAAAAAAAAAAAAAAAAAAAAAAAAAAAAAAAAAAAAAAAAAAAAAAAA
AAAAAAAAAAAAA

SEQ ID NO: 56

>I'-Delta24-B mRNA

GAGUUAGGAGUUGGUGGCAGUUUCAAUAACAGGUCAUUGCCGAGAAAAG
GAUAGCACUAUAAUAUGUUCGUGUUCCUGGUGCUGCUGCCCCUGGUGAG
CUCUCAGUGCGUGAACCUGAGGACAAGAACACAGCUGCCCCCGCCUACA
CCAACAGCUUCACAAGAGGCGUGUACUACCCCGACAAGGUGUUCAGAAGC
AGCGUGCUGCACUCCACCCAAGACCUGUUUCUGCCCUUCUUCAGCAACGU
GACCUGGUUCCACGCCAUCCACGUCAGCGGCACCAACGGCACCAAGAGAU
UCGACAACCCCGUGCUGCCCUUCAACGACGGCGUGUACUUCGCUAGCACC
GAAAAGAGCAACAUCAUCAGAGGCUGGAUCUUCGGCACCACCCUAGACUC
CAAGACACAAAGCCUGCUGAUCGUUAACAACGCGACCAACGUGGUGAUCA
AGGUGUGCGAGUUUCAGUUCUGCAACGACCCCUUCCUGGACGUGUACUAC
CACAAGAACAACAAGAGCUGGAUGGAGAGCGGGGUCUACAGCAGCGCCA
ACAACUGCACCUUCGAGUACGUGUCUCAGCCCUUCCUGAUGGACCUGGAG
GGCAAGCAAGGCAAUUUCAAGAACCUAAGAGAGUUCGUGUUCAAGAACA
UCGACGGCUACUUCAAGAUCUACAGCAAGCACACCCCCAUCAACCUGGUG
AGAGACCUGCCCCAAGGCUUCAGCGCCCUGGAGCCCCUGGUGGACCUGCC
CAUCGGCAUCAACAUCACAAGAUUUCAGACCCUGCUGGCCCUGCACCGUA
GCUACCUAACCCCCGGCGAUAGUUCCUCUGGCUGGACGGCCGGCGCUGCC
GCCUACUACGUGGGCUACCUGCAGCCUAGAACCUUCCUGCUGAAGUACAA
CGAGAACGGCACUAUCACUGAUGCCGUGGACUGUGCCCUGGACCCCCUGA
GCGAGACCAAGUGCACCCUCAAGAGCUUCACCGUGGAGAAGGGCAUCUAU
CAGACAAGCAACUUCAGAGUGCAGCCCACCGAGAGCAUCGUGAGAUUCCC
CAACAUCACCAACCUGUGCCCCUUCGGCGAGGUGUUCAACGCCACAAGAU

UCGCUAGCGUGUACGCGUGGAACAGAAAGAGAAUCAGCAACUGCGUGGC
CGACUACAGCGUGCUGUACAACAGCGCUAGCUUCAGCACCUUCAAGUGCU
AUGGCGUCAGCCCCACCAAGCUGAACGACCUGUGCUUCACCAACGUGUAC
GCCGACAGCUUCGUGAUCAGAGGCGACGAGGUGAGACAGAUUGCCCCCGG
GCAAACCGGCAAGAUCGCCGACUACAACUACAAGCUGCCCGACGACUUCA
CCGGCUGCGUGAUCGCGUGGAACAGCAAUAACCUGGAUUCCAAGGUGGGC
GGCAACUACAACUACCGGUACAGACUGUUCAGAAAGAGCAACCUGAAGCC
CUUCGAGAGAGACAUCAGCACCGAGAUCUACCAAGCCGGCAGCAAGCCCU
GCAACGGCGUGGAGGGCUUCAACUGCUACUUCCCCUGCAGAGCUACGGC
UUUCAGCCCACCAACGGCGUGGGCUAUCAGCCCUACAGAGUGGUGGUGCU
GAGCUUCGAGCUGCUGCACGCCCCCGCAACCGUGUGCGGCCCCAAAAAGA
GCACCAACCUGGUGAAGAACAAGUGCGUGAACUUCAACUUUAAUGGCCU
UACCGGCACGGGCGUGCUGACCGAGAGCAACAAGAAGUUCCUGCCAUUUC
AGCAAUUCGGCAGAGACAUCGCCGACACCACCGACGCGGUCAGAGACCCU
CAGACCCUGGAGAUCCUGGACAUCACCCCCUGUAGCUUCGGCGGCGUGAG
CGUGAUCACCCCCGGCACUAACACAAGCAACCAAGUGGCCGUGCUGUACC
AAGGCGUGAACUGCACCGAGGUGCCCGUGGCCAUCCACGCCGAUCAGCUG
ACCCCCACCUGGAGAGUAUACAGCACCGGAAGCAACGUGUUUCAGACAAG
AGCCGGCUGCCUGAUCGGCGCCGAGCACGUGAACAACAGCUACGAGUGCG
ACAUCCCCAUCGGCGCCGGCAUCUGCGCUAGCUAUCAGACACAGACCAAC
AGCCGCCGGAGAGCUAGAAGCGUGGCUUCUCAGAGCAUCAUCGCCUACAC
CAUGAGCCUGGGCGCCGAGAACAGCGUGGCCUACAGCAACAACAGCAUCG
CCAUCCCCACCAACUUCACCAUCAGCGUGACCACCGAGAUCCUGCCCGUA
AGUAUGACCAAGACAAGCGUGGACUGCACCAUGUACAUCUGCGGCGACAG
CACCGAGUGCAGCAACCUGCUGCUGCAGUACGGCAGCUUCUGCACACAGC
UGAACAGAGCCCUGACCGGCAUCGCCGUGGAGCAAGACAAGAACACCCAA
GAGGUGUUCGCCCAAGUGAAGCAGAUCUACAAGACCCCCCCCAUCAAGGA
CUUCGGCGGCUUCAACUUCAGCCAAAUCCUGCCCGACCCUAGCAAGCCUA
GCAAGCGGAGCUUCAUCGAGGACCUGCUGUUCAACAAGGUGACCCUGGCC
GACGCCGGCUUCAUCAAGCAGUACGGCGACUGCCUAGGCGACAUCGCAGC
UAGAGACCUGAUCUGCGCUCAGAAGUUCAAUGGACUCACCGUGCUCCCCC
CCCUGCUGACCGACGAGAUGAUCGCUCAGUACACCUCUGCCCUGCUGGCG
GGAACCAUAACAAGCGGUUGGACAUUUGGCGCCGGGGCCGCACUGCAAAU
CCCCUUCGCCAUGCAGAUGGCCUACAGAUUCAACGGCAUCGGCGUGACAC
AGAACGUGCUGUACGAGAAUCAGAAGCUGAUCGCCAAUCAGUUCAACAG
CGCCAUCGGCAAGAUCCAAGACAGCCUGAGCAGCACCGCUAGCGCCCUGG
GCAAGCUGCAAAACGUGGUGAAUCAGAACGCCCAAGCCCUGAACACCCUG
GUGAAGCAGCUGAGCAGCAACUUCGGCGCCAUCAGCAGCGUAUUGAAUG
ACAUCCUGUCUAGACUGGACCCCCCCGAGGCCGAGGUGCAGAUCGACAGA
CUGAUCACCGGCAGACUGCAGAGCCUGCAGACCUACGUGACACAGCAGCU
GAUCAGAGCCGCCGAGAUCAGAGCUAGCGCCAACCUGGCCGCCACCAAGA

UGAGCGAGUGCGUGCUGGGGCAGAGCAAGAGAGUGGACUUCUGCGGCAA
GGGCUACCACCUGAUGAGCUUCCCUCAGAGCGCCCCCCACGGCGUGGUGU
UCCUGCACGUGACCUACGUGCCCGCCCAAGAGAAGAACUUCACCACCGCU
CCCGCCAUCUGCCACGACGGCAAGGCCCACUUCCCUAGAGAGGGCGUGUU
CGUGAGCAACGGCACCCACUGGUUCGUGACACAGAGAAACUUCUACGAGC
CUCAGAUCAUCACCACCGACAACACCUUCGUGAGCGGCAACUGCGACGUG
GUGAUCGGCAUAGUGAACAACACCGUAUACGACCCCCUGCAGCCCGAGCU
GGACAGCUUCAAGGAGGAGCUGGACAAGUACUUCAAAAACCAUACUAGC
CCCGACGUGGACCUGGGAGACAUCUCUGGCAUCAACGCUAGCGUGGUGAA
CAUUCAGAAGGAGAUCGACCGGCUGAAUGAGGUGGCCAAGAACCUGAAC
GAGAGCCUGAUCGACCUGCAAGAGCUGGGCAAGUACGAGCAGUACAUCA
AGUGGCCCUGGUACAUCUGGCUGGGCUUCAUCGCCGGCCUGAUCGCCAUC
GUGAUGGUGACCAUCAUGCUGUGCUGCAUGACAAGCUGUUGCAGCUGCC
UGAAAGGUUGCUGCAGCUGUGGCUCGUGCUGCAAGUUCGACGAGGACGA
CAGCGAGCCCGUGCUGAAGGGCGUGAAGCUGCACUACACCUGAUGACUCG
AGGCUCGCUUUCUUGCUGUCCAAUUUCUAUUAAAGGUUCCUUUGUUCCCU
AAGUCCAACUACUAAACUGGGGGAUAUUAUGAAGGGCCUUGAGCAUCUG
GAUUCUGCCUAAUAAAAACAUUUAUUUCAUUGCAAAAAAAAAAAAAAAAA
AAAAAAAAAAAAAAAAAAAGCAUAUGACUAAAAAAAAAAAAAAAAAAAAA
AAAAAAAAAAAAAAAAAAAAAAAAAAAAAAAAAAAAAAAAAAAAAAAAAA
AA

SEQ ID NO: 57

>I'-OC24-B mRNA

GAGUUAGGAGUUGGUGGCAGUUUCAAUAACAGGUCAUUGCCGAGAAAAG
GAUAGCACUAUAAUAUGUUCGUGUUCCUGGUGCUGCUGCCCCUGGUGAG
CUCUCAGUGCGUGAACCUGACCACAAGAACACAGCUGCCCCCGCCUACA
CCAACAGCUUCACAAGAGGCGUGUACUACCCCGACAAGGUGUUCAGAAGC
AGCGUGCUGCACUCCACCCAAGACCUGUUUCUGCCCUUCUUCAGCAACGU
GACCUGGUUCCACGUGAUCAGCGGCACCAACGGCACCAAGAGAUUCGACA
ACCCCGUGCUGCCCUUCAACGACGGCGUGUACUUCGCUAGCAUCGAAAAG
AGCAACAUCAUCAGAGGCUGGAUCUUCGGCACCACCCUAGACUCCAAGAC
ACAAAGCCUGCUGAUCGUUAACAACGCGACCAACGUGGUGAUCAAGGUG
UGCGAGUUUCAGUUCUGCAACGACCCCUUCCUGGACCACAAGAACAACAA
GAGCUGGAUGGAGAGCGAGUUCAGAGUGUACAGCAGCGCCAACAACUGC
ACCUUCGAGUACGUGUCUCAGCCCUUCCUGAUGGACCUGGAGGGCAAGCA
AGGCAAUUUCAAGAACCUAAGAGAGUUCGUGUUCAAGAACAUCGACGGC
UACUUCAAGAUCUACAGCAAGCACACCCCCAUCAUCGUGAGAGAGCCCGA
GGACCUGCCCCAAGGCUUCAGCGCCCUGGAGCCCCUGGUGGACCUGCCCA
UCGGCAUCAACAUCACAAGAUUUCAGACCCUGCUGGCCCUGCACCGUAGC
UACCUAACCCCCGGCGAUAGUUCCUCUGGCUGGACGGCCGGCGCUGCCGC
CUACUACGUGGGCUACCUGCAGCCUAGAACCUUCCUGCUGAAGUACAACG

AGAACGGCACUAUCACUGAUGCCGUGGACUGUGCCCUGGACCCCCUGAGC
GAGACCAAGUGCACCCUCAAGAGCUUCACCGUGGAGAAGGGCAUCUAUCA
GACAAGCAACUUCAGAGUGCAGCCCACCGAGAGCAUCGUGAGAUUCCCCA
ACAUCACCAACCUGUGCCCCUUCGACGAGGUGUUCAACGCCACCAGAUUU
GCUAGCGUGUACGCCUGGAACCGGAAGAGAAUCAGCAACUGCGUGGCCGA
CUACAGCGUGCUGUACAACCUGGCCCCCUUCUUCACCUUCAAGUGCUAUG
GCGUCAGCCCCACCAAGCUGAACGACCUGUGCUUCACCAACGUGUACGCC
GACAGCUUCGUGAUCAGAGGCGACGAGGUGAGACAGAUCGCCCCCGGGCA
GACCGGCAACAUCGCCGACUACAACUACAAGCUGCCCGACGACUUCACCG
GCUGCGUGAUCGCCUGGAACAGCAACAAGCUGGAUAGCAAGGUGAGCGG
CAACUACAAUUACCUGUACAGACUGUUCAGAAAGAGCAACCUGAAGCCCU
UCGAGAGAGACAUCAGCACCGAGAUCUACCAAGCCGGCAACAAGCCCUGC
AACGGCGUGGCGGGCUUCAACUGCUACUUCCCCUCAGGAGCUACAGCUU
CAGACCCACCUACGGCGUGGGCCAUCAGCCCUACAGAGUGGUCGUGCUGA
GCUUCGAGCUGCUGCACGCCCCUGCCACAGUGUGCGGCCCCAAAAAGAGC
ACCAACCUGGUGAAGAACAAGUGCGUGAACUUCAACUUCAACGGCCUGAA
GGGCACCGGCGUGCUGACCGAGAGCAACAAGAAGUUCCUGCCAUUUCAGC
AAUUCGGCAGAGACAUCGCCGACACCACCGACGCGGUCAGAGACCCUCAG
ACCCUGGAGAUCCUGGACAUCACCCCCUGUAGCUUCGGCGGCGUGAGCGU
GAUCACCCCCGGCACUAACACAAGCAACCAAGUGGCCGUGCUGUACCAAG
GCGUGAACUGCACCGAGGUGCCCGUGGCCAUCCACGCCGAUCAGCUGACC
CCCACCUGGAGAGUGUAUAGCACCGGCAGCAACGUGUUUCAGACAAGAGC
CGGCUGCCUGAUCGGCGCCGAGUACGUGAACAACAGCUACGAGUGCGACA
UCCCCAUCGGCGCCGGCAUCUGCGCUAGCUAUCAGACACAGACCAAGAGC
CACCGGAGAGCUAGAAGCGUGGCUAGCCAAAGCAUCAUCGCCUACACCAU
GAGCCUGGGCGCCGAGAACAGCGUGGCCUACAGCAACAACAGCAUCGCCA
UCCCCACCAACUUCACCAUCAGCGUGACCACCGAGAUCCUGCCCGUAAGU
AUGACCAAGACAAGCGUGGACUGCACCAUGUACAUCUGCGGCGACAGCAC
CGAGUGCAGCAACCUGCUCCUGCAGUACGGCAGCUUCUGCACACAGCUGA
AGAGAGCCCUGACCGGCAUCGCCGUGGAGCAAGACAAGAACACCCAAGAG
GUGUUCGCCCAAGUGAAGCAGAUCUACAAGACCCCCCCCAUCAAGUACUU
CGGCGGCUUCAACUUCAGCCAAAUCCUGCCCGACCCUAGCAAGCCUAGCA
AGCGGAGCUUCAUCGAGGACCUGCUGUUCAACAAGGUGACCCUGGCCGAC
GCCGGCUUCAUCAAGCAGUACGGCGACUGCCUAGGCGACAUCGCAGCUAG
AGACCUGAUCUGCGCUCAGAAGUUCAAGGGACUCACCGUGCUCCCCCCCC
UGCUGACCGACGAGAUGAUCGCUCAGUACACCUCUGCCCUGCUGGCGGGA
ACCAUAACAAGCGGUUGGACAUUUGGCGCCGGGGCCGCACUGCAAAUCCC
CUUCGCCAUGCAGAUGGCCUACAGAUUCAACGGCAUCGGCGUGACACAGA
ACGUGCUGUACGAGAAUCAGAAGCUGAUCGCCAAUCAGUUCAACAGCGCC
AUCGGCAAGAUCCAAGACAGCCUGAGCAGCACCGCUAGCGCCCUGGGCAA
GCUGCAAGACGUGGUGAACCACAACGCCCAAGCCCUGAACACCCUGGUGA

AGCAGCUGAGCAGCAAGUUCGGCGCCAUCAGCAGCGUGCUGAAUGACAUC
UUCUCUAGACUGGACCCCCCCGAGGCCGAGGUGCAGAUCGACAGACUGAU
CACCGGCAGACUGCAGAGCCUGCAGACCUACGUGACACAGCAGCUGAUCA
GAGCCGCCGAGAUCAGAGCUAGCGCCAACCUGGCCGCCACCAAGAUGAGC
GAGUGCGUGCUGGGGCAGAGCAAGAGAGUGGACUUCUGCGGCAAGGGCU
ACCACCUGAUGAGCUUCCCUCAGAGCGCCCCCCACGGCGUGGUGUUCCUG
CACGUGACCUACGUGCCCGCCCAAGAGAAGAACUUCACCACCGCUCCCGC
CAUCUGCCACGACGGCAAGGCCCACUUCCUAGAGAGGGCGUGUUCGUGA
GCAACGGCACCCACUGGUUCGUGACACAGAGAAACUUCUACGAGCCUCAG
AUCAUCACCACCGACAACACCUUCGUGAGCGGCAACUGCGACGUGGUGAU
CGGCAUAGUGAACAACACCGUAUACGACCCCCUGCAGCCCGAGCUGGACA
GCUUCAAGGAGGAGCUGGACAAGUACUUCAAAAACCAUACUAGCCCCGAC
GUGGACCUGGGAGACAUCUCUGGCAUCAACGCUAGCGUGGUGAACAUUC
AGAAGGAGAUCGACCGGCUGAAUGAGGUGGCCAAGAACCUGAACGAGAG
CCUGAUCGACCUGCAAGAGCUGGGCAAGUACGAGCAGUACAUCAAGUGGC
CCUGGUACAUCUGGCUGGGCUUCAUCGCCGGCCUGAUCGCCAUCGUGAUG
GUGACCAUCAUGCUGUGCUGCAUGACAAGCUGUUGCAGCUGCCUGAAAG
GUUGCUGCAGCUGUGGCUCGUGCUGCAAGUUCGACGAGGACGACAGCGA
GCCCGUGCUGAAGGGCGUGAAGCUGCACUACACCUGAUGACUCGAGGCUC
GCUUUCUUGCUGUCCAAUUUCUAUUAAAGGUUCCUUUGUUCCCUAAGUCC
AACUACUAAACUGGGGGAUAUUAUGAAGGGCCUUGAGCAUCUGGAUUCU
GCCUAAUAAAAACAUUUAUUUUCAUUGCAAAAAAAAAAAAAAAAAAAAAA
AAAAAAAAAAAAGCAUAUGACUAAAAAAAAAAAAAAAAAAAAAAAAAAAA
AAAAAAAAAAAAAAAAAAAAAAAAAAAAAAAAAAAAAAAAAAA

SEQ ID NO: 58

>B.1.351 spike protein mRNA

AUGUUCGUGUUCCUGGUGCUGCUGCCCCUGGUGAGCUCUCAGUGCGUGAA
CUUCACGACAAGAACACAGCUGCCCCCGCCUACACCAACAGCUUCACAA
GAGGCGUGUACUACCCCGACAAGGUGUUCAGAAGCAGCGUGCUGCACUCC
ACCCAAGACCUGUUUCUGCCCUUCUUCAGCAACGUGACCUGGUUCCACGC
CAUCCACGUGAGCGGCACCAACGGCACCAAGAGAUUCGCCAACCCCGUGC
UGCCCUUCAACGACGGCGUGUACUUCGCUAGCACCGAAAAGAGCAACAUC
AUCAGAGGCUGGAUCUUCGGCACCACCCUAGACUCCAAGACACAAAGCCU
GCUGAUCGUUAACAACGCGACCAACGUGGUGAUCAAGGUGUGCGAGUUU
CAGUUCUGCAACGACCCCUUCCUGGGCGUGUACUACCACAAGAACAACAA
GAGCUGGAUGGAGAGCGAGUUCCGUGUCUACAGCAGCGCCAACAACUGCA
CCUUCGAGUACGUGUCUCAGCCCUUCCUGAUGGACCUGGAGGGCAAGCAA
GGCAAUUUCAAGAACCUAAGAGAGUUCGUGUUCAAGAACAUCGACGGCU
ACUUCAAGAUCUACAGCAAGCACACCCCCAUCAACCUGGUGAGAGGCCUG
CCCCAAGGCUUCAGCGCCCUGGAGCCCCUGGUGGACCUGCCCAUCGGCAU
CAACAUCACAAGAUUUCAGACCCUGCACCGUAGCUACCUAACCCCCGGCG

AUAGUUCCUCUGGCUGGACGGCCGGCGCUGCCGCCUACUACGUGGGCUAC
CUGCAGCCUAGAACCUUCCUGCUGAAGUACAACGAGAACGGCACUAUCAC
UGAUGCCGUGGACUGUGCCCUGGACCCCCUGAGCGAGACCAAGUGCACCC
UCAAGAGCUUCACCGUGGAGAAGGGCAUCUAUCAGACAAGCAACUUCAG
AGUGCAGCCCACCGAGAGCAUCGUGAGAUUCCCCAACAUCACCAACCUGU
GCCCCUUCGGCGAGGUGUUCAACGCCACAAGAUUCGCUAGCGUGUACGCG
UGGAACAGAAAGAGAAUCAGCAACUGCGUGGCCGACUACAGCGUGCUGU
ACAACAGCGCUAGCUUCAGCACCUUCAAGUGCUAUGGCGUCAGCCCCACC
AAGCUGAACGACCUGUGCUUCACCAACGUGUACGCCGACAGCUUCGUGAU
CAGAGGCGACGAGGUGAGACAGAUUGCCCCCGGGCAAACCGGCAACAUCG
CCGACUACAACUACAAGCUGCCCGACGACUUCACCGGCUGCGUGAUCGCG
UGGAACAGCAAUAACCUGGAUUCCAAGGUGGGCGGCAACUACAACUACCU
GUACAGACUGUUCAGAAAGAGCAACCUGAAGCCCUUCGAGAGAGACAUC
AGCACCGAGAUCUACCAAGCCGGCAGCACCCCUGCAACGGCGUGAAGGG
CUUCAACUGCUACUUCCCCCUGCAGAGCUACGGCUUUCAGCCCACCUACG
GCGUGGGCUAUCAGCCCUACAGAGUGGUGGUGCUGAGCUUCGAGCUGCU
GCACGCCCCCGCAACCGUGUGCGGCCCCAAAAAGAGCACCAACCUGGUGA
AGAACAAGUGCGUGAACUUCAACUUUAAUGGCCUUACCGGCACGGGCGU
GCUGACCGAGAGCAACAAGAAGUUCCUGCCAUUUCAGCAAUUCGGCAGAG
ACAUCGCCGACACCACCGACGCGGUCAGAGACCCUCAGACCCUGGAGAUC
CUGGACAUCACCCCUGUAGCUUCGGCGGCGUGAGCGUGAUCACCCCCGG
CACUAACACAAGCAACCAAGUGGCCGUGCUGUACCAAGGCGUGAACUGCA
CCGAGGUGCCCGUGGCCAUCCACGCCGAUCAGCUGACCCCCACCUGGAGA
GUAUACAGCACCGGAAGCAACGUGUUUCAGACAAGAGCCGGCUGCCUGAU
CGGCGCCGAGCACGUGAACAACAGCUACGAGUGCGACAUCCCCAUCGGCG
CCGGCAUCUGCGCUAGCUAUCAGACACAGACCAACAGCCCCCGGAGAGCU
AGAAGCGUGGCUUCUCAGAGCAUCAUCGCCUACACCAUGAGCCUGGGCGU
CGAGAACAGCGUGGCCUACAGCAACAACAGCAUCGCCAUCCCCACCAACU
UCACCAUCAGCGUGACCACCGAGAUCCUGCCCGUAAGUAUGACCAAGACA
AGCGUGGACUGCACCAUGUACAUCUGCGGCGACAGCACCGAGUGCAGCAA
CCUGCUGCUGCAGUACGGCAGCUUCUGCACACAGCUGAACAGAGCCCUGA
CCGGCAUCGCCGUGGAGCAAGACAAGAACACCCAAGAGGUGUUCGCCCAA
GUGAAGCAGAUCUACAAGACCCCCCCCAUCAAGGACUUCGGCGGCUUCAA
CUUCAGCCAAAUCCUGCCCGACCCUAGCAAGCCUAGCAAGCGGAGCUUCA
UCGAGGACCUGCUGUUCAACAAGGUGACCCUGGCCGACGCCGGCUUCAUC
AAGCAGUACGGCGACUGCCUAGGCGACAUCGCAGCUAGAGACCUGAUCUG
CGCUCAGAAGUUCAAUGGACUCACCGUGCUCCCCCCCCUGCUGACCGACG
AGAUGAUCGCUCAGUACACCUCUGCCCUGCUGGCGGGAACCAUAACAAGC
GGUUGGACAUUUGGCGCCGGGGCCGCACUGCAAAUCCCCUUCGCCAUGCA
GAUGGCCUACAGAUUCAACGGCAUCGGCGUGACACAGAACGUGCUGUACG
AGAAUCAGAAGCUGAUCGCCAAUCAGUUCAACAGCGCCAUCGGCAAGAUC

CAAGACAGCCUGAGCAGCACCGCUAGCGCCCUGGGCAAGCUGCAAGACGU
GGUGAAUCAGAACGCCCAAGCCCUGAACACCCUGGUGAAGCAGCUGAGCA
GCAACUUCGGCGCCAUCAGCAGCGUAUUGAAUGACAUCCUGUCUAGACUG
GACCCCCCCGAGGCCGAGGUGCAGAUCGACAGACUGAUCACCGGCAGACU
GCAGAGCCUGCAGACCUACGUGACACAGCAGCUGAUCAGAGCCGCCGAGA
UCAGAGCUAGCGCCAACCUGGCCGCCACCAAGAUGAGCGAGUGCGUGCUG
GGGCAGAGCAAGAGAGUGGACUUCUGCGGCAAGGGCUACCACCUGAUGA
GCUUCCCUCAGAGCGCCCCCACGGCGUGGUGUUCCUGCACGUGACCUAC
GUGCCCGCCCAAGAGAAGAACUUCACCACCGCUCCCGCCAUCUGCCACGA
CGGCAAGGCCCACUUCCCUAGAGAGGGCGUGUUCGUGAGCAACGGCACCC
ACUGGUUCGUGACACAGAGAAACUUCUACGAGCCUCAGAUCAUCACCACC
GACAACACCUUCGUGAGCGGCAACUGCGACGUGGUGAUCGGCAUAGUGA
ACAACACCGUAUACGACCCCCUGCAGCCCGAGCUGGACAGCUUCAAGGAG
GAGCUGGACAAGUACUUCAAAAACCAUACUAGCCCCGACGUGGACCUGGG
AGACAUCUCUGGCAUCAACGCUAGCGUGGUGAACAUUCAGAAGGAGAUC
GACCGGCUGAAUGAGGUGGCCAAGAACCUGAACGAGAGCCUGAUCGACCU
GCAAGAGCUGGGCAAGUACGAGCAGUACAUCAAGUGGCCCUGGUACAUC
UGGCUGGGCUUCAUCGCCGGCCUGAUCGCCAUCGUGAUGGUGACCAUCAU
GCUGUGCUGCAUGACAAGCUGUUGCAGCUGCCUGAAAGGUUGCUGCAGC
UGUGGCUCGUGCUGCAAGUUCGACGAGGACGACAGCGAGCCCGUGCUGAA
GGGCGUGAAGCUGCACUACACCUGAUGA

SEQ ID NO: 59

>B.1.1.7 spike protein mRNA

AUGUUCGUGUUCCUGGUGCUGCUGCCCCUGGUGAGCUCUCAGUGCGUGAA
CCUGACCACAAGAACACAGCUGCCCCCGCCUACACCAACAGCUUCACAA
GAGGCGUGUACUACCCCGACAAGGUGUUCAGAAGCAGCGUGCUGCACUCC
ACCCAAGACCUGUUUCUGCCCUUCUUCAGCAACGUGACCUGGUUCCACGC
CAUCAGCGGCACCAACGGCACCAAGAGAUUCGACAACCCCGUGCUGCCCU
UCAACGACGGCGUGUACUUCGCUAGCACCGAAAAGAGCAACAUCAUCAGA
GGCUGGAUCUUCGGCACCACCCUAGACUCCAAGACACAAAGCCUGCUGAU
CGUUAACAACGCGACCAACGUGGUGAUCAAGGUGUGCGAGUUUCAGUUC
UGCAACGACCCCUUCCUGGGCGUGUACCACAAGAACAACAAGAGCUGGAU
GGAGAGCGAGUUCCGUGUCUACAGCAGCGCCAACAACUGCACCUUCGAGU
ACGUGUCUCAGCCCUUCCUGAUGGACCUGGAGGGCAAGCAAGGCAAUUUC
AAGAACCUAAGAGAGUUCGUGUUCAAGAACAUCGACGGCUACUUCAAGA
UCUACAGCAAGCACACCCCCAUCAACCUGGUGAGAGACCUGCCCCAAGGC
UUCAGCGCCCUGGAGCCCCUGGUGGACCUGCCCAUCGGCAUCAACAUCAC
AAGAUUUCAGACCCUGCUGGCCCUGCACCGUAGCUACCUAACCCCCGGCG
AUAGUUCCUCUGGCUGGACGGCCGGCGCUGCCGCCUACUACGUGGGCUAC
CUGCAGCCUAGAACCUUCCUGCUGAAGUACAACGAGAACGGCACUAUCAC
UGAUGCCGUGGACUGUGCCCUGGACCCCCUGAGCGAGACCAAGUGCACCC

UCAAGAGCUUCACCGUGGAGAAGGGCAUCUAUCAGACAAGCAACUUCAG
AGUGCAGCCCACCGAGAGCAUCGUGAGAUUCCCCAACAUCACCAACCUGU
GCCCCUUCGGCGAGGUGUUCAACGCCACAAGAUUCGCUAGCGUGUACGCG
UGGAACAGAAAGAGAAUCAGCAACUGCGUGGCCGACUACAGCGUGCUGU
ACAACAGCGCUAGCUUCAGCACCUUCAAGUGCUAUGGCGUCAGCCCCACC
AAGCUGAACGACCUGUGCUUCACCAACGUGUACGCCGACAGCUUCGUGAU
CAGAGGCGACGAGGUGAGACAGAUUGCCCCCGGGCAAACCGGCAAGAUCG
CCGACUACAACUACAAGCUGCCCGACGACUUCACCGGCUGCGUGAUCGCG
UGGAACAGCAAUAACCUGGAUUCCAAGGUGGGCGGCAACUACAACUACCU
GUACAGACUGUUCAGAAAGAGCAACCUGAAGCCCUUCGAGAGAGACAUC
AGCACCGAGAUCUACCAAGCCGGCAGCACCCCCUGCAACGGCGUGGAGGG
CUUCAACUGCUACUUCCCCCUGCAGAGCUACGGCUUUCAGCCCACCUACG
GCGUGGGCUAUCAGCCCUACAGAGUGGUGGUGCUGAGCUUCGAGCUGCU
GCACGCCCCGCAACCGUGUGCGGCCCCAAAAAGAGCACCAACCUGGUGA
AGAACAAGUGCGUGAACUUCAACUUUAAUGGCCUUACCGGCACGGGCGU
GCUGACCGAGAGCAACAAGAAGUUCCUGCCAUUUCAGCAAUUCGGCAGAG
ACAUCGACGACACCACCGACGCGGUCAGAGACCCUCAGACCCUGGAGAUC
CUGGACAUCACCCCCUGUAGCUUCGGCGGCGUGAGCGUGAUCACCCCCGG
CACUAACACAAGCAACCAAGUGGCCGUGCUGUACCAAGGCGUGAACUGCA
CCGAGGUGCCCGUGGCCAUCCACGCCGAUCAGCUGACCCCCACCUGGAGA
GUAUACAGCACCGGAAGCAACGUGUUUCAGACAAGAGCCGGCUGCCUGAU
CGGCGCCGAGCACGUGAACAACAGCUACGAGUGCGACAUCCCCAUCGGCG
CCGGCAUCUGCGCUAGCUAUCAGACACAGACCAACAGCCACCGGAGAGCU
AGAAGCGUGGCUUCUCAGAGCAUCAUCGCCUACACCAUGAGCCUGGGCGC
CGAGAACAGCGUGGCCUACAGCAACAACAGCAUCGCCAUCCCCAUCAACU
UCACCAUCAGCGUGACCACCGAGAUCCUGCCCGUAAGUAUGACCAAGACA
AGCGUGGACUGCACCAUGUACAUCUGCGGCGACAGCACCGAGUGCAGCAA
CCUGCUGCUGCAGUACGGCAGCUUCUGCACACAGCUGAACAGAGCCCUGA
CCGGCAUCGCCGUGGAGCAAGACAAGAACACCCAAGAGGUGUUCGCCCAA
GUGAAGCAGAUCUACAAGACCCCCCCCAUCAAGGACUUCGGCGGCUUCAA
CUUCAGCCAAAUCCUGCCCGACCCUAGCAAGCCUAGCAAGCGGAGCUUCA
UCGAGGACCUGCUGUUCAACAAGGUGACCCUGGCCGACGCCGGCUUCAUC
AAGCAGUACGGCGACUGCCUAGGCGACAUCGCAGCUAGAGACCUGAUCUG
CGCUCAGAAGUUCAAUGGACUCACCGUGCUCCCCCCCCUGCUGACCGACG
AGAUGAUCGCUCAGUACACCUCUGCCCUGCUGGCGGGAACCAUAACAAGC
GGUUGGACAUUUGGCGCCGGGGCCGCACUGCAAAUCCCCUUCGCCAUGCA
GAUGGCCUACAGAUUCAACGGCAUCGGCGUGACACAGAACGUGCUGUACG
AGAAUCAGAAGCUGAUCGCCAAUCAGUUCAACAGCGCCAUCGGCAAGAUC
CAAGACAGCCUGAGCAGCACCGCUAGCGCCCUGGGCAAGCUGCAAGACGU
GGUGAAUCAGAACGCCCAAGCCCUGAACACCCUGGUGAAGCAGCUGAGCA
GCAACUUCGGCGCCAUCAGCAGCGUAUUGAAUGACAUCCUGGCUAGACUG

GACCCCCCCGAGGCCGAGGUGCAGAUCGACAGACUGAUCACCGGCAGACU
GCAGAGCCUGCAGACCUACGUGACACAGCAGCUGAUCAGAGCCGCCGAGA
UCAGAGCUAGCGCCAACCUGGCCGCCACCAAGAUGAGCGAGUGCGUGCUG
GGGCAGAGCAAGAGAGUGGACUUCUGCGGCAAGGGCUACCACCUGAUGA
GCUUCCCUCAGAGCGCCCCCCACGGCGUGGUGUUCCUGCACGUGACCUAC
GUGCCCGCCCAAGAGAAGAACUUCACCACCGCUCCCGCCAUCUGCCACGA
CGGCAAGGCCCACUUCCCUAGAGAGGGCGUGUUCGUGAGCAACGGCACCC
ACUGGUUCGUGACACAGAGAAACUUCUACGAGCCUCAGAUCAUCACCACC
CACAACACCUUCGUGAGCGGCAACUGCGACGUGGUGAUCGGCAUAGUGAA
CAACACCGUAUACGACCCCCUGCAGCCCGAGCUGGACAGCUUCAAGGAGG
AGCUGGACAAGUACUUCAAAAACCAUACUAGCCCCGACGUGGACCUGGGA
GACAUCUCUGGCAUCAACGCUAGCGUGGUGAACAUUCAGAAGGAGAUCG
ACCGGCUGAAUGAGGUGGCCAAGAACCUGAACGAGAGCCUGAUCGACCUG
CAAGAGCUGGGCAAGUACGAGCAGUACAUCAAGUGGCCCUGGUACAUCU
GGCUGGGCUUCAUCGCCGGCCUGAUCGCCAUCGUGAUGGUGACCAUCAUG
CUGUGCUGCAUGACAAGCUGUUGCAGCUGCCUGAAAGGUUGCUGCAGCU
GUGGCUCGUGCUGCAAGUUCGACGAGGACGACAGCGAGCCCGUGCUGAAG
GGCGUGAAGCUGCACUACACCUGAUGA

<div align="right">SEQ ID NO: 60</div>

>SARS-CoV-2 spike protein mRNA

AUGUUCGUGUUCCUGGUGCUGCUGCCCCUGGUGAGCUCUCAGUGCGUGAA
CCUGACCACAAGAACACAGCUGCCCCCCGCCUACACCAACAGCUUCACAA
GAGGCGUGUACUACCCCGACAAGGUGUUCAGAAGCAGCGUGCUGCACUCC
ACCCAAGACCUGUUUCUGCCCUUCUUCAGCAACGUGACCUGGUUCCACGC
CAUCCACGUGAGCGGCACCAACGGCACCAAGAGAUUCGACAACCCCGUGC
UGCCCUUCAACGACGGCGUGUACUUCGCUAGCACCGAAAAGAGCAACAUC
AUCAGAGGCUGGAUCUUCGGCACCACCCUAGACUCCAAGACACAAAGCCU
GCUGAUCGUUAACAACGCGACCAACGUGGUGAUCAAGGUGUGCGAGUUU
CAGUUCUGCAACGACCCCUUCCUGGGCGUGUACUACCACAAGAACAACAA
GAGCUGGAUGGAGAGCGAGUUCCGUGUCUACAGCAGCGCCAACAACUGCA
CCUUCGAGUACGUGUCUCAGCCCUUCCUGAUGGACCUGGAGGGCAAGCAA
GGCAAUUUCAAGAACCUAAGAGAGUUCGUGUUCAAGAACAUCGACGGCU
ACUUCAAGAUCUACAGCAAGCACACCCCCAUCAACCUGGUGAGAGACCUG
CCCCAAGGCUUCAGCGCCCUGGAGCCCCUGGUGGACCUGCCCAUCGGCAU
CAACAUCACAAGAUUUCAGACCCUGCUGGCCCUGCACCGUAGCUACCUAA
CCCCCGGCGAUAGUUCCUCUGGCUGGACGGCCGGCGCUGCCGCCUACUAC
GUGGGCUACCUGCAGCCUAGAACCUUCCUGCUGAAGUACAACGAGAACGG
CACUAUCACUGAUGCCGUGGACUGUGCCCUGGACCCCCUGAGCGAGACCA
AGUGCACCCUCAAGAGCUUCACCGUGGAGAAGGGCAUCUAUCAGACAAGC
AACUUCAGAGUGCAGCCCACCGAGAGCAUCGUGAGAUUCCCCAACAUCAC
CAACCUGUGCCCCUUCGGCGAGGUGUUCAACGCCACAAGAUUCGCUAGCG

UGUACGCGUGGAACAGAAAGAGAAUCAGCAACUGCGUGGCCGACUACAG
CGUGCUGUACAACAGCGCUAGCUUCAGCACCUUCAAGUGCUAUGGCGUCA
GCCCCACCAAGCUGAACGACCUGUGCUUCACCAACGUGUACGCCGACAGC
UUCGUGAUCAGAGGCGACGAGGUGAGACAGAUUGCCCCCGGGCA5AACCG
GCAAGAUCGCCGACUACAACUACAAGCUGCCCGACGACUUCACCGGCUGC
GUGAUCGCGUGGAACAGCAAUAACCUGGAUUCCAAGGUGGGCGGCAACU
ACAACUACCUGUACAGACUGUUCAGAAAGAGCAACCUGAAGCCCUUCGAG
AGAGACAUCAGCACCGAGAUCUACCAAGCCGGCAGCACCCCCUGCAACGG
CGUGGAGGGCUUCAACUGCUACUUCCCCCUGCAGAGCUACGGCUUUCAGC
CCACCAAUGGCGUGGGCUAUCAGCCCUACAGAGUGGUGGUGCUGAGCUUC
GAGCUGCUGCACGCCCCCGCAACCGUGUGCGGCCCCAAAAAGAGCACCAA
CCUGGUGAAGAACAAGUGCGUGAACUUCAACUUUAAUGGCCUUACCGGC
ACGGGCGUGCUGACCGAGAGCAACAAGAAGUUCCUGCCAUUUCAGCAAUU
CGGCAGAGACAUCGCCGACACCACCGACGCGGUCAGAGACCCUCAGACCC
UGGAGAUCCUGGACAUCACCCCCUGUAGCUUCGGCGGCGUGAGCGUGAUC
ACCCCCGGCACUAACACAAGCAACCAAGUGGCCGUGCUGUACCAAGACGU
GAACUGCACCGAGGUGCCCGUGGCCAUCCACGCCGAUCAGCUGACCCCCA
CCUGGAGAGUAUACAGCACCGGAAGCAACGUGUUUCAGACAAGAGCCGGC
UGCCUGAUCGGCGCCGAGCACGUGAACAACAGCUACGAGUGCGACAUCCC
CAUCGGCGCCGGCAUCUGCGCUAGCUAUCAGACACAGACCAACAGCCCUC
GGAGAGCUAGAAGCGUGGCUUCUCAGAGCAUCAUCGCCUACACCAUGAGC
CUGGGCGCCGAGAACAGCGUGGCCUACAGCAACAACAGCAUCGCCAUCCC
CACCAACUUCACCAUCAGCGUGACCACCGAGAUCCUGCCCGUAAGUAUGA
CCAAGACAAGCGUGGACUGCACCAUGUACAUCUGCGGCGACAGCACCGAG
UGCAGCAACCUGCUGCUGCAGUACGGCAGCUUCUGCACACAGCUGAACAG
AGCCCUGACCGGCAUCGCCGUGGAGCAAGACAAGAACACCCAAGAGGUGU
UCGCCCAAGUGAAGCAGAUCUACAAGACCCCCCCCAUCAAGGACUUCGGC
GGCUUCAACUUCAGCCAAAUCCUGCCCGACCCUAGCAAGCCUAGCAAGCG
GAGCUUCAUCGAGGACCUGCUGUUCAACAAGGUGACCCUGGCCGACGCCG
GCUUCAUCAAGCAGUACGGCGACUGCCUAGGCGACAUCGCAGCUAGAGAC
CUGAUCUGCGCUCAGAAGUUCAAUGGACUCACCGUGCUCCCCCCCCUGCU
GACCGACGAGAUGAUCGCUCAGUACACCUCUGCCCUGCUGGCGGGAACCA
UAACAAGCGGUUGGACAUUUGGCGCCGGGGCCGCACUGCAAAUCCCCUUC
GCCAUGCAGAUGGCCUACAGAUUCAACGGCAUCGGCGUGACACAGAACGU
GCUGUACGAGAAUCAGAAGCUGAUCGCCAAUCAGUUCAACAGCGCCAUCG
GCAAGAUCCAAGACAGCCUGAGCAGCACCGCUAGCGCCCUGGGCAAGCUG
CAAGACGUGGUGAAUCAGAACGCCCAAGCCCUGAACACCCUGGUGAAGCA
GCUGAGCAGCAACUUCGGCGCCAUCAGCAGCGUAUUGAAUGACAUCCUGA
GCAGACUGGACCCCCCCGAGGCCGAGGUGCAGAUCGACAGACUGAUCACC
GGCAGACUGCAGAGCCUGCAGACCUACGUGACACAGCAGCUGAUCAGAGC
CGCCGAGAUCAGAGCUAGCGCCAACCUGGCCGCCACCAAGAUGAGCGAGU

GCGUGCUGGGGCAGAGCAAGAGAGUGGACUUCUGCGGCAAGGGCUACCA
CCUGAUGAGCUUCCCUCAGAGCGCCCCCCACGGCGUGGUGUUCCUGCACG
UGACCUACGUGCCCGCCCAAGAGAAGAACUUCACCACCGCUCCCGCCAUC
UGCCACGACGGCAAGGCCCACUUCCCUAGAGAGGGCGUGUUCGUGAGCAA
CGGCACCCACUGGUUCGUGACACAGAGAAACUUCUACGAGCCUCAGAUCA
UCACCACCGACAACACCUUCGUGAGCGGCAACUGCGACGUGGUGAUCGGC
AUAGUGAACAACACCGUAUACGACCCCCUGCAGCCCGAGCUGGACAGCUU
CAAGGAGGAGCUGGACAAGUACUUCAAAAACCAUACUAGCCCCGACGUGG
ACCUGGGAGACAUCUCUGGCAUCAACGCUAGCGUGGUGAACAUUCAGAA
GGAGAUCGACCGGCUGAAUGAGGUGGCCAAGAACCUGAACGAGAGCCUG
AUCGACCUGCAAGAGCUGGGCAAGUACGAGCAGUACAUCAAGUGGCCCUG
GUACAUCUGGCUGGGCUUCAUCGCCGGCCUGAUCGCCAUCGUGAUGGUGA
CCAUCAUGCUGUGCUGCAUGACAAGCUGUUGCAGCUGCCUGAAAGGUUGC
UGCAGCUGUGGCUCGUGCUGCAAGUUCGACGAGGACGACAGCGAGCCCGU
GCUGAAGGGCGUGAAGCUGCACUACACCUGAUGA

SEQ ID NO: 61

>Luciferase mRNA

AUGGAGGACGCCAAGAACAUCAAGAAAGGCCCCGCCCCCUUCUACCCCC
UGGAGGACGGCACCGCCGGCGAGCAGCUGCACAAAGCCAUGAAAAGAUA
CGCUCUGGUGCCCGGCACCAUCGCCUUCACCGACGCCCACAUCGAGGUGA
ACAUCACCUACGCCGAGUACUUCGAGAUGAGCGUGAGACUGGCCGAGGC
UAUGAAGAGAUACGGCUUGAACACCAACCACAGAAUCGUGGUGUGCAGC
GAGAACAGCCUGCAGUUCUUCAUGCCCGUGCUGGGCGCCCUGUUCAUCG
GCGUGGCCGUUGCCCCCGCCAACGACAUCUACAACGAGAGAGAGCUGCU
GAACAGCAUGAACAUCUCUCAGCCCACCGUGGUUUUCGUGUCCAAAAAG
GGCCUGCAGAAGAUCCUGAACGUGCAGAAGAAGCUGCCCAUCAUUCAGA
AGAUCAUCAUGGACAGCAAGACCGACUACCAAGGCUUUCAGAGCAU
GUACACCUUCGUGACAAGCCACCUGCCCCCGGCUUCAACGAGUACGAC
UUCGUGCCCGAGAGCUUCGACAGAGACAAGACCAUCGCCCUGAUCAUGA
ACAGCAGCGGCAGCACCGGCCUGCCCAAGGGCGUGGCCCUGCCCCACAGA
ACCGCCUGCGUGAGAUUCAGCCACGCUAGAGACCCCAUCUUCGGCAAUC
AGAUCAUCCCCGACACCGCCAUCCUGAGCGUAGUGCCUUUCCAUCAUGG
CUUCGGCAUGUUCACCACCCUGGGCUACCUGAUCUGCGGCUUCAGAGUG
GUGCUGAUGUACAGAUUCGAGGAGGAGCUGUUCCUGAGAAGCCUGCAAG
ACUACAAGAUUCAGAGCGCCCUGCUGGUGCCCACCCUGUUCAGCUUCUU
CGCCAAGAGCACCCUGAUCGACAAGUAUGACCUGAGCAACCUGCACGAA
AUUGCGAGCGGCGGAGCGCCGCUCAGCAAAGAGGUGGGCGAGGCCGUGG
CCAAGAGAUUCCACCUGCCCGGCAUCAGACAAGGCUACGGCCUGACCGA

GACCACAAGCGCCAUCCUGAUCACCCCCGAGGGCGACGAUAAGCCCGGC
GCCGUGGGCAAGGUGGUACCAUUCUUCGAGGCCAAGGUGGUGGACCUGG
ACACCGGCAAGACCCUGGGCGUGAAUCAGAGAGGCGAGCUGUGCGUGAG
AGGCCCCAUGAUCAUGAGCGGCUACGUGAACAACCCCGAGGCCACCAAC
GCCCUCAUCGAUAAGGAUGGGUGGCUGCACAGCGGCGACAUCGCCUACU
GGGACGAGGACGAGCACUUCUUCAUCGUGGACAGACUCAAGAGCCUGAU
CAAGUACAAGGGCUACCAAGUGGCCCCCGCCGAGUUGGAGAGCAUCCUG
CUGCAGCACCCCAAUAUCUUCGACGCUGGCGUGGCCGGACUACCCGACG
ACGACGCCGGCGAGUUACCCGCCGCCGUGGUGGUGCUGGAGCACGGCAA
GACCAUGACCGAGAAGGAGAUCGUGGACUACGUGGCUAGCCAAGUGACC
ACCGCCAAGAAGCUGAGAGGCGGCGUGGUGUUCGUUGACGAAGUGCCCA
AGGGCCUGACCGGCAAGCUGGACGCUAGAAAGAUCAGAGAGAUCCUGAU
CAAGGCCAAGAAGGGCGGCAAGAGCAAGCUGUGA

SEQ ID NO: 62

>LUC1- I' truncation

UAGGAGUUGGUGGCAGUUUCAAUAACAGGUCAUUGCCGAGAAAAGGAUA
GCACUAUAAU

SEQ ID NO: 63

>LUC2- I' truncation

AGUUGGUGGCAGUUUCAAUAACAGGUCAUUGCCGAGAAAAGGAUAGCAC
UAUAAU

SEQ ID NO: 64

>LUC3- I' truncation

GGUGGCAGUUUCAAUAACAGGUCAUUGCCGAGAAAAGGAUAGCACUAUA
AU

SEQ ID NO: 65

>LUC4- I' truncation
GCAGUUUCAAUAACAGGUCAUUGCCGAGAAAAGGAUAGCACUAUAAU
SEQ ID NO: 66
>LUC5- I' truncation
UUUCAAUAACAGGUCAUUGCCGAGAAAAGGAUAGCACUAUAAU
SEQ ID NO: 67
>LUC6- I' truncation
AAUAACAGGUCAUUGCCGAGAAAAGGAUAGCACUAUAAU
SEQ ID NO: 68
> LUC7 - I' truncation
ACAGGUCAUUGCCGAGAAAAGGAUAGCACUAUAAU
SEQ ID NO: 69
>LUC8- I' truncation
GUCAUUGCCGAGAAAAGGAUAGCACUAUAAU
SEQ ID NO: 70
>LUC9- I' truncation
UUGCCGAGAAAAGGAUAGCACUAUAAU
SEQ ID NO: 71
>LUC10- I' truncation
CGAGAAAAGGAUAGCACUAUAAU
SEQ ID NO: 72
>LUC11- I' truncation
AAAAGGAUAGCACUAUAAU
SEQ ID NO: 73
>LUC12- I' truncation
GGAUAGCACUAUAAU
SEQ ID NO: 74
>LUC13- I' truncation
AGCACUAUAAU
SEQ ID NO: 75
>LUC14- I' truncation
CUAUAAU
>LUC15- I' mutant

GAGUUAGGAGUUGCUGGCAGUUUCAAUAACAGGUCAUUGCCGAGAAAA
GGAUAGCACUAUAAU

SEQ ID NO: 77

>LUC16- I' mutant

GAGUUAGGAGUUGUUGGCAGUUUCAAUAACAGGUCAUUGCCGAGAAAA
GGAUAGCACUAUAAU

SEQ ID NO: 78

>5'UTR I general formula

GAGUUAGGAGUUGN$_2$UGGCAGUUUCAAUAACAGGUCAUUGCCGAGAAAAG
GAUAGCACUAUAAU

SEQ ID NO: 79

N$_2$ is selected from the group consisting of A, G, C, and U
>Wildtype SARS-CoV-2 spike protein amino acid sequence

MFVFLVLLPLVSSQCVNLTTRTQLPPAYTNSFTRGVYYPDKVFRSSVLHSTQDL
FLPFFSNVTWFHAIHVSGTNGTKRFDNPVLPFNDGVYFASTEKSNIIRGWIFGTT
LDSKTQSLLIVNNATNVVIKVCEFQFCNDPFLGVYYHKNNKSWMESEFRVYSS

ANNCTFEYVSQPFLMDLEGKQGNFKNLREFVFKNIDGYFKIYSKHTPINLVRDL
PQGFSALEPLVDLPIGINITRFQTLLALHRSYLTPGDSSSGWTAGAAAYYVGYL
QPRTFLLKYNENGTITDAVDCALDPLSETKCTLKSFTVEKGIYQTSNFRVQPTES
IVRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFN
CYFPLQSYGFQPTNGVGYQPYRVVVLSFELLHAPATVCGPKKSTNLVKNKCVN
FNFNGLTGTGVLTESNKKFLPFQQFGRDIADTTDAVRDPQTLEILDITPCSFGGV
SVITPGTNTSNQVAVLYQDVNCTEVPVAIHADQLTPTWRVYSTGSNVFQTRAG
CLIGAEHVNNSYECDIPIGAGICASYQTQTNSPRRARSVASQSIIAYTMSLGAEN
SVAYSNNSIAIPTNFTISVTTEILPVSMTKTSVDCTMYICGDSTECSNLLLQYGSF
CTQLNRALTGIAVEQDKNTQEVFAQVKQIYKTPPIKDFGGFNFSQILPDPSKPSK
RSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQ
KLIANQFNSAIGKIQDSLSSTASALGKLQDVVNQNAQALNTLVKQLSSNFGAIS
SVLNDILSRLDKVEAEVQIDRLITGRLQSLQTYVTQQLIRAAEIRASANLAATK
MSECVLGQSKRVDFCGKGYHLMSFPQSAPHGVVFLHVTYVPAQEKNFTTAPAI
CHDGKAHFPREGVFVSNGTHWFVTQRNFYEPQIITTDNTFVSGNCDVVIGIVN
NTVYDPLQPELDSFKEELDKYFKNHTSPDVDLGDISGINASVVNIQKEIDRLNE
VAKNLNESLIDLQELGKYEQYIKWPWYIWLGFIAGLIAIVMVTIMLCCMTSCCS
CLKGCCSCGSCCKFDEDDSEPVLKGVKLHYT

SEQ ID NO: 80

>Wildtype SARS-CoV-2 spike protein CDS (NC_045512.2)

ATGTTTGTTTTTCTTGTTTTATTGCCACTAGTCTCTAGTCAGTGTGTTAATCTT
ACAACCAGAACTCAATTACCCCCTGCATACACTAATTCTTTCACACGTGGTG
TTTATTACCCTGACAAAGTTTTCAGATCCTCAGTTTTACATTCAACTCAGGAC
TTGTTCTTACCTTTCTTTTCCAATGTTACTTGGTTCCATGCTATACATGTCTCT
GGGACCAATGGTACTAAGAGGTTTGATAACCCTGTCCTACCATTTAATGATG
GTGTTTATTTTGCTTCCACTGAGAAGTCTAACATAATAAGAGGCTGGATTTTT
GGTACTACTTTAGATTCGAAGACCCAGTCCCTACTTATTGTTAATAACGCTAC
TAATGTTGTTATTAAAGTCTGTGAATTTCAATTTTGTAATGATCCATTTTTGGG
TGTTTATTACCACAAAAACAACAAAGTTGGATGGAAAGTGAGTTCAGAGT
TTATTCTAGTGCGAATAATTGCACTTTTGAATATGTCTCTCAGCCTTTTCTTAT
GGACCTTGAAGGAAACAGGGTAATTTCAAAAATCTTAGGGAATTTGTGTTT
AAGAATATTGATGGTTATTTTAAAATATATTCTAAGCACACGCCTATTAATTTA
GTGCGTGATCTCCCTCAGGGTTTTTCGGCTTTAGAACCATTGGTAGATTTGC

CAATAGGTATTAACATCACTAGGTTTCAAACTTTACTTGCTTTACATAGAAGT

TATTTGACTCCTGGTGATTCTTCTTCAGGTTGGACAGCTGGTGCTGCAGCTTA

TTATGTGGGTTATCTTCAACCTAGGACTTTTCTATTAAAATATAATGAAAATGG

AACCATTACAGATGCTGTAGACTGTGCACTTGACCCTCTCTCAGAAACAAA

GTGTACGTTGAAATCCTTCACTGTAGAAAAGGAATCTATCAAACTTCTAAC

TTTAGAGTCCAACCAACAGAATCTATTGTTAGATTTCCTAATATTACAAACTT

GTGCCCTTTTGGTGAAGTTTTTAACGCCACCAGATTTGCATCTGTTTATGCTT

GGAACAGGAAGAGAATCAGCAACTGTGTTGCTGATTATTCTGTCCTATATAA

TTCCGCATCATTTTCCACTTTTAAGTGTTATGGAGTGTCTCCTACTAAATTAAA

TGATCTCTGCTTTACTAATGTCTATGCAGATTCATTTGTAATTAGAGGTGATGA

AGTCAGACAAATCGCTCCAGGGCAAACTGGAAAGATTGCTGATTATAATTAT

AAATTACCAGATGATTTTACAGGCTGCGTTATAGCTTGGAATTCTAACAATCT

TGATTCTAAGGTTGGTGGTAATTATAATTACCTGTATAGATTGTTTAGGAAGT

CTAATCTCAAACCTTTTGAGAGAGATATTTCAACTGAAATCTATCAGGCCGG

TAGCACACCTTGTAATGGTGTTGAAGGTTTTAATTGTTACTTTCCTTTACAAT

CATATGGTTTCCAACCCACTAATGGTGTTGGTTACCAACCATACAGAGTAGT

AGTACTTTCTTTTGAACTTCTACATGCACCAGCAACTGTTTGTGGACCTAAA

AAGTCTACTAATTTGGTTAAAAACAAATGTGTCAATTTCAACTTCAATGGTTT

AACAGGCACAGGTGTTCTTACTGAGTCTAACAAAAGTTTCTGCCTTTCCA

ACAATTTGGCAGAGACATTGCTGACACTACTGATGCTGTCCGTGATCCACAG

ACACTTGAGATTCTTGACATTACACCATGTTCTTTTGGTGGTGTCAGTGTTAT

AACACCAGGAACAAATACTTCTAACCAGGTTGCTGTTCTTTATCAGGATGTT

AACTGCACAGAAGTCCCTGTTGCTATTCATGCAGATCAACTTACTCCTACTT

GGCGTGTTTATTCTACAGGTTCTAATGTTTTTCAAACACGTGCAGGCTGTTTA

ATAGGGGCTGAACATGTCAACAACTCATATGAGTGTGACATACCCATTGGTG

CAGGTATATGCGCTAGTTATCAGACTCAGACTAATTCTCCTCGGCGGGCACG

TAGTGTAGCTAGTCAATCCATCATTGCCTACACTATGTCACTTGGTGCAGAAA

ATTCAGTTGCTTACTCTAATAACTCTATTGCCATACCCACAAATTTTACTATTA

GTGTTACCACAGAAATTCTACCAGTGTCTATGACCAAGACATCAGTAGATTG

TACAATGTACATTTGTGGTGATTCAACTGAATGCAGCAATCTTTTGTTGCAAT

ATGGCAGTTTTTGTACACAATTAAACCGTGCTTTAACTGGAATAGCTGTTGA

ACAAGACAAAAACACCCAAGAAGTTTTTGCACAAGTCAAACAAATTTACA

AAACACCACCAATTAAAGATTTTGGTGGTTTTAATTTTTCACAAATATTACCA

GATCCATCAAAACCAAGCAAGAGGTCATTTATTGAAGATCTACTTTTCAACA

AAGTGACACTTGCAGATGCTGGCTTCATCAAACAATATGGTGATTGCCTTGG

TGATATTGCTGCTAGAGACCTCATTTGTGCACAAAAGTTTAACGGCCTTACT

GTTTTGCCACCTTTGCTCACAGATGAAATGATTGCTCAATACACTTCTGCACT
GTTAGCGGGTACAATCACTTCTGGTTGGACCTTTGGTGCAGGTGCTGCATTA
CAAATACCATTTGCTATGCAAATGGCTTATAGGTTTAATGGTATTGGAGTTAC
ACAGAATGTTCTCTATGAGAACCAAAAATTGATTGCCAACCAATTTAATAGT
GCTATTGGCAAAATTCAAGACTCACTTTCTTCCACAGCAAGTGCACTTGGAA
AACTTCAAGATGTGGTCAACCAAAATGCACAAGCTTTAAACACGCTTGTTA
AACAACTTAGCTCCAATTTTGGTGCAATTTCAAGTGTTTTAAATGATATCCTT
TCACGTCTTGACAAAGTTGAGGCTGAAGTGCAAATTGATAGGTTGATCACA
GGCAGACTTCAAAGTTTGCAGACATATGTGACTCAACAATTAATTAGAGCTG
CAGAAATCAGAGCTTCTGCTAATCTTGCTGCTACTAAAATGTCAGAGTGTGT
ACTTGGACAATCAAAAAGAGTTGATTTTTGTGGAAAGGGCTATCATCTTATG
TCCTTCCCTCAGTCAGCACCTCATGGTGTAGTCTTCTTGCATGTGACTTATGT
CCCTGCACAAGAAAAGAACTTCACAACTGCTCCTGCCATTTGTCATGATGG
AAAAGCACACTTTCCTCGTGAAGGTGTCTTTGTTTCAAATGGCACACACTG
GTTGTAACACAAAGGAATTTTTATGAACCACAAATCATTACTACAGACAAC
ACATTTGTGTCTGGTAACTGTGATGTTGTAATAGGAATTGTCAACAACACAG
TTTATGATCCTTTGCAACCTGAATTAGACTCATTCAAGGAGGAGTTAGATAAA
TATTTTAAGAATCATACATCACCAGATGTTGATTAGGTGACATCTCTGGCATT
AATGCTTCAGTTGTAAACATTCAAAAAGAAATTGACCGCCTCAATGAGGTTG
CCAAGAATTTAAATGAATCTCTCATCGATCTCCAAGAACTTGGAAAGTATGA
GCAGTATATAAAATGGCCATGGTACATTTGGCTAGGTTTTATAGCTGGCTTGA
TTGCCATAGTAATGGTGACAATTATGCTTTGCTGTATGACCAGTTGCTGTAGT
TGTCTCAAGGGCTGTTGTTCTTGTGGATCCTGCTGCAAATTTGATGAAGACG
ACTCTGAGCCAGTGCTCAAAGGAGTCAAATTACATTACACATAA

SEQ ID NO: 81

>Wildtype SARS-CoV-2 spike protein mRNA

AUGUUUGUUUUUCUUGUUUUAUUGCCACUAGUCUCUAGUCAGUGUGUUA
AUCUUACAACCAGAACUCAAUUACCCCCUGCAUACACUAAUUCUUUCAC
ACGUGGUGUUUAUUACCCUGACAAAGUUUUCAGAUCCUCAGUUUUACAU
UCAACUCAGGACUUGUUCUUACCUUUCUUUUCCAAUGUUACUUGGUUCC
AUGCUAUACAUGUCUCUGGGACCAAUGGUACUAAGAGGUUUGAUAACCC
UGUCCUACCAUUUAAUGAUGGUGUUUAUUUUGCUUCCACUGAGAAGUCU
AACAUAAUAAGAGGCUGGAUUUUUGGUACUACUUUAGAUUCGAAGACCC
AGUCCCUACUUAUUGUUAAUAACGCUACUAAUGUUGUUAUUAAAGUCUG
UGAAUUUCAAUUUUGUAAUGAUCCAUUUUUGGGUGUUUAUUACCACAA
AAACAACAAAAGUUGGAUGGAAAGUGAGUUCAGAGUUUAUUCUAGUGC

GAAUAAUUGCACUUUUGAAUAUGUCUCUCAGCCUUUUCUUAUGGACCUU
GAAGGAAAACAGGGUAAUUUCAAAAAUCUUAGGGAAUUUGUGUUUAAG
AAUAUUGAUGGUUAUUUUAAAAUAUAUUCUAAGCACACGCCUAUUAAU
UUAGUGCGUGAUCUCCCUCAGGGUUUUUCGGCUUUAGAACCAUUGGUAG
AUUUGCCAAUAGGUAUUAACAUCACUAGGUUUCAAACUUUACUUGCUUU
ACAUAGAAGUUAUUUGACUCCUGGUGAUUCUUCUUCAGGUUGGACAGCU
GGUGCUGCAGCUUAUUAUGUGGGUUAUCUUCAACCUAGGACUUUUCUAU
UAAAAUAUAAUGAAAAUGGAACCAUUACAGAUGCUGUAGACUGUGCACU
UGACCCUCUCUCAGAAACAAAGUGUACGUUGAAAUCCUUCACUGUAGAA
AAAGGAAUCUAUCAAACUUCUAACUUUAGAGUCCAACCAACAGAAUCUA
UUGUUAGAUUUCCUAAUAUUACAAACUUGUGCCCUUUUGGUGAAGUUUU
UAACGCCACCAGAUUUGCAUCUGUUUAUGCUUGGAACAGGAAGAGAAUC
AGCAACUGUGUUGCUGAUUAUUCUGUCCUAUAUAAUUCCGCAUCAUUUU
CCACUUUUAAGUGUUAUGGAGUGUCUCCUACUAAAUUAAAUGAUCUCUG
CUUUACUAAUGUCUAUGCAGAUUCAUUUGUAAUUAGAGGUGAUGAAGU
CAGACAAAUCGCUCCAGGGCAAACUGGAAAGAUUGCUGAUUAUAAUUAU
AAAUUACCAGAUGAUUUUACAGGCUGCGUUAUAGCUUGGAAUUCUAACA
AUCUUGAUUCUAAGGUUGGUGGUAAUUAUAAUUACCUGUAUAGAUUGU
UUAGGAAGUCUAAUCUCAAACCUUUUGAGAGAGAUAUUUCAACUGAAAU
CUAUCAGGCCGGUAGCACACCUUGUAAUGGUGUUGAAGGUUUUAAUUGU
UACUUUCCUUUACAAUCAUAUGGUUUCCAACCCACUAAUGGUGUUGGUU
ACCAACCAUACAGAGUAGUAGUACUUUCUUUUGAACUUCUACAUGCACC
AGCAACUGUUUGUGGACCUAAAAAGUCUACUAAUUUGGUUAAAAACAAA
UGUGUCAAUUUCAACUUCAAUGGUUUAACAGGCACAGGUGUUCUUACUG
AGUCUAACAAAAAGUUUCUGCCUUUCCAACAAUUUGGCAGAGACAUUGC
UGACACUACUGAUGCUGUCCGUGAUCCACAGACACUUGAGAUUCUUGAC
AUUACACCAUGUUCUUUUGGUGGUGUCAGUGUUAUAACACCAGGAACAA
AUACUUCUAACCAGGUUGCUGUUCUUUAUCAGGAUGUUAACUGCACAGA
AGUCCCUGUUGCUAUUCAUGCAGAUCAACUUACUCCUACUUGGCGUGUU
UAUUCUACAGGUUCUAAUGUUUUUCAAACACGUGCAGGCUGUUUAAUAG
GGGCUGAACAUGUCAACAACUCAUAUGAGUGUGACAUACCCAUUGGUGC
AGGUAUAUGCGCUAGUUAUCAGACUCAGACUAAUUCUCCUCGGCGGGCA
CGUAGUGUAGCUAGUCAAUCCAUCAUUGCCUACACUAUGUCACUUGGUG
CAGAAAAUUCAGUUGCUUACUCUAAUAACUCUAUUGCCAUACCCACAAA
UUUUACUAUUAGUGUUACCACAGAAAUUCUACCAGUGUCUAUGACCAAG
ACAUCAGUAGAUUGUACAAUGUACAUUUGUGGUGAUUCAACUGAAUGCA

GCAAUCUUUUGUUGCAAUAUGGCAGUUUUUGUACACAAUUAAACCGUGC
UUUAACUGGAAUAGCUGUUGAACAAGACAAAAACACCCAAGAAGUUUUU
GCACAAGUCAAACAAAUUUACAAACACCACCAAUUAAAGAUUUUGGUG
GUUUUAAUUUUUCACAAAUAUUACCAGAUCCAUCAAAACCAAGCAAGAG
GUCAUUUAUUGAAGAUCUACUUUCAACAAAGUGACACUUGCAGAUGCU
GGCUUCAUCAAACAAUAUGGUGAUUGCCUUGGUGAUAUUGCUGCUAGAG
ACCUCAUUUGUGCACAAAAGUUUAACGGCCUUACUGUUUUGCCACCUUU
GCUCACAGAUGAAAUGAUUGCUCAAUACACUUCUGCACUGUUAGCGGGU
ACAAUCACUUCUGGUUGGACCUUUGGUGCAGGUGCUGCAUUACAAAUAC
CAUUUGCUAUGCAAAUGGCUUAUAGGUUUAAUGGUAUUGGAGUUACAC
AGAAUGUUCUCUAUGAGAACCAAAAAUUGAUUGCCAACCAAUUUAAUAG
UGCUAUUGGCAAAAUUCAAGACUCACUUUCUUCCACAGCAAGUGCACUU
GGAAAACUUCAAGAUGUGGUCAACCAAAAUGCACAAGCUUUAAACACGC
UUGUUAAACAACUUAGCUCCAAUUUUGGUGCAAUUUCAAGUGUUUUAAA
UGAUAUCCUUUCACGUCUUGACAAAGUUGAGGCUGAAGUGCAAAUUGAU
AGGUUGAUCACAGGCAGACUUCAAAGUUUGCAGACAUAUGUGACUCAAC
AAUUAAUUAGAGCUGCAGAAAUCAGAGCUUCUGCUAAUCUUGCUGCUAC
UAAAAUGUCAGAGUGUGUACUUGGACAAUCAAAAAGAGUUGAUUUUUG
UGGAAAGGGCUAUCAUCUUAUGUCCUUCCCUCAGUCAGCACCUCAUGGU
GUAGUCUUCUUGCAUGUGACUUAUGUCCCUGCACAAGAAAAGAACUUCA
CAACUGCUCCUGCCAUUUGUCAUGAUGGAAAAGCACACUUUCCUCGUGA
AGGUGUCUUUGUUUCAAAUGGCACACACUGGUUUGUAACACAAAGGAAU
UUUUAUGAACCACAAAUCAUUACUACAGACAACACAUUUGUGUCUGGUA
ACUGUGAUGUUGUAAUAGGAAUUGUCAACAACACAGUUUAUGAUCCUUU
GCAACCUGAAUUAGACUCAUUCAAGGAGGAGUUAGAUAAAUAUUUUAA
GAAUCAUACAUCACCAGAUGUUGAUUUAGGUGACAUCUCUGGCAUUAAU
GCUUCAGUUGUAAACAUUCAAAAAGAAAUUGACCGCCUCAAUGAGGUUG
CCAAGAAUUUAAAUGAAUCUCUCAUCGAUCUCCAAGAACUUGGAAAGUA
UGAGCAGUAUAUAAAAUGGCCAUGGUACAUUUGGCUAGGUUUUAUAGC
UGGCUUGAUUGCCAUAGUAAUGGUGACAAUUAUGCUUUGCUGUAUGACC
AGUUGCUGUAGUUGUCUCAAGGGCUGUUGUUCUUGUGGAUCCUGCUGCA
AAUUUGAUGAAGACGACUCUGAGCCAGUGCUCAAAGGAGUCAAAUUACA
UUACACAUAA

SEQ ID NO: 82

>Influenza A virus H1N1 (A/Wisconsin/588/2019) HA amino acid sequence

MKAILVVMLYTFTTANADTLCIGYHANNSTDTVDTVLEKNVTVTHSVNLLED

KHNGKLCKLRGVAPLHLGKCNIAGWILGNPECESLSTARSWSYIVETSNSDNG
TCYPGDFINYEELREQLSSVSSFERFEIFPKTSSWPNHDSDNGVTAACPHAGAK
SFYKNLIWLVKKGKSYPKINQTYINDKGKEVLVLWGIHHPPTIADQQSLYQNA
DAYVFVGTSRYSKKFKPEIATRPKVRDQEGRMNYYWTLVEPGDKITFEATGNL
VAPRYAFTMERDAGSGIIISDTPVHDCNTTCQTPEGAINTSLPFQNVHPITIGKCP
KYVKSTKLRLATGLRNVPSIQSRGLFGAIAGFIEGGWTGMVDGWYGYHHQNE
QGSGYAADLKSTQNAIDKITNKVNSVIEKMNTQFTAVGKEFNHLEKRIENLNK
KVDDGFLDIWTYNAELLVLLENERTLDYHDSNVKNLYEKVRNQLKNNAKEIG
NGCFEFYHKCDNTCMESVKNGTYDYPKYSEEAKLNREKIDGVKLDSTRIYQIL
AIYSTVASSLVLVVSLGAISFWMCSNGSLQCRICI

SEQ ID NO: 83

>Influenza A virus H3N2 (A/Cambodia/e0826360/2020) HA amino acid sequence

MKTIIALSYILCLVFAQKIPGNDNSTATLCLGHHAVPNGTIVKTITNDRIEVTNAT
ELVQNSSIGEICDSPHQILDGGNCTLIDALLGDPQCDGFQNKEWDLFVERSRAN
SNCYPYDVPDYASLRSLVASSGTLEFKNESFNWTGVKQNGTSSACIRGSSSSFFS
RLNWLTHLNYTYPALNVTMPNNEQFDKLYIWGVHHPSTDKDQISLFAQPSGRI
TVSTKRSQQAVIPNIGSRPRIRDIPSRISIYWTIVKPGDILLINSTGNLIAPRGYFKI
RSGKSSIMRSDAPIGKCKSECITPNGSIPNDKPFQNVNRITYGACPRYVKQSTLK
LATGMRNVPEKQTRGIFGAIAGFIENGWEGMVDGWYGFRHQNSEGRGQAAD
LKSTQAAIDQINGKLNRLIGKTNEKFHQIEKEFSEVEGRVQDLEKYVEDTKIDL
WSYNAELLVALENQHTIDLTDSEMNKLFEKTKKQLRENAEDMGNGCFKIYHK
CDNACIGSIRNETYDHNVYRDEALNNRFQIKGVELKSGYKDWILWISFAMSCF
LLCIALLGFIMWACQKGNIRCNICI

SEQ ID NO: 84

>Influenza B virus Victoria lineage (B/Washington/02/2019) HA amino acid sequence

MKAIIVLLMVVTSNADRICTGITSSNSPHVVKTATQGEVNVTGVIPLTTTPTKSH
FANLKGTETRGKLCPKCLNCTDLDVALGRPKCTGKIPSARVSILHEVRPVTSGC
FPIMHDRTKIRQLPNLLRGYEHVRLSTHNVINAEDAPGRPYEIGTSGSCPNITNG
NGFFATMAWAVPKNKTATNPLTIEVPYICTEGEDQITVWGFHSDNETQMAKLY
GDSKPQKFTSSANGVTTHYVSQIGGFPNQTEDGGLPQSGRIVVDYMVQKSGKT
GTITYQRGILLPQKVWCASGRSKVIKGSLPLIGEADCLHEKYGGLNKSKPYYT
GEHAKAIGNCPIWVKTPLKLANGTKYRPPAKLLKERGFFGAIAGFLEGGWEG
MIAGWHGYTSHGAHGVAVAADLKSTQEAINKITKNLNSLSELEVKNLQRLSGA
MDELHNEILELDEKVDDLRADTISSQIELAVLLSNEGIINSEDEHLLALERKLKK
MLGPSAVEIGNGCFETKHKCNQTCLDRIAAGTFDAGEFSLPTFDSLNITAASLN
DDGLDNHTILLYYSTAASSLAVTLMIAIFVVYMVSRDNVSCSICL

<div align="center">SEQ ID NO: 85</div>

>Influenza B virus Yamagata lineage (B/Phuket/3073/2013) HA amino acid sequence

MKAIIVLLMVVTSNADRICTGITSSNSPHVVKTATQGEVNVTGVIPLTTTPTKSY
FANLKGTRTRGKLCPDCLNCTDLDVALGRPMCVGTTPSAKASILHEVRPVTSG
CFPIMHDRTKIRQLPNLLRGYEKIRLSTQNVIDAEKAPGGPYRLGTSGSCPNATS
KIGFFATMAWAVPKDNYKNATNPLTVEVPYICTEGEDQITVWGFHSDNKTQMK
SLYGDSNPQKFTSSANGVTTHYVSQIGDFPDQTEDGGLPQSGRIVVDYMMQKP
GKTGTIVYQRGVLLPQKVWCASGRSKVIKGSLPLIGEADCLHEEYGGLNKSKP
YYTGKHAKAIGNCPIWVKTPLKLANGTKYRPPAKLLKERGFFGAIAGFLEGG
WEGMIAGWHGYTSHGAHGVAVAADLKSTQEAINKITKNLNSLSELEVKNLQR
LSGAMDELHNEILELDEKVDDLRADTISSQIELAVLLSNEGIINSEDEHLLALER
KLKKMLGPSAVDIGNGCFETKHKCNQTCLDRIAAGTFNAGEFSLPTFDSLNITA
ASLNDDGLDNHTILLYYSTAASSLAVTLMLAIFIVYMVSRDNVSCSICL

<div align="right">SEQ ID NO: 86</div>

>Influenza A virus H1N1 (A/Wisconsin/588/2019) HA CDS

ATGAAGGCCATCCTGGTGGTGATGCTGTACACCTTCACCACCGCCAACGCC
GACACCCTGTGCATCGGCTACCACGCCAACAACAGCACCGACACCGTGGAC
ACCGTGCTGGAGAAGAACGTGACCGTGACCCACAGCGTGAACCTGCTGGA
GGACAAGCACAACGGCAAGCTGTGCAAGCTGCGGGGCGTGGCCCCCCTGC
ACCTGGGCAAGTGCAACATCGCCGGCTGGATCCTGGGCAACCCCGAGTGCG
AGTCCCTGAGCACCGCCCGCTCCTGGAGCTACATCGTGGAGACCTCCAACA
GCGACAACGGCACCTGCTACCCCGGGGACTTCATCAACTACGAGGAGCTGC
GGGAGCAGCTGTCCAGCGTGTCCAGCTTCGAGCGCTTCGAGATCTTCCCCA
AGACCTCCAGCTGGCCCAACCACGACTCCGACAACGGCGTGACCGCCGCC
TGCCCCCACGCCGGGGCCAAGAGCTTCTACAAGAACCTGATCTGGCTGGTG
AAGAAGGGCAAGTCCTACCCCAAGATCAACCAGACCTACATCAACGACAA
GGGGAAGGAGGTCCTCGTGCTGTGGGGCATCCACCACCCCCCCACCATCGC
CGACCAGCAGAGCCTCTACCAGAACGCCGACGCCTACGTCTTCGTGGGGGAC
CTCCCGGTACAGCAAGAAGTTCAAGCCCGAGATCGCCACCCGCCCCAAGGT
CCGGGACCAGGAGGGCCGCATGAACTACTACTGGACCCTGGTGGAGCCGG
GGGACAAGATCACCTTCGAGGCCACCGGCAACCTCGTCGCCCCGCGGTACG
CCTTCACGATGGAGCGCGACGCCGGGTCCGGCATCATCATCAGCGACACCC
CCGTGCACGACTGCAACACGACCTGCCAGACGCCCGAGGGGGCGATCAAC
ACCTCCCTGCCGTTCCAGAACGTCCACCCGATCACGATCGGCAAGTGCCCC
AAGTACGTGAAGTCCACCAAGCTCCGGCTGGCCACGGGGCTCCGCAACGTC
CCCTCCATCCAGAGCCGGGGCCTGTTCGGGGCGATCGCCGGCTTCATCGAG

GGGGGCTGGACCGGGATGGTGGACGGCTGGTACGGGTACCACCACCAGAA
CGAGCAGGGCTCCGGGTACGCGGCCGACCTCAAGTCGACGCAGAACGCGA
TCGACAAGATCACCAACAAGGTCAACAGCGTGATCGAGAAGATGAACACG
CAGTTCACCGCCGTCGGCAAGGAGTTCAACCACCTGGAGAAGCGCATCGA
GAACCTCAACAAGAAGGTGGACGACGGGTTCCTGGACATCTGGACGTACA
ACGCGGAGCTCCTGGTCCTCCTGGAGAACGAGCGGACCCTCGACTACCACG
ACTCCAACGTGAAGAACCTGTACGAGAAGGTCCGCAACCAGCTCAAGAAC
AACGCCAAGGAGATCGGCAACGGGTGCTTCGAGTTCTACCACAAGTGCGAC
AACACGTGCATGGAGTCGGTGAAGAACGGCACCTACGACTACCCGAAGTAC
AGCGAGGAGGCGAAGCTGAACCGGGAGAAGATCGACGGGGTCAAGCTCGA
CTCCACGCGCATCTACCAGATCCTGGCCATCTACTCGACCGTGGCGAGCTCC
CTCGTCCTGGTGGTCTCGCTCGGCGCCATCAGCTTCTGGATGTGCTCCAACG
GGTCGCTGCAGTGCCGGATCTGCATCTGA

<div align="right">SEQ ID NO: 87</div>

>Influenza A virus H3N2 (A/Cambodia/e0826360/2020) HA CDS

ATGAAGACCATCATCGCCCTGAGCTACATCCTGTGCCTGGTGTTCGCCCAGA
AGATCCCCGGCAACGACAACAGCACCGCCACCCTGTGCCTGGGCCACCAC
GCCGTGCCCAACGGCACCATCGTGAAGACCATCACCAACGACCGGATCGAG
GTGACCAACGCCACCGAGCTGGTGCAGAACTCCAGCATCGGCGAGATCTGC
GACTCCCCCACCAGATCCTGGACGGCGGCAACTGCACCCTGATCGACGCC
CTGCTGGGCGACCCCCAGTGCGACGGGTTCCAGAACAAGGAGTGGGACCT
GTTCGTGGAGCGGAGCCGCGCCAACTCCAACTGCTACCCCTACGACGTGCC
CGACTACGCCAGCCTGCGGTCCCTGGTGGCCAGCTCCGGCACCCTGGAGTT
CAAGAACGAGAGCTTCAACTGGACCGGGGTCAAGCAGAACGGCACCTCCA
GCGCCTGCATCCGCGGGTCCAGCTCCAGCTTCTTCTCCCGGCTGAACTGGCT
CACCCACCTGAACTACACCTACCCCGCCCTCAACGTGACCATGCCCAACAA
CGAGCAGTTCGACAAGCTGTACATCTGGGGCGTCCACCACCCCAGCACCGA
CAAGGACCAGATCTCCCTCTTCGCCCAGCCCAGCGGGCGCATCACCGTGTC
CACGAAGCGGAGCCAGCAGGCCGTCATCCCCAACATCGGCTCCCGCCCGCG
GATCCGCGACATCCCCAGCCGGATCTCCATCTACTGGACGATCGTGAAGCCG
GGGGACATCCTGCTCATCAACAGCACCGGCAACCTGATCGCCCCCGCGGG
TACTTCAAGATCCGGTCCGGCAAGAGCTCGATCATGCGCTCCGACGCCCCGA
TCGGGAAGTGCAAGAGCGAGTGCATCACCCCCAACGGCTCGATCCCGAACG
ACAAGCCCTTCCAGAACGTCAACCGGATCACGTACGGGGCCTGCCCGCGCT
ACGTGAAGCAGTCCACGCTCAAGCTGGCCACCGGCATGCGGAACGTCCCCG
AGAAGCAGACCCGCGGGATCTTCGGCGCCATCGCGGGGTTCATCGAGAACG

GCTGGGAGGGGATGGTGGACGGCTGGTACGGGTTCCGGCACCAGAACAGC
GAGGGCCGCGGGCAGGCCGCGGACCTCAAGTCGACGCAGGCCGCGATCGA
CCAGATCAACGGCAAGCTGAACCGGCTCATCGGGAAGACGAACGAGAAGT
TCCACCAGATCGAGAAGGAGTTCTCCGAGGTCGAGGGCCGCGTGCAGGAC
CTGGAGAAGTACGTCGAGGACACCAAGATCGACCTCTGGAGCTACAACGCC
GAGCTGCTCGTGGCGCTGGAGAACCAGCACACCATCGACCTCACGGACTCG
GAGATGAACAAGCTGTTCGAGAAGACGAAGAAGCAGCTCCGGGAGAACGC
CGAGGACATGGGGAACGGCTGCTTCAAGATCTACCACAAGTGCGACAACGC
GTGCATCGGGTCCATCCGCAACGAGACCTACGACCACAACGTCTACCGGGA
CGAGGCCCTGAACAACCGCTTCCAGATCAAGGGCGTGGAGCTCAAGAGCG
GGTACAAGGACTGGATCCTGTGGATCTCGTTCGCGATGTCCTGCTTCCTCCT
GTGCATCGCCCTCCTGGGCTTCATCATGTGGGCGTGCCAGAAGGGGAACAT
CCGGTGCAACATCTGCATCTGA

SEQ ID NO: 88

>Influenza B virus Victoria lineage (B/Washington/02/2019) HA CDS

ATGAAGGCCATCATCGTGCTGCTGATGGTGGTGACCAGCAACGCCGACCGG
ATCTGCACCGGCATCACCAGCTCCAACAGCCCCACGTGGTGAAGACCGCC
ACCCAGGGCGAGGTGAACGTGACCGGCGTGATCCCCCTGACCACCACCCCC
ACCAAGTCCCACTTCGCCAACCTGAAGGGCACCGAGACCCGGGGCAAGCT
GTGCCCCAAGTGCCTGAACTGCACCGACCTGGACGTGGCCCTGGGCCGCCC
CAAGTGCACCGGCAAGATCCCCAGCGCCCGGGTGTCCATCCTGCACGAGGT
GCGCCCCGTGACCAGCGGCTGCTTCCCCATCATGCACGACCGCACCAAGAT
CCGGCAGCTGCCCAACCTGCTGCGGGGGTACGAGCACGTCCGCCTGTCCAC
CCACAACGTGATCAACGCCGAGGACGCCCCCGGCCGGCCCTACGAGATCGG
GACCAGCGGCTCCTGCCCCAACATCACCAACGGGAACGGCTTCTTCGCCAC
CATGGCCTGGGCCGTCCCCAAGAACAAGACCGCCACCAACCCCCTGACCAT
CGAGGTGCCGTACATCTGCACCGAGGGGGAGGACCAGATCACCGTCTGGGG
CTTCCACAGCGACAACGAGACGCAGATGGCCAAGCTGTACGGGGACTCCA
AGCCCCAGAAGTTCACGAGCTCCGCCAACGGCGTGACCACCCACTACGTCA
GCCAGATCGGGGGCTTCCCGAACCAGACGGAGGACGGGGGCCTGCCCCAG
TCCGGGCGCATCGTGGTCGACTACATGGTGCAGAAGTCCGGCAAGACGGGG
ACCATCACGTACCAGCGGGGCATCCTGCTGCCGCAGAAGGTCTGGTGCGCC
TCCGGGCGCAGCAAGGTGATCAAGGGCTCCCTGCCCCTCATCGGGGAGGCC
GACTGCCTGCACGAGAAGTACGGCGGGCTCAACAAGAGCAAGCCGTACTA
CACCGGCGAGCACGCCAAGGCCATCGGGAACTGCCCCATCTGGGTCAAGAC
CCCGCTGAAGCTCGCCAACGGCACGAAGTACCGGCCCCGGCCAAGCTGCT

CAAGGAGCGCGGGTTCTTCGGCGCCATCGCCGGGTTCCTGGAGGGCGGGTG
GGAGGGCATGATCGCCGGGTGGCACGGCTACACGTCCCACGGGGCCCACG
GCGTGGCGGTCGCCGCGGACCTCAAGAGCACCCAGGAGGCCATCAACAAG
ATCACGAAGAACCTGAACTCCCTCAGCGAGCTGGAGGTGAAGAACCTCCA
GCGCCTGTCCGGGGCGATGGACGAGCTCCACAACGAGATCCTGGAGCTCGA
CGAGAAGGTCGACGACCTGCGGGCCGACACCATCAGCTCGCAGATCGAGCT
CGCGGTGCTGCTCTCCAACGAGGGCATCATCAACAGCGAGGACGAGCACCT
GCTCGCCCTGGAGCGGAAGCTCAAGAAGATGCTGGGGCCCTCGGCGGTCG
AGATCGGCAACGGGTGCTTCGAGACCAAGCACAAGTGCAACCAGACGTGC
CTCGACCGCATCGCCGCGGGCACGTTCGACGCCGGGGAGTTCTCCCTGCCG
ACCTTCGACAGCCTCAACATCACGGCGGCCTCGCTGAACGACGACGGCCTC
GACAACCACACCATCCTGCTCTACTACTCCACCGCGGCCAGCTCGCTGGCG
GTGACGCTCATGATCGCCATCTTCGTCGTGTACATGGTCTCCCGCGACAACG
TGAGCTGCTCGATCTGCCTGTGA

SEQ ID NO: 89

>Influenza B virus/Yamagata lineage (B/Phuket/3073/2013) HA CDS

ATGAAGGCCATCATCGTGCTGCTGATGGTGGTGACCAGCAACGCCGACCGG
ATCTGCACCGGCATCACCAGCTCCAACAGCCCCCACGTGGTGAAGACCGCC
ACCCAGGGCGAGGTGAACGTGACCGGCGTGATCCCCCTGACCACCACCCCC
ACCAAGTCCTACTTCGCCAACCTGAAGGGCACCCGGACCCGCGGCAAGCTG
TGCCCCGACTGCCTGAACTGCACCGACCTGGACGTGGCCCTGGGCCGGCCC
ATGTGCGTGGGCACCACCCCCAGCGCCAAGGCCTCCATCCTGCACGAGGTG
CGCCCCGTGACCAGCGGCTGCTTCCCCATCATGCACGACCGCACCAAGATC
CGGCAGCTGCCCAACCTGCTGCGGGGGTACGAGAAGATCCGCCTGTCCACC
CAGAACGTCATCGACGCCGAGAAGGCCCCCGGCGGGCCCTACCGGCTGGG
CACCAGCGGGTCCTGCCCCAACGCCACCAGCAAGATCGGCTTCTTCGCCAC
CATGGCCTGGGCCGTGCCCAAGGACAACTACAAGAACGCCACCAACCCCCT
GACCGTCGAGGTGCCCTACATCTGCACCGAGGGGGAGGACCAGATCACGGT
CTGGGGCTTCCACTCCGACAACAAGACCCAGATGAAGTCCCTGTACGGGGA
CTCCAACCCGCAGAAGTTCACGAGCTCCGCCAACGGCGTGACCACGCACTA
CGTCAGCCAGATCGGGGACTTCCCGGACCAGACCGAGGACGGCGGGCTGC
CCCAGTCCGGCCGCATCGTGGTCGACTACATGATGCAGAAGCCCGGGAAGA
CGGGCACCATCGTGTACCAGCGGGGGGTCCTGCTGCCGCAGAAGGTGTGGT
GCGCCAGCGGCCGCTCCAAGGTCATCAAGGGGAGCCTGCCCCTCATCGGCG
AGGCCGACTGCCTGCACGAGGAGTACGGGGGCCTCAACAAGTCCAAGCCG
TACTACACGGGGAAGCACGCCAAGGCCATCGGCAACTGCCCGATCTGGGTG

AAGACCCCCCTGAAGCTCGCCAACGGGACGAAGTACCGGCCGCCCGCCAA
GCTGCTCAAGGAGCGCGGCTTCTTCGGGGCCATCGCCGGCTTCCTGGAGGG
GGGCTGGGAGGGGATGATCGCCGGCTGGCACGGGTACACCAGCCACGGCG
CCCACGGGGTCGCGGTGGCCGCGGACCTCAAGTCCACGCAGGAGGCCATC
AACAAGATCACCAAGAACCTGAACAGCTCTCCGAGCTGGAGGTCAAGAA
CCTCCAGCGCCTGAGCGGCGCGATGGACGAGCTCCACAACGAGATCCTGGA
GCTCGACGAGAAGGTGGACGACCTGCGGGCCGACACGATCTCGTCCCAGAT
CGAGCTCGCGGTCCTGCTCAGCAACGAGGGGATCATCAACTCGGAGGACGA
GCACCTGCTCGCCCTGGAGCGGAAGCTCAAGAAGATGCTGGGCCCGTCCGC
GGTGGACATCGGGAACGGCTGCTTCGAGACCAAGCACAAGTGCAACCAGA
CGTGCCTCGACCGCATCGCCGCGGGGACCTTCAACGCCGGCGAGTTCAGCC
TGCCCACGTTCGACTCGCTCAACATCACCGCGGCCTCCCTGAACGACGACG
GGCTCGACAACCACACGATCCTGCTCTACTACAGCACCGCGGCCTCGTCCC
TGGCGGTCACGCTCATGCTGGCCATCTTCATCGTGTACATGGTCAGCCGCGA
CAACGTGTCGTGCTCCATCTGCCTCTGA

SEQ ID NO: 90

> 5'UTR - Moderna

GGGAAATAAGAGAGAAAGAAGAGTAAGAAGAAATATAAGACCCCGGCGC
CGCCACC

SEQ ID NO: 91

>3'UTR-Moderna

GCTGGAGCCTCGGTGGCCTAGCTTCTTGCCCCTTGGGCCTCCCCCCAGCCCC
TCCTCCCCTTCCTGCACCCGTACCCCGTGGTCTTTGAATAAAGTCTGAGTG
GGCGGC

SEQ ID NO: 92

>Influenza A virus H1N1 (A/Wisconsin/588/2019) HA Moderna

ATGAAGGCCATCCTGGTCGTGATGCTGTACACCTTCACCACCGCCAACGCCG
ACACCCTGTGCATCGGCTACCACGCCAACAACAGCACCGACACCGTGGACA
CCGTGCTGGAGAAGAACGTGACCGTGACCCACAGCGTGAACCTGCTGGAG
GACAAGCACAACGGCAAGCTGTGCAAGCTGAGGGGAGTGGCACCCCTGCA
CCTGGGCAAGTGCAACATCGCCGGCTGGATCCTGGGCAACCCCGAGTGCGA
GAGCCTGAGCACAGCCCGGAGCTGGAGCTACATCGTGGAGACCAGCAACA
GCGACAACGGCACCTGTTACCCCGGCGACTTCATCAACTACGAGGAGCTGC
GGGAGCAGCTGAGCAGCGTGAGCAGCTTCGAGCGGTTCGAGATCTTCCCCA
AGACCAGCAGCTGGCCCAACCACGACAGCGACAACGGCGTGACAGCAGCC
TGTCCACACGCCGGAGCCAAGAGCTTCTACAAGAACCTGATCTGGCTGGTG

AAGAAGGGCAAGAGCTACCCCAAGATCAACCAGACCTACATCAACGACAA
GGGCAAGGAGGTGCTGGTGCTGTGGGGCATCCACCACCCACCTACCATCGC
CGACCAGCAGAGCCTGTACCAGAACGCCGACGCCTACGTGTTCGTGGGCAC
CAGCCGGTACAGCAAGAAGTTCAAGCCAGAGATCGCCACCCGGCCCAAGG
TGAGAGACCAGGAGGGCCGGATGAACTACTACTGGACCCTGGTGGAGCCC
GGAGACAAGATTACCTTCGAGGCCACCGGCAACCTGGTGGCCCCTCGGTAC
GCCTTCACCATGGAACGGGACGCTGGCAGCGGCATCATCATCAGCGACACT
CCCGTGCACGACTGCAACACCACCTGCCAGACTCCCGAGGGCGCTATCAAC
ACCAGCCTGCCCTTCCAGAACGTGCACCCCATCACCATCGGCAAGTGCCCC
AAGTACGTAAAGAGCACCAAATTGCGGCTGGCCACCGGACTCAGGAACGT
GCCCAGCATCCAAAGCCGGGGCCTGTTTGGCGCAATCGCCGGCTTCATCGA
GGGCGGCTGGACTGGCATGGTGGACGGCTGGTACGGCTACCACCACCAGA
ACGAACAGGGGAGCGGCTACGCAGCTGACCTGAAGAGCACCCAGAACGCC
ATCGACAAGATCACCAACAAGGTGAACAGCGTGATCGAGAAGATGAACAC
CCAGTTCACCGCCGTGGGCAAGGAGTTCAACCACCTGGAGAAGCGGATCG
AGAACCTGAACAAGAAGGTGGACGACGGCTTCCTGGACATCTGGACCTAC
AACGCCGAGCTGCTGGTTCTGCTGGAGAACGAGCGGACCCTGGACTATCAC
GACAGCAACGTGAAGAACCTGTACGAGAAGGTGCGGAACCAGCTGAAGAA
CAACGCCAAGGAGATCGGCAACGGCTGCTTCGAGTTCTACCACAAGTGCGA
CAACACCTGCATGGAGAGCGTGAAGAACGGCACCTACGACTACCCCAAGTA
CAGCGAGGAGGCCAAGCTGAACCGGGAGAAGATCGACGGCGTGAAGCTGG
ACAGCACCCGGATCTACCAGATCCTGGCCATCTACAGCACCGTGGCCAGCA
GCCTGGTGCTGGTGGTGAGCCTGGGCGCCATCAGCTTCTGGATGTGCAGCA
ACGGCAGCCTGCAGTGCCGGATCTGCATC

SEQ ID NO: 93

>Influenza A virus H3N2 (A/Cambodia/e0826360/2020) HA Moderna

ATGAAGACCATCATCGCCCTGAGCTACATCCTGTGCCTGGTGTTCGCCCAGA
AGATCCCCGGCAACGATAACAGCACCGCCACCCTGTGTCTGGGACACCACG
CCGTGCCCAACGGCACCATCGTGAAGACTATCACCAACGACCGGATCGAGG
TGACCAACGCCACCGAGCTGGTGCAGAACAGCAGCATCGGCGAGATCTGC
GACAGCCCTCACCAGATCCTGGACGGCGGCAACTGCACCCTGATCGACGCA
CTGCTGGGCGACCCTCAGTGCGACGGCTTTCAGAACAAGGAGTGGGACCT
GTTCGTGGAGAGATCGCGGGCCAACAGCAACTGCTACCCCTACGACGTCCC
CGACTACGCAAGCCTGAGAAGCCTCGTGGCCTCAAGCGGCACCCTGGAGTT
CAAGAACGAGAGCTTCAACTGGACCGGCGTGAAGCAGAACGGCACCTCAA
GCGCCTGCATCCGGGGCTCCAGCAGCAGCTTCTTCTCACGGCTGAACTGGC

TGACCCACCTGAACTACACCTACCCCGCCCTGAACGTGACCATGCCCAACA
ACGAGCAGTTCGACAAGCTGTACATCTGGGGAGTGCACCATCCCAGCACCG
ACAAGGACCAGATTAGCCTGTTCGCCCAGCCCAGCGGCCGGATCACCGTGA
GCACCAAGCGGAGCCAGCAGGCCGTGATCCCCAACATCGGCTCTCGGCCCA
GAATCCGGGACATCCCCAGCCGGATCAGCATCTACTGGACCATTGTGAAGCC
CGGCGACATCCTGCTGATCAACTCCACCGGCAACCTGATCGCCCCTCGGGGG
CTATTTCAAGATCCGGAGCGGCAAGAGCAGCATCATGCGGAGCGACGCCCC
TATCGGCAAGTGCAAGAGCGAGTGCATCACACCCAACGGAAGCATCCCCAA
CGACAAGCCCTTCCAGAACGTGAACCGGATAACCTACGGCGCCTGCCCTAG
ATACGTGAAGCAGAGCACCCTGAAGCTGGCCACCGGCATGCGGAACGTGCC
CGAGAAGCAGACTCGGGGCATCTTCGGCGCCATCGCCGGCTTCATCGAGAA
CGGCTGGGAGGGCATGGTGGACGGCTGGTACGGCTTCCGGCACCAGAACTC
TGAGGGCAGAGGACAGGCCGCAGACCTGAAGAGCACCCAGGCCGCCATCG
ACCAGATCAACGGCAAGCTGAACCGGCTGATCGGCAAGACCAACGAGAAG
TTCCACCAGATCGAGAAGGAGTTCAGCGAGGTGGAGGGCAGGGTACAGGA
CCTGGAGAAGTACGTGGAGGACACCAAGATCGACCTGTGGAGCTACAACG
CCGAGCTGCTGGTAGCCCTGGAGAACCAGCACACCATCGACCTGACCGACA
GCGAGATGAACAAGCTGTTCGAGAAGACCAAGAAGCAGCTGCGGGAGAAC
GCCGAGGACATGGGCAACGGCTGCTTCAAGATCTACCACAAGTGCGACAAC
GCCTGCATCGGCAGCATCCGGAACGAGACCTACGACCACAACGTGTACCGG
GACGAGGCCCTGAACAACCGGTTCCAGATCAAGGGCGTGGAGCTGAAGAG
CGGCTACAAGGACTGGATCCTGTGGATCAGCTTCGCCATGTCCTGCTTCCTG
CTGTGCATCGCCCTGCTGGGTTTCATCATGTGGGCCTGCCAGAAGGGCAACA
TCCGGTGCAACATCTGCATC

SEQ ID NO: 94

>Influenza B virus Victoria lineage (B/Washington/02/2019) HA CDS Moderna

ATGAAGGCCATCATCGTGCTGTTAATGGTGGTGACCAGCAACGCCGACCGG
ATCTGCACCGGCATCACCTCTAGCAACAGCCCTCACGTGGTGAAGACCGCC
ACACAGGGCGAGGTGAACGTGACCGGCGTGATTCCCCTGACCACCACCCCT
ACCAAGAGCCACTTCGCCAACCTGAAGGGAACCGAGACCCGGGGCAAGCT
GTGTCCCAAGTGCCTGAACTGCACCGACCTGGACGTGGCCCTGGGCAGACC
CAAGTGCACCGGCAAGATCCCCAGCGCCCGGGTGTCTATCCTGCACGAAGT
GCGGCCCGTGACTAGCGGCTGCTTCCCCATCATGCACGACCGGACCAAGAT
CCGGCAGCTGCCCAACCTGCTGCGGGGCTACGAGCACGTGCGGCTGAGCA
CCCACAACGTGATCAACGCCGAAGACGCACCCGGGAGACCATACGAGATCG
GCACCAGCGGCTCTTGCCCCAACATCACCAACGGCAACGGCTTCTTCGCTA

CCATGGCCTGGGCCGTGCCAAAGAACAAGACTGCCACCAACCCTCTGACCA
TCGAGGTGCCCTACATCTGCACCGAGGGCGAGGACCAGATCACCGTGTGGG
GCTTCCACAGCGACAGCGAGACCCAGATGGCCAAGCTGTACGGCGACAGC
AAGCCCCAGAAGTTCACCAGCAGCGCCAACGGCGTGACCACCCACTACGT
GAGCCAGATCGGCGGCTTCCCCAACCAGACCGAGGACGGCGGCTTACCCCA
GAGCGGCCGGATCGTGGTGGACTACATGGTGCAGAAGAGCGGCAAGACCG
GCACCATCACCTACCAGCGGGGCATCCTGCTGCCACAGAAGGTGTGGTGCG
CCTCAGGGCGGTCAAAGGTGATCAAGGGCAGCCTGCCACTGATTGGCGAGG
CCGACTGCCTGCACGAGAAGTACGGCGGCCTGAACAAGAGCAAGCCCTACT
ACACCGGCGAGCACGCCAAGGCAATCGGCAACTGCCCCATCTGGGTGAAG
ACACCCCTGAAGCTGGCCAACGGCACCAAGTACCGGCCACCCGCCAAACT
GCTGAAGGAGCGGGGCTTCTTCGGCGCCATTGCCGGCTTCCTCGAAGGCGG
TTGGGAGGGCATGATCGCCGGCTGGCACGGCTACACTAGCCACGGCGCACA
CGGAGTAGCAGTGGCCGCCGACCTGAAGAGCACCCAGGAGGCCATCAACA
AGATCACCAAGAACCTGAACAGCCTGAGCGAGCTGGAGGTGAAGAATCTG
CAGCGGCTGTCTGGCGCTATGGACGAGCTGCACAACGAGATCCTGGAGCTG
GACGAGAAGGTGGACGACTTACGGGCCGACACCATCAGCAGCCAGATCGA
GCTGGCCGTGCTGCTCTCCAACGAGGGCATCATCAACAGCGAGGACGAGCA
CCTGCTCGCCCTGGAGCGGAAGCTGAAGAAGATGCTGGGCCCTTCTGCCGT
GGAGATCGGTAACGGCTGCTTCGAGACCAAGCACAAGTGCAACCAGACCT
GCCTGGATCGGATCGCAGCCGGCACCTTTGACGCCGGGGAGTTCAGCCTGC
CCACCTTCGACAGCCTGAACATCACCGCCGCCAGCCTGAACGACGACGGCC
TGGACAACCACACCATCCTGCTGTACTACTCTACAGCCGCTAGCAGCCTGGC
CGTGACCCTGATGATCGCCATCTTCGTGGTGTACATGGTGAGCCGGGACAAC
GTGAGCTGCAGCATCTGCCTG

<div align="right">SEQ ID NO: 95</div>

>Influenza B virus Yamagata lineage (B/Phuket/3073/2013) HA CDS Moderna

ATGAAGGCCATCATCGTGCTACTGATGGTGGTGACCAGCAACGCCGACCGG
ATCTGCACCGGCATCACCAGCAGCAACAGCCCGCACGTGGTGAAGACCGCC
ACCCAAGGCGAGGTGAACGTGACCGGCGTGATCCCACTGACCACCACTCCC
ACCAAGAGCTACTTCGCCAACCTGAAGGGCACACGGACTCGGGGCAAGCT
GTGCCCCGACTGCCTGAACTGCACCGACCTGGACGTGGCCCTGGGCAGACC
CATGTGCGTGGGCACCACCCCTTCTGCCAAGGCCAGCATCCTGCACGAGGT
GAGACCCGTGACCAGCGGGTGCTTCCCCATCATGCACGACCGGACCAAGAT
CCGGCAGCTGCCCAACCTGCTGCGGGGCTACGAGAAGATCCGGCTGAGCAC
CCAGAACGTGATCGACGCCGAGAAGGCCCCTGGAGGTCCCTACCGGCTGG

GCACCAGCGGAAGCTGCCCCAACGCCACCAGCAAGATCGGCTTCTTCGCCA
CCATGGCCTGGGCTGTGCCCAAGGACAACTACAAGAACGCCACCAATCCCC
TGACCGTGGAGGTGCCCTACATCTGCACCGAGGGCGAGGACCAGATCACCG
TGTGGGGCTTCCACAGCGACAACAAGACCCAGATGAAGAGCCTGTACGGC
GACAGCAATCCCAGAAGTTCACAAGCAGCGCCAACGGCGTGACCACCCA
CTACGTGAGCCAGATCGGCGACTTCCCCGACCAGACCGAGGACGGAGGGC
TGCCTCAGAGTGGCCGGATCGTGGTGGACTACATGATGCAGAAGCCCGGCA
AGACCGGCACCATCGTGTACCAGCGGGGCGTGCTGTTGCCTCAGAAAGTTT
GGTGTGCCAGCGGCAGGAGCAAGGTGATCAAGGGCAGCCTGCCCCTGATC
GGCGAGGCAGACTGCCTCCACGAGGAGTACGGCGGCCTGAACAAGAGCAA
GCCCTACTACACCGGCAAGCACGCCAAGGCCATCGGCAACTGCCCCATCTG
GGTGAAGACCCCTCTGAAGCTGGCCAACGGCACCAAGTACCGGCCACCAG
CCAAGCTGCTGAAGGAGCGGGGCTTCTTTGGCGCCATTGCCGGCTTCCTCG
AGGGAGGCTGGGAGGGCATGATCGCCGGCTGGCACGGCTACACAAGCCAC
GGCGCACGGAGTGGCTGTGGCTGCCGACCTGAAGAGCACCCAGGAGGC
CATCAACAAGATCACCAAGAACCTGAACAGCCTGAGCGAGCTGGAGGTGA
AGAACCTGCAGCGGCTGTCAGGCGCCATGGACGAGCTGCACAACGAGATC
CTGGAGCTGGACGAGAAGGTGGACGACCTGCGTGCCGACACCATCAGCAG
CCAGATCGAGCTGGCCGTGCTGCTGAGCAACGAGGGCATCATCAACAGCGA
GGACGAGCACCTGCTGGCCCTGGAGCGGAAACTGAAGAAGATGCTGGGAC
CCTCTGCCGTGGACATCGGCAACGGCTGCTTCGAGACCAAGCACAAGTGCA
ACCAGACCTGCCTGGATCGGATCGCCGCCGGAACCTTCAACGCCGGCGAGT
TCAGCCTGCCCACCTTCGACAGCCTGAACATCACCGCCGCCAGCCTGAACG
ACGACGGCCTGGACAACCACACCATCCTGCTGTACTACAGCACTGCCGCCT
CAAGCCTGGCCGTGACCCTGATGCTGGCCATCTTCATCGTGTACATGGTGAG
CCGGGACAACGTGAGCTGCAGCATCTGCCTG

SEQ ID NO: 96

> SARS-CoV-2 BA.4 spike protein CDS

ATGTTCGTGTTCCTGGTGCTGCTGCCCCTGGTGAGCTCTCAGTGCGTGAACC
TGATCACAAGAACACAGTCGTACACCAACAGCTTCACAAGAGGCGTGTACT
ACCCCGACAAGGTGTTCAGAAGCAGCGTGCTGCACTCCACCCAAGACCTGT
TTCTGCCCTTCTTCAGCAACGTGACCTGGTTCCACGCCATCAGCGGCACCAA
CGGCACCAAGAGATTCGACAACCCCGTGCTGCCCTTCAACGACGGCGTGTA
CTTCGCTAGCACCGAAAAGAGCAACATCATCAGAGGCTGGATCTTCGGCAC
CACCCTAGACTCCAAGACACAAAGCCTGCTGATCGTTAACAACGCGACCAA
CGTGGTGATCAAGGTGTGCGAGTTTCAGTTCTGCAACGACCCCTTCCTGGA

CGTGTACTACCACAAGAACAACAAGAGCTGGATGGAGAGCGAGTTCAGAG
TCTACAGCAGCGCCAACAACTGCACCTTCGAGTACGTGTCTCAGCCCTTCCT
GATGGACCTGGAGGGCAAGCAAGGCAATTTCAAGAACCTAAGAGAGTTCG
TGTTCAAGAACATCGACGGCTACTTCAAGATCTACAGCAAGCACACCCCCA
TCAACCTGGGGAGAGACCTGCCCCAAGGCTTCAGCGCCCTGGAGCCCCTGG
TGGACCTGCCCATCGGCATCAACATCACAAGATTTCAGACCCTGCTGGCCCT
GCACCGTAGCTACCTAACCCCCGGCGATAGTTCCTCTGGCTGGACGGCCGG
CGCTGCCGCCTACTACGTGGGCTACCTGCAGCCTAGAACCTTCCTGCTGAAG
TACAACGAGAACGGCACTATCACTGATGCCGTGGACTGTGCCCTGGACCCC
CTGAGCGAGACCAAGTGCACCCTCAAGAGCTTCACCGTGGAGAAGGGCAT
CTATCAGACAAGCAACTTCAGAGTGCAGCCCACCGAGAGCATCGTGAGATT
CCCCAACATCACCAACCTGTGCCCCTTCGACGAGGTGTTCAACGCCACAAG
ATTCGCTAGCGTGTACGCGTGGAACAGAAAGAGAATCAGCAACTGCGTGGC
CGACTACAGCGTGCTGTACAACTTCGCTCCCTTCTTCGCCTTCAAGTGCTAT
GGCGTCAGCCCCACCAAGCTGAACGACCTGTGCTTCACCAACGTGTACGCC
GACAGCTTCGTGATCAGAGGCAACGAGGTGAGCCAGATTGCCCCCGGGCA
AACCGGCAACATCGCCGACTACAACTACAAGCTGCCCGACGACTTCACCGG
CTGCGTGATCGCGTGGAACAGCAATAAGCTGGATTCCAAGGTGGGCGGCAA
CTACAACTACCGGTACAGACTGTTCAGAAAGAGCAACCTGAAGCCCTTCGA
GAGAGACATCAGCACCGAGATCTACCAAGCCGGCAACAAGCCCTGCAACG
GCGTGGCGGGCGTCAACTGCTACTTCCCCCTGCAGAGCTACGGCTTTAGGC
CCACCTACGGCGTGGGCCATCAGCCCTACAGAGTGGTGGTGCTGAGCTTCG
AGCTGCTGCACGCCCCGCAACCGTGTGCGGCCCCAAAAAGAGCACCAAC
CTGGTGAAGAACAAGTGCGTGAACTTCAACTTTAATGGCCTTAAGGGCACG
GGCGTGCTGACCGAGAGCAACAAGAAGTTCCTGCCATTTCAGCAATTCGGC
AGAGACATCGCCGACACCACCGACGCGGTCAGAGACCCTCAGACCCTGGA
GATCCTGGACATCACCCCCTGTAGCTTCGGCGGCGTGAGCGTGATCACCCCC
GGCACTAACACAAGCAACCAAGTGGCCGTGCTGTACCAAGGCGTGAACTG
CACCGAGGTGCCCGTGGCCATCCACGCCGATCAGCTGACCCCCACCTGGAG
AGTATACAGCACCGGAAGCAACGTGTTTCAGACAAGAGCCGGCTGCCTGAT
CGGCGCCGAGTACGTGAACAACAGCTACGAGTGCGACATCCCCATCGGCGC
CGGCATCTGCGCTAGCTATCAGACACAGACCAAGAGCCACCGGAGAGCTAG
AAGCGTGGCTTCTCAGAGCATCATCGCCTACACCATGAGCCTGGGCGCCGA
GAACAGCGTGGCCTACAGCAACAACAGCATCGCCATCCCCACCAACTTCAC
CATCAGCGTGACCACCGAGATCCTGCCCGTAAGTATGACCAAGACAAGCGT
GGACTGCACCATGTACATCTGCGGCGACAGCACCGAGTGCAGCAACCTGCT

GCTGCAGTACGGCAGCTTCTGCACACAGCTGAAGAGAGCCCTGACCGGCAT

CGCCGTGGAGCAAGACAAGAACACCCAAGAGGTGTTCGCCCAAGTGAAGC

AGATCTACAAGACCCCCCCCATCAAGTACTTCGGCGGCTTCAACTTCAGCCA

AATCCTGCCCGACCCTAGCAAGCCTAGCAAGCGGAGCTTCATCGAGGACCT

GCTGTTCAACAAGGTGACCCTGGCCGACGCCGGCTTCATCAAGCAGTACGG

CGACTGCCTAGGCGACATCGCAGCTAGAGACCTGATCTGCGCTCAGAAGTT

CAATGGACTCACCGTGCTCCCCCCCCTGCTGACCGACGAGATGATCGCTCA

GTACACCTCTGCCCTGCTGGCGGGAACCATAACAAGCGGTTGGACATTTGG

CGCCGGGGCCGCACTGCAAATCCCCTTCGCCATGCAGATGGCCTACAGATTC

AACGGCATCGGCGTGACACAGAACGTGCTGTACGAGAATCAGAAGCTGATC

GCCAATCAGTTCAACAGCGCCATCGGCAAGATCCAAGACAGCCTGAGCAGC

ACCGCTAGCGCCCTGGGCAAGCTGCAAGACGTGGTGAATCACAACGCCCA

AGCCCTGAACACCCTGGTGAAGCAGCTGAGCAGCAAGTTCGGCGCCATCA

GCAGCGTATTGAATGACATCCTGTCTAGACTGGACCCCCCCGAGGCCGAGG

TGCAGATCGACAGACTGATCACCGGCAGACTGCAGAGCCTGCAGACCTACG

TGACACAGCAGCTGATCAGAGCCGCCGAGATCAGAGCTAGCGCCAACCTGG

CCGCCACCAAGATGAGCGAGTGCGTGCTGGGGCAGAGCAAGAGAGTGGAC

TTCTGCGGCAAGGGCTACCACCTGATGAGCTTCCCTCAGAGCGCCCCCCAC

GGCGTGGTGTTCCTGCACGTGACCTACGTGCCCGCCCAAGAGAAGAACTTC

ACCACCGCTCCCGCCATCTGCCACGACGGCAAGGCCCACTTCCCTAGAGAG

GGCGTGTTCGTGAGCAACGGCACCCACTGGTTCGTGACACAGAGAAACTTC

TACGAGCCTCAGATCATCACCACCGACAACACCTTCGTGAGCGGCAACTGC

GACGTGGTGATCGGCATAGTGAACAACACCGTATACGACCCCCTGCAGCCC

GAGCTGGACAGCTTCAAGGAGGAGCTGGACAAGTACTTCAAAAACCATACT

AGCCCCGACGTGGACCTGGGAGACATCTCTGGCATCAACGCTAGCGTGGTG

AACATTCAGAAGGAGATCGACCGGCTGAATGAGGTGGCCAAGAACCTGAA

CGAGAGCCTGATCGACCTGCAAGAGCTGGGCAAGTACGAGCAGTACATCAA

GTGGCCCTGGTACATCTGGCTGGGCTTCATCGCCGGCCTGATCGCCATCGTG

ATGGTGACCATCATGCTGTGCTGCATGACAAGCTGTTGCAGCTGCCTGAAAG

GTTGCTGCAGCTGTGGCTCGTGCTGCAAGTTCGACGAGGACGACAGCGAGC

CCGTGCTGAAGGGCGTGAAGCTGCACTACCTGATGA

SEQ ID NO: 97

> Influenza A virus H1N1 HA0 mutant amino acid sequence

MKAILVVMLYTFTTANADTLCIGYHANNSTDTVDTVLEKNVTVTHSVNLLED
KHNGKLCKLRGVAPLHLGKCNIAGWILGNPECESLSTARSWSYIVETSNSDNG
TCYPGDFINYEELREQLSSVSSFERFEIFPKTSSWPNHDSDNGVTAACPHAGAK

SFYKNLIWLVKKGKSYPKINQTYINDKGKEVLVLWGIHHPPTIADQQSLYQNA
DAYVFVGTSRYSKKFKPEIATRPKVRDQEGRMNYYWTLVEPGDKITFEATGNL
VAPRYAFTMERDAGSGIIISDTPVHDCNTTCQTPEGAINTSLPFQNVHPITIGKCP
KYVKSTKLRLATGLRNVPSIQSQGLFGAIAGFIEGGWTGMVDGWYGYHHQNE
QGSGYAADLKSTQNAIDKITNKVNSVIEKMNTQFTAVGKEFNHLEKRIENLNK
KVDDGFLDIWTYNAELLVLLENERTLDYHDSNVKNLYEKVRNQLKNNAKEIG
NGCFEFYHKCDNTCMESVKNGTYDYPKYSEEAKLNREKIDGVKLDSTRIYQIL
AIYSTVASSLVLVVSLGAISFWMCSNGSLQCRICI

SEQ ID NO: 98

>Influenza A virus H3N2 HA0 mutant amino acid sequence

MKTIIALSYILCLVFAQKIPGNDNSTATLCLGHHAVPNGTIVKTITNDRIEVTNAT
ELVQNSSIGEICDSPHQILDGGNCTLIDALLGDPQCDGFQNKEWDLFVERSRAN
SNCYPYDVPDYASLRSLVASSGTLEFKNESFNWTGVKQNGTSSACIRGSSSSFFS
RLNWLTHLNYTYPALNVTMPNNEQFDKLYIWGVHHPSTDKDQISLFAQPSGRI
TVSTKRSQQAVIPNIGSRPRIRDIPSRISIYWTIVKPGDILLINSTGNLIAPRGYFKI
RSGKSSIMRSDAPIGKCKSECITPNGSIPNDKPFQNVNRITYGACPRYVKQSTLK
LATGMRNVPEKQTQGIFGAIAGFIENGWEGMVDGWYGFRHQNSEGRGQAAD
LKSTQAAIDQINGKLNRLIGKTNEKFHQIEKEFSEVEGRVQDLEKYVEDTKIDL
WSYNAELLVALENQHTIDLTDSEMNKLFEKTKKQLRENAEDMGNGCFKIYHK
CDNACIGSIRNETYDHNVYRDEALNNRFQIKGVELKSGYKDWILWISFAMSCF
LLCIALLGFIMWACQKGNIRCNICI

SEQ ID NO: 99

>Influenza B virus/Victoria HA0 mutant amino acid sequence

MKAIIVLLMVVTSNADRICTGITSSNSPHVVKTATQGEVNVTGVIPLTTTPTKSH
FANLKGTETRGKLCPKCLNCTDLDVALGRPKCTGKIPSARVSILHEVRPVTSGC
FPIMHDRTKIRQLPNLLRGYEHVRLSTHNVINAEDAPGRPYEIGTSGSCPNITNG
NGFFATMAWAVPKNKTATNPLTIEVPYICTEGEDQITVWGFHSDNETQMAKLY
GDSKPQKFTSSANGVTTHYVSQIGGFPNQTEDGGLPQSGRIVVDYMVQKSGKT
GTITYQRGILLPQKVWCASGRSKVIKGSLPLIGEADCLHEKYGGLNKSKPYYT
GEHAKAIGNCPIWVKTPLKLANGTKYRPPAKLLKEQGFFGAIAGFLEGGWEG
MIAGWHGYTSHGAHGVAVAADLKSTQEAINKITKNLNSLSELEVKNLQRLSGA
MDELHNEILELDEKVDDLRADTISSQIELAVLLSNEGIIINSEDEHLLALERKLKK
MLGPSAVEIGNGCFETKHKCNQTCLDRIAAGTFDAGEFSLPTFDSLNITAASLN
DDGLDNHTILLYYSTAASSLAVTLMIAIFVVYMVSRDNVSCSICL

SEQ ID NO: 100

>Influenza B virus/Yamagata HA0 mutant amino acid sequence

MKAIIVLLMVVTSNADRICTGITSSNSPHVVKTATQGEVNVTGVIPLTTTPTKSY
FANLKGTRTRGKLCPDCLNCTDLDVALGRPMCVGTTPSAKASILHEVRPVTSG
CFPIMHDRTKIRQLPNLLRGYEKIRLSTQNVIDAEKAPGGPYRLGTSGSCPNATS
KIGFFATMAWAVPKDNYKNATNPLTVEVPYICTEGEDQITVWGFHSDNKTQMK
SLYGDSNPQKFTSSANGVTTHYVSQIGDFPDQTEDGGLPQSGRIVVDYMMQKP
GKTGTIVYQRGVLLPQKVWCASGRSKVIKGSLPLIGEADCLHEEYGGLNKSKP
YYTGKHAKAIGNCPIWVKTPLKLANGTKYRPPAKLLKEQGFFGAIAGFLEGG
WEGMIAGWHGYTSHGAHGVAVAADLKSTQEAINKITKNLNSLSELEVKNLQR
LSGAMDELHNEILELDEKVDDLRADTISSQIELAVLLSNEGIIINSEDEHLLALER
KLKKMLGPSAVDIGNGCFETKHKCNQTCLDRIAAGTFNAGEFSLPTFDSLNITA
ASLNDDGLDNHTILLYYSTAASSLAVTLMLAIFIVYMVSRDNVSCSICL

SEQ ID NO: 101

> Influenza A virus H1N1 HA0 mutant CDS

ATGAAGGCCATCCTGGTGGTGATGCTGTACACCTTCACCACCGCCAACGCC
GACACCCTGTGCATCGGCTACCACGCCAACAACAGCACCGACACCGTGGAC
ACCGTGCTGGAGAAGAACGTGACCGTGACCCACAGCGTGAACCTGCTGGA
GGACAAGCACAACGGCAAGCTGTGCAAGCTGCGGGGCGTGGCCCCCCTGC
ACCTGGGCAAGTGCAACATCGCCGGCTGGATCCTGGGCAACCCCGAGTGCG
AGTCCCTGAGCACCGCCCGCTCCTGGAGCTACATCGTGGAGACCTCCAACA
GCGACAACGGCACCTGCTACCCCGGGGACTTCATCAACTACGAGGAGCTGC
GGGAGCAGCTGTCCAGCGTGTCCAGCTTCGAGCGCTTCGAGATCTTCCCCA
AGACCTCCAGCTGGCCCAACCACGACTCCGACAACGGCGTGACCGCCGCC
TGCCCCCACGCCGGGGCCAAGAGCTTCTACAAGAACCTGATCTGGCTGGTG
AAGAAGGGCAAGTCCTACCCCAAGATCAACCAGACCTACATCAACGACAA
GGGGAAGGAGGTCCTCGTGCTGTGGGGCATCCACCACCCCCCCACCATCGC
CGACCAGCAGAGCCTCTACCAGAACGCCGACGCCTACGTCTTCGTGGGGGAC
CTCCCGGTACAGCAAGAAGTTCAAGCCCGAGATCGCCACCCGCCCCAAGGT
CCGGGACCAGGAGGGCCGCATGAACTACTACTGGACCCTGGTGGAGCCGG
GGGACAAGATCACCTTCGAGGCCACCGGCAACCTCGTCGCCCCGCGGTACG
CCTTCACGATGGAGCGCGACGCCGGGTCCGGCATCATCATCAGCGACACCC
CCGTGCACGACTGCAACACGACCTGCCAGACGCCCGAGGGGGCGATCAAC
ACCTCCCTGCCGTTCCAGAACGTCCACCCGATCACGATCGGCAAGTGCCCC
AAGTACGTGAAGTCCACCAAGCTCCGGCTGGCCACGGGGCTCCGCAACGTC
CCCTCCATCCAGAGCCAGGGCCTGTTCGGGGCGATCGCCGGCTTCATCGAG
GGGGGCTGGACCGGGATGGTGGACGGCTGGTACGGGTACCACCACCAGAA
CGAGCAGGGCTCCGGGTACGCGGCCGACCTCAAGTCGACGCAGAACGCGA

TCGACAAGATCACCAACAAGGTCAACAGCGTGATCGAGAAGATGAACACG
CAGTTCACCGCCGTCGGCAAGGAGTTCAACCACCTGGAGAAGCGCATCGA
GAACCTCAACAAGAAGGTGGACGACGGGTTCCTGGACATCTGGACGTACA
ACGCGGAGCTCCTGGTCCTCCTGGAGAACGAGCGGACCCTCGACTACCACG
ACTCCAACGTGAAGAACCTGTACGAGAAGGTCCGCAACCAGCTCAAGAAC
AACGCCAAGGAGATCGGCAACGGGTGCTTCGAGTTCTACCACAAGTGCGAC
AACACGTGCATGGAGTCGGTGAAGAACGGCACCTACGACTACCCGAAGTAC
AGCGAGGAGGCGAAGCTGAACCGGGAGAAGATCGACGGGGTCAAGCTCGA
CTCCACGCGCATCTACCAGATCCTGGCCATCTACTCGACCGTGGCGAGCTCC
CTCGTCCTGGTGGTCTCGCTCGGCGCCATCAGCTTCTGGATGTGCTCCAACG
GGTCGCTGCAGTGCCGGATCTGCATCTGA

SEQ ID NO: 102

>Influenza A virus H3N2 HA0 mutant CDS

ATGAAGACCATCATCGCCCTGAGCTACATCCTGTGCCTGGTGTTCGCCCAGA
AGATCCCCGGCAACGACAACAGCACCGCCACCCTGTGCCTGGGCCACCAC
GCCGTGCCCAACGGCACCATCGTGAAGACCATCACCAACGACCGGATCGAG
GTGACCAACGCCACCGAGCTGGTGCAGAACTCCAGCATCGGCGAGATCTGC
GACTCCCCCACCAGATCCTGGACGGCGGCAACTGCACCCTGATCGACGCC
CTGCTGGGCGACCCCAGTGCGACGGGTTCCAGAACAAGGAGTGGGACCT
GTTCGTGGAGCGGAGCCGCGCCAACTCCAACTGCTACCCCTACGACGTGCC
CGACTACGCCAGCCTGCGGTCCCTGGTGGCCAGCTCCGGCACCCTGGAGTT
CAAGAACGAGAGCTTCAACTGGACCGGGGTCAAGCAGAACGGCACCTCCA
GCGCCTGCATCCGCGGGTCCAGCTCCAGCTTCTTCTCCCGGCTGAACTGGCT
CACCCACCTGAACTACACCTACCCCGCCCTCAACGTGACCATGCCCAACAA
CGAGCAGTTCGACAAGCTGTACATCTGGGGCGTCCACCACCCCAGCACCGA
CAAGGACCAGATCTCCCTCTTCGCCCAGCCCAGCGGGCGCATCACCGTGTC
CACGAAGCGGAGCCAGCAGGCCGTCATCCCCAACATCGGCTCCCGCCCGCG
GATCCGCGACATCCCCAGCCGGATCTCCATCTACTGGACGATCGTGAAGCCG
GGGGACATCCTGCTCATCAACAGCACCGGCAACCTGATCGCCCCCGCGGG
TACTTCAAGATCCGGTCCGGCAAGAGCTCGATCATGCGCTCCGACGCCCCGA
TCGGGAAGTGCAAGAGCGAGTGCATCACCCCCAACGGCTCGATCCCGAACG
ACAAGCCCTTCCAGAACGTCAACCGGATCACGTACGGGGCCTGCCCGCGCT
ACGTGAAGCAGTCCACGCTCAAGCTGGCCACCGGCATGCGGAACGTCCCCG
AGAAGCAGACCCAGGGGATCTTCGGCGCCATCGCGGGGTTCATCGAGAACG
GCTGGGAGGGGATGGTGGACGGCTGGTACGGGTTCCGGCACCAGAACAGC
GAGGGCCGCGGGCAGGCCGCGGACCTCAAGTCGACGCAGGCCGCGATCGA

CCAGATCAACGGCAAGCTGAACCGGCTCATCGGGAAGACGAACGAGAAGT
TCCACCAGATCGAGAAGGAGTTCTCCGAGGTCGAGGGCCGCGTGCAGGAC
CTGGAGAAGTACGTCGAGGACACCAAGATCGACCTCTGGAGCTACAACGCC
GAGCTGCTCGTGGCGCTGGAGAACCAGCACACCATCGACCTCACGGACTCG
GAGATGAACAAGCTGTTCGAGAAGACGAAGAAGCAGCTCCGGGAGAACGC
CGAGGACATGGGGAACGGCTGCTTCAAGATCTACCACAAGTGCGACAACGC
GTGCATCGGGTCCATCCGCAACGAGACCTACGACCACAACGTCTACCGGGA
CGAGGCCCTGAACAACCGCTTCCAGATCAAGGGCGTGGAGCTCAAGAGCG
GGTACAAGGACTGGATCCTGTGGATCTCGTTCGCGATGTCCTGCTTCCTCCT
GTGCATCGCCCTCCTGGGCTTCATCATGTGGGCGTGCCAGAAGGGGAACAT
CCGGTGCAACATCTGCATCTGA

SEQ ID NO: 103

>Influenza B virus/Victoria HA0 mutant CDS

ATGAAGGCCATCATCGTGCTGCTGATGGTGGTGACCAGCAACGCCGACCGG
ATCTGCACCGGCATCACCAGCTCCAACAGCCCCCACGTGGTGAAGACCGCC
ACCCAGGGCGAGGTGAACGTGACCGGCGTGATCCCCCTGACCACCACCCCC
ACCAAGTCCCACTTCGCCAACCTGAAGGGCACCGAGACCCGGGGCAAGCT
GTGCCCCAAGTGCCTGAACTGCACCGACCTGGACGTGGCCCTGGGCCGCCC
CAAGTGCACCGGCAAGATCCCCAGCGCCCGGGTGTCCATCCTGCACGAGGT
GCGCCCCGTGACCAGCGGCTGCTTCCCCATCATGCACGACCGCACCAAGAT
CCGGCAGCTGCCCAACCTGCTGCGGGGGTACGAGCACGTCCGCCTGTCCAC
CCACAACGTGATCAACGCCGAGGACGCCCCCGGCCGGCCCTACGAGATCGG
GACCAGCGGCTCCTGCCCCAACATCACCAACGGGAACGGCTTCTTCGCCAC
CATGGCCTGGGCCGTCCCCAAGAACAAGACCGCCACCAACCCCCTGACCAT
CGAGGTGCCGTACATCTGCACCGAGGGGGAGGACCAGATCACCGTCTGGGG
CTTCCACAGCGACAACGAGACGCAGATGGCCAAGCTGTACGGGGACTCCA
AGCCCCAGAAGTTCACGAGCTCCGCCAACGGCGTGACCACCCACTACGTCA
GCCAGATCGGGGGCTTCCCGAACCAGACGGAGGACGGGGGCCTGCCCCAG
TCCGGGCGCATCGTGGTCGACTACATGGTGCAGAAGTCCGGCAAGACGGGG
ACCATCACGTACCAGCGGGGCATCCTGCTGCCGCAGAAGGTCTGGTGCGCC
TCCGGGCGCAGCAAGGTGATCAAGGGCTCCCTGCCCCTCATCGGGGAGGCC
GACTGCCTGCACGAGAAGTACGGCGGGCTCAACAAGAGCAAGCCGTACTA
CACCGGCGAGCACGCCAAGGCCATCGGGAACTGCCCCATCTGGGTCAAGAC
CCCGCTGAAGCTCGCCAACGGCACGAAGTACCGGCCCCGGCCAAGCTGCT
CAAGGAGCAGGGGTTCTTCGGCGCCATCGCCGGGTTCCTGGAGGGCGGGTG
GGAGGGCATGATCGCCGGGTGGCACGGCTACGTCCCACGGGGCCCACG

GCGTGGCGGTCGCCGCGGACCTCAAGAGCACCCAGGAGGCCATCAACAAG
ATCACGAAGAACCTGAACTCCCTCAGCGAGCTGGAGGTGAAGAACCTCCA
GCGCCTGTCCGGGGCGATGGACGAGCTCCACAACGAGATCCTGGAGCTCGA
CGAGAAGGTCGACGACCTGCGGGCCGACACCATCAGCTCGCAGATCGAGCT
CGCGGTGCTGCTCTCCAACGAGGGCATCATCAACAGCGAGGACGAGCACCT
GCTCGCCCTGGAGCGGAAGCTCAAGAAGATGCTGGGGCCCTCGGCGGTCG
AGATCGGCAACGGGTGCTTCGAGACCAAGCACAAGTGCAACCAGACGTGC
CTCGACCGCATCGCCGCGGGCACGTTCGACGCCGGGGAGTTCTCCCTGCCG
ACCTTCGACAGCCTCAACATCACGGCGGCCTCGCTGAACGACGACGGCCTC
GACAACCACACCATCCTGCTCTACTACTCCACCGCGGCCAGCTCGCTGGCG
GTGACGCTCATGATCGCCATCTTCGTCGTGTACATGGTCTCCCGCGACAACG
TGAGCTGCTCGATCTGCCTGTGA

SEQ ID NO: 104

>Influenza B virus/Yamagata HA0 mutant CDS

ATGAAGGCCATCATCGTGCTGCTGATGGTGGTGACCAGCAACGCCGACCGG
ATCTGCACCGGCATCACCAGCTCCAACAGCCCCCACGTGGTGAAGACCGCC
ACCCAGGGCGAGGTGAACGTGACCGGCGTGATCCCCCTGACCACCACCCCC
ACCAAGTCCTACTTCGCCAACCTGAAGGGCACCCGGACCCGCGGCAAGCTG
TGCCCCGACTGCCTGAACTGCACCGACCTGGACGTGGCCCTGGGCCGGCCC
ATGTGCGTGGGCACCACCCCCAGCGCCAAGGCCTCCATCCTGCACGAGGTG
CGCCCCGTGACCAGCGGCTGCTTCCCCATCATGCACGACCGCACCAAGATC
CGGCAGCTGCCCAACCTGCTGCGGGGGTACGAGAAGATCCGCCTGTCCACC
CAGAACGTCATCGACGCCGAGAAGGCCCCCGGCGGGCCCTACCGGCTGGG
CACCAGCGGGTCCTGCCCCAACGCCACCAGCAAGATCGGCTTCTTCGCCAC
CATGGCCTGGGCCGTGCCCAAGGACAACTACAAGAACGCCACCAACCCCCT
GACCGTCGAGGTGCCCTACATCTGCACCGAGGGGGAGGACCAGATCACGGT
CTGGGGCTTCCACTCCGACAACAAGACCCAGATGAAGTCCCTGTACGGGGA
CTCCAACCCGCAGAAGTTCACGAGCTCCGCCAACGGCGTGACCACGCACTA
CGTCAGCCAGATCGGGGACTTCCCGGACCAGACCGAGGACGGCGGGCTGC
CCCAGTCCGGCCGCATCGTGGTCGACTACATGATGCAGAAGCCCGGGAAGA
CGGGCACCATCGTGTACCAGCGGGGGGTCCTGCTGCCGCAGAAGGTGTGGT
GCGCCAGCGGCCGCTCCAAGGTCATCAAGGGGAGCCTGCCCCTCATCGGCG
AGGCCGACTGCCTGCACGAGGAGTACGGGGGCCTCAACAAGTCCAAGCCG
TACTACACGGGGAAGCACGCCAAGGCCATCGGCAACTGCCCGATCTGGGTG
AAGACCCCCCTGAAGCTCGCCAACGGGACGAAGTACCGGCCGCCCGCCAA
GCTGCTCAAGGAGCAGGGCTTCTTCGGGGCCATCGCCGGCTTCCTGGAGGG

GGGCTGGGAGGGGATGATCGCCGGCTGGCACGGGTACACCAGCCACGGCG
CCCACGGGGTCGCGGTGGCCGCGGACCTCAAGTCCACGCAGGAGGCCATC
AACAAGATCACCAAGAACCTGAACAGCCTCTCCGAGCTGGAGGTCAAGAA
CCTCCAGCGCCTGAGCGGCGCGATGGACGAGCTCCACAACGAGATCCTGGA
GCTCGACGAGAAGGTGGACGACCTGCGGGCCGACACGATCTCGTCCCAGAT
CGAGCTCGCGGTCCTGCTCAGCAACGAGGGGATCATCAACTCGGAGGACGA
GCACCTGCTCGCCCTGGAGCGGAAGCTCAAGAAGATGCTGGGCCCGTCCGC
GGTGGACATCGGGAACGGCTGCTTCGAGACCAAGCACAAGTGCAACCAGA
CGTGCCTCGACCGCATCGCCGCGGGGACCTTCAACGCCGGCGAGTTCAGCC
TGCCCACGTTCGACTCGCTCAACATCACCGCGGCCTCCCTGAACGACGACG
GGCTCGACAACCACACGATCCTGCTCTACTACAGCACCGCGGCCTCGTCCC
TGGCGGTCACGCTCATGCTGGCCATCTTCATCGTGTACATGGTCAGCCGCGA
CAACGTGTCGTGCTCCATCTGCCTCTGA

SEQ ID NO: 105

>I'-Delta24-B-120A mRNA

GAGUUAGGAGUUGGUGGCAGUUUCAAUAACAGGUCAUUGCCGAGAAAAG
GAUAGCACUAUAAUAUGUUCGUGUUCCUGGUGCUGCUGCCCCUGGUGAG
CUCUCAGUGCGUGAACCUGAGGACAAGAACACAGCUGCCCCCGCCUACA
CCAACAGCUUCACAAGAGGCGUGUACUACCCCGACAAGGUGUUCAGAAGC
AGCGUGCUGCACUCCACCCAAGACCUGUUUCUGCCCUUCUUCAGCAACGU
GACCUGGUUCCACGCCAUCCACGUCAGCGGCACCAACGGCACCAAGAGAU
UCGACAACCCCGUGCUGCCCUUCAACGACGGCGUGUACUUCGCUAGCACC
GAAAAGAGCAACAUCAUCAGAGGCUGGAUCUUCGGCACCACCCUAGACUC
CAAGACACAAAGCCUGCUGAUCGUUAACAACGCGACCAACGUGGUGAUCA
AGGUGUGCGAGUUUCAGUUCUGCAACGACCCCUUCCUGGACGUGUACUAC
CACAAGAACAACAAGAGCUGGAUGGAGAGCGGGGUCUACAGCAGCGCCA
ACAACUGCACCUUCGAGUACGUGUCUCAGCCCUUCCUGAUGGACCUGGAG
GGCAAGCAAGGCAAUUUCAAGAACCUAAGAGAGUUCGUGUUCAAGAACA
UCGACGGCUACUUCAAGAUCUACAGCAAGCACACCCCCAUCAACCUGGUG
AGAGACCUGCCCCAAGGCUUCAGCGCCCUGGAGCCCCUGGUGGACCUGCC
CAUCGGCAUCAACAUCACAAGAUUUCAGACCCUGCUGGCCCUGCACCGUA
GCUACCUAACCCCCGGCGAUAGUUCCUCUGGCUGGACGGCCGGCGCUGCC
GCCUACUACGUGGGCUACCUGCAGCCUAGAACCUUCCUGCUGAAGUACAA
CGAGAACGGCACUAUCACUGAUGCCGUGGACUGUGCCCUGGACCCCCUGA
GCGAGACCAAGUGCACCCUCAAGAGCUUCACCGUGGAGAAGGGCAUCUAU
CAGACAAGCAACUUCAGAGUGCAGCCCACCGAGAGCAUCGUGAGAUUCCC
CAACAUCACCAACCUGUGCCCCUUCGGCGAGGUGUUCAACGCCACAAGAU
UCGCUAGCGUGUACGCGUGGAACAGAAAGAGAAUCAGCAACUGCGUGGC
CGACUACAGCGUGCUGUACAACAGCGCUAGCUUCAGCACCUUCAAGUGCU

AUGGCGUCAGCCCCACCAAGCUGAACGACCUGUGCUUCACCAACGUGUAC
GCCGACAGCUUCGUGAUCAGAGGCGACGAGGUGAGACAGAUUGCCCCCGG
GCAAACCGGCAAGAUCGCCGACUACAACUACAAGCUGCCCGACGACUUCA
CCGGCUGCGUGAUCGCGUGGAACAGCAAUAACCUGGAUUCCAAGGUGGGC
GGCAACUACAACUACCGGUACAGACUGUUCAGAAAGAGCAACCUGAAGCC
CUUCGAGAGAGACAUCAGCACCGAGAUCUACCAAGCCGGCAGCAAGCCCU
GCAACGGCGUGGAGGGCUUCAACUGCUACUUCCCCCUGCAGAGCUACGGC
UUUCAGCCCACCAACGGCGUGGGCUAUCAGCCCUACAGAGUGGUGGUGCU
GAGCUUCGAGCUGCUGCACGCCCCCGCAACCGUGUGCGGCCCCAAAAAGA
GCACCAACCUGGUGAAGAACAAGUGCGUGAACUUCAACUUUAAUGGCCU
UACCGGCACGGGCGUGCUGACCGAGAGCAACAAGAAGUUCCUGCCAUUUC
AGCAAUUCGGCAGAGACAUCGCCGACACCACCGACGCGGUCAGAGACCCU
CAGACCCUGGAGAUCCUGGACAUCACCCCCUGUAGCUUCGGCGGCGUGAG
CGUGAUCACCCCCGGCACUAACACAAGCAACCAAGUGGCCGUGCUGUACC
AAGGCGUGAACUGCACCGAGGUGCCCGUGGCCAUCCACGCCGAUCAGCUG
ACCCCCACCUGGAGAGUAUACAGCACCGGAAGCAACGUGUUUCAGACAAG
AGCCGGCUGCCUGAUCGGCGCCGAGCACGUGAACAACAGCUACGAGUGCG
ACAUCCCCAUCGGCGCCGGCAUCUGCGCUAGCUAUCAGACACAGACCAAC
AGCCGCCGGAGAGCUAGAAGCGUGGCUUCUCAGAGCAUCAUCGCCUACAC
CAUGAGCCUGGGCGCCGAGAACAGCGUGGCCUACAGCAACAACAGCAUCG
CCAUCCCCACCAACUUCACCAUCAGCGUGACCACCGAGAUCCUGCCCGUA
AGUAUGACCAAGACAAGCGUGGACUGCACCAUGUACAUCUGCGGCGACAG
CACCGAGUGCAGCAACCUGCUGCUGCAGUACGGCAGCUUCUGCACACAGC
UGAACAGAGCCCUGACCGGCAUCGCCGUGGAGCAAGACAAGAACACCCAA
GAGGUGUUCGCCCAAGUGAAGCAGAUCUACAAGACCCCCCCCAUCAAGGA
CUUCGGCGGCUUCAACUUCAGCCAAAUCCUGCCCGACCCUAGCAAGCCUA
GCAAGCGGAGCUUCAUCGAGGACCUGCUGUUCAACAAGGUGACCCUGGCC
GACGCCGGCUUCAUCAAGCAGUACGGCGACUGCCUAGGCGACAUCGCAGC
UAGAGACCUGAUCUGCGCUCAGAAGUUCAAUGGACUCACCGUGCUCCCCC
CCCUGCUGACCGACGAGAUGAUCGCUCAGUACACCUCUGCCCUGCUGGCG
GGAACCAUAACAAGCGGUUGGACAUUUGGCGCCGGGGCCGCACUGCAAAU
CCCCUUCGCCAUGCAGAUGGCCUACAGAUUCAACGGCAUCGGCGUGACAC
AGAACGUGCUGUACGAGAAUCAGAAGCUGAUCGCCAAUCAGUUCAACAG
CGCCAUCGGCAAGAUCCAAGACAGCCUGAGCAGCACCGCUAGCGCCCUGG
GCAAGCUGCAAAACGUGGUGAAUCAGAACGCCCAAGCCCUGAACACCCUG
GUGAAGCAGCUGAGCAGCAACUUCGGCGCCAUCAGCAGCGUAUUGAAUG
ACAUCCUGUCUAGACUGGACCCCCCCGAGGCCGAGGUGCAGAUCGACAGA
CUGAUCACCGGCAGACUGCAGAGCCUGCAGACCUACGUGACACAGCAGCU
GAUCAGAGCCGCCGAGAUCAGAGCUAGCGCCAACCUGGCCGCCACCAAGA
UGAGCGAGUGCGUGCUGGGGCAGAGCAAGAGAGUGGACUUCUGCGGCAA
GGGCUACCACCUGAUGAGCUUCCCUCAGAGCGCCCCCCACGGCGUGGUGU

UCCUGCACGUGACCUACGUGCCCGCCCAAGAGAAGAACUUCACCACCGCU
CCCGCCAUCUGCCACGACGGCAAGGCCCACUUCCCUAGAGAGGGCGUGUU
CGUGAGCAACGGCACCCACUGGUUCGUGACACAGAGAAACUUCUACGAGC
CUCAGAUCAUCACCACCGACAACACCUUCGUGAGCGGCAACUGCGACGUG
GUGAUCGGCAUAGUGAACAACACCGUAUACGACCCCCUGCAGCCCGAGCU
GGACAGCUUCAAGGAGGAGCUGGACAAGUACUUCAAAAACCAUACUAGC
CCCGACGUGGACCUGGGAGACAUCUCUGGCAUCAACGCUAGCGUGGUGAA
CAUUCAGAAGGAGAUCGACCGGCUGAAUGAGGUGGCCAAGAACCUGAAC
GAGAGCCUGAUCGACCUGCAAGAGCUGGGCAAGUACGAGCAGUACAUCA
AGUGGCCCUGGUACAUCUGGCUGGGCUUCAUCGCCGGCCUGAUCGCCAUC
GUGAUGGUGACCAUCAUGCUGUGCUGCAUGACAAGCUGUUGCAGCUGCC
UGAAAGGUUGCUGCAGCUGUGGCUCGUGCUGCAAGUUCGACGAGGACGA
CAGCGAGCCCGUGCUGAAGGGCGUGAAGCUGCACUACACCUGAUGACUCG
AGGCUCGCUUUCUUGCUGUCCAAUUUCUAUUAAAGGUUCCUUUGUUCCCU
AAGUCCAACUACUAAACUGGGGGAUAUUAUGAAGGGCCUUGAGCAUCUG
GAUUCUGCCUAAUAAAAACAUUUAUUUUCAUUGCAAAAAAAAAAAAAAA
AAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAA
AAAAAAAAAA

SEQ ID NO: 106

>I'-OC24-B-120A mRNA

GAGUUAGGAGUUGGUGGCAGUUUCAAUAACAGGUCAUUGCCGAGAAAAG
GAUAGCACUAUAAUAUGUUCGUGUUCCUGGUGCUGCUGCCCCUGGUGAG
CUCUCAGUGCGUGAACCUGACCACAAGAACACAGCUGCCCCCGCCUACA
CCAACAGCUUCACAAGAGGCGUGUACUACCCCGACAAGGUGUUCAGAAGC
AGCGUGCUGCACUCCACCCAAGACCUGUUUCUGCCCUUCUUCAGCAACGU
GACCUGGUUCCACGUGAUCAGCGGCACCAACGGCACCAAGAGAUUCGACA
ACCCCGUGCUGCCCUUCAACGACGGCGUGUACUUCGCUAGCAUCGAAAAG
AGCAACAUCAUCAGAGGCUGGAUCUUCGGCACCACCCUAGACUCCAAGAC
ACAAAGCCUGCUGAUCGUUAACAACGCGACCAACGUGGUGAUCAAGGUG
UGCGAGUUUCAGUUCUGCAACGACCCCUUCCUGGACCACAAGAACAACAA
GAGCUGGAUGGAGAGCGAGUUCAGAGUGUACAGCAGCGCCAACAACUGC
ACCUUCGAGUACGUGUCUCAGCCCUUCCUGAUGGACCUGGAGGGCAAGCA
AGGCAAUUUCAAGAACCUAAGAGAGUUCGUGUUCAAGAACAUCGACGGC
UACUUCAAGAUCUACAGCAAGCACACCCCCAUCAUCGUGAGAGAGCCCGA
GGACCUGCCCCAAGGCUUCAGCGCCCUGGAGCCCCUGGUGGACCUGCCCA
UCGGCAUCAACAUCACAAGAUUUCAGACCCUGCUGGCCCUGCACCGUAGC
UACCUAACCCCCGGCGAUAGUUCCUCUGGCUGGACGGCCGGCGCUGCCGC
CUACUACGUGGGCUACCUGCAGCCUAGAACCUUCCUGCUGAAGUACAACG
AGAACGGCACUAUCACUGAUGCCGUGGACUGUGCCCUGGACCCCCUGAGC
GAGACCAAGUGCACCCUCAAGAGCUUCACCGUGGAGAAGGGCAUCUAUCA

GACAAGCAACUUCAGAGUGCAGCCCACCGAGAGCAUCGUGAGAUUCCCCA
ACAUCACCAACCUGUGCCCCUUCGACGAGGUGUUCAACGCCACCAGAUUU
GCUAGCGUGUACGCCUGGAACCGGAAGAGAAUCAGCAACUGCGUGGCCGA
CUACAGCGUGCUGUACAACCUGGCCCCCUUCUUCACCUUCAAGUGCUAUG
GCGUCAGCCCCACCAAGCUGAACGACCUGUGCUUCACCAACGUGUACGCC
GACAGCUUCGUGAUCAGAGGCGACGAGGUGAGACAGAUCGCCCCCGGGCA
GACCGGCAACAUCGCCGACUACAACUACAAGCUGCCCGACGACUUCACCG
GCUGCGUGAUCGCCUGGAACAGCAACAAGCUGGAUAGCAAGGUGAGCGG
CAACUACAAUUACCUGUACAGACUGUUCAGAAAGAGCAACCUGAAGCCCU
UCGAGAGAGACAUCAGCACCGAGAUCUACCAAGCCGGCAACAAGCCCUGC
AACGGCGUGGCGGGCUUCAACUGCUACUUCCCCCUCAGGAGCUACAGCUU
CAGACCCACCUACGGCGUGGGCCAUCAGCCCUACAGAGUGGUCGUGCUGA
GCUUCGAGCUGCUGCACGCCCCUGCCACAGUGUGCGGCCCCAAAAAGAGC
ACCAACCUGGUGAAGAACAAGUGCGUGAACUUCAACUUCAACGGCCUGAA
GGGCACCGGCGUGCUGACCGAGAGCAACAAGAAGUUCCUGCCAUUUCAGC
AAUUCGGCAGAGACAUCGCCGACACCACCGACGCGGUCAGAGACCCUCAG
ACCCUGGAGAUCCUGGACAUCACCCCCUGUAGCUUCGGCGGCGUGAGCGU
GAUCACCCCCGGCACUAACACAAGCAACCAAGUGGCCGUGCUGUACCAAG
GCGUGAACUGCACCGAGGUGCCCGUGGCCAUCCACGCCGAUCAGCUGACC
CCCACCUGGAGAGUGUAUAGCACCGGCAGCAACGUGUUCAGACAAGAGC
CGGCUGCCUGAUCGGCGCCGAGUACGUGAACAACAGCUACGAGUGCGACA
UCCCCAUCGGCGCCGGCAUCUGCGCUAGCUAUCAGACACAGACCAAGAGC
CACCGGAGAGCUAGAAGCGUGGCUAGCCAAAGCAUCAUCGCCUACACCAU
GAGCCUGGGCGCCGAGAACAGCGUGGCCUACAGCAACAACAGCAUCGCCA
UCCCCACCAACUUCACCAUCAGCGUGACCACCGAGAUCCUGCCCGUAAGU
AUGACCAAGACAAGCGUGGACUGCACCAUGUACAUCUGCGGCGACAGCAC
CGAGUGCAGCAACCUGCUCCUGCAGUACGGCAGCUUCUGCACACAGCUGA
AGAGAGCCCUGACCGGCAUCGCCGUGGAGCAAGACAAGAACACCCAAGAG
GUGUUCGCCCAAGUGAAGCAGAUCUACAAGACCCCCCCCAUCAAGUACUU
CGGCGGCUUCAACUUCAGCCAAAUCCUGCCCGACCCUAGCAAGCCUAGCA
AGCGGAGCUUCAUCGAGGACCUGCUGUUCAACAAGGUGACCCUGGCCGAC
GCCGGCUUCAUCAAGCAGUACGGCGACUGCCUAGGCGACAUCGCAGCUAG
AGACCUGAUCUGCGCUCAGAAGUUCAAGGGACUCACCGUGCUCCCCCCCC
UGCUGACCGACGAGAUGAUCGCUCAGUACACCUCUGCCCUGCUGGCGGGA
ACCAUAACAAGCGGUUGGACAUUUGGCGCCGGGGCCGCACUGCAAAUCCC
CUUCGCCAUGCAGAUGGCCUACAGAUUCAACGGCAUCGGCGUGACACAGA
ACGUGCUGUACGAGAAUCAGAAGCUGAUCGCCAAUCAGUUCAACAGCGCC
AUCGGCAAGAUCCAAGACAGCCUGAGCAGCACCGCUAGCGCCCUGGGCAA
GCUGCAAGACGUGGUGAACCACAACGCCCAAGCCCUGAACACCCUGGUGA
AGCAGCUGAGCAGCAAGUUCGGCGCCAUCAGCAGCGUGCUGAAUGACAUC
UUCUCUAGACUGGACCCCCCCGAGGCCGAGGUGCAGAUCGACAGACUGAU

CACCGGCAGACUGCAGAGCCUGCAGACCUACGUGACACAGCAGCUGAUCA
GAGCCGCCGAGAUCAGAGCUAGCGCCAACCUGGCCGCCACCAAGAUGAGC
GAGUGCGUGCUGGGGCAGAGCAAGAGAGUGGACUUCUGCGGCAAGGGCU
ACCACCUGAUGAGCUUCCCUCAGAGCGCCCCCCACGGCGUGGUGUUCCUG
CACGUGACCUACGUGCCCGCCCAAGAGAAGAACUUCACCACCGCUCCCGC
CAUCUGCCACGACGGCAAGGCCCACUUCCCUAGAGAGGGCGUGUUCGUGA
GCAACGGCACCCACUGGUUCGUGACACAGAGAAACUUCUACGAGCCUCAG
AUCAUCACCACCGACAACACCUUCGUGAGCGGCAACUGCGACGUGGUGAU
CGGCAUAGUGAACAACACCGUAUACGACCCCCUGCAGCCCGAGCUGGACA
GCUUCAAGGAGGAGCUGGACAAGUACUUCAAAAACCAUACUAGCCCCGAC
GUGGACCUGGGAGACAUCUCUGGCAUCAACGCUAGCGUGGUGAACAUUC
AGAAGGAGAUCGACCGGCUGAAUGAGGUGGCCAAGAACCUGAACGAGAG
CCUGAUCGACCUGCAAGAGCUGGGCAAGUACGAGCAGUACAUCAAGUGGC
CCUGGUACAUCUGGCUGGGCUUCAUCGCCGGCCUGAUCGCCAUCGUGAUG
GUGACCAUCAUGCUGUGCUGCAUGACAAGCUGUUGCAGCUGCCUGAAAG
GUUGCUGCAGCUGUGGCUCGUGCUGCAAGUUCGACGAGGACGACAGCGA
GCCCGUGCUGAAGGGCGUGAAGCUGCACUACACCUGAUGACUCGAGGCUC
GCUUUCUUGCUGUCCAAUUUCUAUUAAAGGUUCCUUUGUUCCCUAAGUCC
AACUACUAAACUGGGGGAUAUUAUGAAGGGCCUUGAGCAUCUGGAUUCU
GCCUAAUAAAAACAUUUAUUUUCAUUGCAAAAAAAAAAAAAAAAAAAAAA
AAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAA
AAAA

SEQ ID NO: 107

>I'-BA4-B-120A mRNA

GAGUUAGGAGUUGGUGGCAGUUUCAAUAACAGGUCAUUGCCGAGAAAA
GGAUAGCACUAUAAUAUGUUCGUGUUCCUGGUGCUGCUGCCCCUGGUGA
GCUCUCAGUGCGUGAACCUGAUCACAAGAACACAGUCGUACACCAACAG
CUUCACAAGAGGCGUGUACUACCCCGACAAGGUGUUCAGAAGCAGCGUG
CUGCACUCCACCCAAGACCUGUUUCUGCCCUUCUUCAGCAACGUGACCU
GGUUCCACGCCAUCAGCGGCACCAACGGCACCAAGAGAUUCGACAACCC
CGUGCUGCCCUUCAACGACGGCGUGUACUUCGCUAGCACCGAAAAGAGC
AACAUCAUCAGAGGCUGGAUCUUCGGCACCACCCUAGACUCCAAGACAC
AAAGCCUGCUGAUCGUUAACAACGCGACCAACGUGGUGAUCAAGGUGUG
CGAGUUUCAGUUCUGCAACGACCCCUUCCUGGACGUGUACUACCACAAG
AACAACAAGAGCUGGAUGGAGAGCGAGUUCAGAGUCUACAGCAGCGCCA
ACAACUGCACCUUCGAGUACGUGUCUCAGCCCUUCCUGAUGGACCUGGA
GGGCAAGCAAGGCAAUUUCAAGAACCUAAGAGAGUUCGUGUUCAAGAAC
AUCGACGGCUACUUCAAGAUCUACAGCAAGCACACCCCCAUCAACCUGG

GGAGAGACCUGCCCCAAGGCUUCAGCGCCCUGGAGCCCCUGGUGGACCU
GCCCAUCGGCAUCAACAUCACAAGAUUUCAGACCCUGCUGGCCCUGCAC
CGUAGCUACCUAACCCCCGGCGAUAGUUCCUCUGGCUGGACGGCCGGCG
CUGCCGCCUACUACGUGGGCUACCUGCAGCCUAGAACCUUCCUGCUGAA
GUACAACGAGAACGGCACUAUCACUGAUGCCGUGGACUGUGCCCUGGAC
CCCCUGAGCGAGACCAAGUGCACCCUCAAGAGCUUCACCGUGGAGAAGG
GCAUCUAUCAGACAAGCAACUUCAGAGUGCAGCCCACCGAGAGCAUCGU
GAGAUUCCCCAACAUCACCAACCUGUGCCCCUUCGACGAGGUGUUCAAC
GCCACAAGAUUCGCUAGCGUGUACGCGUGGAACAGAAAGAGAAUCAGCA
ACUGCGUGGCCGACUACAGCGUGCUGUACAACUUCGCUCCCUUCUUCGC
CUUCAAGUGCUAUGGCGUCAGCCCCACCAAGCUGAACGACCUGUGCUUC
ACCAACGUGUACGCCGACAGCUUCGUGAUCAGAGGCAACGAGGUGAGCC
AGAUUGCCCCCGGGCAAACCGGCAACAUCGCCGACUACAACUACAAGCU
GCCCGACGACUUCACCGGCUGCGUGAUCGCGUGGAACAGCAAUAAGCUG
GAUUCCAAGGUGGGCGGCAACUACAACUACCGGUACAGACUGUUCAGAA
AGAGCAACCUGAAGCCCUUCGAGAGAGACAUCAGCACCGAGAUCUACCA
AGCCGGCAACAAGCCCUGCAACGGCGUGGCGGGCGUCAACUGCUACUUC
CCCCUGCAGAGCUACGGCUUUAGGCCCACCUACGGCGUGGGCCAUCAGC
CCUACAGAGUGGUGGUGCUGAGCUUCGAGCUGCUGCACGCCCCCGCAAC
CGUGUGCGGCCCCAAAAAGAGCACCAACCUGGUGAAGAACAAGUGCGUG
AACUUCAACUUUAAUGGCCUUAAGGGCACGGGCGUGCUGACCGAGAGCA
ACAAGAAGUUCCUGCCAUUUCAGCAAUUCGGCAGAGACAUCGCCGACAC
CACCGACGCGGUCAGAGACCCUCAGACCCUGGAGAUCCUGGACAUCACC
CCCUGUAGCUUCGGCGGCGUGAGCGUGAUCACCCCCGGCACUAACACAA
GCAACCAAGUGGCCGUGCUGUACCAAGGCGUGAACUGCACCGAGGUGCC
CGUGGCCAUCCACGCCGAUCAGCUGACCCCCACCUGGAGAGUAUACAGC
ACCGGAAGCAACGUGUUCAGACAAGAGCCGGCUGCCUGAUCGGCGCCG
AGUACGUGAACAACAGCUACGAGUGCGACAUCCCCAUCGGCGCCGGCAU
CUGCGCUAGCUAUCAGACACAGACCAAGAGCCACCGGAGAGCUAGAAGC
GUGGCUUCUCAGAGCAUCAUCGCCUACACCAUGAGCCUGGGCGCCGAGA
ACAGCGUGGCCUACAGCAACAACAGCAUCGCCAUCCCCACCAACUUCACC
AUCAGCGUGACCACCGAGAUCCUGCCCGUAAGUAUGACCAAGACAAGCG
UGGACUGCACCAUGUACAUCUGCGGCGACAGCACCGAGUGCAGCAACCU
GCUGCUGCAGUACGGCAGCUUCUGCACACAGCUGAAGAGAGCCCUGACC
GGCAUCGCCGUGGAGCAAGACAAGAACACCCAAGAGGUGUUCGCCCAAG
UGAAGCAGAUCUACAAGACCCCCCCCAUCAAGUACUUCGGCGGCUUCAA

CUUCAGCCAAAUCCUGCCCGACCCUAGCAAGCCUAGCAAGCGGAGCUUC
AUCGAGGACCUGCUGUUCAACAAGGUGACCCUGGCCGACGCCGGCUUCA
UCAAGCAGUACGGCGACUGCCUAGGCGACAUCGCAGCUAGAGACCUGAU
CUGCGCUCAGAAGUUCAAUGGACUCACCGUGCUCCCCCCCCUGCUGACCG
ACGAGAUGAUCGCUCAGUACACCUCUGCCCUGCUGGCGGGAACCAUAAC
AAGCGGUUGGACAUUUGGCGCCGGGGCCGCACUGCAAAUCCCCUUCGCC
AUGCAGAUGGCCUACAGAUUCAACGGCAUCGGCGUGACACAGAACGUGC
UGUACGAGAAUCAGAAGCUGAUCGCCAAUCAGUUCAACAGCGCCAUCGG
CAAGAUCCAAGACAGCCUGAGCAGCACCGCUAGCGCCCUGGGCAAGCUG
CAAGACGUGGUGAAUCACAACGCCCAAGCCCUGAACACCCUGGUGAAGC
AGCUGAGCAGCAAGUUCGGCGCCAUCAGCAGCGUAUUGAAUGACAUCCU
GUCUAGACUGGACCCCCCCGAGGCCGAGGUGCAGAUCGACAGACUGAUC
ACCGGCAGACUGCAGAGCCUGCAGACCUACGUGACACAGCAGCUGAUCA
GAGCCGCCGAGAUCAGAGCUAGCGCCAACCUGGCCGCCACCAAGAUGAG
CGAGUGCGUGCUGGGGCAGAGCAAGAGAGUGGACUUCUGCGGCAAGGGC
UACCACCUGAUGAGCUUCCCUCAGAGCGCCCCCCACGGCGUGGUGUUCC
UGCACGUGACCUACGUGCCCGCCCAAGAGAAGAACUUCACCACCGCUCCC
GCCAUCUGCCACGACGGCAAGGCCCACUUCCCUAGAGAGGGCGUGUUCG
UGAGCAACGGCACCCACUGGUUCGUGACACAGAGAAACUUCUACGAGCC
UCAGAUCAUCACCACCGACAACACCUUCGUGAGCGGCAACUGCGACGUG
GUGAUCGGCAUAGUGAACAACACCGUAUACGACCCCCUGCAGCCCGAGC
UGGACAGCUUCAAGGAGGAGCUGGACAAGUACUUCAAAAACCAUACUAG
CCCCGACGUGGACCUGGGAGACAUCUCUGGCAUCAACGCUAGCGUGGUG
AACAUUCAGAAGGAGAUCGACCGGCUGAAUGAGGUGGCCAAGAACCUGA
ACGAGAGCCUGAUCGACCUGCAAGAGCUGGGCAAGUACGAGCAGUACAU
CAAGUGGCCCUGGUACAUCUGGCUGGGCUUCAUCGCCGGCCUGAUCGCC
AUCGUGAUGGUGACCAUCAUGCUGUGCUGCAUGACAAGCUGUUGCAGCU
GCCUGAAAGGUUGCUGCAGCUGUGGCUCGUGCUGCAAGUUCGACGAGGA
CGACAGCGAGCCCGUGCUGAAGGGCGUGAAGCUGCACUACACCUGAUGA
CUCGAGGCUCGCUUUCUUGCUGUCCAAUUUCUAUUAAAGGUUCCUUUGU
UCCCUAAGUCCAACUACUAAACUGGGGGAUAUUAUGAAGGGCCUUGAGC
AUCUGGAUUCUGCCUAAUAAAAACAUUUAUUUUCAUUGCAAAAAAAAA
AAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAA
AAAAAAAAAAAAAAAAA

SEQ ID NO: 108

>I'-BA4-B mRNA

>I'-BA4-B mRNA

GAGUUAGGAGUUGGUGGCAGUUUCAAUAACAGGUCAUUGCCGAGAAAA
GGAUAGCACUAUAAUAUGUUCGUGUUCCUGGUGCUGCUGCCCCUGGUGA
GCUCUCAGUGCGUGAACCUGAUCACAAGAACACAGUCGUACACCAACAG
CUUCACAAGAGGCGUGUACUACCCCGACAAGGUGUUCAGAAGCAGCGUG
CUGCACUCCACCCAAGACCUGUUUCUGCCCUUCUUCAGCAACGUGACCU
GGUUCCACGCCAUCAGCGGCACCAACGGCACCAAGAGAUUCGACAACCC
CGUGCUGCCCUUCAACGACGGCGUGUACUUCGCUAGCACCGAAAAGAGC
AACAUCAUCAGAGGCUGGAUCUUCGGCACCACCCUAGACUCCAAGACAC
AAAGCCUGCUGAUCGUUAACAACGCGACCAACGUGGUGAUCAAGGUGUG
CGAGUUUCAGUUCUGCAACGACCCCUUCCUGGACGUGUACUACCACAAG
AACAACAAGAGCUGGAUGGAGAGCGAGUUCAGAGUCUACAGCAGCGCCA
ACAACUGCACCUUCGAGUACGUGUCUCAGCCCUUCCUGAUGGACCUGGA
GGGCAAGCAAGGCAAUUUCAAGAACCUAAGAGAGUUCGUGUUCAAGAAC
AUCGACGGCUACUUCAAGAUCUACAGCAAGCACACCCCCAUCAACCUGG
GGAGAGACCUGCCCCAAGGCUUCAGCGCCCUGGAGCCCCUGGUGGACCU
GCCCAUCGGCAUCAACAUCACAAGAUUUCAGACCCUGCUGGCCCUGCAC
CGUAGCUACCUAACCCCCGGCGAUAGUUCCUCUGGCUGGACGGCCGGCG
CUGCCGCCUACUACGUGGGCUACCUGCAGCCUAGAACCUUCCUGCUGAA
GUACAACGAGAACGGCACUAUCACUGAUGCCGUGGACUGUGCCCUGGAC
CCCCUGAGCGAGACCAAGUGCACCCUCAAGAGCUUCACCGUGGAGAAGG
GCAUCUAUCAGACAAGCAACUUCAGAGUGCAGCCCACCGAGAGCAUCGU
GAGAUUCCCCAACAUCACCAACCUGUGCCCCUUCGACGAGGUGUUCAAC
GCCACAAGAUUCGCUAGCGUGUACGCGUGGAACAGAAAGAGAAUCAGCA
ACUGCGUGGCCGACUACAGCGUGCUGUACAACUUCGCUCCCUUCUUCGC
CUUCAAGUGCUAUGGCGUCAGCCCCACCAAGCUGAACGACCUGUGCUUC
ACCAACGUGUACGCCGACAGCUUCGUGAUCAGAGGCAACGAGGUGAGCC
AGAUUGCCCCCGGGCAAACCGGCAACAUCGCCGACUACAACUACAAGCU
GCCCGACGACUUCACCGGCUGCGUGAUCGCGUGGAACAGCAAUAAGCUG
GAUUCCAAGGUGGGCGGCAACUACAACUACCGGUACAGACUGUUCAGAA
AGAGCAACCUGAAGCCCUUCGAGAGAGACAUCAGCACCGAGAUCUACCA
AGCCGGCAACAAGCCCUGCAACGGCGUGGCGGGCGUCAACUGCUACUUC
CCCCUGCAGAGCUACGGCUUUAGGCCCACCUACGGCGUGGGCCAUCAGC
CCUACAGAGUGGUGGUGCUGAGCUUCGAGCUGCUGCACGCCCCCGCAAC
CGUGUGCGGCCCCAAAAAGAGCACCAACCUGGUGAAGAACAAGUGCGUG
AACUUCAACUUUAAUGGCCUUAAGGGCACGGGCGUGCUGACCGAGAGCA

ACAAGAAGUUCCUGCCAUUUCAGCAAUUCGGCAGAGACAUCGCCGACAC
CACCGACGCGGUCAGAGACCCUCAGACCCUGGAGAUCCUGGACAUCACC
CCCUGUAGCUUCGGCGGCGUGAGCGUGAUCACCCCCGGCACUAACACAA
GCAACCAAGUGGCCGUGCUGUACCAAGGCGUGAACUGCACCGAGGUGCC
CGUGGCCAUCCACGCCGAUCAGCUGACCCCCACCUGGAGAGUAUACAGC
ACCGGAAGCAACGUGUUUCAGACAAGAGCCGGCUGCCUGAUCGGCGCCG
AGUACGUGAACAACAGCUACGAGUGCGACAUCCCCAUCGGCGCCGGCAU
CUGCGCUAGCUAUCAGACACAGACCAAGAGCCACCGGAGAGCUAGAAGC
GUGGCUUCUCAGAGCAUCAUCGCCUACACCAUGAGCCUGGGCGCCGAGA
ACAGCGUGGCCUACAGCAACAACAGCAUCGCCAUCCCCACCAACUUCACC
AUCAGCGUGACCACCGAGAUCCUGCCCGUAAGUAUGACCAAGACAAGCG
UGGACUGCACCAUGUACAUCUGCGGCGACAGCACCGAGUGCAGCAACCU
GCUGCUGCAGUACGGCAGCUUCUGCACACAGCUGAAGAGAGCCCUGACC
GGCAUCGCCGUGGAGCAAGACAAGAACACCCAAGAGGUGUUCGCCCAAG
UGAAGCAGAUCUACAAGACCCCCCCCAUCAAGUACUUCGGCGGCUUCAA
CUUCAGCCAAAUCCUGCCCGACCCUAGCAAGCCUAGCAAGCGGAGCUUC
AUCGAGGACCUGCUGUUCAACAAGGUGACCCUGGCCGACGCCGGCUUCA
UCAAGCAGUACGGCGACUGCCUAGGCGACAUCGCAGCUAGAGACCUGAU
CUGCGCUCAGAAGUUCAAUGGACUCACCGUGCUCCCCCCCCUGCUGACCG
ACGAGAUGAUCGCUCAGUACACCUCUGCCCUGCUGGCGGGAACCAUAAC
AAGCGGUUGGACAUUUGGCGCCGGGGCCGCACUGCAAAUCCCCUUCGCC
AUGCAGAUGGCCUACAGAUUCAACGGCAUCGGCGUGACACAGAACGUGC
UGUACGAGAAUCAGAAGCUGAUCGCCAAUCAGUUCAACAGCGCCAUCGG
CAAGAUCCAAGACAGCCUGAGCAGCACCGCUAGCGCCCUGGGCAAGCUG
CAAGACGUGGUGAAUCACAACGCCCAAGCCCUGAACACCCUGGUGAAGC
AGCUGAGCAGCAAGUUCGGCGCCAUCAGCAGCGUAUUGAAUGACAUCCU
GUCUAGACUGGACCCCCCCGAGGCCGAGGUGCAGAUCGACAGACUGAUC
ACCGGCAGACUGCAGAGCCUGCAGACCUACGUGACACAGCAGCUGAUCA
GAGCCGCCGAGAUCAGAGCUAGCGCCAACCUGGCCGCCACCAAGAUGAG
CGAGUGCGUGCUGGGGCAGAGCAAGAGAGUGGACUUCUGCGGCAAGGGC
UACCACCUGAUGAGCUUCCCUCAGAGCGCCCCCCACGGCGUGGUGUUCC
UGCACGUGACCUACGUGCCCGCCCAAGAGAAGAACUUCACCACCGCUCCC
GCCAUCUGCCACGACGGCAAGGCCCACUUCCCUAGAGAGGGCGUGUUCG
UGAGCAACGGCACCCACUGGUUCGUGACACAGAGAAACUUCUACGAGCC
UCAGAUCAUCACCACCGACAACACCUUCGUGAGCGGCAACUGCGACGUG
GUGAUCGGCAUAGUGAACAACACCGUAUACGACCCCCUGCAGCCCGAGC

UGGACAGCUUCAAGGAGGAGCUGGACAAGUACUUCAAAAACCAUACUAG
CCCCGACGUGGACCUGGGAGACAUCUCUGGCAUCAACGCUAGCGUGGUG
AACAUUCAGAAGGAGAUCGACCGGCUGAAUGAGGUGGCCAAGAACCUGA
ACGAGAGCCUGAUCGACCUGCAAGAGCUGGGCAAGUACGAGCAGUACAU
CAAGUGGCCCUGGUACAUCUGGCUGGGCUUCAUCGCCGGCCUGAUCGCC
AUCGUGAUGGUGACCAUCAUGCUGUGCUGCAUGACAAGCUGUUGCAGCU
GCCUGAAAGGUUGCUGCAGCUGUGGCUCGUGCUGCAAGUUCGACGAGGA
CGACAGCGAGCCCGUGCUGAAGGGCGUGAAGCUGCACUACACCUGAUGA
CUCGAGGCUCGCUUUCUUGCUGUCCAAUUUCUAUUAAAGGUUCCUUUGU
UCCCUAAGUCCAACUACUAAACUGGGGGAUAUUAUGAAGGGCCUUGAGC
AUCUGGAUUCUGCCUAAUAAAAAACAUUUAUUUUCAUUGCAAAAAAAAA
AAAAAAAAAAAAAAAAAAAAGCAUAUGACUAAAAAAAAAAAAAAA
AAAAAAAAAAAAAAAAAAAAAAAAAAAAAAAAAAAAAAAAAAAAA
AAAAAAAA

SEQ ID NO: 109

>I'-H1N1-B mRNA

GAGUUAGGAGUUGGUGGCAGUUUCAAUAACAGGUCAUUGCCGAGAAAA
GGAUAGCACUAUAAUAUGAAGGCCAUCCUGGUGGUGAUGCUGUACACCU
UCACCACCGCCAACGCCGACACCCUGUGCAUCGGCUACCACGCCAACAAC
AGCACCGACACCGUGGACACCGUGCUGGAGAAGAACGUGACCGUGACCC
ACAGCGUGAACCUGCUGGAGGACAAGCACAACGGCAAGCUGUGCAAGCU
GCGGGGCGUGGCCCCCUGCACCUGGGCAAGUGCAACAUCGCCGGCUGG
AUCCUGGGCAACCCCGAGUGCGAGUCCCUGAGCACCGCCCGCUCCUGGA
GCUACAUCGUGGAGACCUCCAACAGCGACAACGGCACCUGCUACCCCGG
GGACUUCAUCAACUACGAGGAGCUGCGGGAGCAGCUGUCCAGCGUGUCC
AGCUUCGAGCGCUUCGAGAUCUUCCCCAAGACCUCCAGCUGGCCCAACC
ACGACUCCGACAACGGCGUGACCGCCGCCUGCCCCCACGCCGGGGCCAAG
AGCUUCUACAAGAACCUGAUCUGGCUGGUGAAGAAGGGCAAGUCCUACC
CCAAGAUCAACCAGACCUACAUCAACGACAAGGGGAAGGAGGUCCUCGU
GCUGUGGGGCAUCCACCACCCCCCCACCAUCGCCGACCAGCAGAGCCUCU
ACCAGAACGCCGACGCCUACGUCUUCGUGGGGACCUCCCGGUACAGCAA
GAAGUUCAAGCCCGAGAUCGCCACCCGCCCCAAGGUCCGGGACCAGGAG
GGCCGCAUGAACUACUACUGGACCCUGGUGGAGCCGGGGGACAAGAUCA
CCUUCGAGGCCACCGGCAACCUCGUCGCCCCGCGGUACGCCUUCACGAUG
GAGCGCGACGCCGGGUCCGGCAUCAUCAUCAGCGACACCCCCGUGCACG
ACUGCAACACGACCUGCCAGACGCCCGAGGGGGCGAUCAACACCUCCCU

GCCGUUCCAGAACGUCCACCCGAUCACGAUCGGCAAGUGCCCCAAGUAC
GUGAAGUCCACCAAGCUCCGGCUGGCCACGGGGCUCCGCAACGUCCCCUC
CAUCCAGAGCCGGGGCCUGUUCGGGGCGAUCGCCGGCUUCAUCGAGGGG
GGCUGGACCGGGAUGGUGGACGGCUGGUACGGGUACCACCACCAGAACG
AGCAGGGCUCCGGGUACGCGGCCGACCUCAAGUCGACGCAGAACGCGAU
CGACAAGAUCACCAACAAGGUCAACAGCGUGAUCGAGAAGAUGAACACG
CAGUUCACCGCCGUCGGCAAGGAGUUCAACCACCUGGAGAAGCGCAUCG
AGAACCUCAACAAGAAGGUGGACGACGGGUUCCUGGACAUCUGGACGUA
CAACGCGGAGCUCCUGGUCCUCCUGGAGAACGAGCGGACCCUCGACUAC
CACGACUCCAACGUGAAGAACCUGUACGAGAAGGUCCGCAACCAGCUCA
AGAACAACGCCAAGGAGAUCGGCAACGGGUGCUUCGAGUUCUACCACAA
GUGCGACAACACGUGCAUGGAGUCGGUGAAGAACGGCACCUACGACUAC
CCGAAGUACAGCGAGGAGGCGAAGCUGAACCGGGAGAAGAUCGACGGGG
UCAAGCUCGACUCCACGCGCAUCUACCAGAUCCUGGCCAUCUACUCGACC
GUGGCGAGCUCCCUCGUCCUGGUGGUCUCGCUCGGCGCCAUCAGCUUCU
GGAUGUGCUCCAACGGGUCGCUGCAGUGCCGGAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUUCAUUGCAAAAAAAAAAAA
AAAAAAAAAAAAAAAAAAAAGCAUAUGACUAAAAAAAAAAAAAAAAAAA
AAAAAAAAAAAAAAAAAAAAAAAAAAAAAAAAAAAAAAAAAAAAAAAAA
AAAAAAA

SEQ ID NO: 110

>I'-H3N2-B mRNA

GAGUUAGGAGUUGGUGGCAGUUUCAAUAACAGGUCAUUGCCGAGAAAA
GGAUAGCACUAUAAUAUGAAGACCAUCAUCGCCCUGAGCUACAUCCUGU
GCCUGGUGUUCGCCCAGAAGAUCCCCGGCAACGACAACAGCACCGCCACC
CUGUGCCUGGGCCACCACGCCGUGCCCAACGGCACCAUCGUGAAGACCA
UCACCAACGACCGGAUCGAGGUGACCAACGCCACCGAGCUGGUGCAGAA
CUCCAGCAUCGGCGAGAUCUGCGACUCCCCCACCAGAUCCUGGACGGCG
GCAACUGCACCCUGAUCGACGCCCUGCUGGGCGACCCCAGUGCGACGG
GUUCCAGAACAAGGAGUGGGACCUGUUCGUGGAGCGGAGCCGCGCCAAC
UCCAACUGCUACCCCUACGACGUGCCCGACUACGCCAGCCUGCGGUCCCU
GGUGGCCAGCUCCGGCACCCUGGAGUUCAAGAACGAGAGCUUCAACUGG
ACCGGGGUCAAGCAGAACGGCACCUCCAGCGCCUGCAUCCGCGGGUCCA
GCUCCAGCUUCUUCUCCCGGCUGAACUGGCUCACCCACCUGAACUACACC

UACCCCGCCCUCAACGUGACCAUGCCCAACAACGAGCAGUUCGACAAGC
UGUACAUCUGGGGCGUCCACCACCCCAGCACCGACAAGGACCAGAUCUC
CCUCUUCGCCCAGCCCAGCGGGCGCAUCACCGUGUCCACGAAGCGGAGCC
AGCAGGCCGUCAUCCCCAACAUCGGCUCCCGCCCGCGGAUCCGCGACAUC
CCCAGCCGGAUCUCCAUCUACUGGACGAUCGUGAAGCCGGGGGACAUCC
UGCUCAUCAACAGCACCGGCAACCUGAUCGCCCCCGCGGGUACUUCAA
GAUCCGGUCCGGCAAGAGCUCGAUCAUGCGCUCCGACGCCCCGAUCGGG
AAGUGCAAGAGCGAGUGCAUCACCCCCAACGGCUCGAUCCCGAACGACA
AGCCCUUCCAGAACGUCAACCGGAUCACGUACGGGGCCUGCCCGCGCUA
CGUGAAGCAGUCCACGCUCAAGCUGGCCACCGGCAUGCGGAACGUCCCC
GAGAAGCAGACCCGCGGGAUCUUCGGCGCCAUCGCGGGGUUCAUCGAGA
ACGGCUGGGAGGGGAUGGUGGACGGCUGGUACGGGUUCCGGCACCAGAA
CAGCGAGGGCCGCGGGCAGGCCGCGGACCUCAAGUCGACGCAGGCCGCG
AUCGACCAGAUCAACGGCAAGCUGAACCGGCUCAUCGGGAAGACGAACG
AGAAGUUCCACCAGAUCGAGAAGGAGUUCUCCGAGGUCGAGGGCCGCGU
GCAGGACCUGGAGAAGUACGUCGAGGACACCAAGAUCGACCUCUGGAGC
UACAACGCCGAGCUGCUCGUGGCGCUGGAGAACCAGCACACCAUCGACC
UCACGGACUCGGAGAUGAACAAGCUGUUCGAGAAGACGAAGAAGCAGCU
CCGGGAGAACGCCGAGGACAUGGGGAACGGCUGCUUCAAGAUCUACCAC
AAGUGCGACAACGCGUGCAUCGGGUCCAUCCGCAACGAGACCUACGACC
ACAACGUCUACCGGGACGAGGCCCUGAACAACCGCUUCCAGAUCAAGGG
CGUGGAGCUCAAGAGCGGGUACAAGGACUGGAUCCUGUGGAUCUCGUUC
GCGAUGUCCUGCUUCCUCCUGUGCAUCGCCCUCCUGGGCUUCAUCAUGU
GGGCGUGCCAGAAGGGGAACAUCCGGUGCAACAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUUCAUUGCAAAAAAAAAA
AAAAAAAAAAAAAAAAAAAAGCAUAUGACUAAAAAAAAAAAAAAAAAA
AAAAAAAAAAAAAAAAAAAAAAAAAAAAAAAAAAAAAAAAAAAAAAAA
AAAAAAA

SEQ ID NO: 111

>I'-B-Victoria-B mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA

UCCCCCUGACCACCACCCCCACCAAGUCCCACUUCGCCAACCUGAAGGGC
ACCGAGACCCGGGGCAAGCUGUGCCCCAAGUGCCUGAACUGCACCGACC
UGGACGUGGCCCUGGGCCGCCCCAAGUGCACCGGCAAGAUCCCCAGCGCC
CGGGUGUCCAUCCUGCACGAGGUGCGCCCCGUGACCAGCGGCUGCUUCC
CCAUCAUGCACGACCGCACCAAGAUCCGGCAGCUGCCCAACCUGCUGCGG
GGGUACGAGCACGUCCGCCUGUCCACCCACAACGUGAUCAACGCCGAGG
ACGCCCCGGCCGGCCCUACGAGAUCGGGACCAGCGGCUCCUGCCCCAAC
AUCACCAACGGGAACGGCUUCUUCGCCACCAUGGCCUGGGCCGUCCCCA
AGAACAAGACCGCCACCAACCCCCUGACCAUCGAGGUGCCGUACAUCUG
CACCGAGGGGGAGGACCAGAUCACCGUCUGGGGCUUCCACAGCGACAAC
GAGACGCAGAUGGCCAAGCUGUACGGGGACUCCAAGCCCCAGAAGUUCA
CGAGCUCCGCCAACGGCGUGACCACCCACUACGUCAGCCAGAUCGGGGG
CUUCCCGAACCAGACGGAGGACGGGGGCCUGCCCCAGUCCGGGCGCAUC
GUGGUCGACUACAUGGUGCAGAAGUCCGGCAAGACGGGGACCAUCACGU
ACCAGCGGGGCAUCCUGCUGCCGCAGAAGGUCUGGUGCGCCUCCGGGCG
CAGCAAGGUGAUCAAGGGCUCCCUGCCCCUCAUCGGGGAGGCCGACUGC
CUGCACGAGAAGUACGGCGGGCUCAACAAGAGCAAGCCGUACUACACCG
GCGAGCACGCCAAGGCCAUCGGGAACUGCCCCAUCUGGGUCAAGACCCC
GCUGAAGCUCGCCAACGGCACGAAGUACCGGCCCCCGGCCAAGCUGCUC
AAGGAGCGCGGGUUCUUCGGCGCCAUCGCCGGGUUCCUGGAGGGCGGGU
GGGAGGGCAUGAUCGCCGGGUGGCACGGCUACACGUCCCACGGGGCCCA
CGGCGUGGCGGUCGCCGCGGACCUCAAGAGCACCCAGGAGGCCAUCAAC
AAGAUCACGAAGAACCUGAACUCCCUCAGCGAGCUGGAGGUGAAGAACC
UCCAGCGCCUGUCCGGGGCGAUGGACGAGCUCCACAACGAGAUCCUGGA
GCUCGACGAGAAGGUCGACGACCUGCGGGCCGACACCAUCAGCUCGCAG
AUCGAGCUCGCGGUGCUGCUCUCCAACGAGGGCAUCAUCAACAGCGAGG
ACGAGCACCUGCUCGCCCUGGAGCGGAAGCUCAAGAAGAUGCUGGGGCC
CUCGGCGGUCGAGAUCGGCAACGGGUGCUUCGAGACCAAGCACAAGUGC
AACCAGACGUGCCUCGACCGCAUCGCCGCGGGCACGUUCGACGCCGGGG
AGUUCUCCCUGCCGACCUUCGACAGCCUCAACAUCACGGCGGCCUCGCUG
AACGACGACGGCCUCGACAACCACACCAUCCUGCUCUACUACUCCACCGC
GGCCAGCUCGCUGGCGGUGACGCUCAUGAUCGCCAUCUUCGUCGUGUAC
AUGGUCUCCCGCGACAACGUGAGCUGCUCGAUCUGCCUGUGAUGACUCG
AGGCUCGCUUUCUUGCUGUCCAAUUUCUAUUAAAGGUUCCUUUGUUCCC
UAAGUCCAACUACUAAACUGGGGGAUAUUAUGAAGGGCCUUGAGCAUCU
GGAUUCUGCCUAAUAAAAACAUUUAUUUUCAUUGCAAAAAAAAAAAAA

AAAAAAAAAAAAAAAAAAAAAGCAUAUGACUAAAAAAAAAAAAAAAAAA
AAAAAAAAAAAAAAAAAAAAAAAAAAAAAAAAAAAAAAAAAAAAAAAAA
AAAAA

SEQ ID NO: 112

>I'-B-Yamagata-B mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCUACUUCGCCAACCUGAAGGGC
ACCCGGACCCGCGGCAAGCUGUGCCCCGACUGCCUGAACUGCACCGACCU
GGACGUGGCCCUGGGCCGGCCCAUGUGCGUGGGCACCACCCCCAGCGCCA
AGGCCUCCAUCCUGCACGAGGUGCGCCCCGUGACCAGCGGCUGCUUCCCC
AUCAUGCACGACCGCACCAAGAUCCGGCAGCUGCCCAACCUGCUGCGGG
GGUACGAGAAGAUCCGCCUGUCCACCCAGAACGUCAUCGACGCCGAGAA
GGCCCCCGGCGGGCCCUACCGGCUGGGCACCAGCGGGUCCUGCCCCAACG
CCACCAGCAAGAUCGGCUUCUUCGCCACCAUGGCCUGGGCCGUGCCCAA
GGACAACUACAAGAACGCCACCAACCCCCUGACCGUCGAGGUGCCCUAC
AUCUGCACCGAGGGGGAGGACCAGAUCACGGUCUGGGGCUUCCACUCCG
ACAACAAGACCCAGAUGAAGUCCCUGUACGGGGACUCCAACCCGCAGAA
GUUCACGAGCUCCGCCAACGGCGUGACCACGCACUACGUCAGCCAGAUC
GGGGACUUCCCGGACCAGACCGAGGACGGCGGGCUGCCCCAGUCCGGCC
GCAUCGUGGUCGACUACAUGAUGCAGAAGCCCGGGAAGACGGGCACCAU
CGUGUACCAGCGGGGGGGUCCUGCUGCCGCAGAAGGUGUGGUGCGCCAGC
GGCCGCUCCAAGGUCAUCAAGGGGAGCCUGCCCCUCAUCGGCGAGGCCG
ACUGCCUGCACGAGGAGUACGGGGGCCUCAACAAGUCCAAGCCGUACUA
CACGGGGAAGCACGCCAAGGCCAUCGGCAACUGCCCGAUCUGGGUGAAG
ACCCCCUGAAGCUCGCCAACGGGACGAAGUACCGGCCGCCCGCCAAGCU
GCUCAAGGAGCGCGGCUUCUUCGGGGCCAUCGCCGGCUUCCUGGAGGGG
GGCUGGGAGGGGAUGAUCGCCGGCUGGCACGGGUACACCAGCCACGGCG
CCCACGGGGUCGCGGUGGCCGCGGACCUCAAGUCCACGCAGGAGGCCAU
CAACAAGAUCACCAAGAACCUGAACAGCCUCUCCGAGCUGGAGGUCAAG
AACCUCCAGCGCCUGAGCGGCGCGAUGGACGAGCUCCACAACGAGAUCC
UGGAGCUCGACGAGAAGGUGGACGACCUGCGGGCCGACACGAUCUCGUC
CCAGAUCGAGCUCGCGGUCCUGCUCAGCAACGAGGGGAUCAUCAACUCG
GAGGACGAGCACCUGCUCGCCCUGGAGCGGAAGCUCAAGAAGAUGCUGG

GCCCGUCCGCGGUGGACAUCGGGAACGGCUGCUUCGAGACCAAGCACAA
GUGCAACCAGACGUGCCUCGACCGCAUCGCCGCGGGGACCUUCAACGCC
GGCGAGUUCAGCCUGCCCACGUUCGACUCGCUCAACAUCACCGCGGCCUC
CCUGAACGACGACGGGCUCGACAACCACACGAUCCUGCUCUACUACAGC
ACCGCGGCCUCGUCCCUGGCGGUCACGCUCAUGCUGGCCAUCUUCAUCG
UGUACAUGGUCAGCCGCGACAACGUGUCGUGCUCCAUCUGCCUCUGAUG
ACUCGAGGCUCGCUUUCUUGCUGUCCAAUUUCUAUUAAAGGUUCCUUUG
UUCCCUAAGUCCAACUACUAAACUGGGGGAUAUUAUGAAGGGCCUUGAG
CAUCUGGAUUCUGCCUAAUAAAAACAUUUAUUUUCAUUGCAAAAAAAA
AAAAAAAAAAAAAAAAAAAAAAAGCAUAUGACUAAAAAAAAAAAAAAA
AAAAAAAAAAAAAAAAAAAAAAAAAAAAAAAAAAAAAAAAAAAAAAAA
AAAAAAAAA

SEQ ID NO: 113

>I'-H1N1-HA0-mut-B mRNA

GAGUUAGGAGUUGGUGGCAGUUUCAAUAACAGGUCAUUGCCGAGAAAA
GGAUAGCACUAUAAUAUGAAGGCCAUCCUGGUGGUGAUGCUGUACACCU
UCACCACCGCCAACGCCGACACCCUGUGCAUCGGCUACCACGCCAACAAC
AGCACCGACACCGUGGACACCGUGCUGGAGAAGAACGUGACCGUGACCC
ACAGCGUGAACCUGCUGGAGGACAAGCACAACGGCAAGCUGUGCAAGCU
GCGGGGCGUGGCCCCCUGCACCUGGGCAAGUGCAACAUCGCCGGCUGG
AUCCUGGGCAACCCCGAGUGCGAGUCCCUGAGCACCGCCCGCUCCUGGA
GCUACAUCGUGGAGACCUCCAACAGCGACAACGGCACCUGCUACCCCGG
GGACUUCAUCAACUACGAGGAGCUGCGGGAGCAGCUGUCCAGCGUGUCC
AGCUUCGAGCGCUUCGAGAUCUUCCCCAAGACCUCCAGCUGGCCCAACC
ACGACUCCGACAACGGCGUGACCGCCGCCUGCCCCCACGCCGGGGCCAAG
AGCUUCUACAAGAACCUGAUCUGGCUGGUGAAGAAGGGCAAGUCCUACC
CCAAGAUCAACCAGACCUACAUCAACGACAAGGGGAAGGAGGUCCUCGU
GCUGUGGGGCAUCCACCACCCCCCCACCAUCGCCGACCAGCAGAGCCUCU
ACCAGAACGCCGACGCCUACGUCUUCGUGGGGACCUCCCGGUACAGCAA
GAAGUUCAAGCCCGAGAUCGCCACCCGCCCCAAGGUCCGGGACCAGGAG
GGCCGCAUGAACUACUACUGGACCCUGGUGGAGCCGGGGGACAAGAUCA
CCUUCGAGGCCACCGGCAACCUCGUCGCCCCGCGGUACGCCUUCACGAUG
GAGCGCGACGCCGGGUCCGGCAUCAUCAUCAGCGACACCCCCGUGCACG
ACUGCAACACGACCUGCCAGACGCCCGAGGGGGCGAUCAACACCUCCCU
GCCGUUCCAGAACGUCCACCCGAUCACGAUCGGCAAGUGCCCCAAGUAC
GUGAAGUCCACCAAGCUCCGGCUGGCCACGGGGCUCCGCAACGUCCCUC

CAUCCAGAGCCAGGGCCUGUUCGGGGCGAUCGCCGGCUUCAUCGAGGGG
GGCUGGACCGGGAUGGUGGACGGCUGGUACGGGUACCACCACCAGAACG
AGCAGGGCUCCGGGUACGCGGCCGACCUCAAGUCGACGCAGAACGCGAU
CGACAAGAUCACCAACAAGGUCAACAGCGUGAUCGAGAAGAUGAACACG
CAGUUCACCGCCGUCGGCAAGGAGUUCAACCACCUGGAGAAGCGCAUCG
AGAACCUCAACAAGAAGGUGGACGACGGGUUCCUGGACAUCUGGACGUA
CAACGCGGAGCUCCUGGUCCUCCUGGAGAACGAGCGGACCCUCGACUAC
CACGACUCCAACGUGAAGAACCUGUACGAGAAGGUCCGCAACCAGCUCA
AGAACAACGCCAAGGAGAUCGGCAACGGGUGCUUCGAGUUCUACCACAA
GUGCGACAACACGUGCAUGGAGUCGGUGAAGAACGGCACCUACGACUAC
CCGAAGUACAGCGAGGAGGCGAAGCUGAACCGGGAGAAGAUCGACGGGG
UCAAGCUCGACUCCACGCGCAUCUACCAGAUCCUGGCCAUCUACUCGACC
GUGGCGAGCUCCCUCGUCCUGGUGGUCUCGCUCGGCGCCAUCAGCUUCU
GGAUGUGCUCCAACGGGUCGCUGCAGUGCCGGAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUCAUUGCAAAAAAAAAA
AAAAAAAAAAAAAAAAAAAAAAGCAUAUGACUAAAAAAAAAAAAAAAAA
AAAAAAAAAAAAAAAAAAAAAAAAAAAAAAAAAAAAAAAAAAAAAAAAA
AAAAAAA

SEQ ID NO: 114

>I'-H3N2-HA0-mut-B mRNA

GAGUUAGGAGUUGGUGGCAGUUUCAAUAACAGGUCAUUGCCGAGAAAA
GGAUAGCACUAUAAUAUGAAGACCAUCAUCGCCCUGAGCUACAUCCUGU
GCCUGGUGUUCGCCCAGAAGAUCCCCGGCAACGACAACAGCACCGCCACC
CUGUGCCUGGGCCACCACGCCGUGCCCAACGGCACCAUCGUGAAGACCA
UCACCAACGACCGGAUCGAGGUGACCAACGCCACCGAGCUGGUGCAGAA
CUCCAGCAUCGGCGAGAUCUGCGACUCCCCCACCAGAUCCUGGACGGCG
GCAACUGCACCCUGAUCGACGCCCUGCUGGGCGACCCCCAGUGCGACGG
GUUCCAGAACAAGGAGUGGGACCUGUUCGUGGAGCGGAGCCGCGCCAAC
UCCAACUGCUACCCCUACGACGUGCCCGACUACGCCAGCCUGCGGUCCCU
GGUGGCCAGCUCCGGCACCCUGGAGUUCAAGAACGAGAGCUUCAACUGG
ACCGGGGUCAAGCAGAACGGCACCUCCAGCGCCUGCAUCCGCGGGUCCA
GCUCCAGCUUCUUCUCCCGGCUGAACUGGCUCACCCACCUGAACUACACC
UACCCCGCCCUCAACGUGACCAUGCCCAACAACGAGCAGUUCGACAAGC
UGUACAUCUGGGGCGUCCACCACCCCAGCACCGACAAGGACCAGAUCUC

CCUCUUCGCCCAGCCCAGCGGGCGCAUCACCGUGUCCACGAAGCGGAGCC
AGCAGGCCGUCAUCCCCAACAUCGGCUCCCGCCCGCGGAUCCGCGACAUC
CCCAGCCGGAUCUCCAUCUACUGGACGAUCGUGAAGCCGGGGGGACAUCC
UGCUCAUCAACAGCACCGGCAACCUGAUCGCCCCCGCGGGUACUUCAA
GAUCCGGUCCGGCAAGAGCUCGAUCAUGCGCUCCGACGCCCCGAUCGGG
AAGUGCAAGAGCGAGUGCAUCACCCCCAACGGCUCGAUCCCGAACGACA
AGCCCUUCCAGAACGUCAACCGGAUCACGUACGGGGCCUGCCCGCGCUA
CGUGAAGCAGUCCACGCUCAAGCUGGCCACCGGCAUGCGGAACGUCCCC
GAGAAGCAGACCCAGGGGAUCUUCGGCGCCAUCGCGGGGUUCAUCGAGA
ACGGCUGGGAGGGGAUGGUGGACGGCUGGUACGGGUUCCGGCACCAGAA
CAGCGAGGGCCGCGGGCAGGCCGCGGACCUCAAGUCGACGCAGGCCGCG
AUCGACCAGAUCAACGGCAAGCUGAACCGGCUCAUCGGGAAGACGAACG
AGAAGUUCCACCAGAUCGAGAAGGAGUUCUCCGAGGUCGAGGGCCGCGU
GCAGGACCUGGAGAAGUACGUCGAGGACACCAAGAUCGACCUCUGGAGC
UACAACGCCGAGCUGCUCGUGGCGCUGGAGAACCAGCACACCAUCGACC
UCACGGACUCGGAGAUGAACAAGCUGUUCGAGAAGACGAAGAAGCAGCU
CCGGGAGAACGCCGAGGACAUGGGGAACGGCUGCUUCAAGAUCUACCAC
AAGUGCGACAACGCGUGCAUCGGGUCCAUCCGCAACGAGACCUACGACC
ACAACGUCUACCGGGACGAGGCCCUGAACAACCGCUUCCAGAUCAAGGG
CGUGGAGCUCAAGAGCGGGUACAAGGACUGGAUCCUGUGGAUCUCGUUC
GCGAUGUCCUGCUUCCUCCUGUGCAUCGCCCUCCUGGGCUUCAUCAUGU
GGGCGUGCCAGAAGGGGAACAUCCGGUGCAACAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUCAUUGCAAAAAAAAAAA
AAAAAAAAAAAAAAAAAAAAAGCAUAUGACUAAAAAAAAAAAAAAAAA
AAAAAAAAAAAAAAAAAAAAAAAAAAAAAAAAAAAAAAAAAAAAAAAA
AAAAAAA

<div align="right">SEQ ID NO: 115</div>

>I'-B-Victoria-HA0-mut-B mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCCACUUCGCCAACCUGAAGGGC
ACCGAGACCCGGGGCAAGCUGUGCCCCAAGUGCCUGAACUGCACCGACC

UGGACGUGGCCCUGGGCCGCCCCAAGUGCACCGGCAAGAUCCCCAGCGCC
CGGGUGUCCAUCCUGCACGAGGUGCGCCCCGUGACCAGCGGCUGCUUCC
CCAUCAUGCACGACCGCACCAAGAUCCGGCAGCUGCCCAACCUGCUGCGG
GGGUACGAGCACGUCCGCCUGUCCACCCACAACGUGAUCAACGCCGAGG
ACGCCCCCGGCCGGCCCUACGAGAUCGGGACCAGCGGCUCCUGCCCCAAC
AUCACCAACGGGAACGGCUUCUUCGCCACCAUGGCCUGGGCCGUCCCCA
AGAACAAGACCGCCACCAACCCCCUGACCAUCGAGGUGCCGUACAUCUG
CACCGAGGGGGAGGACCAGAUCACCGUCUGGGGCUUCCACAGCGACAAC
GAGACGCAGAUGGCCAAGCUGUACGGGGACUCCAAGCCCCAGAAGUUCA
CGAGCUCCGCCAACGGCGUGACCACCCACUACGUCAGCCAGAUCGGGGG
CUUCCCGAACCAGACGGAGGACGGGGGCCUGCCCCAGUCCGGGCGCAUC
GUGGUCGACUACAUGGUGCAGAAGUCCGGCAAGACGGGGACCAUCACGU
ACCAGCGGGGCAUCCUGCUGCCGCAGAAGGUCUGGUGCGCCUCCGGGCG
CAGCAAGGUGAUCAAGGGCUCCCUGCCCCUCAUCGGGGAGGCCGACUGC
CUGCACGAGAAGUACGGCGGGCUCAACAAGAGCAAGCCGUACUACACCG
GCGAGCACGCCAAGGCCAUCGGGAACUGCCCCAUCUGGGUCAAGACCCC
GCUGAAGCUCGCCAACGGCACGAAGUACGGCCCCCGGCCAAGCUGCUC
AAGGAGCAGGGGUUCUUCGGCGCCAUCGCCGGGUUCCUGGAGGGCGGGU
GGGAGGGCAUGAUCGCCGGGUGGCACGGCUACACGUCCCACGGGGCCCA
CGGCGUGGCGGUCGCCGCGGACCUCAAGAGCACCCAGGAGGCCAUCAAC
AAGAUCACGAAGAACCUGAACUCCCUCAGCGAGCUGGAGGUGAAGAACC
UCCAGCGCCUGUCCGGGGCGAUGGACGAGCUCCACAACGAGAUCCUGGA
GCUCGACGAGAAGGUCGACGACCUGCGGGCCGACACCAUCAGCUCGCAG
AUCGAGCUCGCGGUGCUGCUCUCCAACGAGGGCAUCAUCAACAGCGAGG
ACGAGCACCUGCUCGCCCUGGAGCGGAAGCUCAAGAAGAUGCUGGGGCC
CUCGGCGGUCGAGAUCGGCAACGGGUGCUUCGAGACCAAGCACAAGUGC
AACCAGACGUGCCUCGACCGCAUCGCCGCGGGCACGUUCGACGCCGGGG
AGUUCUCCCUGCCGACCUUCGACAGCCUCAACAUCACGGCGGCCUCGCUG
AACGACGACGGCCUCGACAACCACACCAUCCUGCUCUACUACUCCACCGC
GGCCAGCUCGCUGGCGGUGACGCUCAUGAUCGCCAUCUUCGUCGUGUAC
AUGGUCUCCCGCGACAACGUGAGCUGCUCGAUCUGCCUGUGAUGACUCG
AGGCUCGCUUUCUUGCUGUCCAAUUUCUAUUAAAGGUUCCUUUGUUCCC
UAAGUCCAACUACUAAACUGGGGGAUAUUAUGAAGGGCCUUGAGCAUCU
GGAUUCUGCCUAAUAAAAACAUUUAUUUUCAUUGCAAAAAAAAAAAA
AAAAAAAAAAAAAAAAAAAGCAUAUGACUAAAAAAAAAAAAAAAAAAA
AAAAAAAAAAAAAAAAAAAAAAAAAAAAAAAAAAAAAAAAAAAAAAAA

AAAAAA

SEQ ID NO: 116

>I'-B-Yamagata-HA0-mut-B mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCUACUUCGCCAACCUGAAGGGC
ACCCGGACCCGCGGCAAGCUGUGCCCCGACUGCCUGAACUGCACCGACCU
GGACGUGGCCCUGGGCCGGCCCAUGUGCGUGGGCACCACCCCCAGCGCCA
AGGCCUCCAUCCUGCACGAGGUGCGCCCCGUGACCAGCGGCUGCUUCCCC
AUCAUGCACGACCGCACCAAGAUCCGGCAGCUGCCCAACCUGCUGCGGG
GGUACGAGAAGAUCCGCCUGUCCACCCAGAACGUCAUCGACGCCGAGAA
GGCCCCCGGCGGGCCCUACCGGCUGGGCACCAGCGGGUCCUGCCCCAACG
CCACCAGCAAGAUCGGCUUCUUCGCCACCAUGGCCUGGGCCGUGCCCAA
GGACAACUACAAGAACGCCACCAACCCCCUGACCGUCGAGGUGCCCUAC
AUCUGCACCGAGGGGGAGGACCAGAUCACGGUCUGGGGCUUCCACUCCG
ACAACAAGACCCAGAUGAAGUCCCUGUACGGGGACUCCAACCCGCAGAA
GUUCACGAGCUCCGCCAACGGCGUGACCACGCACUACGUCAGCCAGAUC
GGGGACUUCCCGGACCAGACCGAGGACGGCGGGCUGCCCCAGUCCGGCC
GCAUCGUGGUCGACUACAUGAUGCAGAAGCCCGGGAAGACGGGCACCAU
CGUGUACCAGCGGGGGGGUCCUGCUGCCGCAGAAGGUGUGGUGCGCCAGC
GGCCGCUCCAAGGUCAUCAAGGGGAGCCUGCCCCUCAUCGGCGAGGCCG
ACUGCCUGCACGAGGAGUACGGGGGCCUCAACAAGUCCAAGCCGUACUA
CACGGGGAAGCACGCCAAGGCCAUCGGCAACUGCCCGAUCUGGGUGAAG
ACCCCCCUGAAGCUCGCCAACGGGACGAAGUACCGGCCGCCCGCCAAGCU
GCUCAAGGAGCAGGGCUUCUUCGGGGCCAUCGCCGGCUUCCUGGAGGGG
GGCUGGGAGGGGAUGAUCGCCGGCUGGCACGGGUACACCAGCCACGGCG
CCCACGGGGUCGCGGUGGCCGCGGACCUCAAGUCCACGCAGGAGGCCAU
CAACAAGAUCACCAAGAACCUGAACAGCCUCUCCGAGCUGGAGGUCAAG
AACCUCCAGCGCCUGAGCGGCGCGAUGGACGAGCUCCACAACGAGAUCC
UGGAGCUCGACGAGAAGGUGGACGACCUGCGGGCCGACACGAUCUCGUC
CCAGAUCGAGCUCGCGGUCCUGCUCAGCAACGAGGGGAUCAUCAACUCG
GAGGACGAGCACCUGCUCGCCCUGGAGCGGAAGCUCAAGAAGAUGCUGG
GCCCGUCCGCGGUGGACAUCGGGAACGGCUGCUUCGAGACCAAGCACAA
GUGCAACCAGACGUGCCUCGACCGCAUCGCCGCGGGGACCUUCAACGCC

GGCGAGUUCAGCCUGCCCACGUUCGACUCGCUCAACAUCACCGCGGCCUC
CCUGAACGACGACGGGCUCGACAACCACACGAUCCUGCUCUACUACAGC
ACCGCGGCCUCGUCCUGGCGGUCACGCUCAUGCUGGCCAUCUUCAUCG
UGUACAUGGUCAGCCGCGACAACGUGUCGUGCUCCAUCUGCCUCUGAUG
ACUCGAGGCUCGCUUUCUUGCUGUCCAAUUUCUAUUAAAGGUUCCUUUG
UUCCCUAAGUCCAACUACUAAACUGGGGGAUAUUAUGAAGGGCCUUGAG
CAUCUGGAUUCUGCCUAAUAAAAACAUUUAUUUUCAUUGCAAAAAAAA
AAAAAAAAAAAAAAAAAAAAAAAGCAUAUGACUAAAAAAAAAAAAAAA
AAAAAAAAAAAAAAAAAAAAAAAAAAAAAAAAAAAAAAAAAAAAAAAA
AAAAAAAAA

SEQ ID NO: 117

>I'-H1N1-B-120A mRNA

GAGUUAGGAGUUGGUGGCAGUUUCAAUAACAGGUCAUUGCCGAGAAAA
GGAUAGCACUAUAAUAUGAAGGCCAUCCUGGUGGUGAUGCUGUACACCU
UCACCACCGCCAACGCCGACACCCUGUGCAUCGGCUACCACGCCAACAAC
AGCACCGACACCGUGGACACCGUGCUGGAGAAGAACGUGACCGUGACCC
ACAGCGUGAACCUGCUGGAGGACAAGCACAACGGCAAGCUGUGCAAGCU
GCGGGGCGUGGCCCCCUGCACCUGGGCAAGUGCAACAUCGCCGGCUGG
AUCCUGGGCAACCCCGAGUGCGAGUCCCUGAGCACCGCCCGCUCCUGGA
GCUACAUCGUGGAGACCUCCAACAGCGACAACGGCACCUGCUACCCCGG
GGACUUCAUCAACUACGAGGAGCUGCGGGAGCAGCUGUCCAGCGUGUCC
AGCUUCGAGCGCUUCGAGAUCUUCCCCAAGACCUCCAGCUGGCCCAACC
ACGACUCCGACAACGGCGUGACCGCCGCCUGCCCCCACGCCGGGGCCAAG
AGCUUCUACAAGAACCUGAUCUGGCUGGUGAAGAAGGGCAAGUCCUACC
CCAAGAUCAACCAGACCUACAUCAACGACAAGGGGAAGGAGGUCCUCGU
GCUGUGGGGCAUCCACCACCCCCCCACCAUCGCCGACCAGCAGAGCCUCU
ACCAGAACGCCGACGCCUACGUCUUCGUGGGGACCUCCCGGUACAGCAA
GAAGUUCAAGCCCGAGAUCGCCACCCGCCCCAAGGUCCGGGACCAGGAG
GGCCGCAUGAACUACUACUGGACCCUGGUGGAGCCGGGGGACAAGAUCA
CCUUCGAGGCCACCGGCAACCUCGUCGCCCCGCGGUACGCCUUCACGAUG
GAGCGCGACGCCGGGUCCGGCAUCAUCAUCAGCGACACCCCCGUGCACG
ACUGCAACACGACCUGCCAGACGCCCGAGGGGGCGAUCAACACCUCCCU
GCCGUUCCAGAACGUCCACCCGAUCACGAUCGGCAAGUGCCCCAAGUAC
GUGAAGUCCACCAAGCUCCGGCUGGCCACGGGGCUCCGCAACGUCCCCUC
CAUCCAGAGCCGGGGCCUGUUCGGGGCGAUCGCCGGCUUCAUCGAGGGG
GGCUGGACCGGGAUGGUGGACGGCUGGUACGGGUACCACCACCAGAACG

AGCAGGGCUCCGGGUACGCGGCCGACCUCAAGUCGACGCAGAACGCGAU
CGACAAGAUCACCAACAAGGUCAACAGCGUGAUCGAGAAGAUGAACACG
CAGUUCACCGCCGUCGGCAAGGAGUUCAACCACCUGGAGAAGCGCAUCG
AGAACCUCAACAAGAAGGUGGACGACGGGUUCCUGGACAUCUGGACGUA
CAACGCGGAGCUCCUGGUCCUCCUGGAGAACGAGCGGACCCUCGACUAC
CACGACUCCAACGUGAAGAACCUGUACGAGAAGGUCCGCAACCAGCUCA
AGAACAACGCCAAGGAGAUCGGCAACGGGUGCUUCGAGUUCUACCACAA
GUGCGACAACACGUGCAUGGAGUCGGUGAAGAACGGCACCUACGACUAC
CCGAAGUACAGCGAGGAGGCGAAGCUGAACCGGGAGAAGAUCGACGGGG
UCAAGCUCGACUCCACGCGCAUCUACCAGAUCCUGGCCAUCUACUCGACC
GUGGCGAGCUCCCUCGUCCUGGUGGUCUCGCUCGGCGCCAUCAGCUUCU
GGAUGUGCUCCAACGGGUCGCUGCAGUGCCGGAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUCAUUGCAAAAAAAAAAA
AAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAA
AAAAAAAAAAAAAA

SEQ ID NO: 118

>I'-H3N2-B-120A mRNA

GAGUUAGGAGUUGGUGGCAGUUUCAAUAACAGGUCAUUGCCGAGAAAA
GGAUAGCACUAUAAUAUGAAGACCAUCAUCGCCCUGAGCUACAUCCUGU
GCCUGGUGUUCGCCCAGAAGAUCCCCGGCAACGACAACAGCACCGCCACC
CUGUGCCUGGGCCACCACGCCGUGCCCAACGGCACCAUCGUGAAGACCA
UCACCAACGACCGGAUCGAGGUGACCAACGCCACCGAGCUGGUGCAGAA
CUCCAGCAUCGGCGAGAUCUGCGACUCCCCCACCAGAUCCUGGACGGCG
GCAACUGCACCCUGAUCGACGCCCUGCUGGGCGACCCCCAGUGCGACGG
GUUCCAGAACAAGGAGUGGGACCUGUUCGUGGAGCGGAGCCGCGCCAAC
UCCAACUGCUACCCCUACGACGUGCCCGACUACGCCAGCCUGCGGUCCCU
GGUGGCCAGCUCCGGCACCCUGGAGUUCAAGAACGAGAGCUUCAACUGG
ACCGGGGUCAAGCAGAACGGCACCUCCAGCGCCUGCAUCCGCGGGUCCA
GCUCCAGCUUCUUCUCCCGGCUGAACUGGCUCACCCACCUGAACUACACC
UACCCCGCCCUCAACGUGACCAUGCCCAACAACGAGCAGUUCGACAAGC
UGUACAUCUGGGGCGUCCACCACCCCAGCACCGACAAGGACCAGAUCUC
CCUCUUCGCCCAGCCCAGCGGGCGCAUCACCGUGUCCACGAAGCGGAGCC
AGCAGGCCGUCAUCCCCAACAUCGGCUCCCGCCCGCGGAUCCGCGACAUC

CCCAGCCGGAUCUCCAUCUACUGGACGAUCGUGAAGCCGGGGGGACAUCC
UGCUCAUCAACAGCACCGGCAACCUGAUCGCCCCCGCGGGUACUUCAA
GAUCCGGUCCGGCAAGAGCUCGAUCAUGCGCUCCGACGCCCGAUCGGG
AAGUGCAAGAGCGAGUGCAUCACCCCCAACGGCUCGAUCCCGAACGACA
AGCCCUUCCAGAACGUCAACCGGAUCACGUACGGGGCCUGCCCGCGCUA
CGUGAAGCAGUCCACGCUCAAGCUGGCCACCGGCAUGCGGAACGUCCCC
GAGAAGCAGACCCGCGGGAUCUUCGGCGCCAUCGCGGGGUUCAUCGAGA
ACGGCUGGGAGGGGAUGGUGGACGGCUGGUACGGGUUCCGGCACCAGAA
CAGCGAGGGCCGCGGGCAGGCCGCGGACCUCAAGUCGACGCAGGCCGCG
AUCGACCAGAUCAACGGCAAGCUGAACCGGCUCAUCGGGAAGACGAACG
AGAAGUUCCACCAGAUCGAGAAGGAGUUCUCCGAGGUCGAGGGCCGCGU
GCAGGACCUGGAGAAGUACGUCGAGGACACCAAGAUCGACCUCUGGAGC
UACAACGCCGAGCUGCUCGUGGCGCUGGAGAACCAGCACACCAUCGACC
UCACGGACUCGGAGAUGAACAAGCUGUUCGAGAAGACGAAGAAGCAGCU
CCGGGAGAACGCCGAGGACAUGGGGAACGGCUGCUUCAAGAUCUACCAC
AAGUGCGACAACGCGUGCAUCGGGUCCAUCCGCAACGAGACCUACGACC
ACAACGUCUACCGGGACGAGGCCCUGAACAACCGCUUCCAGAUCAAGGG
CGUGGAGCUCAAGAGCGGGUACAAGGACUGGAUCCUGUGGAUCUCGUUC
GCGAUGUCCUGCUUCCUCCUGUGCAUCGCCCUCCUGGGCUUCAUCAUGU
GGGCGUGCCAGAAGGGGAACAUCCGGUGCAACAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUUCAUUGCAAAAAAAAAAA
AAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAA
AAAAAAAAAAAAAA

SEQ ID NO: 119

>I'-B-Victoria-B-120A mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCCACUUCGCCAACCUGAAGGGC
ACCGAGACCCGGGGCAAGCUGUGCCCCAAGUGCCUGAACUGCACCGACC
UGGACGUGGCCCUGGGCCGCCCCAAGUGCACCGGCAAGAUCCCCAGCGCC
CGGGUGUCCAUCCUGCACGAGGUGCGCCCCGUGACCAGCGGCUGCUUCC

CCAUCAUGCACGACCGCACCAAGAUCCGGCAGCUGCCCAACCUGCUGCGG
GGGUACGAGCACGUCCGCCUGUCCACCCACAACGUGAUCAACGCCGAGG
ACGCCCCCGGCCGGCCCUACGAGAUCGGGACCAGCGGCUCCUGCCCCAAC
AUCACCAACGGGAACGGCUUCUUCGCCACCAUGGCCUGGGCCGUCCCCA
AGAACAAGACCGCCACCAACCCCUGACCAUCGAGGUGCCGUACAUCUG
CACCGAGGGGGAGGACCAGAUCACCGUCUGGGGCUUCCACAGCGACAAC
GAGACGCAGAUGGCCAAGCUGUACGGGGACUCCAAGCCCCAGAAGUUCA
CGAGCUCCGCCAACGGCGUGACCACCCACUACGUCAGCCAGAUCGGGGG
CUUCCCGAACCAGACGGAGGACGGGGGCCUGCCCCAGUCCGGGCGCAUC
GUGGUCGACUACAUGGUGCAGAAGUCCGGCAAGACGGGGACCAUCACGU
ACCAGCGGGGCAUCCUGCUGCCGCAGAAGGUCUGGUGCGCCUCCGGGCG
CAGCAAGGUGAUCAAGGGCUCCCUGCCCCUCAUCGGGGAGGCCGACUGC
CUGCACGAGAAGUACGGCGGGCUCAACAAGAGCAAGCCGUACUACACCG
GCGAGCACGCCAAGGCCAUCGGGAACUGCCCCAUCUGGGUCAAGACCCC
GCUGAAGCUCGCCAACGGCACGAAGUACCGGCCCCCGGCCAAGCUGCUC
AAGGAGCGCGGGUUCUUCGGCGCCAUCGCCGGGUUCCUGGAGGGCGGGU
GGGAGGGCAUGAUCGCCGGGUGGCACGGCUACACGUCCCACGGGGCCCA
CGGCGUGGCGGUCGCCGCGGACCUCAAGAGCACCCAGGAGGCCAUCAAC
AAGAUCACGAAGAACCUGAACUCCCUCAGCGAGCUGGAGGUGAAGAACC
UCCAGCGCCUGUCCGGGGCGAUGGACGAGCUCCACAACGAGAUCCUGGA
GCUCGACGAGAAGGUCGACGACCUGCGGGCCGACACCAUCAGCUCGCAG
AUCGAGCUCGCGGUGCUGCUCUCCAACGAGGGCAUCAUCAACAGCGAGG
ACGAGCACCUGCUCGCCCUGGAGCGGAAGCUCAAGAAGAUGCUGGGGCC
CUCGGCGGUCGAGAUCGGCAACGGGUGCUUCGAGACCAAGCACAAGUGC
AACCAGACGUGCCUCGACCGCAUCGCCGCGGGCACGUUCGACGCCGGGG
AGUUCUCCCUGCCGACCUUCGACAGCCUCAACAUCACGGCGGCCUCGCUG
AACGACGACGGCCUCGACAACCACACCAUCCUGCUCUACUACUCCACCGC
GGCCAGCUCGCUGGCGGUGACGCUCAUGAUCGCCAUCUUCGUCGUGUAC
AUGGUCUCCCGCGACAACGUGAGCUGCUCGAUCUGCCUGUGAUGACUCG
AGGCUCGCUUUCUUGCUGUCCAAUUUCUAUUAAAGGUUCCUUUGUUCCC
UAAGUCCAACUACUAAACUGGGGGAUAUUAUGAAGGGCCUUGAGCAUCU
GGAUUCUGCCUAAUAAAAACAUUUAUUUUCAUUGCAAAAAAAAAAA
AAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAA
AAAAAAAAAAAAA

SEQ ID NO: 120

>I'-B-Yamagata-B-120A mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCUACUUCGCCAACCUGAAGGGC
ACCCGGACCCGCGGCAAGCUGUGCCCCGACUGCCUGAACUGCACCGACCU
GGACGUGGCCCUGGGCCGGCCCAUGUGCGUGGGCACCACCCCCAGCGCCA
AGGCCUCCAUCCUGCACGAGGUGCGCCCCGUGACCAGCGGCUGCUUCCCC
AUCAUGCACGACCGCACCAAGAUCCGGCAGCUGCCCAACCUGCUGCGGG
GGUACGAGAAGAUCCGCCUGUCCACCCAGAACGUCAUCGACGCCGAGAA
GGCCCCCGGCGGGCCCUACCGGCUGGGCACCAGCGGGUCCUGCCCCAACG
CCACCAGCAAGAUCGGCUUCUUCGCCACCAUGGCCUGGGCCGUGCCCAA
GGACAACUACAAGAACGCCACCAACCCCCUGACCGUCGAGGUGCCCUAC
AUCUGCACCGAGGGGGAGGACCAGAUCACGGUCUGGGGCUUCCACUCCG
ACAACAAGACCCAGAUGAAGUCCCUGUACGGGGACUCCAACCCGCAGAA
GUUCACGAGCUCCGCCAACGGCGUGACCACGCACUACGUCAGCCAGAUC
GGGGACUUCCCGGACCAGACCGAGGACGGCGGGCUGCCCCAGUCCGGCC
GCAUCGUGGUCGACUACAUGAUGCAGAAGCCCGGGAAGACGGGCACCAU
CGUGUACCAGCGGGGGGGUCCUGCUGCCGCAGAAGGUGUGGUGCGCCAGC
GGCCGCUCCAAGGUCAUCAAGGGGAGCCUGCCCCUCAUCGGCGAGGCCG
ACUGCCUGCACGAGGAGUACGGGGGCCUCAACAAGUCCAAGCCGUACUA
CACGGGGAAGCACGCCAAGGCCAUCGGCAACUGCCCGAUCUGGGUGAAG
ACCCCCCUGAAGCUCGCCAACGGGACGAAGUACCGGCCGCCCGCCAAGCU
GCUCAAGGAGCGCGGCUUCUUCGGGGCCAUCGCCGGCUUCCUGGAGGGG
GGCUGGGAGGGGAUGAUCGCCGGCUGGCACGGGUACACCAGCCACGGCG
CCCACGGGGUCGCGGUGGCCGCGGACCUCAAGUCCACGCAGGAGGCCAU
CAACAAGAUCACCAAGAACCUGAACAGCCUCUCCGAGCUGGAGGUCAAG
AACCUCCAGCGCCUGAGCGGCGCGAUGGACGAGCUCCACAACGAGAUCC
UGGAGCUCGACGAGAAGGUGGACGACCUGCGGGCCGACACGAUCUCGUC
CCAGAUCGAGCUCGCGGUCCUGCUCAGCAACGAGGGGAUCAUCAACUCG
GAGGACGAGCACCUGCUCGCCCUGGAGCGGAAGCUCAAGAAGAUGCUGG
GCCCGUCCGCGGUGGACAUCGGGAACGGCUGCUUCGAGACCAAGCACAA
GUGCAACCAGACGUGCCUCGACCGCAUCGCCGCGGGGACCUUCAACGCC
GGCGAGUUCAGCCUGCCCACGUUCGACUCGCUCAACAUCACCGCGGCCUC
CCUGAACGACGACGGGCUCGACAACCACACGAUCCUGCUCUACUACAGC

ACCGCGGCCUCGUCCCUGGCGGUCACGCUCAUGCUGGCCAUCUUCAUCG
UGUACAUGGUCAGCCGCGACAACGUGUCGUGCUCCAUCUGCCUCUGAUG
ACUCGAGGCUCGCUUUCUUGCUGUCCAAUUUCUAUUAAAGGUUCCUUUG
UUCCCUAAGUCCAACUACUAAACUGGGGGAUAUUAUGAAGGGCCUUGAG
CAUCUGGAUUCUGCCUAAUAAAAACAUUUAUUUUCAUUGCAAAAAAAA
AAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAA
AAAAAAAAAAAAAAAAAA

SEQ ID NO: 121

>I'-H1N1-HA0-mut-B-120A mRNA

GAGUUAGGAGUUGGUGGCAGUUUCAAUAACAGGUCAUUGCCGAGAAAA
GGAUAGCACUAUAAUAUGAAGGCCAUCCUGGUGGUGAUGCUGUACACCU
UCACCACCGCCAACGCCGACACCCUGUGCAUCGGCUACCACGCCAACAAC
AGCACCGACACCGUGGACACCGUGCUGGAGAAGAACGUGACCGUGACCC
ACAGCGUGAACCUGCUGGAGGACAAGCACAACGGCAAGCUGUGCAAGCU
GCGGGGCGUGGCCCCCUGCACCUGGGCAAGUGCAACAUCGCCGGCUGG
AUCCUGGGCAACCCCGAGUGCGAGUCCCUGAGCACCGCCCGCUCCUGGA
GCUACAUCGUGGAGACCUCCAACAGCGACAACGGCACCUGCUACCCCGG
GGACUUCAUCAACUACGAGGAGCUGCGGGAGCAGCUGUCCAGCGUGUCC
AGCUUCGAGCGCUUCGAGAUCUUCCCCAAGACCUCCAGCUGGCCCAACC
ACGACUCCGACAACGGCGUGACCGCCGCCUGCCCCCACGCCGGGGCCAAG
AGCUUCUACAAGAACCUGAUCUGGCUGGUGAAGAAGGGCAAGUCCUACC
CCAAGAUCAACCAGACCUACAUCAACGACAAGGGGAAGGAGGUCCUCGU
GCUGUGGGGCAUCCACCACCCCCCCACCAUCGCCGACCAGCAGAGCCUCU
ACCAGAACGCCGACGCCUACGUCUUCGUGGGGACCUCCCGGUACAGCAA
GAAGUUCAAGCCCGAGAUCGCCACCCGCCCCAAGGUCCGGGACCAGGAG
GGCCGCAUGAACUACUACUGGACCCUGGUGGAGCCGGGGGGACAAGAUCA
CCUUCGAGGCCACCGGCAACCUCGUCGCCCCGCGGUACGCCUUCACGAUG
GAGCGCGACGCCGGGUCCGGCAUCAUCAUCAGCGACACCCCCGUGCACG
ACUGCAACACGACCUGCCAGACGCCCGAGGGGGCGAUCAACACCUCCCU
GCCGUUCCAGAACGUCCACCCGAUCACGAUCGGCAAGUGCCCCAAGUAC
GUGAAGUCCACCAAGCUCCGGCUGGCCACGGGGCUCCGCAACGUCCCCUC
CAUCCAGAGCCAGGGCCUGUUCGGGGCGAUCGCCGGCUUCAUCGAGGGG
GGCUGGACCGGGAUGGUGGACGGCUGGUACGGGUACCACCACCAGAACG
AGCAGGGCUCCGGGUACGCGGCCGACCUCAAGUCGACGCAGAACGCGAU
CGACAAGAUCACCAACAAGGUCAACAGCGUGAUCGAGAAGAUGAACACG

CAGUUCACCGCCGUCGGCAAGGAGUUCAACCACCUGGAGAAGCGCAUCG
AGAACCUCAACAAGAAGGUGGACGACGGGUUCCUGGACAUCUGGACGUA
CAACGCGGAGCUCCUGGUCCUCCUGGAGAACGAGCGGACCCUCGACUAC
CACGACUCCAACGUGAAGAACCUGUACGAGAAGGUCCGCAACCAGCUCA
AGAACAACGCCAAGGAGAUCGGCAACGGGUGCUUCGAGUUCUACCACAA
GUGCGACAACACGUGCAUGGAGUCGGUGAAGAACGGCACCUACGACUAC
CCGAAGUACAGCGAGGAGGCGAAGCUGAACCGGGAGAAGAUCGACGGGG
UCAAGCUCGACUCCACGCGCAUCUACCAGAUCCUGGCCAUCUACUCGACC
GUGGCGAGCUCCCUCGUCCUGGUGGUCUCGCUCGGCGCCAUCAGCUUCU
GGAUGUGCUCCAACGGGUCGCUGCAGUGCCGGAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUUCAUUGCAAAAAAAAAAAA
AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAAAAAAAAAAA

SEQ ID NO: 122

>I'-H3N2-HA0-mut-B-120A mRNA

GAGUUAGGAGUUGGUGGCAGUUUCAAUAACAGGUCAUUGCCGAGAAAA
GGAUAGCACUAUAAUAUGAAGACCAUCAUCGCCCUGAGCUACAUCCUGU
GCCUGGUGUUCGCCCAGAAGAUCCCCGGCAACGACAACAGCACCGCCACC
CUGUGCCUGGGCCACCACGCCGUGCCCAACGGCACCAUCGUGAAGACCA
UCACCAACGACCGGAUCGAGGUGACCAACGCCACCGAGCUGGUGCAGAA
CUCCAGCAUCGGCGAGAUCUGCGACUCCCCCACCAGAUCCUGGACGGCG
GCAACUGCACCCUGAUCGACGCCCUGCUGGGCGACCCCAGUGCGACGG
GUUCCAGAACAAGGAGUGGGACCUGUUCGUGGAGCGGAGCCGCGCCAAC
UCCAACUGCUACCCCUACGACGUGCCCGACUACGCCAGCCUGCGGUCCCU
GGUGGCCAGCUCCGGCACCCUGGAGUUCAAGAACGAGAGCUUCAACUGG
ACCGGGGUCAAGCAGAACGGCACCUCCAGCGCCUGCAUCCGCGGGUCCA
GCUCCAGCUUCUUCUCCCGGCUGAACUGGCUCACCCACCUGAACUACACC
UACCCCGCCCUCAACGUGACCAUGCCCAACAACGAGCAGUUCGACAAGC
UGUACAUCUGGGGCGUCCACCACCCCAGCACCGACAAGGACCAGAUCUC
CCUCUUCGCCCAGCCCAGCGGGCGCAUCACCGUGUCCACGAAGCGGAGCC
AGCAGGCCGUCAUCCCCAACAUCGGCUCCCGCCCGCGGAUCCGCGACAUC
CCCAGCCGGAUCUCCAUCUACUGGACGAUCGUGAAGCCGGGGGACAUCC
UGCUCAUCAACAGCACCGGCAACCUGAUCGCCCCCGCGGGUACUUCAA

GAUCCGGUCCGGCAAGAGCUCGAUCAUGCGCUCCGACGCCCCGAUCGGG
AAGUGCAAGAGCGAGUGCAUCACCCCCAACGGCUCGAUCCCGAACGACA
AGCCCUUCCAGAACGUCAACCGGAUCACGUACGGGGCCUGCCCGCGCUA
CGUGAAGCAGUCCACGCUCAAGCUGGCCACCGGCAUGCGGAACGUCCCC
GAGAAGCAGACCCAGGGGAUCUUCGGCGCCAUCGCGGGGUUCAUCGAGA
ACGGCUGGGAGGGGAUGGUGGACGGCUGGUACGGGUUCCGGCACCAGAA
CAGCGAGGGCCGCGGGCAGGCCGCGGACCUCAAGUCGACGCAGGCCGCG
AUCGACCAGAUCAACGGCAAGCUGAACCGGCUCAUCGGGAAGACGAACG
AGAAGUUCCACCAGAUCGAGAAGGAGUUCUCCGAGGUCGAGGGCCGCGU
GCAGGACCUGGAGAAGUACGUCGAGGACACCAAGAUCGACCUCUGGAGC
UACAACGCCGAGCUGCUCGUGGCGCUGGAGAACCAGCACACCAUCGACC
UCACGGACUCGGAGAUGAACAAGCUGUUCGAGAAGACGAAGAAGCAGCU
CCGGGAGAACGCCGAGGACAUGGGGAACGGCUGCUUCAAGAUCUACCAC
AAGUGCGACAACGCGUGCAUCGGGUCCAUCCGCAACGAGACCUACGACC
ACAACGUCUACCGGGACGAGGCCCUGAACAACCGCUUCCAGAUCAAGGG
CGUGGAGCUCAAGAGCGGGUACAAGGACUGGAUCCUGUGGAUCUCGUUC
GCGAUGUCCUGCUUCCUCCUGUGCAUCGCCCUCCUGGGCUUCAUCAUGU
GGGCGUGCCAGAAGGGGAACAUCCGGUGCAACAUCUGCAUCUGAUGACU
CGAGGCUCGCUUUCUUGCUGUCCAAUUUCUAUUAAAGGUUCCUUUGUUC
CCUAAGUCCAACUACUAAACUGGGGGAUAUUAUGAAGGGCCUUGAGCAU
CUGGAUUCUGCCUAAUAAAAACAUUUAUUUCAUUGCAAAAAAAAAAAA
AAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAA
AAAAAAAAAAAAAA

SEQ ID NO: 123

>I'-B-Victoria-HA0-mut-B-120A mRNA

GAGUUAGGAGUUGGUGGCAGUUUCAAUAACAGGUCAUUGCCGAGAAAA
GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCCACUUCGCCAACCUGAAGGGC
ACCGAGACCCGGGGCAAGCUGUGCCCCAAGUGCCUGAACUGCACCGACC
UGGACGUGGCCCUGGGCCGCCCCAAGUGCACCGGCAAGAUCCCCAGCGCC
CGGGUGUCCAUCCUGCACGAGGUGCGCCCCGUGACCAGCGGCUGCUUCC
CCAUCAUGCACGACCGCACCAAGAUCCGGCAGCUGCCCAACCUGCUGCGG
GGGUACGAGCACGUCCGCCUGUCCACCCACAACGUGAUCAACGCCGAGG

ACGCCCCCGGCCGGCCCUACGAGAUCGGGACCAGCGGCUCCUGCCCCAAC
AUCACCAACGGGAACGGCUUCUUCGCCACCAUGGCCUGGGCCGUCCCCA
AGAACAAGACCGCCACCAACCCCCUGACCAUCGAGGUGCCGUACAUCUG
CACCGAGGGGGAGGACCAGAUCACCGUCUGGGGCUUCCACAGCGACAAC
GAGACGCAGAUGGCCAAGCUGUACGGGGACUCCAAGCCCCAGAAGUUCA
CGAGCUCCGCCAACGGCGUGACCACCCACUACGUCAGCCAGAUCGGGGG
CUUCCCGAACCAGACGGAGGACGGGGGCCUGCCCCAGUCCGGGCGCAUC
GUGGUCGACUACAUGGUGCAGAAGUCCGGCAAGACGGGGACCAUCACGU
ACCAGCGGGGCAUCCUGCUGCCGCAGAAGGUCUGGUGCGCCUCCGGGCG
CAGCAAGGUGAUCAAGGGCUCCCUGCCCCUCAUCGGGGAGGCCGACUGC
CUGCACGAGAAGUACGGCGGGCUCAACAAGAGCAAGCCGUACUACACCG
GCGAGCACGCCAAGGCCAUCGGGAACUGCCCCAUCUGGGUCAAGACCCC
GCUGAAGCUCGCCAACGGCACGAAGUACCGGCCCCCGGCCAAGCUGCUC
AAGGAGCAGGGGUUCUUCGGCGCCAUCGCCGGGUUCCUGGAGGGCGGGU
GGGAGGGCAUGAUCGCCGGGUGGCACGGCUACACGUCCCACGGGGCCCA
CGGCGUGGCGGUCGCCGCGGACCUCAAGAGCACCCAGGAGGCCAUCAAC
AAGAUCACGAAGAACCUGAACUCCCUCAGCGAGCUGGAGGUGAAGAACC
UCCAGCGCCUGUCCGGGGCGAUGGACGAGCUCCACAACGAGAUCCUGGA
GCUCGACGAGAAGGUCGACGACCUGCGGGCCGACACCAUCAGCUCGCAG
AUCGAGCUCGCGGUGCUGCUCUCCAACGAGGGCAUCAUCAACAGCGAGG
ACGAGCACCUGCUCGCCCUGGAGCGGAAGCUCAAGAAGAUGCUGGGGCC
CUCGGCGGUCGAGAUCGGCAACGGGUGCUUCGAGACCAAGCACAAGUGC
AACCAGACGUGCCUCGACCGCAUCGCCGCGGGCACGUUCGACGCCGGGG
AGUUCUCCCUGCCGACCUUCGACAGCCUCAACAUCACGGCGGCCUCGCUG
AACGACGACGGCCUCGACAACCACACCAUCCUGCUCUACUACUCCACCGC
GGCCAGCUCGCUGGCGGUGACGCUCAUGAUCGCCAUCUUCGUCGUGUAC
AUGGUCUCCCGCGACAACGUGAGCUGCUCGAUCUGCCUGUGAUGACUCG
AGGCUCGCUUUCUUGCUGUCCAAUUUCUAUUAAAGGUUCCUUUGUUCCC
UAAGUCCAACUACUAAACUGGGGGAUAUUAUGAAGGGCCUUGAGCAUCU
GGAUUCUGCCUAAUAAAAACAUUUAUUUUCAUUGCAAAAAAAAAAAA
AAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAA
AAAAAAAAAAAAA

SEQ ID NO: 124

>I'-B-Yamagata-HA0-mut-B-120A mRNA

GAGUUAGGAGUUGGUGGCAGUUUCAAUAACAGGUCAUUGCCGAGAAAA

GGAUAGCACUAUAAUAUGAAGGCCAUCAUCGUGCUGCUGAUGGUGGUGA
CCAGCAACGCCGACCGGAUCUGCACCGGCAUCACCAGCUCCAACAGCCCC
CACGUGGUGAAGACCGCCACCCAGGGCGAGGUGAACGUGACCGGCGUGA
UCCCCCUGACCACCACCCCCACCAAGUCCUACUUCGCCAACCUGAAGGGC
ACCCGGACCCGCGGCAAGCUGUGCCCCGACUGCCUGAACUGCACCGACCU
GGACGUGGCCCUGGGCCGGCCCAUGUGCGUGGGCACCACCCCCAGCGCCA
AGGCCUCCAUCCUGCACGAGGUGCGCCCCGUGACCAGCGGCUGCUUCCCC
AUCAUGCACGACCGCACCAAGAUCCGGCAGCUGCCCAACCUGCUGCGGG
GGUACGAGAAGAUCCGCCUGUCCACCCAGAACGUCAUCGACGCCGAGAA
GGCCCCCGGCGGGCCCUACCGGCUGGGCACCAGCGGGUCCUGCCCCAACG
CCACCAGCAAGAUCGGCUUCUUCGCCACCAUGGCCUGGGCCGUGCCCAA
GGACAACUACAAGAACGCCACCAACCCCCUGACCGUCGAGGUGCCCUAC
AUCUGCACCGAGGGGGAGGACCAGAUCACGGUCUGGGGCUUCCACUCCG
ACAACAAGACCCAGAUGAAGUCCCUGUACGGGGACUCCAACCCGCAGAA
GUUCACGAGCUCCGCCAACGGCGUGACCACGCACUACGUCAGCCAGAUC
GGGGACUUCCCGGACCAGACCGAGGACGGCGGGCUGCCCCAGUCCGGCC
GCAUCGUGGUCGACUACAUGAUGCAGAAGCCCGGGAAGACGGGCACCAU
CGUGUACCAGCGGGGGGUCCUGCUGCCGCAGAAGGUGUGGUGCGCCAGC
GGCCGCUCCAAGGUCAUCAAGGGGAGCCUGCCCCUCAUCGGCGAGGCCG
ACUGCCUGCACGAGGAGUACGGGGGCCUCAACAAGUCCAAGCCGUACUA
CACGGGGAAGCACGCCAAGGCCAUCGGCAACUGCCCGAUCUGGGUGAAG
ACCCCCUGAAGCUCGCCAACGGGACGAAGUACCGGCCGCCCGCCAAGCU
GCUCAAGGAGCAGGGCUUCUUCGGGGCCAUCGCCGGCUUCCUGGAGGGG
GGCUGGGAGGGGAUGAUCGCCGGCUGGCACGGGUACACCAGCCACGGCG
CCCACGGGGUCGCGGUGGCCGCGGACCUCAAGUCCACGCAGGAGGCCAU
CAACAAGAUCACCAAGAACCUGAACAGCCUCUCCGAGCUGGAGGUCAAG
AACCUCCAGCGCCUGAGCGGCGCGAUGGACGAGCUCCACAACGAGAUCC
UGGAGCUCGACGAGAAGGUGGACGACCUGCGGGCCGACACGAUCUCGUC
CCAGAUCGAGCUCGCGGUCCUGCUCAGCAACGAGGGGAUCAUCAACUCG
GAGGACGAGCACCUGCUCGCCCUGGAGCGGAAGCUCAAGAAGAUGCUGG
GCCCGUCCGCGGUGGACAUCGGGAACGGCUGCUUCGAGACCAAGCACAA
GUGCAACCAGACGUGCCUCGACCGCAUCGCCGCGGGGACCUUCAACGCC
GGCGAGUUCAGCCUGCCCACGUUCGACUCGCUCAACAUCACCGCGGCCUC
CCUGAACGACGACGGGCUCGACAACCACACGAUCCUGCUCUACUACAGC
ACCGCGGCCUCGUCCCUGGCGGUCACGCUCAUGCUGGCCAUCUUCAUCG
UGUACAUGGUCAGCCGCGACAACGUGUCGUGCUCCAUCUGCCUCUGAUG

ACUCGAGGCUCGCUUUCUUGCUGUCCAAUUUCUAUUAAAGGUUCCUUUG
UUCCCUAAGUCCAACUACUAAACUGGGGGAUAUUAUGAAGGGCCUUGAG
CAUCUGGAUUCUGCCUAAUAAAAACAUUUAUUUUCAUUGCAAAAAAAA
AAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAA
AAAAAAAAAAAAAAAAAA

SEQ ID NO: 125

>Influenza A virus H1N1 (A/Wisconsin/588/2019) HA mRNA

AUGAAGGCCAUCCUGGUGGUGAUGCUGUACACCUUCACCACCGCCAACG
CCGACACCCUGUGCAUCGGCUACCACGCCAACAACAGCACCGACACCGUG
GACACCGUGCUGGAGAAGAACGUGACCGUGACCCACAGCGUGAACCUGC
UGGAGGACAAGCACAACGGCAAGCUGUGCAAGCUGCGGGGCGUGGCCCC
CCUGCACCUGGGCAAGUGCAACAUCGCCGGCUGGAUCCUGGGCAACCCC
GAGUGCGAGUCCCUGAGCACCGCCCGCUCCUGGAGCUACAUCGUGGAGA
CCUCCAACAGCGACAACGGCACCUGCUACCCCGGGGACUUCAUCAACUAC
GAGGAGCUGCGGGAGCAGCUGUCCAGCGUGUCCAGCUUCGAGCGCUUCG
AGAUCUUCCCCAAGACCUCCAGCUGGCCCAACCACGACUCCGACAACGGC
GUGACCGCCGCCUGCCCCCACGCCGGGGCCAAGAGCUUCUACAAGAACCU
GAUCUGGCUGGUGAAGAAGGGCAAGUCCUACCCCAAGAUCAACCAGACC
UACAUCAACGACAAGGGGAAGGAGGUCCUCGUGCUGUGGGGCAUCCACC
ACCCCCCCACCAUCGCCGACCAGCAGAGCCUCUACCAGAACGCCGACGCC
UACGUCUUCGUGGGGACCUCCCGGUACAGCAAGAAGUUCAAGCCCGAGA
UCGCCACCCGCCCCAAGGUCCGGGACCAGGAGGGCCGCAUGAACUACUA
CUGGACCCUGGUGGAGCCGGGGGACAAGAUCACCUUCGAGGCCACCGGC
AACCUCGUCGCCCCGCGGUACGCCUUCACGAUGGAGCGCGACGCCGGGU
CCGGCAUCAUCAUCAGCGACACCCCCGUGCACGACUGCAACACGACCUGC
CAGACGCCCGAGGGGGCGAUCAACACCUCCCUGCCGUUCCAGAACGUCC
ACCCGAUCACGAUCGGCAAGUGCCCCAAGUACGUGAAGUCCACCAAGCU
CCGGCUGGCCACGGGGCUCCGCAACGUCCCCUCCAUCCAGAGCCGGGGCC
UGUUCGGGGCGAUCGCCGGCUUCAUCGAGGGGGGCUGGACCGGGAUGGU
GGACGGCUGGUACGGGUACCACCACCAGAACGAGCAGGGCUCCGGGUAC
GCGGCCGACCUCAAGUCGACGCAGAACGCGAUCGACAAGAUCACCAACA
AGGUCAACAGCGUGAUCGAGAAGAUGAACACGCAGUUCACCGCCGUCGG
CAAGGAGUUCAACCACCUGGAGAAGCGCAUCGAGAACCUCAACAAGAAG
GUGGACGACGGGUUCCUGGACAUCUGGACGUACAACGCGGAGCUCCUGG
UCCUCCUGGAGAACGAGCGGACCCUCGACUACCACGACUCCAACGUGAA

GAACCUGUACGAGAAGGUCCGCAACCAGCUCAAGAACAACGCCAAGGAG
AUCGGCAACGGGUGCUUCGAGUUCUACCACAAGUGCGACAACACGUGCA
UGGAGUCGGUGAAGAACGGCACCUACGACUACCCGAAGUACAGCGAGGA
GGCGAAGCUGAACCGGGAGAAGAUCGACGGGGUCAAGCUCGACUCCACG
CGCAUCUACCAGAUCCUGGCCAUCUACUCGACCGUGGCGAGCUCCCUCG
UCCUGGUGGUCUCGCUCGGCGCCAUCAGCUUCUGGAUGUGCUCCAACGG
GUCGCUGCAGUGCCGGAUCUGCAUCUGA

<div align="right">SEQ ID NO: 126</div>

>Influenza A virus H3N2 (A/Cambodia/e0826360/2020) HA mRNA

AUGAAGACCAUCAUCGCCCUGAGCUACAUCCUGUGCCUGGUGUUCGCCC
AGAAGAUCCCCGGCAACGACAACAGCACCGCCACCCUGUGCCUGGGCCAC
CACGCCGUGCCCAACGGCACCAUCGUGAAGACCAUCACCAACGACCGGA
UCGAGGUGACCAACGCCACCGAGCUGGUGCAGAACUCCAGCAUCGGCGA
GAUCUGCGACUCCCCCACCAGAUCCUGGACGGCGGCAACUGCACCCUGA
UCGACGCCCUGCUGGGCGACCCCCAGUGCGACGGGUUCCAGAACAAGGA
GUGGGACCUGUUCGUGGAGCGGAGCCGCGCCAACUCCAACUGCUACCCC
UACGACGUGCCCGACUACGCCAGCCUGCGGUCCCUGGUGGCCAGCUCCG
GCACCCUGGAGUUCAAGAACGAGAGCUUCAACUGGACCGGGGUCAAGCA
GAACGGCACCUCCAGCGCCUGCAUCCGCGGGUCCAGCUCCAGCUUCUUCU
CCCGGCUGAACUGGCUCACCCACCUGAACUACACCUACCCCGCCCUCAAC
GUGACCAUGCCCAACAACGAGCAGUUCGACAAGCUGUACAUCUGGGGCG
UCCACCACCCCAGCACCGACAAGGACCAGAUCUCCCUCUUCGCCCAGCCC
AGCGGGCGCAUCACCGUGUCCACGAAGCGGAGCCAGCAGGCCGUCAUCC
CCAACAUCGGCUCCCGCCCGCGGAUCCGCGACAUCCCCAGCCGGAUCUCC
AUCUACUGGACGAUCGUGAAGCCGGGGGACAUCCUGCUCAUCAACAGCA
CCGGCAACCUGAUCGCCCCCGCGGGUACUUCAAGAUCCGGUCCGGCAA
GAGCUCGAUCAUGCGCUCCGACGCCCCGAUCGGGAAGUGCAAGAGCGAG
UGCAUCACCCCCAACGGCUCGAUCCCGAACGACAAGCCCUUCCAGAACGU
CAACCGGAUCACGUACGGGGCCUGCCCGCGCUACGUGAAGCAGUCCACG
CUCAAGCUGGCCACCGGCAUGCGGAACGUCCCCGAGAAGCAGACCCGCG
GGAUCUUCGGCGCCAUCGCGGGGUUCAUCGAGAACGGCUGGGAGGGGAU
GGUGGACGGCUGGUACGGGUUCCGGCACCAGAACAGCGAGGGCCGCGGG
CAGGCCGCGGACCUCAAGUCGACGCAGGCCGCGAUCGACCAGAUCAACG
GCAAGCUGAACCGGCUCAUCGGGAAGACGAACGAGAAGUUCCACCAGAU
CGAGAAGGAGUUCUCCGAGGUCGAGGGCCGCGUGCAGGACCUGGAGAAG
UACGUCGAGGACACCAAGAUCGACCUCUGGAGCUACAACGCCGAGCUGC

UCGUGGCGCUGGAGAACCAGCACACCAUCGACCUCACGGACUCGGAGAU
GAACAAGCUGUUCGAGAAGACGAAGAAGCAGCUCCGGGAGAACGCCGAG
GACAUGGGGAACGGCUGCUUCAAGAUCUACCACAAGUGCGACAACGCGU
GCAUCGGGUCCAUCCGCAACGAGACCUACGACCACAACGUCUACCGGGA
CGAGGCCCUGAACAACCGCUUCCAGAUCAAGGGCGUGGAGCUCAAGAGC
GGGUACAAGGACUGGAUCCUGUGGAUCUCGUUCGCGAUGUCCUGCUUCC
UCCUGUGCAUCGCCCUCCUGGGCUUCAUCAUGUGGGCGUGCCAGAAGGG
GAACAUCCGGUGCAACAUCUGCAUCUGA

SEQ ID NO: 127

>Influenza B virus Victoria lineage (B/Washington/02/2019) HA mRNA

AUGAAGGCCAUCAUCGUGCUGCUGAUGGUGGUGACCAGCAACGCCGACC
GGAUCUGCACCGGCAUCACCAGCUCCAACAGCCCCCACGUGGUGAAGAC
CGCCACCCAGGGCGAGGUGAACGUGACCGGCGUGAUCCCCUGACCACC
ACCCCCACCAAGUCCCACUUCGCCAACCUGAAGGGCACCGAGACCCGGGG
CAAGCUGUGCCCCAAGUGCCUGAACUGCACCGACCUGGACGUGGCCCUG
GGCCGCCCCAAGUGCACCGGCAAGAUCCCCAGCGCCCGGGUGUCCAUCCU
GCACGAGGUGCGCCCCGUGACCAGCGGCUGCUUCCCCAUCAUGCACGACC
GCACCAAGAUCCGGCAGCUGCCCAACCUGCUGCGGGGGUACGAGCACGU
CCGCCUGUCCACCCACAACGUGAUCAACGCCGAGGACGCCCCCGGCCGGC
CCUACGAGAUCGGGACCAGCGGCUCCUGCCCCAACAUCACCAACGGGAA
CGGCUUCUUCGCCACCAUGGCCUGGGCCGUCCCCAAGAACAAGACCGCCA
CCAACCCCCUGACCAUCGAGGUGCCGUACAUCUGCACCGAGGGGGAGGA
CCAGAUCACCGUCUGGGGCUUCCACAGCGACAACGAGACGCAGAUGGCC
AAGCUGUACGGGGACUCCAAGCCCCAGAAGUUCACGAGCUCCGCCAACG
GCGUGACCACCCACUACGUCAGCCAGAUCGGGGGGCUUCCCGAACCAGAC
GGAGGACGGGGGCCUGCCCCAGUCCGGGCGCAUCGUGGUCGACUACAUG
GUGCAGAAGUCCGGCAAGACGGGGACCAUCACGUACCAGCGGGGCAUCC
UGCUGCCGCAGAAGGUCUGGUGCGCCUCCGGGCGCAGCAAGGUGAUCAA
GGGCUCCCUGCCCCUCAUCGGGGAGGCCGACUGCCUGCACGAGAAGUAC
GGCGGGCUCAACAAGAGCAAGCCGUACUACACCGGCGAGCACGCCAAGG
CCAUCGGGAACUGCCCCAUCUGGGUCAAGACCCCGCUGAAGCUCGCCAA
CGGCACGAAGUACCGGCCCCCGGCCAAGCUGCUCAAGGAGCGCGGGUUC
UUCGGCGCCAUCGCCGGGUUCCUGGAGGGCGGGUGGGAGGGCAUGAUCG
CCGGGUGGCACGGCUACACGUCCCACGGGGCCCACGGCGUGGCGGUCGC
CGCGGACCUCAAGAGCACCCAGGAGGCCAUCAACAAGAUCACGAAGAAC
CUGAACUCCCUCAGCGAGCUGGAGGUGAAGAACCUCCAGCGCCUGUCCG

GGGCGAUGGACGAGCUCCACAACGAGAUCCUGGAGCUCGACGAGAAGGU
CGACGACCUGCGGGCCGACACCAUCAGCUCGCAGAUCGAGCUCGCGGUG
CUGCUCUCCAACGAGGGCAUCAUCAACAGCGAGGACGAGCACCUGCUCG
CCCUGGAGCGGAAGCUCAAGAAGAUGCUGGGGCCCUCGGCGGUCGAGAU
CGGCAACGGGUGCUUCGAGACCAAGCACAAGUGCAACCAGACGUGCCUC
GACCGCAUCGCCGCGGGCACGUUCGACGCCGGGGAGUUCUCCCUGCCGA
CCUUCGACAGCCUCAACAUCACGGCGGCCUCGCUGAACGACGACGGCCUC
GACAACCACACCAUCCUGCUCUACUACUCCACCGCGGCCAGCUCGCUGGC
GGUGACGCUCAUGAUCGCCAUCUUCGUCGUGUACAUGGUCUCCCGCGAC
AACGUGAGCUGCUCGAUCUGCCUGUGA

SEQ ID NO: 128

>Influenza B virus /Yamagata lineage (B/Phuket/3073/2013) HA mRNA

AUGAAGGCCAUCAUCGUGCUGCUGAUGGUGGUGACCAGCAACGCCGACC
GGAUCUGCACCGGCAUCACCAGCUCCAACAGCCCCCACGUGGUGAAGAC
CGCCACCCAGGGCGAGGUGAACGUGACCGGCGUGAUCCCCCUGACCACC
ACCCCCACCAAGUCCUACUUCGCCAACCUGAAGGGCACCCGGACCCGCGG
CAAGCUGUGCCCCGACUGCCUGAACUGCACCGACCUGGACGUGGCCCUG
GGCCGGCCCAUGUGCGUGGGCACCACCCCCAGCGCCAAGGCCUCCAUCCU
GCACGAGGUGCGCCCCGUGACCAGCGGCUGCUUCCCCAUCAUGCACGACC
GCACCAAGAUCCGGCAGCUGCCCAACCUGCUGCGGGGGUACGAGAAGAU
CCGCCUGUCCACCCAGAACGUCAUCGACGCCGAGAAGGCCCCCGGCGGGC
CCUACCGGCUGGGCACCAGCGGGUCCUGCCCCAACGCCACCAGCAAGAUC
GGCUUCUUCGCCACCAUGGCCUGGGCCGUGCCCAAGGACAACUACAAGA
ACGCCACCAACCCCCUGACCGUCGAGGUGCCCUACAUCUGCACCGAGGGG
GAGGACCAGAUCACGGUCUGGGGCUUCCACUCCGACAACAAGACCCAGA
UGAAGUCCCUGUACGGGGACUCCAACCCGCAGAAGUUCACGAGCUCCGC
CAACGGCGUGACCACGCACUACGUCAGCCAGAUCGGGGACUUCCCGGAC
CAGACCGAGGACGGCGGGCUGCCCCAGUCCGGCCGCAUCGUGGUCGACU
ACAUGAUGCAGAAGCCCGGGAAGACGGGCACCAUCGUGUACCAGCGGGG
GGUCCUGCUGCCGCAGAAGGUGUGGUGCGCCAGCGGCCGCUCCAAGGUC
AUCAAGGGGAGCCUGCCCCUCAUCGGCGAGGCCGACUGCCUGCACGAGG
AGUACGGGGGCCUCAACAAGUCCAAGCCGUACUACACGGGGAAGCACGC
CAAGGCCAUCGGCAACUGCCCGAUCUGGGUGAAGACCCCCUGAAGCUC
GCCAACGGGACGAAGUACCGGCCGCCCGCCAAGCUGCUCAAGGAGCGCG
GCUUCUUCGGGGCCAUCGCCGGCUUCCUGGAGGGGGGCUGGGAGGGGAU
GAUCGCCGGCUGGCACGGGUACACCAGCCACGGCGCCCACGGGGUCGCG

GUGGCCGCGGACCUCAAGUCCACGCAGGAGGCCAUCAACAAGAUCACCA
AGAACCUGAACAGCCUCUCCGAGCUGGAGGUCAAGAACCUCCAGCGCCU
GAGCGGCGCGAUGGACGAGCUCCACAACGAGAUCCUGGAGCUCGACGAG
AAGGUGGACGACCUGCGGGCCGACACGAUCUCGUCCCAGAUCGAGCUCG
CGGUCCUGCUCAGCAACGAGGGGAUCAUCAACUCGGAGGACGAGCACCU
GCUCGCCCUGGAGCGGAAGCUCAAGAAGAUGCUGGGCCCGUCCGCGGUG
GACAUCGGGAACGGCUGCUUCGAGACCAAGCACAAGUGCAACCAGACGU
GCCUCGACCGCAUCGCCGCGGGGACCUUCAACGCCGGCGAGUUCAGCCU
GCCCACGUUCGACUCGCUCAACAUCACCGCGGCCUCCCUGAACGACGACG
GGCUCGACAACCACACGAUCCUGCUCUACUACAGCACCGCGGCCUCGUCC
CUGGCGGUCACGCUCAUGCUGGCCAUCUUCAUCGUGUACAUGGUCAGCC
GCGACAACGUGUCGUGCUCCAUCUGCCUCUGA

SEQ ID NO: 129

>SARS-CoV-2 BA.4 spike protein mRNA

AUGUUCGUGUUCCUGGUGCUGCUGCCCCUGGUGAGCUCUCAGUGCGUGA
ACCUGAUCACAAGAACACAGUCGUACACCAACAGCUUCACAAGAGGCGU
GUACUACCCCGACAAGGUGUUCAGAAGCAGCGUGCUGCACUCCACCCAA
GACCUGUUUCUGCCCUUCUUCAGCAACGUGACCUGGUUCCACGCCAUCA
GCGGCACCAACGGCACCAAGAGAUUCGACAACCCCGUGCUGCCCUUCAA
CGACGGCGUGUACUUCGCUAGCACCGAAAAGAGCAACAUCAUCAGAGGC
UGGAUCUUCGGCACCACCCUAGACUCCAAGACACAAAGCCUGCUGAUCG
UUAACAACGCGACCAACGUGGUGAUCAAGGUGUGCGAGUUUCAGUUCUG
CAACGACCCCUUCCUGGACGUGUACUACCACAAGAACAACAAGAGCUGG
AUGGAGAGCGAGUUCAGAGUCUACAGCAGCGCCAACAACUGCACCUUCG
AGUACGUGUCUCAGCCCUUCCUGAUGGACCUGGAGGGCAAGCAAGGCAA
UUUCAAGAACCUAAGAGAGUUCGUGUUCAAGAACAUCGACGGCUACUUC
AAGAUCUACAGCAAGCACACCCCCAUCAACCUGGGGAGAGACCUGCCCC
AAGGCUUCAGCGCCCUGGAGCCCCUGGUGGACCUGCCCAUCGGCAUCAA
CAUCACAAGAUUUCAGACCCUGCUGGCCCUGCACCGUAGCUACCUAACC
CCCGGCGAUAGUUCCUCUGGCUGGACGGCCGGCGCUGCCGCCUACUACG
UGGGCUACCUGCAGCCUAGAACCUUCCUGCUGAAGUACAACGAGAACGG
CACUAUCACUGAUGCCGUGGACUGUGCCCUGGACCCCCUGAGCGAGACC
AAGUGCACCCUCAAGAGCUUCACCGUGGAGAAGGGCAUCUAUCAGACAA
GCAACUUCAGAGUGCAGCCCACCGAGAGCAUCGUGAGAUUCCCCAACAU
CACCAACCUGUGCCCCUUCGACGAGGUGUUCAACGCCACAAGAUUCGCU

AGCGUGUACGCGUGGAACAGAAAGAGAAUCAGCAACUGCGUGGCCGACU
ACAGCGUGCUGUACAACUUCGCUCCCUUCUUCGCCUUCAAGUGCUAUGG
CGUCAGCCCCACCAAGCUGAACGACCUGUGCUUCACCAACGUGUACGCC
GACAGCUUCGUGAUCAGAGGCAACGAGGUGAGCCAGAUUGCCCCCGGGC
AAACCGGCAACAUCGCCGACUACAACUACAAGCUGCCCGACGACUUCAC
CGGCUGCGUGAUCGCGUGGAACAGCAAUAAGCUGGAUUCCAAGGUGGGC
GGCAACUACAACUACCGGUACAGACUGUUCAGAAAGAGCAACCUGAAGC
CCUUCGAGAGAGACAUCAGCACCGAGAUCUACCAAGCCGGCAACAAGCC
CUGCAACGGCGUGGCGGGCGUCAACUGCUACUUCCCCCUGCAGAGCUAC
GGCUUUAGGCCCACCUACGGCGUGGGCCAUCAGCCCUACAGAGUGGUGG
UGCUGAGCUUCGAGCUGCUGCACGCCCCCGCAACCGUGUGCGGCCCCAA
AAAGAGCACCAACCUGGUGAAGAACAAGUGCGUGAACUUCAACUUUAAU
GGCCUUAAGGGCACGGGCGUGCUGACCGAGAGCAACAAGAAGUUCCUGC
CAUUUCAGCAAUUCGGCAGAGACAUCGCCGACACCACCGACGCGGUCAG
AGACCCUCAGACCCUGGAGAUCCUGGACAUCACCCCCUGUAGCUUCGGC
GGCGUGAGCGUGAUCACCCCCGGCACUAACACAAGCAACCAAGUGGCCG
UGCUGUACCAAGGCGUGAACUGCACCGAGGUGCCCGUGGCCAUCCACGC
CGAUCAGCUGACCCCCACCUGGAGAGUAUACAGCACCGGAAGCAACGUG
UUUCAGACAAGAGCCGGCUGCCUGAUCGGCGCCGAGUACGUGAACAACA
GCUACGAGUGCGACAUCCCCAUCGGCGCCGGCAUCUGCGCUAGCUAUCA
GACACAGACCAAGAGCCACCGGAGAGCUAGAAGCGUGGCUUCUCAGAGC
AUCAUCGCCUACACCAUGAGCCUGGGCGCCGAGAACAGCGUGGCCUACA
GCAACAACAGCAUCGCCAUCCCCACCAACUUCACCAUCAGCGUGACCACC
GAGAUCCUGCCCGUAAGUAUGACCAAGACAAGCGUGGACUGCACCAUGU
ACAUCUGCGGCGACAGCACCGAGUGCAGCAACCUGCUGCUGCAGUACGG
CAGCUUCUGCACACAGCUGAAGAGAGCCCUGACCGGCAUCGCCGUGGAG
CAAGACAAGAACACCCAAGAGGUGUUCGCCCAAGUGAAGCAGAUCUACA
AGACCCCCCCCAUCAAGUACUUCGGCGGCUUCAACUUCAGCCAAAUCCU
GCCCGACCCUAGCAAGCCUAGCAAGCGGAGCUUCAUCGAGGACCUGCUG
UUCAACAAGGUGACCCUGGCCGACGCCGGCUUCAUCAAGCAGUACGGCG
ACUGCCUAGGCGACAUCGCAGCUAGAGACCUGAUCUGCGCUCAGAAGUU
CAAUGGACUCACCGUGCUCCCCCCCUGCUGACCGACGAGAUGAUCGCUC
AGUACACCUCUGCCCUGCUGGCGGGAACCAUAACAAGCGGUUGGACAUU
UGGCGCCGGGGCCGCACUGCAAAUCCCCUUCGCCAUGCAGAUGGCCUAC
AGAUUCAACGGCAUCGGCGUGACACAGAACGUGCUGUACGAGAAUCAGA
AGCUGAUCGCCAAUCAGUUCAACAGCGCCAUCGGCAAGAUCCAAGACAG

CCUGAGCAGCACCGCUAGCGCCCUGGGCAAGCUGCAAGACGUGGUGAAU
CACAACGCCCAAGCCCUGAACACCCUGGUGAAGCAGCUGAGCAGCAAGU
UCGGCGCCAUCAGCAGCGUAUUGAAUGACAUCCUGUCUAGACUGGACCC
CCCCGAGGCCGAGGUGCAGAUCGACAGACUGAUCACCGGCAGACUGCAG
AGCCUGCAGACCUACGUGACACAGCAGCUGAUCAGAGCCGCCGAGAUCA
GAGCUAGCGCCAACCUGGCCGCCACCAAGAUGAGCGAGUGCGUGCUGGG
GCAGAGCAAGAGAGUGGACUUCUGCGGCAAGGGCUACCACCUGAUGAGC
UUCCCUCAGAGCGCCCCCACGGCGUGGUGUUCCUGCACGUGACCUACG
UGCCCGCCCAAGAGAAGAACUUCACCACCGCUCCCGCCAUCUGCCACGAC
GGCAAGGCCCACUUCCCUAGAGAGGGCGUGUUCGUGAGCAACGGCACCC
ACUGGUUCGUGACACAGAGAAACUUCUACGAGCCUCAGAUCAUCACCAC
CGACAACACCUUCGUGAGCGGCAACUGCGACGUGGUGAUCGGCAUAGUG
AACAACACCGUAUACGACCCCCUGCAGCCCGAGCUGGACAGCUUCAAGG
AGGAGCUGGACAAGUACUUCAAAAACCAUACUAGCCCCGACGUGGACCU
GGGAGACAUCUCUGGCAUCAACGCUAGCGUGGUGAACAUUCAGAAGGAG
AUCGACCGGCUGAAUGAGGUGGCCAAGAACCUGAACGAGAGCCUGAUCG
ACCUGCAAGAGCUGGGCAAGUACGAGCAGUACAUCAAGUGGCCCUGGUA
CAUCUGGCUGGGCUUCAUCGCCGGCCUGAUCGCCAUCGUGAUGGUGACC
AUCAUGCUGUGCUGCAUGACAAGCUGUUGCAGCUGCCUGAAAGGUUGCU
GCAGCUGUGGCUCGUGCUGCAAGUUCGACGAGGACGACAGCGAGCCCGU
GCUGAAGGGCGUGAAGCUGCACUACACCUGAUGA

<div align="right">SEQ ID NO: 130</div>

> Influenza A virus H1N1 HA0 mutant mRNA

AUGAAGGCCAUCCUGGUGGUGAUGCUGUACACCUUCACCACCGCCAACG
CCGACACCCUGUGCAUCGGCUACCACGCCAACAACAGCACCGACACCGUG
GACACCGUGCUGGAGAAGAACGUGACCGUGACCCACAGCGUGAACCUGC
UGGAGGACAAGCACAACGGCAAGCUGUGCAAGCUGCGGGGCGUGGCCCC
CCUGCACCUGGGCAAGUGCAACAUCGCCGGCUGGAUCCUGGGCAACCCC
GAGUGCGAGUCCCUGAGCACCGCCCGCUCCUGGAGCUACAUCGUGGAGA
CCUCCAACAGCGACAACGGCACCUGCUACCCCGGGGACUUCAUCAACUAC
GAGGAGCUGCGGGAGCAGCUGUCCAGCGUGUCCAGCUUCGAGCGCUUCG
AGAUCUUCCCCAAGACCUCCAGCUGGCCCAACCACGACUCCGACAACGGC
GUGACCGCCGCCUGCCCCCACGCCGGGGCCAAGAGCUUCUACAAGAACCU
GAUCUGGCUGGUGAAGAAGGGCAAGUCCUACCCCAAGAUCAACCAGACC
UACAUCAACGACAAGGGGAAGGAGGUCCUCGUGCUGUGGGGCAUCCACC
ACCCCCCCACCAUCGCCGACCAGCAGAGCCUCUACCAGAACGCCGACGCC

UACGUCUUCGUGGGGACCUCCCGGUACAGCAAGAAGUUCAAGCCCGAGA
UCGCCACCCGCCCCAAGGUCCGGGACCAGGAGGGCCGCAUGAACUACUA
CUGGACCCUGGUGGAGCCGGGGGACAAGAUCACCUUCGAGGCCACCGGC
AACCUCGUCGCCCCGCGGUACGCCUUCACGAUGGAGCGCGACGCCGGGU
CCGGCAUCAUCAUCAGCGACACCCCCGUGCACGACUGCAACACGACCUGC
CAGACGCCCGAGGGGGCGAUCAACACCUCCCUGCCGUUCCAGAACGUCC
ACCCGAUCACGAUCGGCAAGUGCCCCAAGUACGUGAAGUCCACCAAGCU
CCGGCUGGCCACGGGGCUCCGCAACGUCCCCUCCAUCCAGAGCCAGGGCC
UGUUCGGGGCGAUCGCCGGCUUCAUCGAGGGGGGCUGGACCGGGAUGGU
GGACGGCUGGUACGGGUACCACCACCAGAACGAGCAGGGCUCCGGGUAC
GCGGCCGACCUCAAGUCGACGCAGAACGCGAUCGACAAGAUCACCAACA
AGGUCAACAGCGUGAUCGAGAAGAUGAACACGCAGUUCACCGCCGUCGG
CAAGGAGUUCAACCACCUGGAGAAGCGCAUCGAGAACCUCAACAAGAAG
GUGGACGACGGGUUCCUGGACAUCUGGACGUACAACGCGGAGCUCCUGG
UCCUCCUGGAGAACGAGCGGACCCUCGACUACCACGACUCCAACGUGAA
GAACCUGUACGAGAAGGUCCGCAACCAGCUCAAGAACAACGCCAAGGAG
AUCGGCAACGGGUGCUUCGAGUUCUACCACAAGUGCGACAACACGUGCA
UGGAGUCGGUGAAGAACGGCACCUACGACUACCCGAAGUACAGCGAGGA
GGCGAAGCUGAACCGGGAGAAGAUCGACGGGGUCAAGCUCGACUCCACG
CGCAUCUACCAGAUCCUGGCCAUCUACUCGACCGUGGCGAGCUCCCUCG
UCCUGGUGGUCUCGCUCGGCGCCAUCAGCUUCUGGAUGUGCUCCAACGG
GUCGCUGCAGUGCCGGAUCUGCAUCUGA

SEQ ID NO: 131

>Influenza A virus H3N2 HA0 mutant mRNA

AUGAAGACCAUCAUCGCCCUGAGCUACAUCCUGUGCCUGGUGUUCGCCC
AGAAGAUCCCCGGCAACGACAACAGCACCGCCACCCUGUGCCUGGGCCAC
CACGCCGUGCCCAACGGCACCAUCGUGAAGACCAUCACCAACGACCGGA
UCGAGGUGACCAACGCCACCGAGCUGGUGCAGAACUCCAGCAUCGGCGA
GAUCUGCGACUCCCCCACCAGAUCCUGGACGGCGGCAACUGCACCCUGA
UCGACGCCCUGCUGGGCGACCCCAGUGCGACGGGUUCCAGAACAAGGA
GUGGGACCUGUUCGUGGAGCGGAGCCGCGCCAACUCCAACUGCUACCCC
UACGACGUGCCCGACUACGCCAGCCUGCGGUCCCUGGUGGCCAGCUCCG
GCACCCUGGAGUUCAAGAACGAGAGCUUCAACUGGACCGGGGUCAAGCA
GAACGGCACCUCCAGCGCCUGCAUCCGCGGGUCCAGCUCCAGCUUCUUCU
CCCGGCUGAACUGGCUCACCCACCUGAACUACACCUACCCCGCCCUCAAC
GUGACCAUGCCCAACAACGAGCAGUUCGACAAGCUGUACAUCUGGGGCG

UCCACCACCCCAGCACCGACAAGGACCAGAUCUCCCUCUUCGCCCAGCCC
AGCGGGCGCAUCACCGUGUCCACGAAGCGGAGCCAGCAGGCCGUCAUCC
CCAACAUCGGCUCCCGCCCGCGGAUCCGCGACAUCCCCAGCCGGAUCUCC
AUCUACUGGACGAUCGUGAAGCCGGGGGACAUCCUGCUCAUCAACAGCA
CCGGCAACCUGAUCGCCCCCGCGGGUACUUCAAGAUCCGGUCCGGCAA
GAGCUCGAUCAUGCGCUCCGACGCCCCGAUCGGGAAGUGCAAGAGCGAG
UGCAUCACCCCCAACGGCUCGAUCCCGAACGACAAGCCCUUCCAGAACGU
CAACCGGAUCACGUACGGGGCCUGCCCGCGCUACGUGAAGCAGUCCACG
CUCAAGCUGGCCACCGGCAUGCGGAACGUCCCCGAGAAGCAGACCCAGG
GGAUCUUCGGCGCCAUCGCGGGGUUCAUCGAGAACGGCUGGGAGGGGAU
GGUGGACGGCUGGUACGGGUUCCGGCACCAGAACAGCGAGGGCCGCGGG
CAGGCCGCGGACCUCAAGUCGACGCAGGCCGCGAUCGACCAGAUCAACG
GCAAGCUGAACCGGCUCAUCGGGAAGACGAACGAGAAGUUCCACCAGAU
CGAGAAGGAGUUCUCCGAGGUCGAGGGCCGCGUGCAGGACCUGGAGAAG
UACGUCGAGGACACCAAGAUCGACCUCGGAGCUACAACGCCGAGCUGC
UCGUGGCGCUGGAGAACCAGCACACCAUCGACCUCACGGACUCGGAGAU
GAACAAGCUGUUCGAGAAGACGAAGAAGCAGCUCCGGGAGAACGCCGAG
GACAUGGGGAACGGCUGCUUCAAGAUCUACCACAAGUGCGACAACGCGU
GCAUCGGGUCCAUCCGCAACGAGACCUACGACCACAACGUCUACCGGGA
CGAGGCCCUGAACAACCGCUUCCAGAUCAAGGGCGUGGAGCUCAAGAGC
GGGUACAAGGACUGGAUCCUGUGGAUCUCGUUCGCGAUGUCCUGCUUCC
UCCUGUGCAUCGCCCUCCUGGGCUUCAUCAUGUGGGCGUGCCAGAAGGG
GAACAUCCGGUGCAACAUCUGCAUCUGA

<div align="right">SEQ ID NO: 132</div>

>Influenza B virus/Victoria HA0 mutant mRNA

AUGAAGGCCAUCAUCGUGCUGCUGAUGGUGGUGACCAGCAACGCCGACC
GGAUCUGCACCGGCAUCACCAGCUCCAACAGCCCCACGUGGUGAAGAC
CGCCACCCAGGGCGAGGUGAACGUGACCGGCGUGAUCCCCCUGACCACC
ACCCCCACCAAGUCCCACUUCGCCAACCUGAAGGGCACCGAGACCCGGGG
CAAGCUGUGCCCCAAGUGCCUGAACUGCACCGACCUGGACGUGGCCCUG
GGCCGCCCCAAGUGCACCGGCAAGAUCCCCAGCGCCCGGGUGUCCAUCCU
GCACGAGGUGCGCCCCGUGACCAGCGGCUGCUUCCCCAUCAUGCACGACC
GCACCAAGAUCCGGCAGCUGCCCAACCUGCUGCGGGGGUACGAGCACGU
CCGCCUGUCCACCCACAACGUGAUCAACGCCGAGGACGCCCCGGCCGGC
CCUACGAGAUCGGGACCAGCGGCUCCUGCCCCAACAUCACCAACGGGAA
CGGCUUCUUCGCCACCAUGGCCUGGGCCGUCCCCAAGAACAAGACCGCCA

CCAACCCCCUGACCAUCGAGGUGCCGUACAUCUGCACCGAGGGGGAGGA
CCAGAUCACCGUCUGGGGCUUCCACAGCGACAACGAGACGCAGAUGGCC
AAGCUGUACGGGGACUCCAAGCCCCAGAAGUUCACGAGCUCCGCCAACG
GCGUGACCACCCACUACGUCAGCCAGAUCGGGGGCUUCCCGAACCAGAC
GGAGGACGGGGGCCUGCCCCAGUCCGGGCGCAUCGUGGUCGACUACAUG
GUGCAGAAGUCCGGCAAGACGGGGACCAUCACGUACCAGCGGGGCAUCC
UGCUGCCGCAGAAGGUCUGGUGCGCCUCCGGGCGCAGCAAGGUGAUCAA
GGGCUCCCUGCCCUCAUCGGGGAGGCCGACUGCCUGCACGAGAAGUAC
GGCGGGCUCAACAAGAGCAAGCCGUACUACACCGGCGAGCACGCCAAGG
CCAUCGGGAACUGCCCCAUCUGGGUCAAGACCCCGCUGAAGCUCGCCAA
CGGCACGAAGUACCGGCCCCGGCCAAGCUGCUCAAGGAGCAGGGGUUC
UUCGGCGCCAUCGCCGGGUUCCUGGAGGGCGGGUGGGAGGGCAUGAUCG
CCGGGUGGCACGGCUACACGUCCCACGGGGCCCACGGCGUGGCGGUCGC
CGCGGACCUCAAGAGCACCCAGGAGGCCAUCAACAAGAUCACGAAGAAC
CUGAACUCCCUCAGCGAGCUGGAGGUGAAGAACCUCCAGCGCCUGUCCG
GGGCGAUGGACGAGCUCCACAACGAGAUCCUGGAGCUCGACGAGAAGGU
CGACGACCUGCGGGCCGACACCAUCAGCUCGCAGAUCGAGCUCGCGGUG
CUGCUCUCCAACGAGGGCAUCAUCAACAGCGAGGACGAGCACCUGCUCG
CCCUGGAGCGGAAGCUCAAGAAGAUGCUGGGGCCCUCGGCGGUCGAGAU
CGGCAACGGGUGCUUCGAGACCAAGCACAAGUGCAACCAGACGUGCCUC
GACCGCAUCGCCGCGGGCACGUUCGACGCCGGGGAGUUCUCCCUGCCGA
CCUUCGACAGCCUCAACAUCACGGCGGCCUCGCUGAACGACGACGGCCUC
GACAACCACACCAUCCUGCUCUACUACUCCACCGCGGCCAGCUCGCUGGC
GGUGACGCUCAUGAUCGCCAUCUUCGUCGUGUACAUGGUCUCCCGCGAC
AACGUGAGCUGCUCGAUCUGCCUGUGA

SEQ ID NO: 133

>Influenza B virus/Yamagata HA0 mutant mRNA

AUGAAGGCCAUCAUCGUGCUGCUGAUGGUGGUGACCAGCAACGCCGACC
GGAUCUGCACCGGCAUCACCAGCUCCAACAGCCCCCACGUGGUGAAGAC
CGCCACCCAGGGCGAGGUGAACGUGACCGGCGUGAUCCCCUGACCACC
ACCCCCACCAAGUCCUACUUCGCCAACCUGAAGGGCACCCGGACCCGCGG
CAAGCUGUGCCCCGACUGCCUGAACUGCACCGACCUGGACGUGGCCCUG
GGCCGGCCCAUGUGCGUGGGCACCACCCCCAGCGCCAAGGCCUCCAUCCU
GCACGAGGUGCGCCCCGUGACCAGCGGCUGCUUCCCCAUCAUGCACGACC
GCACCAAGAUCCGGCAGCUGCCCAACCUGCUGCGGGGGUACGAGAAGAU
CCGCCUGUCCACCCAGAACGUCAUCGACGCCGAGAAGGCCCCCGGCGGGC

CCUACCGGCUGGGCACCAGCGGGUCCUGCCCCAACGCCACCAGCAAGAUC
GGCUUCUUCGCCACCAUGGCCUGGGCCGUGCCCAAGGACAACUACAAGA
ACGCCACCAACCCCCUGACCGUCGAGGUGCCCUACAUCUGCACCGAGGGG
GAGGACCAGAUCACGGUCUGGGGCUUCCACUCCGACAACAAGACCCAGA
UGAAGUCCCUGUACGGGGACUCCAACCCGCAGAAGUUCACGAGCUCCGC
CAACGGCGUGACCACGCACUACGUCAGCCAGAUCGGGGACUUCCCGGAC
CAGACCGAGGACGGCGGGCUGCCCCAGUCCGGCCGCAUCGUGGUCGACU
ACAUGAUGCAGAAGCCCGGGAAGACGGGCACCAUCGUGUACCAGCGGGG
GGUCCUGCUGCCGCAGAAGGUGUGGUGCGCCAGCGGCCGCUCCAAGGUC
AUCAAGGGGAGCCUGCCCCUCAUCGGCGAGGCCGACUGCCUGCACGAGG
AGUACGGGGGCCUCAACAAGUCCAAGCCGUACUACACGGGGAAGCACGC
CAAGGCCAUCGGCAACUGCCCGAUCUGGGUGAAGACCCCCUGAAGCUC
GCCAACGGGACGAAGUACCGGCCGCCCGCCAAGCUGCUCAAGGAGCAGG
GCUUCUUCGGGGCCAUCGCCGGCUUCCUGGAGGGGGGCUGGGAGGGGAU
GAUCGCCGGCUGGCACGGGUACACCAGCCACGGCGCCCACGGGGUCGCG
GUGGCCGCGGACCUCAAGUCCACGCAGGAGGCCAUCAACAAGAUCACCA
AGAACCUGAACAGCCUCUCCGAGCUGGAGGUCAAGAACCUCCAGCGCCU
GAGCGGCGCGAUGGACGAGCUCCACAACGAGAUCCUGGAGCUCGACGAG
AAGGUGGACGACCUGCGGGCCGACACGAUCUCGUCCCAGAUCGAGCUCG
CGGUCCUGCUCAGCAACGAGGGGAUCAUCAACUCGGAGGACGAGCACCU
GCUCGCCCUGGAGCGGAAGCUCAAGAAGAUGCUGGGCCCGUCCGCGGUG
GACAUCGGGAACGGCUGCUUCGAGACCAAGCACAAGUGCAACCAGACGU
GCCUCGACCGCAUCGCCGCGGGGACCUUCAACGCCGGCGAGUUCAGCCU
GCCCACGUUCGACUCGCUCAACAUCACCGCGGCCUCCCUGAACGACGACG
GGCUCGACAACCACACGAUCCUGCUCUACUACAGCACCGCGGCCUCGUCC
CUGGCGGUCACGCUCAUGCUGGCCAUCUUCAUCGUGUACAUGGUCAGCC
GCGACAACGUGUCGUGCUCCAUCUGCCUCUGA

SEQ ID NO: 134

>Poly A

AAAAAAAAAAAAAAAAAAAAAAAAAAAAAAAAAAAAAAAAAAAAAAAAAA
AAAAAAAAAAAAAAAAAAAAAAAAAAAAAAAAAAAAAAAAAAAAAAAAAA
AAAAAAAAAAAAAAAAAAAAAAAA

SEQ ID NO: 135

>SARS-CoV-2 BA.4 spike protein amino acid sequence

MFVFLVLLPLVSSQCVNLITRTQSYTNSFTRGVYYPDKVFRSSVLHSTQDLFLPF
FSNVTWFHAISGTNGTKRFDNPVLPFNDGVYFASTEKSNIIRGWIFGTTLDSKT
QSLLIVNNATNVVIKVCEFQFCNDPFLDVYYHKNNKSWMESEFRVYSSANNCT
FEYVSQPFLMDLEGKQGNFKNLREFVFKNIDGYFKIYSKHTPINLGRDLPQGFS
ALEPLVDLPIGINITRFQTLLALHRSYLTPGDXSSGWTAGAAAYYVGYLQPRTF
LLKYNENGTITDAVDCALDPLSETKCTLKSFTVEKGIYQTSNFRVQPTESIVRFP
NITNLCPFDEVFNATRFASVYAWNRKRISNCVADYSVLYNFAPFFAFKCYGVSP
TKLNDLCFTNVYADSFVIRGNEVSQIAPGQTGNIADYNYKLPDDFTGCVIAWN
SNKLDSKVGGNYNYRYRLFRKSNLKPFERDISTEIYQAGNKPCNGVAGVNCYF
PLQSYGFRPTYGVGHQPYRVVVLSFELLHAPATVCGPKKSTNLVKNKCVNFNF
NGLKGTGVLTESNKKFLPFQQFGRDIADTTDAVRDPQTLEILDITPCSFGGVSVI
TPGTNTSNQVAVLYQGVNCTEVPVAIHADQLTPTWRVYSTGSNVFQTRAGCLI
GAEYVNNSYECDIPIGAGICASYQTQTKSHRRARSVASQSIIAYTMSLGAENSVA
YSNNSIAIPTNFTISVTTEILPVXMTKTSVDCTMYICGDSTECSNLLLQYGSFCT
QLKRALTGIAVEQDKNTQEVFAQVKQIYKTPPIKYFGGFNFSQILPDPSKPSKRS
FIEDLLFNKVTLADAGFIKQYGDCLGDIAARDLICAQKFNGLTVLPPLLTDEMI
AQYTSALLAGTITSGWTFGAGAALQIPFAMQMAYRFNGIGVTQNVLYENQKLI
ANQFNSAIGKIQDSLSSTASALGKLQDVVNHNAQALNTLVKQLSSKFGAISSVL
NDILSRLDPPEAEVQIDRLITGRLQSLQTYVTQQLIRAAEIRASANLAATKMSEC
VLGQSKRVDFCGKGYHLMSFPQSAPHGVVFLHVTYVPAQEKNFTTAPAICHDG
KAHFPREGVFVSNGTHWFVTQRNFYEPQIITTDNTFVSGNCDVVIGIVNNTVY
DPLQPELDSFKEELDKYFKNHTSPDVDLGDISGINASVVNIQKEIDRLNEVAKN
LNESLIDLQELGKYEQYIKWPWYIWLGFIAGLIAIVMVTIMLCCMTSCCSCLKG
CCSCGSCCKFDEDDSEPVLKGVKLHYT

SEQ ID NO: 136

**Claims**

1. A nucleic acid construct, comprising:

    (a) an open reading frame (ORF), and
    (b) a 5' untranslated region element (5'UTR),
    wherein the 5'UTR is derived from an ARHGAP gene;
    preferably, the ARHGAP is ARHGAP15;
    more preferably, the 5'UTR comprises the nucleotide sequence set forth in SEQ ID NO: 44 or a truncation thereof.

2. The nucleic acid construct according to claim 1, wherein the 5'UTR comprises at least one point mutation capable of being used to inhibit an ATG within the UTR from initiating translation;

    preferably, the point mutation is selected from the group consisting of a mutation at any one or more sites of A, T, or G of the ATG sequence in the 5'UTR;
more preferably, the mutation is to mutate A into G, C, or T.

3. The nucleic acid construct according to claim 1 or 2, wherein the 5'UTR truncation still maintains a function of regulating expression of an ORF-encoded protein; or, the 5'UTR truncation has an enhanced function of regulating expression of an ORF-encoded protein as compared to a natural 5'UTR;
preferably, a truncation mode for the 5'UTR truncation comprises: in a sequence direction from the 5' end to the 3' end, deleting a sequence of contiguous nucleotides from the 5' end; and/or, in a sequence direction from the 3' end to the 5' end, deleting a sequence of contiguous nucleotides from the 3' end.

4. The nucleic acid construct according to any one of claims 1-3, wherein the 5'UTR truncation is formed by, in a sequence direction from the 5' end to the 3' end, deleting a sequence of contiguous nucleotides from the 5' end; and/or, in a sequence direction from the 3' end to the 5' end, deleting a sequence of contiguous nucleotides from the 3' end;
preferably, the 5'UTR truncation comprises a sequence of at least 5 contiguous nucleotides from the sequence set forth in SEQ ID NO: 44, more preferably comprises a sequence of at least 7 contiguous nucleotides from the sequence set forth in SEQ ID NO: 2;

preferably, the nucleotide at the 5' end of the 5'UTR truncation is the nucleotide at any one of positions 1-57, according to natural counting, of the sequence set forth in SEQ ID NO: 44, more preferably the nucleotide at any one of positions 1-13 or 17-29, according to natural counting, of the sequence set forth in SEQ ID NO: 2;
preferably, the 5'UTR truncation comprises CTATAAT or the nucleotide sequence set forth in SEQ ID NO: 193.

5. The nucleic acid construct according to any one of claims 1-3, wherein the 5'UTR comprises the nucleotide sequence set forth in any one of SEQ ID NOs: 2, 28-43, 76, and 137-196 or comprises CTATAAT; or a nucleotide sequence having at least 80% identity to any one of the aforementioned sequences.

6. The nucleic acid construct according to any one of claims 1 to 5, further comprising:

(c) a 3' untranslated region element (3'UTR),
wherein preferably, the 3'UTR comprises a 3'UTR derived from any one of the gene HBB, ARHGAP, CORO1A, or HPX;
more preferably, the 3'UTR comprises a 3'UTR derived from ARHGAP15.

7. The nucleic acid construct according to claim 6, wherein:
the 3'UTR comprises the nucleotide sequence set forth in any one of SEQ ID NOs: 7-10, or, the 3'UTR comprises a nucleotide sequence having at least 80% identity to any one of SEQ ID NOs: 7-10.

8. The nucleic acid construct according to any one of claims 1 to 7, further comprising: (d) a polyadenylic acid (poly-A) tail.

9. The nucleic acid construct according to claim 8, wherein:

the poly-A tail is selected from the group consisting of HGH polyA, SV40polyA, BGH polyA, rbGlob polyA, and SV40late polyA;
preferably, the poly-A tail comprises the nucleotide sequence set forth in SEQ ID NO: 16 or SEQ ID NO: 135 or a nucleotide sequence having at least 80% identity thereto.

10. The nucleic acid construct according to any one of claims 1 to 9, wherein the ORF encodes a viral antigen; preferably, the ORF encodes a coronavirus antigen or an influenza virus antigen;

the coronavirus is preferably SARS-COV-2, and the coronavirus antigen is preferably a spike protein;
more preferably, the spike protein is selected from the group consisting of a spike protein of any one of the viral strain SARS-COV-2, SARS-COV-2 Alpha, SARS-COV-2 Beta, SARS-COV-2 Gamma, SARS-COV-2 Kappa, SARS-COV-2 Delta, or SARS-COV-2 Omicron;
the influenza virus is preferably influenza A virus or influenza B virus, and the antigen is a hemagglutinin protein (HA) and/or a ceramidase (NA);
more preferably, the influenza virus antigen is selected from the group consisting of a hemagglutinin protein and/or a neuraminidase of any one of the viral strains influenza A virus H1N1, influenza A virus H3N2, influenza B virus Victoria, and influenza B virus Yamagata.

11. The nucleic acid construct according to any one of claims 1 to 10, wherein:

a polypeptide sequence encoded by the ORF comprises the amino acid sequence set forth in any one of SEQ ID NOs: 12, 14, 21, 23, 25, 80, 83-86, 98-101, and 136;
preferably, the ORF comprises the nucleotide sequence set forth in any one of SEQ ID NOs: 13, 15, 20, 22, 24, 81, 87-90, 97, and 102-105, or a nucleotide sequence having at least 80%, 85%, 90%, 95%, 96%, 97%, 98%, or 99% identity to the nucleotide sequence set forth in any one of SEQ ID NOs: 13, 15, 20, 22, 24, 81, 87-90, 97, and 102-105.

12. The nucleic acid construct according to any one of the preceding claims, comprising the nucleotide sequences set forth in SEQ ID NOs: 18-19 or nucleotide sequences having at least 80% identity thereto.

13. An RNA molecule, comprising:

(a) an open reading frame (ORF),
(b) a 5' untranslated region element (5'UTR), and
(c) a 3' untranslated region element (3'UTR),
wherein the 5'UTR is selected from the group consisting of a 5'UTR derived from any one of the gene ARHGAP, HSPB1, HBB, or CCL13, and the 3'UTR is selected from the group consisting of a 3'UTR derived from any one of the gene HBB, ARHGAP, CORO1A, or HPX;
the ARHGAP is preferably ARHGAP15, and the 5'UTR preferably comprises the nucleotide sequence set forth in SEQ ID NO: 79 or a truncation thereof.

14. The RNA molecule according to claim 13, wherein the 5'UTR and 3'UTR are selected from the group consisting of any one of the following combinations:

1) the 5'UTR is derived from a 5'UTR of ARHGAP, and the 3'UTR is derived from a 3'UTR of any one of HBB, ARHGAP, CORO1A, or HPX;
2) the 5'UTR is derived from a 5'UTR of HSPB1, and the 3'UTR is derived from a 3'UTR of any one of HBB, ARHGAP, CORO1A, or HPX;
3) the 5'UTR is derived from a 5'UTR of HBB, and the 3'UTR is derived from a 3'UTR of any one of HBB, ARHGAP, CORO1A, or HPX; and
4) the 5'UTR is derived from a 5'UTR of CCL13, and the 3'UTR is derived from a 3'UTR of any one of HBB, ARHGAP, CORO1A, or HPX;

preferably,

the 5'UTR of ARHGAP comprises the nucleotide sequence set forth in any one of SEQ ID NOs: 45-46, 63-75, and 77-78 and CUAUAAU, or comprises CUAUAAU, or a nucleotide sequence having at least 80% identity to any one of the aforementioned sequences;
the 5'UTR derived from HSPB1 comprises the nucleotide sequence set forth in SEQ ID NO: 47 or a nucleotide sequence having at least 80% identity thereto;
the 5'UTR derived from HBB comprises the nucleotide sequence set forth in SEQ ID NO: 48 or a nucleotide sequence having at least 80% identity thereto;
the 5'UTR derived from CCL13 comprises the nucleotide sequence set forth in SEQ ID NO: 49 or a nucleotide sequence having at least 80% identity thereto;
the 3'UTR derived from HBB comprises the nucleotide sequence set forth in SEQ ID NO: 50 or a nucleotide sequence having at least 80% identity thereto;
the 3'UTR derived from ARHGAP comprises the nucleotide sequence set forth in SEQ ID NO: 51 or a nucleotide sequence having at least 80% identity thereto;
the 3'UTR derived from CORO1A comprises the nucleotide sequence set forth in SEQ ID NO: 52 or a nucleotide sequence having at least 80% identity thereto;
the 3'UTR derived from HPX comprises the nucleotide sequence set forth in SEQ ID NO: 53 or a nucleotide sequence having at least 80% identity thereto.

15. The RNA molecule according to any one of claims 13 to 14, wherein the ORF encodes a viral antigen; preferably, the ORF encodes a coronavirus antigen or an influenza virus antigen;

the coronavirus is preferably SARS-COV-2, and the coronavirus antigen is preferably a spike protein;
more preferably, the spike protein is selected from the group consisting of a spike protein of any one of the viral strain SARS-COV-2, SARS-COV-2 Alpha, SARS-COV-2 Beta, SARS-COV-2 Gamma, SARS-COV-2 Kappa, SARS-COV-2 Delta, or SARS-COV-2 Omicron; or,
the influenza virus is selected from the group consisting of influenza A virus and influenza B virus, and the influenza virus antigen is a hemagglutinin protein (HA) and/or a neuraminidase (NA);
more preferably, the influenza virus antigen is selected from the group consisting of a hemagglutinin protein and/or a neuraminidase of any one of the viral strains influenza A virus H1N1, influenza A virus H3N2, influenza B virus Victoria, and influenza B virus Yamagata.

16. The RNA molecule according to claim 15, wherein:

a polypeptide sequence encoded by the ORF comprises the amino acid sequence set forth in any one of SEQ ID NOs: 12, 14, 21, 23, 25, 80, 83-86, 98-101, and 136;
preferably, the nucleotide sequence of the ORF comprises the nucleotide sequence set forth in any one of SEQ ID NOs: 54-55, 59-61, 82, and 126-134, or a nucleotide sequence having at least 80%, 85%, 90%, 95%, 96%, 97%, 98%, or 99% identity to the nucleotide sequence set forth in any one of SEQ ID NOs: 54-55, 59-61, 82, and 126-134.

17. The RNA molecule according to any one of claims 13 to 16, further comprising: (d) a polyadenylic acid (poly-A) tail.

18. The RNA molecule according to claim 17, wherein:

the poly-A tail is selected from the group consisting of HGH polyA, SV40polyA, BGH polyA, rbGlob polyA, and SV40late polyA;
preferably, the poly-A tail comprises the nucleotide sequence set forth in SEQ ID NO: 56 or SEQ ID NO: 135 or a nucleotide sequence having at least 80% identity thereto.

19. The RNA molecule according to any one of claims 13 to 18, comprising (a) an open reading frame (ORF), (b) a 5' untranslated region element (5'UTR), (c) a 3' untranslated region element (3'UTR), and (d) a polyadenylic acid (poly-A) tail, wherein preferably, the RNA molecule comprises the nucleotide sequence set forth in any one of SEQ ID NOs: 57-58 and 106-125.

20. The RNA molecule according to any one of claims 13 to 19, further comprising: (e) a 5' cap structure (5'Cap).

21. The RNA molecule according to claim 20, wherein:

the 5'Cap is selected from the group consisting of Cap0, Cap1, Cap2, Cap3, Cap4, ARCA, modified ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine;
preferably, the 5'Cap is selected from the group consisting of ARCA, 3'OMe-m7G(5')ppp(5')G, m7G(5')ppp(5') (2'OMeA)pU, m7Gppp(A2'O-MOE)pG, m7G(5')ppp(5')(2'OMeA)pG, m7G(5')ppp(5')(2'OMeG)pG, m7(3'OMeG)(5')ppp(5')(2'OMeG)pG, and m7(3'OMeG)(5')ppp(5')(2'OMeA)pG; preferably, the 5'Cap is m7G(5')ppp(5')(2'OMeA)pG.

22. The RNA molecule according to any one of claims 13 to 21, wherein the RNA molecule is an mRNA.

23. The RNA molecule according to any one of claims 13 to 22, further comprising one or more modifications,

wherein preferably, the modifications include a backbone modification, a sugar modification, a base modification, and/or a lipid modification;
more preferably, the base modification is a pseudouridine modification.

24. An RNA molecule, comprising the nucleotide sequence set forth in any one of SEQ ID NOs: 54-55, 59-61, 82, and 126-134 or a nucleotide sequence having at least 80% identity to the nucleotide sequence set forth in any one of SEQ ID NOs: 54-55, 59-61, 82, and 126-134,
wherein preferably, the RNA molecule further comprises one or more modifications; more preferably, the modifications are pseudouridine modifications.

25. Use of the nucleic acid construct according to any one of claims 1-12 or the RNA molecule according to any one of claims 13-24 in any one of the following: (1) preparing a vaccine; (2) encoding a viral antigen in a subject or *in vitro;* (3) preparing a medicament encoding a viral antigen in a subject or *in vitro;* and (4) preparing a medicament.

26. A vaccine, comprising the RNA molecule according to any one of claims 13 to 24, wherein preferably, the RNA molecule encodes one or more antigens of one or more viral strains.

27. A vector, comprising the nucleic acid construct according to any one of claims 1 to 12, or the RNA molecule according to any one of claims 13 to 24.

28. A host cell, comprising the vector according to claim 27.

29. A preparation method for the RNA molecule according to any one of claims 13-24, comprising: reverse-transcribing the nucleic acid construct according to any one of claims 1 to 12 or the vector according to claim 27 to obtain an RNA molecule, wherein preferably, the method further comprises adding a 5'Cap to the 5' end of the RNA molecule.

30. A delivery system, comprising the nucleic acid construct according to any one of claims 1 to 12 or the RNA molecule according to any one of claims 13 to 24, wherein a delivery vehicle of the delivery system is a cationic lipid delivery particle; preferably, the delivery vehicle is a lipid nanoparticle.

31. A pharmaceutical composition, comprising:

   a pharmaceutically acceptable carrier, diluent, or excipient, and
   any one selected from the group consisting of the following:
   the nucleic acid construct according to any one of claims 1 to 12, the RNA molecule according to any one of claims 13 to 24, the vaccine according to claim 26, and/or the delivery system according to claim 30.

32. A product or kit, comprising the nucleic acid construct according to any one of claims 1 to 12, the RNA molecule according to any one of claims 13 to 24, the vaccine according to claim 26, the delivery system according to claim 30, and/or the pharmaceutical composition according to claim 31.

33. Use for preparing a medicament for treating and/or preventing a disease, comprising administering to a subject in need thereof an effective amount of the nucleic acid construct according to any one of claims 1 to 12, the RNA molecule according to any one of claims 13 to 24, the vaccine according to claim 26, the delivery system according to claim 30, the pharmaceutical composition according to claim 31, and/or the product or kit according to claim 32, wherein the disease is a viral infectious disease or a respiratory disease associated with viral infection;

   preferably, the virus is a coronavirus or an influenza virus; more preferably, the virus is SARS-CoV-2, influenza A virus, or influenza B virus;
   preferably, the respiratory disease associated with viral infection includes simple infection, fever, cough, sore throat, rhinitis, headache, pneumonia, acute respiratory infection, severe acute respiratory infection (SARI), hypoxemic respiratory failure, acute respiratory distress syndrome, sepsis, septic shock, and severe acute respiratory syndrome (SARS).

34. A method for treating and/or preventing a disease, comprising administering to a subject in need thereof an effective amount of the nucleic acid construct according to any one of claims 1 to 12, the RNA molecule according to any one of claims 13 to 24, the vaccine according to claim 26, the delivery system according to claim 30, the pharmaceutical composition according to claim 31, and/or the product or kit according to claim 32, wherein the disease is a viral infectious disease or a respiratory disease associated with viral infection;

   preferably, the virus is a coronavirus or an influenza virus; more preferably, the virus is SARS-CoV-2, influenza A virus, or influenza B virus;
   preferably, the respiratory disease associated with viral infection includes simple infection, fever, cough, sore throat, rhinitis, headache, pneumonia, acute respiratory infection, severe acute respiratory infection (SARI), hypoxemic respiratory failure, acute respiratory distress syndrome, sepsis, septic shock, and severe acute respiratory syndrome (SARS).

35. A method for inducing a neutralizing antibody reaction and/or a T cell immune response in a subject, comprising administering to a subject in need thereof an effective amount of the nucleic acid construct according to any one of claims 1 to 12, the RNA molecule according to any one of claims 13 to 24, the vaccine according to claim 26, the delivery system according to claim 30, the pharmaceutical composition according to claim 31, and/or the product or kit according to claim 32,

wherein preferably, the neutralizing antibody reaction is a neutralizing antibody reaction against a viral antigen, and the T cell immune response includes a CD4+ and/or CD8+ T cell immune reaction;
preferably, the virus is a coronavirus or an influenza virus; more preferably, the virus is SARS-CoV-2, influenza A virus, or influenza B virus.

FIG. 1

FIG. 2

FIG. 3

FIG. 4

5'UTR I      gagttaggagttgAtggcagtttcaataacaggtcattgccgagaaaaggatagcactataat   SEQ ID NO: 1

5'UTR I'     gagttaggagttgGtggcagtttcaataacaggtcattgccgagaaaaggatagcactataat   SEQ ID NO: 2

FIG. 5

**EP 4 516 897 A1**

FIG. 6A

FIG. 6B

FIG. 7A

FIG. 7B

FIG. 8

FIG. 9

FIG. 10

<div align="center">**INTERNATIONAL SEARCH REPORT**</div>

| | |
|---|---|
| | International application No. |
| | **PCT/CN2023/091194** |

**A.    CLASSIFICATION OF SUBJECT MATTER**

C12N5/10(2006.01)i;  C12N15/40(2006.01)i;  C12N15/85(2006.01)i;  A61K39/215(2006.01)i;  A61P31/14(2006.01)i;  A61P11/00(2006.01)i

According to International Patent Classification (IPC) or to both national classification and IPC

**B.    FIELDS SEARCHED**

Minimum documentation searched (classification system followed by classification symbols)

IPC: C12N A61K A61P

Documentation searched other than minimum documentation to the extent that such documents are included in the fields searched

Electronic data base consulted during the international search (name of data base and, where practicable, search terms used)

CNABS; CNTXT; WPABS; WPABSC; VEN; ENTXT; ENTXTC; ISI Web of Science; CNKI; 中国专利生物序列检索系统, China Patent Biological Sequence Search System; GenBank: 瑞可迪, Rho GTPase激活蛋白, 5'非翻译区元件, 3'非翻译区元件, β珠蛋白, 冠状病毒, 流感病毒, 抗原, 刺突蛋白, 疫苗, 序列1-196, Rho GTPase activating protein, ARHGAP, ARHGAP15, 5'UTR, 3'UTR, HBB, HSPB1, CCL13, CORO1A, HPX, coronavirus, influenza virus, spike protein, vaccine

**C.    DOCUMENTS CONSIDERED TO BE RELEVANT**

| Category* | Citation of document, with indication, where appropriate, of the relevant passages | Relevant to claim No. |
|---|---|---|
| X | US 2010273220 A1 (MASSACHUSETTS INSTITUTE OF TECHNOLOGY) 28 October 2010 (2010-10-28)<br>claims 45-52, description, paragraph [0220] | 13, 14, 17-23 |
| X | CN 113186203 A (STEMIRNA THERAPEUTICS CO., LTD.) 30 July 2021 (2021-07-30)<br>claims 29-52, description, paragraph [0181] | 24-33, 35 |
| X | CN 113528545 A (ADVACCINE (SUZHOU) BIOPHARMACEUTICALS CO., LTD.) 22 October 2021 (2021-10-22)<br>claims 1-10, description, paragraph [0027] | 24-33, 35 |
| X | CN 108778308 A (CUREVAC AG) 09 November 2018 (2018-11-09)<br>claim 39, description, example 1 | 24-33, 35 |
| X | CN 111671890 A (SOOCHOW UNIVERSITY) 18 September 2020 (2020-09-18)<br>claims 1-6 | 24-33, 35 |

| | |
|---|---|
| ☑ Further documents are listed in the continuation of Box C. | ☑ See patent family annex. |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **08 August 2023** | **10 August 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

# EP 4 516 897 A1

## INTERNATIONAL SEARCH REPORT

International application No.

**PCT/CN2023/091194**

### C.    DOCUMENTS CONSIDERED TO BE RELEVANT

| Category* | Citation of document, with indication, where appropriate, of the relevant passages | Relevant to claim No. |
|---|---|---|
| X | CN 113185613 A (WUHAN UNIVERSITY) 30 July 2021 (2021-07-30)<br>claims 1-11, description, paragraph [0050] | 24-33, 35 |
| X | CN 105007938 A (THE TRUSTEES OF THE UNIVERSITY OF PENNSYLVANIA) 28 October 2015 (2015-10-28)<br>claims 1-20, description, paragraphs [0042], [0087] | 24-33, 35 |
| Y | CN 113186203 A (STEMIRNA THERAPEUTICS CO., LTD.) 30 July 2021 (2021-07-30)<br>claims 29-52, description, paragraph [0181] | 24-33, 35 |
| Y | CN 113528545 A (ADVACCINE (SUZHOU) BIOPHARMACEUTICALS CO., LTD.) 22 October 2021 (2021-10-22)<br>claims 1-10, description, paragraph [0027] | 24-33, 35 |
| Y | CN 108778308 A (CUREVAC AG) 09 November 2018 (2018-11-09)<br>claim 39, description, example 1 | 24-33, 35 |
| Y | CN 111671890 A (SOOCHOW UNIVERSITY) 18 September 2020 (2020-09-18)<br>claims 1-6 | 24-33, 35 |
| Y | CN 113185613 A (WUHAN UNIVERSITY) 30 July 2021 (2021-07-30)<br>claims 1-11, description, paragraph [0050] | 24-33, 35 |
| Y | CN 105007938 A (THE TRUSTEES OF THE UNIVERSITY OF PENNSYLVANIA) 28 October 2015 (2015-10-28)<br>claims 1-20, description, paragraphs [0042], [0087] | 24-33, 35 |
| A | XU, Xiaofeng et al. "BMX-ARHGAP Fusion Protein Maintains the Tumorigenicity of Gastric Cancer Stem Cells by Activating the JAK/STAT3 Signaling Pathway"<br>*Cancer Cell International*, Vol. vol. 19, 31 December 2019 (2019-12-31),<br>pp. 1-15 | 1-33, 35 |
| A | YANG, Tzujing et al. "Effect of SARS-CoV-2 B.1.1.7 Mutations on Spike Protein Structure and Function"<br>*Nature Structural & Molecular Biology*, Vol. vol. 28, 30 September 2021 (2021-09-30),<br>pp. 731-739 | 1-33, 35 |

Form PCT/ISA/210 (second sheet) (July 2022)

214

**INTERNATIONAL SEARCH REPORT**

International application No.

**PCT/CN2023/091194**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.

**PCT/CN2023/091194**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **34, 35**
   because they relate to subject matter not required to be searched by this Authority, namely:

   The subject matter of claim 34 is a method for treating and/or preventing diseases, and the subject matter of claim 35 is a method for inducing a subject to produce a neutralizing antibody reaction and/or a T cell immune response, which relate to methods for treatment of a living human or animal body, and fall within subject matter for which a search is not required by the International Searching Authority as defined in PCT Rule 39.1(iv).

   The reasonably expected amendment regarding claim 35 is to amend the subject matter thereof to the use in the preparation of a drug for inducing a subject to produce a neutralizing antibody reaction and/or a T cell immune response.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/091194**

| Patent document cited in search report | | | Publication date (day/month/year) | Patent family member(s) | | | Publication date (day/month/year) |
|---|---|---|---|---|---|---|---|
| US | 2010273220 | A1 | 28 October 2010 | EP | 2421563 | A1 | 29 February 2012 |
| | | | | EP | 2421563 | A4 | 20 February 2013 |
| | | | | EP | 2421563 | B1 | 12 April 2017 |
| | | | | WO | 2010123501 | A1 | 28 October 2010 |
| CN | 113186203 | A | 30 July 2021 | WO | 2021159985 | A1 | 19 August 2021 |
| | | | | CN | 113186203 | B | 30 December 2022 |
| CN | 113528545 | A | 22 October 2021 | CN | 113528545 | B | 10 December 2021 |
| CN | 108778308 | A | 09 November 2018 | EP | 3701963 | A1 | 02 September 2020 |
| | | | | KR | 20180107109 | A | 01 October 2018 |
| | | | | JP | 2019503678 | A | 14 February 2019 |
| | | | | JP | 6949845 | B2 | 13 October 2021 |
| | | | | CA | 3009551 | A1 | 29 June 2017 |
| | | | | CA | 3009551 | C | 13 December 2022 |
| | | | | IL | 259378 | A | 31 July 2018 |
| | | | | AU | 2016375021 | A1 | 07 June 2018 |
| | | | | AU | 2016375021 | B2 | 03 February 2022 |
| | | | | EP | 3319622 | A1 | 16 May 2018 |
| | | | | EP | 3319622 | B1 | 12 February 2020 |
| | | | | US | 2019083602 | A1 | 21 March 2019 |
| | | | | WO | 2017109134 | A1 | 29 June 2017 |
| | | | | SG | 11201804398 | A1 | 30 July 2018 |
| CN | 111671890 | A | 18 September 2020 | CN | 111671890 | B | 05 August 2022 |
| CN | 113185613 | A | 30 July 2021 | CN | 113185613 | B | 13 September 2022 |
| CN | 105007938 | A | 28 October 2015 | HK | 1216723 | A1 | 02 December 2016 |
| | | | | AU | 2019202628 | A1 | 09 May 2019 |
| | | | | KR | 20150127586 | A | 17 November 2015 |
| | | | | JP | 2016517414 | A | 16 June 2016 |
| | | | | WO | 2014150835 | A1 | 25 September 2014 |
| | | | | CA | 2898235 | A1 | 25 September 2014 |
| | | | | AU | 2017201425 | A1 | 30 March 2017 |
| | | | | AU | 2017201425 | B2 | 17 January 2019 |
| | | | | US | 2016022806 | A1 | 28 January 2016 |
| | | | | US | 10022434 | B2 | 17 July 2018 |
| | | | | AU | 2014235556 | A1 | 30 July 2015 |
| | | | | US | 2018344842 | A1 | 06 December 2018 |
| | | | | EP | 2968523 | A1 | 20 January 2016 |
| | | | | EP | 2968523 | A4 | 20 July 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210456261
- WO 2016193226 A
- WO 2017075531 A
- WO 2018081480 A1
- WO 2017049245 A2
- WO 2017099823 A1
- WO 2022245888 A1
- WO 2022150717 A1
- CN 101291653 A
- CN 102119217 A
- WO 2011000107 A1
- CN 107028886 A
- CN 113521269 A

### Non-patent literature cited in the description

- **IVANOV et al.** *Science*, 2016, vol. 352 (6292), 1413-1416
- **WEINBERG et al.** *J. Infect. Dis.*, 01 June 2010, vol. 201 (11), 1607-10
- Structure-based design of prefusion-stabilized SARS-CoV-2 spikes. *Science*, vol. 369 (6510)
- *Nat Biotechnol. Jul.*, 2019, vol. 37 (7), 803-809